# EUROPEAN PATENT SPECIFICATION

(11) **EP 2 030 021 B1**
(45) Date of publication and mention of the grant of the patent: **21.11.2012**
(21) Application number: 07725374.8
(22) Date of filing: 18.05.2007
(51) Int. Cl.: G01N 33/68

(54) **METHOD FOR DIAGNOSING MITOCHONDRIAL DYSFUNCTION**
VERFAHREN ZUR DIAGNOSE MITOCHONDRIALER FEHLFUNKTION
PROCÉDÉ POUR DIAGNOSTIQUER UN DYSFONCTIONNEMENT MITOCHONDRIAL

(30) Priority: 18.05.2006 US 801484 P
(43) Date of publication of application: 04.03.2009
(73) Proprietor: Reichert, Andreas, 60598 Frankfurt am Main (DE)
(72) Inventor: REICHERT, Andreas, 60598 Frankfurt am Main (DE); DUVEZIN-CAUBET, Stéphane, 33600 Pessac (FR); WAGENER, Johannes, 80639 München (DE); HOFMANN-THIEL, Sabine, 82319 Starnberg-Perchting (DE); NEUPERT, Walter, 82110 Germering (DE)
(74) Representative: Vossius & Partner
(86) International application number: PCT/EP2007/004466
(87) International publication number: WO 2007/134818

(56) References cited:
- WO-A-02/00878
- WO-A-02/27022
- DELETTRE C. ET AL: "Mutation spectrum and splicing variants in the OPA1 gene" HUMAN GENETICS, vol. 109, no. 6, December 2001 (2001-12), pages 584-591, XP001147101 ISSN: 0340-6717 cited in the application
- SATOH MASAAKI ET AL: "Differential sublocalization of the dynamin-related protein OPA1 isoforms in mitochondria." BIOCHEMICAL AND BIOPHYSICAL RESEARCH COMMUNICATIONS, vol. 300, no. 2, 10 January 2003 (2003-01-10), pages 482-493, XP009088982 ISSN: 0006-291X cited in the application

## Description

The present invention relates to means and methods for the diagnosis of mitochondrial disorders/dysfunctions, in particular to a method for determining the susceptibility for, predisposition for or the presence of a disorder correlated with mitochondrial dysfunction or mitochondrial disease, wherein said method comprises the in vitro determination of the ratio of large and/or small isoforms of the optic atrophy 1 protein (OPA1). Furthermore, uses and kits for carrying out these methods are disclosed.

Mitochondria are essential organelles in eukaryotic cells. They perform a plethora of functions and interact with the rest of the cell in a multitude of pathways. Growth, differentiation and maintenance of cells as well as development of tissues and organs is dependent on an adaptive development of mitochondria. This is also apparent from the occurrence of a vast number of human disorders in which mitochondrial function is compromised due to genetic alterations, toxic effects or stress.

The most conspicuous function of mitochondria is in the energy metabolism of the cell. Oxidative phosphorylation is by far the most important pathway of synthesizing ATP and thereby maintaining life. Control of energy metabolism, tissue specific regulation of components of the respiratory chain and ATP synthase are central to tissue performance and therefore an issue in numerous diseases.

Mitochondria have the cellular monopole in housing a considerable number of anabolic pathways leading to the formation of essential components for the cell. Examples are the biosynthesis of haem, of amino acids and of iron-sulfur clusters. In addition, mitochondria harbour essential catabolic pathways such as degradation of fatty acids.

Mitochondria are prominent producers of reactive oxygen species whose, mainly damaging, roles are at the base of numerous processes leading to disease. One of these processes is mutation of mitochondrial DNA promoting defects of oxidative phosphorylation and tissue damage. Damage to mitochondrial DNA has been shown to be causative to premature ageing in mice. Therefore, it is of utmost importance, e.g. for the development of stem cells and generations of engineered tissues, to minimize mitochondrial damage, e.g. due to reactive oxygen species.

Mitochondria play a central role in apoptosis. Apoptosis is a useful and necessary process when it comes to development and destruction of cancer tissue. However, apoptosis can also be responsible for undesired breakdown of cells and tissues.

Mitochondria are highly motile organelles. This property is required for positioning mitochondria in polarized cells, most conspicuously in nerve cells. Mitochondria are also dynamic in terms of continuously occurring and rapid events of fusion and fission. This process is determining mitochondrial structure. Mitochondria form a highly interconnected network in the cell. The integrity of the network is required for mitochondrial competence and for prevention of apoptosis.

Many human diseases are caused or accompanied by mitochondrial dysfunction arising from mutations in mitochondrial or nuclear DNA. Mutations in mitochondrial DNA (mtDNA) affecting essential subunits of the respiratory chain or mitochondrial tRNA genes are associated with common mitochondrial diseases such as MELAS (Myopathy encephalopathy lactic acidosis and stroke-like episodes), myoclonic epilepsy with ragged-red fiber syndrome (MERRF syndrome), cardiomyopathy, Leber's hereditary opticus neuropathy (LHON), Leigh's syndrome, etc., to mention just a few of them (reviewed in Wallace, 2005, Annu Rev Genet, 39, 359-407). Mutations, deletions and insertions in mtDNA have been detected in numerous types of cancer such as renal cell and colorectal carcinoma, early liver, protasta, breast, bladder, primary lung, head and neck tumours, astrocytomas, adenocarcinomas in Barrett's esophagus (Alonso, 1997, Electrophoresis, 18, 682-685; Fliss, 2000, Science, 287, 2017-2019; Habano, 1999, Int J Cancer, 83, 625-629; Horton, 1996, Genes Chromosomes Cancer, 15, 95-101; Jeronimo, 2001, Oncogene, 20, 5195-5198; Kirches, 1999, Genes Chromosomes Cancer, 26, 80-83; Miyazono, 2002, Oncogene, 21, 3780-3783; Modica-Napolitano, 2004, Mitochondrion, 4, 755-762; Parrella, 2001, Cancer Res, 61, 7623-7626; Polyak, 1998, Nat Genet, 20, 291-293; Tan, 2002, Cancer Res, 62, 972-976). Moreover, the occurrence of these mutations appear to be several-fold increased in tumour versus control tissues (Fliss, 2000, Science, 287, 2017-2019; Horton, 1996, Genes Chromosomes Cancer, 15, 95-101). Moreover, a major contributing factor to Parkinson's as well as Alzheimer's disease is the reduced cellular ATP level caused by mitochondrial dysfunction. (Hashimoto, 2003, Curr Biol, 14, 340-345; Orth, 2002, Neurochem Int, 40, 533-541). Impairment of glucose and fatty acid metabolism due to mitochondrial dysfunction are important factors in diabetes and obesity, respectively (Ballinger, 1994, Nat Genet, 7, 458-459; Ballinger, 1992, Nat Genet, 1, 11-15; Gerbitz, 1996, Diabetes, 45, 113-126). Interestingly, the level of mutated mtDNA is also increasing with age - also for non-symptomatic humans. In a transgenic mouse, expression of an error-prone gamma mtDNA polymerase led to mtDNA mutations during mtDNA replication and those mice showed a clear phenotype of premature ageing (Trifunovic, 2004, Nature, 429, 417-423). This study showed that accumulation of mtDNA mutations is causative to ageing.

Mitochondrial morphology appears to be of utmost importance for the maintenance of mitochondrial function. In many cell types, mitochondria form a large network of interconnected tubules that is maintained by a balance of fission and fusion events (Nunnari, 1997, Mol Biol Cell, 8, 1233-1242). The physiological role of this dynamic and energy-consuming process is unclear. Apoptosis requires DRP1-dependent mitochondrial fission (Breckenridge, 2003, J Cell Biol, 160, 1115-1127; Frank, 2001, Dev Cell, 1, 515-525; Jagasia, 2005, Nature, 433, 754-760; Lee, 2004, Mol Biol Cell, 15, 5001-5011). The number and plasticity of dendritic spines and synapses and the mobilization of reserve pool vesicles in synapses depend on the balance between fusion and fission of mitochondria (Li, 2004, Cell, 119, 873-887; Verstreken, 2005, Neuron, 47, 365-378). Mitochondrial fusion was proposed to counteract the effects of mutations in mtDNA by trans-complementation, and therefore to impede the development of mitochondrial diseases (Nakada, 2001, Nat Med, 7, 934-940; Ono, 2001, Nat Genet, 28, 272-275). LETM1 is deleted in the Wolf-Hirschhorn syndrome (Nowikovsky, 2004, J Biol Chem, 279, 30307-30315; Schlickum, 2004, Genomics, 83, 254-261). The yeast orthologue Mdm38 was shown to be essential for wildtype mitochondrial morphology (Dimmer, 2002, Mol Biol Cell, 13, 847-853). Recently, mutations in the ganglioside induced protein 1 that promotes formation of elongated tubular mitochondria were associated with Charcot-Marie-Tooth disease type 4a (Niemann, 2005, J Cell Biol, 170, 1067-78).

Two proteins involved infusion of mitochondria, OPA1 (optic atrophy 1 protein) and Mitofusin2, are known to be affected in optic atrophy type 1 and Charcot-Marie-Tooth disease type 2a, respectively (Alexander, 2000, Nat Genet, 26, 211-215; Delettre, 2000, Nat Genet, 26, 207-210; Zuchner, 2004, Nat Genet, 36, 449-451). Overexpression of OPA1 in mouse embryo fibroblasts promotes tubulation of mitochondria (Cipolat, 2004, Proc Natl Acad Sci USA). Yet, downregulation of OPA1 leads to fragmentation of mitochondria, mitochondrial dysfunction, altered mitochondrial inner membrane morphology and increased propensity to apoptosis (Chen, 2005, J Biol Chem, 280, 26185-26192; Griparic, 2004, J Biol Chem, 279, 18792-18798; Lee, 2004, Mol Biol Cell, 15, 5001-5011; Olichon, 2003, J. Biol. Chem., 278, 7743-7746). The prior art observed fragmentation of mitochondria in over- as well as down-regulation experiments of OPA1 and the corresponding function of OPA1 remains obscure.

Eight alternatively spliced mRNAs transcribed from the OPA1 gene (OPA1 spliceforms 1 to 8) and tissue- and cell line-specific protein isoforms were described (WO 02/00878; Delettre, 2001, Hum Genet, 109,584-591; Olichon, 2002, FEBS Lett, 523,171-176; Satoh, 2003, Biochem Biophys Res Commun, 300, 482-493). The ortholog of OPA1 in baker's yeast, Mgm1, was shown to be essential for mitochondrial fusion (Sesaki, 2003, Mol Biol Cell, 14, 2342-2356; Wong, 2003, J Cell Biol, 160, 303-311). It was demonstrated that Mgm1 is present in two isoforms in the intermembrane space of mitochondria (Herlan, 2003, J Biol Chem, 278, 27781-27788; Wong, 2000, J Cell Biol, 151, 341-352). The exact N-termini of both isoforms were determined with biochemical means and it was found that Mgm1 biogenesis depends on the novel mitochondrial rhomboid protease Pcp1 (Herlan, 2003, J Biol Chem, 278, 27781-27788). Both Mgm1 isoforms appear to be required in roughly equal amounts in order to maintain mitochondrial morphology and mtDNA. Mgm1 is partially cleaved by Pcp1 within a second, so far unrecognized conserved, hydrophobic segment of Mgm1 (Herlan, 2004, J Cell Biol, 165, 167-173). Mutations within the first hydrophobic segment of Mgm1 led to alterations of the ratio of the two isoforms and to fragmentation of mitochondria. It was further shown that formation of the short isoform (s-Mgm1) and mitochondrial morphology depend on a functional import motor and on the ATP level in the matrix. In an atp6 mutant, impaired in mitochondrial ATP synthesis, the formation of s-Mgm1 is reduced. Therefore, mitochondrial morphology depends on an ATP-dependent mechanism which was termed "alternative topogenesis" (Henan, 2004, J Cell Biol, 165, 167-173).

In yeast, any mitochondrial dysfunction that leads to reduced ATP levels in the matrix, would in tum lead to a reduced formation of s-Mgm1, while I-Mgm1 is not affected. The consequence is that such mitochondria cannot fuse anymore with the intact mitochondrial network and are therefore excluded from inheritance to daughter cells.

*A Drosophila melanogaster* homolog of OPA1 (CD8479) is known to enhance the activity of the Drosophila melanogaster Uncoupling Protein (dUCPy), the overexpression of which is assumed to be lethal for the corresponding transgenic flies due to a collaps of the cellular energy production.-WO 03/061681

In humans, many muscular and neurological disorders, various forms of cancer, diabetes, obesity, other disorders and ageing are associated with mitochondrial dysfunction as discussed above and in Wallace, 2005, Annu Rev Genet 39, 359-407, Modica-Napolitano, 2004, Mitochondrion 4, 755-62 or Orth, 2001, Am J Med Genet 106, 27-36). Deficiencies in mitochondrial fusion and fission, two opposing processes that determine mitochondrial morphology (Nunnari, 1997, Mol Biol Cell 8, 1233-42; Okamoto, 2005, Annu Rev Genet 39, 503-36), play an important role in such disorders, in particular in various neurodegenerative disorders (Alexander, 2000, Nat Genet 26, 211-5; Delettre, 2000, Nat Genet 26, 207-10; Zuchner, 2004, Nat Genet 36, 449-51; Niemann, 2005, J Cell Biol 170, 1067-78). Mutations in the OPA1 gene, the human ortholog of Mgm1, are known to cause hereditary autosomal dominant optic atrophy type I, the most prevalent neuropathy of the optic nerve occurring with a prevalence of 1:12000 to 1:50000 dependent on the population (Alexander, 2000, Nat Genet 26, 211; Delettre, 2000, Nat Genet 26, 207). Seven particular mutations of the OPA1 gene were identified in patients suffering from autosomal dominant optic atrophy (WO 02/27022). In addition, 14 mutations which affect the activity of the OPA1 isoforms were identified in humans suffering from optic atrophy and a screening method was developed, wherein the OPA1 isoforms are applied to find modulators of the activity of the OPA1 isoforms to treat diseases that are associated with mitochondrial dysfunction (WO 02/00878). OPA1 appears to be required for mitochondrial fusion (Olichon, 2003, J. Biol. Chem. 278; Cipolat, 2004, Proc Natl Acad Sci USA; Chen, 2005, J Biol Chem 280, 26185). Overexpression of OPA1 in mouse embryo fibroblasts promotes tubulation of mitochondria (Cipolat, 2004, Proc Natl Acad Sci USA, 101, 15927-32). Downregulation of OPA1 leads to fragmentation of mitochondria, mitochondrial dysfunction, altered mitochondrial inner membrane morphology and increased propensity for apoptosis (Lee, 2004, Mol Biol Cell 15, 5001; Olichon, 2003, J. Biol. Chem. 278, 7743; Chen, 2005, J Biol Chem 280, 26185; Griparic, 2004, J Biol Chem 279, 18792).

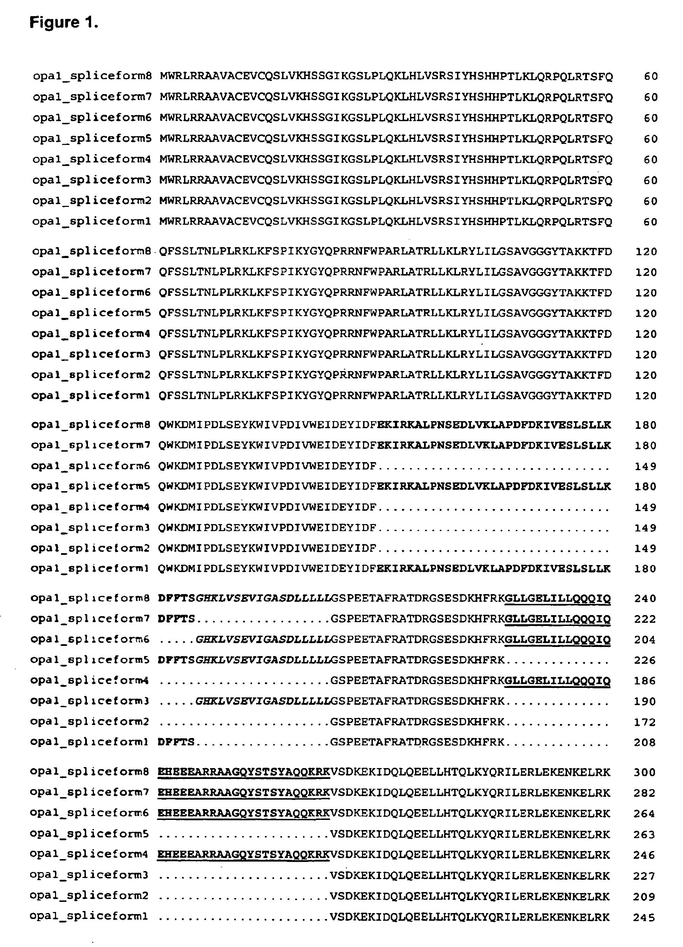

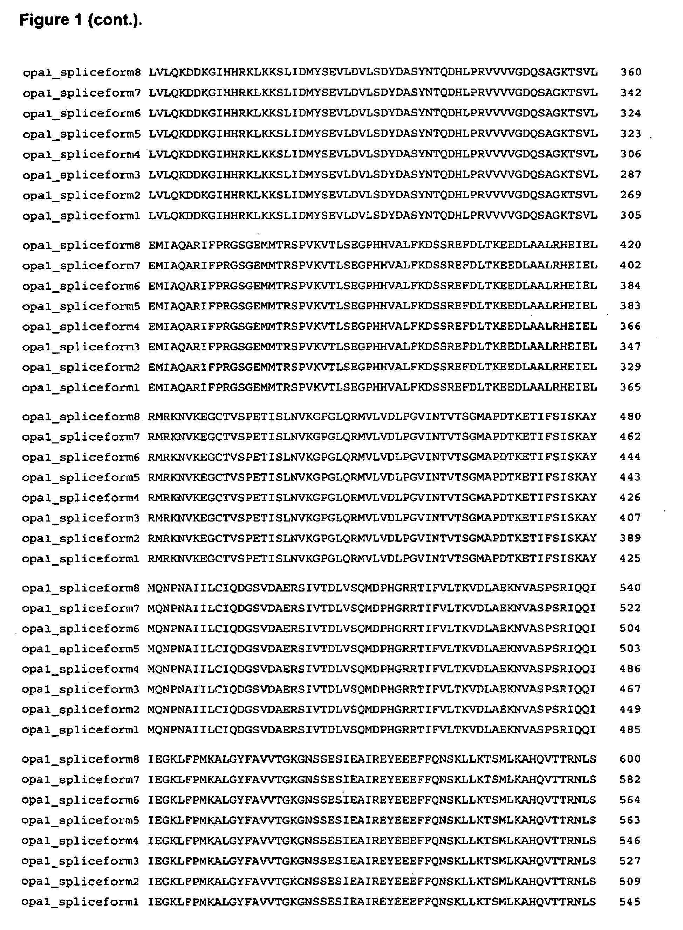

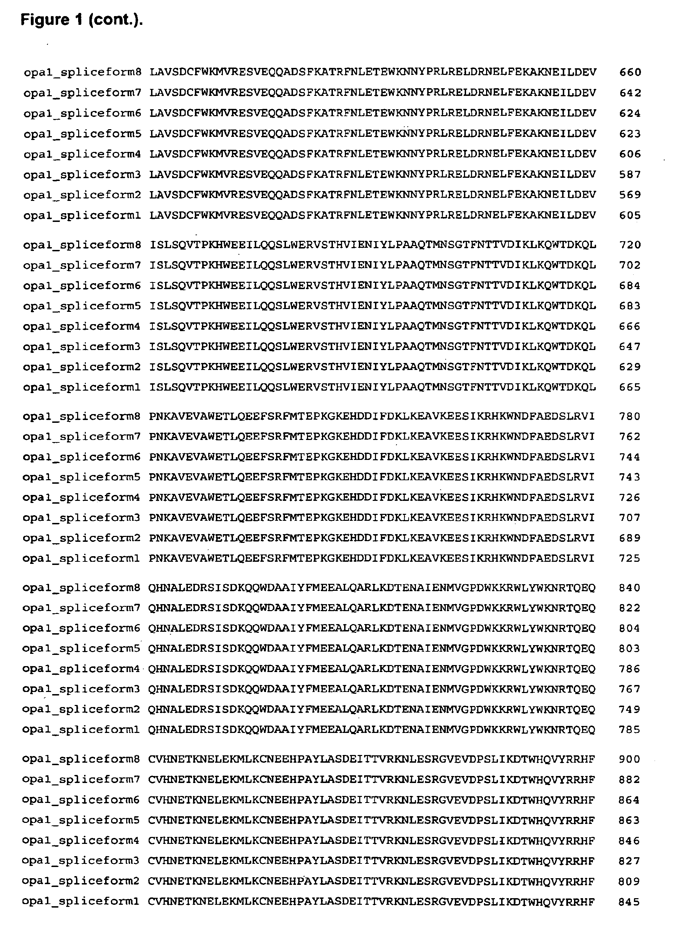

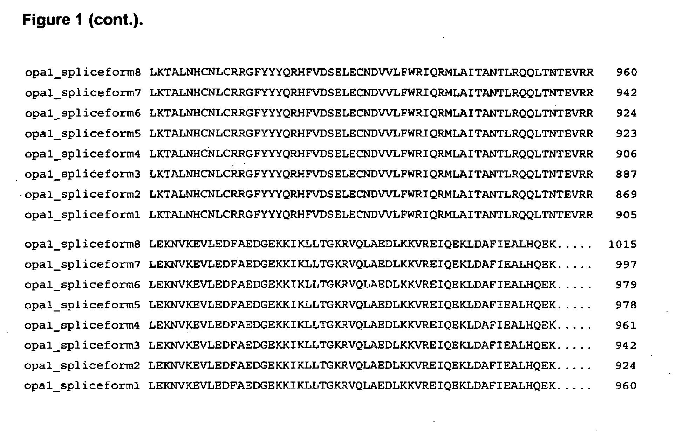

OPA1, is known to be transcribed into eight alternative splice variants which vary in the presence or absence of exon 4, 4b, and/or 5b (see protein sequence alignment in Fig.1). The expression of these splice variants is reported to vary among different tissues. In addition, these splice variants give rise to several protein isoforms (Alexander, 2000, Nat Genet, 26, 211-215; Delettre, 2001, Hum Genet, 109, 584-591; Delettre, 2000, Nat Genet, 26, 207-210; Delettre, 2002, Mol Genet Metab, 75, 97-107; Griparic, 2004, J Biol Chem, 279, 18792-18798; Olichon, 2003, J. Biol. Chem., 278, 7743-7746; Olichon, 2002, FEBS. Lett, 523, 171-176; Pesch, 2004, Invest Ophthalmol Vis Sci, 45, 4217-4225; Pesch, 2001, Hum Mol Genet, 10, 1359-1368; Satoh. 2003, Biochem Biophys Res Commun, 300, 482-493; Thiselton, 2002, Invest Ophthalmol Vis Sci, 43, 1715-1724). The identity and the biogenesis of the different protein isoforms of OPA1 remain elusive in the prior art and are not known.

Diagnosis of mitochondrial dysfunction has so far been based on the following detection systems. Various DNA-based detection/screening methods for nuclear or mitochondrial DNA mutations are commonly used. For example, there are methods to determine enzymatic activities of mitochondria, such as enzymatic activities of the respiratory chain or F₁F_{O}-ATP synthase and various histological methods allowing staining of enzymes of the respiratory chain complexes. Evaluation of the ultrastructure of mitochondria is done by electron microscopy. These methods are very laborious, difficult to automate, too specialized (for only single defects), not robust, not easy to perform, or require large amounts and difficult to get tissue samples. Methods employed in the art mostly determine isolated mitochondrial activities and do not allow a simple and general evaluation of the functionality of mitochondria. Mostly a variety of methods have to be performed and evaluated in a combined fashion to give a general diagnostic overview of mitochondrial function.

The technical problem underlying the present invention is the provision of means and methods for diagnosing mitochondrial dysfunction and diseases related thereto, respectively. In particular, means and methods for the early detection of mitochondrial dysfunction and diseases/disorders related thereto are of need.

The solution to the above technical problem is achieved by providing the embodiments characterized in the claims.

The present invention relates to a method for determining the susceptibility for, predisposition for or the presence of a disorder correlated with mitochondrial dysfunction or mitochondrial disease, comprising the in vitro determination selected from the group consisting of
(a) the in vitro determination of the ratio of at least one large and at least one small isoform of the optic atrophy 1 protein (OPA1), whereby said large isoform has an apparent molecular weight of more than 91 kD and whereby said small isoform has an apparent molecular weight of less than 91 kD, said molecular weights being determined by SDS-PAGE analysis; and/or
   whereby said large isoform has an apparent molecular weight of more than 95 kD and whereby said small isoform has an apparent molecular weight of less than 95 kD, said molecular weights being determined by mass spectrometry;
(b) the in vitro determination of (a) wherein the ratio of at least two OPA1 isoforms is determined; and
(c) the in vitro determination of (a) comprising the step of determining the ratio between at least one large OPA1 isoform and an internal standard and between at least one small OPA1 isoform and an internal standard,
wherein a decrease of the large OPA1 isoforms accompanied by an increase of the small OPA1 isoforms is diagnostic for said disorder or disease.

The present invention solves the above identified technical problem since, as documented herein below and in the appended examples, it was surprisingly found that OPA1 plays a certain role in a pathway selecting against dysfunctional mitochondria. As documented in the appended examples, a (proteolytically) processing of large OPA1 isoforms into small OPA1 isoforms indicates a predisposition for, a susceptibility for or the presence of a disorder correlated with mitochondrial dysfunction. In particular, in context of the present invention, it was found that mitochondrial fragmentation occurs concomitant with processing of OPA1. This happens under various conditions of mitochondrial dysfunction and, e.g., during apoptosis. The proteolytic cleavage or convertion of large OPA1 isoforms into smaller isoforms, in particular (but not limiting) into a small OPA1 isoform denoted as "OPA1-S5", -S5-OPA1-, "OPA1#5" or "s-OPA1#5" herein, is a diagnostic tool for the determination of a mitochondrial disorder/disease which can lead to a manifestation of a corresponding clinical and/or pathological situation, like (but not limiting) neurological disorders (e. g. Alzheimer's disease, bipolar disorders, stroke, Charcot-Marie-Tooth disease, ALS or Parkinson's disease), myopathies (e. g. general myopathy, Ataxia, infantile myopathy, atrophies, ocular myopathy, motor neuron disorders, general encephalomyopathy, Leigh-Syndrom, MELAS (myopathy encephalopathy lactic acidosis and stroke-like episodes), MERRF (myoclonic epilepsy with ragged-red fiber disease) or optic atrophy type 1), metabolic disorders (e. g. diabetes or obesity), infection disorders (e. g. bacterial, fungal or viral infections), neoplastic disorders or cancers, ischemias, oxidative damages and the like.

Further disorders associated with a mitochiondrial dysfunction and to be detected (for example in vitro) by the means and methods provided herein are given herein below.

As mentioned above, the present invention relates to a method for determining the susceptibility for, predisposition for or the presence of a disorder correlated with mitochondrial dysfunction or mitochondrial disease, comprising the in vitro determination selected from the group consisting of
(a) the in vitro determination of the ratio of at least one large and at least one small isoform of the optic atrophy 1 protein (OPA1), whereby said large isoform has an apparent molecular weight of more than 91 kD and whereby said small isoform has an apparent molecular weight of less than 91 kD, said molecular weights being determined by SDS-PAGE analysis; and/or
   whereby said large isoform has an apparent molecular weight of more than 95 kD and whereby said small isoform has an apparent molecular weight of less than 95 kD, said molecular weights being determined by mass spectrometry;
(b) the in vitro determination of (a) wherein the ratio of at least two OPA1 isoforms is determined; and
(c) the in vitro determination of (a) comprising the step of determining the ratio between at least one large OPA1 isoform and an internal standard and between at least one small OPA1 isoform and an internal standard,
wherein a decrease of the large OPA1 isoforms accompanied by an increase of the small OPA1 isoforms is diagnostic for said disorder or disease.

In particular, said large isoform(s) has (have) an apparent molecular weight of more than 91 kD and said small isoform has an apparent molecular weight of less than 91 kD, when said molecular weights being determined by SDS-PAGE analysis, in particular an 10% gel as disclosed herein and described in the appended examples. It is evident for the person skilled in the art that also other SDS gels and means (in particular Western-Blot analysis and the like) are useful and envisaged in context of the present invention. It is of note that the herein given value of 91 kD is, accordingly, an illustrative example and the person skilled in the art can also use other means to deduce the ratio of the herein described OPA isoforms

in a given sample to be analysed. For example, said large OPA1 isoform(s) has (have) an apparent molecular weight of more than 95 kD or, preferably, of more than 99 kD and the small OPA1 isoform(s) has (have) an apparent molecular weight of less than 95 kD or, preferably, of more than 99 kD, when said molecular weights being determined by peptide analysis, e.g. mass spectrometry.

As mentioned herein, in context of the present invention, "OPA" or "OPA1" means the optic atrophy 1 protein/gene, in particular OPA1 of human origin. Yet, in certain embodiments it is also envisaged that OPA1 of other organisms, e. g. of mouse, rat, pig, dog, bovine species or fruit fly, be assessed in context of this invention. The nucleotide and amino acid sequences of human OPA1, particularly of the eight spliceforms of OPA1, are given in the appended sequence listing and examples.

It is of note that the nucleotide and amino acid sequences of OPA1 given herein below are not limiting. Accordingly, the term "OPA" or "OPA1" also encompasses OPA1 proteins/genes having amino acid or nucleotide sequences being derivatives of those given sequences.

In terms of the present invention the term "derivatives" or "derivatives thereof" refers to amino acid or nucleotide sequences being homologous to the amino acid or nucleotide sequences shown herein, e. g. those of human OPA1, and/or amino acid or nucleotide sequences as shown herein, e. g. those of human OPA1, but having (a) particular conservative amino acid(s) exchanged. For instance, in context of the present invention, "homologous" means that amino acid or nucleotide sequences have identities of at least 50%, 60%, 70%, 80%, 90%, 95%, 98% or 99% to the sequences shown herein, e. g. those of human OPA1, wherein the higer identity values are preferred upon the lower ones.

As shown herein, upon drug-induced apoptosis, processing of OPA1 and mitochondrial fragmentation preceeded cytochrome c release. When the mitochondrial membrane potential was dissipated, processing of OPA1 and fragmentation of mitochondria, but not cytochrome c release was observed. The same phenomenon was observed in cybrid cells from a patient with MERRF syndrome and in mouse embryonic fibroblasts harbouring an error-prone mitochondrial DNA (mtDNA) polymerase gamma. Furthermore, processed OPA1 was observed in heart tissue derived from heart-specific TFAM -/- knockout mice suffering from mitochondrial cardiomyopathy and in skeletal muscle from patients with mitochondrial disorders such as MELAS. Processing of OPA1 was inhibited by addition of ortho-phenantroline and partially by addition of DCl (3,4-Dichloroisocoumarin) *in vivo.*

Recovery of mitochondrial fusion was accompanied by resynthesis of large isoforms of OPA1. Fragmentation of mitochondria could be prevented by overexpressing OPA1.

This demonstrates that various forms of mitochondrial dysfunction lead to proteolytic processing of OPA1 resulting in impaired mitochondrial fusion and points out to the existence of a pathway in which processing of OPA1 leads to fragmentation of mitochondria. Without being bound by theory, this fragmentation then serves as an early response in order to segregate dysfunctional mitochondria from the network of functional mitochondria.

These surprising findings demonstrate a proteolytic processing of larger OPA1 isoforms into smaller ones and a corresponding relationship to mitochondrial dysfunction. Accordingly, said processing occurs under various conditions of mitochondrial dysfunction, said dysfunction being linked to pathological status.

The processes of mitochondrial damage leading to dysfunction, breakdown of mitochondrial bioenergetic competence and mitochondrial fragmentation are linked through a cascade of reactions. In context of the present invention, a central molecular player, namely OPA1, that links changes in mitochondrial morphology with mitochondrial dysfunction was identified. This result is exemplary based on a wide variety of established model systems and patient material for, e. g., MERRF, MELAS, mtDNA depletion syndrome, dilated cardiomyopathy, diseases with respiratory deficiencies, e. g. diseases with respiratory deficiencies of unknown origin and aging. Without being bound by theory, it can be deduced how this cascade is organized: Mitochondrial dysfunction leads to impairment of bioenergetic competence of mitochondria. This results in reduced membrane potential and ATP production. In such compromised mitochondria a proteolytic processing of large to small isoforms of OPA1 is activated. As a consequence, fusion of mitochondria is blocked and dysfunctional mitochondria are segregated from the network of intact mitochondria. This in tum triggers further reactions such as removal and degradation of the dysfunctional fragments as reported in several systems (Priault, 2005, Cell Death Differ, online publication, 10 June 2005; Lyamzaev, 2004, Biochem Soc Trans 32, 1070; Skulachev, 2004, Mol Cell Biochem 256-257, 341). A key element of this mechanism is the regulatory inactivation of fusion-promoting OPA1 by proteolytic cleavage.

It was found in the art that, in contrast to OPA1, in the yeast homolog of OPA1 (Mgm1), lack of either the large or the small isoforms leads to an impairment of mitochondrial fusion (Herlan, 2003, J Biol Chem 278, 27781; Herlan, 2004, J Cell Biol 165,167; Sesaki, 2003, Biochem Biophys Res Commun 308, 276). Accordingly, it was speculated, that a proper balance of long and short isoforms of Mgm1 is critical for maintainance of tubular mitochondrial morphology and that, in analogy therto, there is also the possibility that an overexpression of OPA1 can lead to an imbalance in OPA1 isoforms (Chen, 2005, J Biol Chem 280, 26185)

Yet, based on the teaching provided in context of this invention, mitochondrial dysfunction (or a corresponding mitochondrial disease) is not merely correlated with decrease of any one of OPA1 isoforms, but with a decrease of particulary the large isoforms, e. g. OPA1#1 (as defined herein) and OPA1#2 (as defined herein), accompanied by an increase of the small isoforms, e. g. OPA1#3 (as defined herein) and OPA1#5 (as defined herein).

In context of the present invention it was found that the described large and small isoforms of yeast Mgm1 do not correlate to large and small isoforms of OPA1. Accordingly, the above mentioned finding that mitochondrial fragmentation in humans is correlated with a decrease of particularly large isoforms of OPA1 and an an increase of particulary small isoforms of OPA1 was unexpected.

In yeast, mitochondrial dysfunction causes a deficiency in the import of the Mgm1 precursor. Consequently, formation of the small isoform and mitochondrial fusion are impaired (Herlan, 2004, J Cell Biol 165, 167). In humans, mitochondrial dysfunction is sensed in a different fashion which is independent of protein synthesis and consequently protein import into mitochondria. Still, this leads to the specific, rapid, and intramitochondrial inactivation of the orthologous effector protein, OPA1. Taken together, mitochondrial dysfunction leads to a rapid conversion of the large isoforms into the small isoforms in humans whereas in yeast this process occurs in the opposite direction (increase of large isoform) and is slow since it requires protein turnover.

Satoh (2003, Biochem, Biophys Res Commun, 300, 482-493) identified 2 OPA1 isoforms in HeLa cells and speculates that their differential association with the inner and outer mitochondrial membrane suggests the different roles of each of these proteins for controlling the mitochondrial morphology. It is not evident from Satoh that a specific pattern of occurrence of different OPA1 isoforms, not to mention the herein described decrease of large accompanied with an increase of small isoforms, correlates with the fragmentation status of mitochondria.

As mentioned above, herein provided and demonstrated by the appended examples is a method for determining the susceptibility for, predisposition for or the precence of a disorder correlated with mitochondrial dysfunction or mitochondrial disorder/disease.

In context of the present invention, the term "correlated with" means that either a certain clinical and/or pathological situation causes mitochondrial dysfunction or that mitochondrial dysfunction leads to a certain clinical and/or pathological situation. It is of note that the present invention is particularly useful in the determination of a mitochondrial disease, dysfunction or disorder before any clinical and/or pathological symptoms are diagnosed or determined by the attending physician. Accordingly, the present invention is particularly usefull in early determination/diagnosis of a mitochondrial dysfunction and/or correlated disease.

In addition or in iteration to the above, clinical and/or pathological situations correlated with mitochondrial dysfunction or mitochondrial disorder/disease are given in the table below.

| |
|---|
| Ageing: in particular pathological and/or pre-mature aging |
| Alzheimer's disease |
| Amyotrophic lateral sclerosis (ALS) |
| Apoptosis |
| Ataxia |
| Autism |
| Barth syndrome, (familial) |
| Bipolar disorder |
| Cancer (e.g. renal cell and colorectal carcinoma, early liver, protasta, breast, bladder, primary lung, head and neck tumours, astrocytomas, adenocarcinomas in Barrett's esophagus) |
| Cardiomyopathy |
| Charcot-Marie-Tooth disease type 2a |
| Charcot-Marie-Tooth disease type 4a |
| Congenital lactic acidosis |
| Crohn disease |
| Deafness |
| Diabetes |
| Diabetic sensory neuropathy |
| Encephalomyopathy |
| Endotoxemia |
| External ophthalmoplegia (e.g. PEO) |
| Friedreich's ataxia |
| Hepatopathy (e.g. defects in SCO1) |
| Hepato-cerebral form of mtDNA depletion syndrome |
| Hereditary sensory neuropathy |
| Hereditary spastic paraplegia |
| Infantile encephalopathy |
| Infantile myopathy |
| Infectious diseases |
| Inflammatory diseases |
| Ischemia-reperfusion injury / Hypoxic damaqe / Oxidative damaqe |
| Kearns-Sayre syndrome |
| Lactic acidosis |
| Leber's hereditary opticus neuropathy (LHON) |
| Leigh's syndrome |
| Leukodystrophy |
| Mohr-Tranebjaerg-syndrome |
| Metabolic disorders (e.g. defective glucose and fatty acid metabolism) |
| Mitochondrial neurogastrointestinal-encephalomyopathy |
| Motor neuron disorders |
| mtDNA depletion syndrome |
| Myoclonus epilepsy and ragged-red fibers syndrome (MERRF) |
| Myopathy |
| Myopathy encephalopathy lactic acidosis and stroke-like episodes (MELAS) |
| Myositis |
| Neurodegenerative disorders (e.g. autosomal dominant optic atrophy, Charcot-Marie-tooth disease, Wolf-Hirschhom syndrome, ALS) |
| Non-alcoholic fatty liver disease |
| Obesity |
| Ocular myopathy |
| Optic atrophy type 1 |
| Paraganqlioma (e.g. defects in complex II / SDH) |
| Parkinson's disease |
| Pearson's syndrome |
| respiratory chain disorder |
| Rhabdomyolysis |
| Schizophrenia |
| Sideroblastic anemia |
| Stroke |
| Tubulopathy (e.g. defects in BCS1L) |
| Viral and bacterial infections |
| Wolfram syndrome |

The terms "disorder related to mitochondrial dysfunction", "mitochondrial disorder" or "mitochondrial disease" is not to be construed to the disorders provided above.

In context of the present invention, a certain given molecular weigth value , means +/- 3 kD, preferably +/- 2 kD, more preferably +/- 1 kD, more preferably +/- 0.5 kD and most preferably +/- 0.1 kD of said value. Moreover, in context of the present invention, it is envisaged that the term "less than xx kD", for example "less than 91 kD". "less than 95 kD" or "less than 99 kD", also comprises molecular weigth values being equal to xx kD, for example equal to 91, 95 kD or 99 kD.

It is evident for the person skilled in the art that certain given molecular weight values may vary, dependent on the preparational/experimental conditions employed, or, for example with respect to mass spectrometry, dependent on the information content resulting from the preparational/experimental method employed or dependent on an employed modification of the proteins/peptides to be analyzed due to a specific preparational/experimental procedure. It is, for example, known in the art that proteins/peptides to be analyzed via mass spectrometry can be modified, i. e. their theoretical molecular weight can be increased (e.g. by certain chemical modifications) or decreased (e.g. by using (a) certain protease(s)) by a certain value. It is therefore of note in context of the present invention that the molecular weigth values given for certain OPA1 isoforms can change, dependent on the particular preparational/experimental conditions employed during the corresponding mass spectrometry experiment (or other methods for determining molecular weights). The skilled person is readily in the position to deduce whether certain changes/differencies of given molecular weight values result from the particular preparational/experimental method employed or form a specific composition of the protein/peptide analysed.

In context of the present invention, the term "isoform" of OPA1 means a certain form of the OPA1 protein. Without bound by theory, an OPA1 isoform derives from (a protein encoded by) any one of spliceforms 1 to 8 of OPA1, e.g. by posttranslational processing (e. g. proteolytical processing). Without bound by theory, said posttranslational processing (e. g. proteolytical processing) leads to a shortened N-terminus of OPA1, particularly of spliceforms thereof, wherein the C-terminus remains complete. The "isoforms" of OPA1 to be scrutinized in context of the present invention are described herein in more detail. Accordingly, the term "corresponding" in context of OPA1 isoforms and OPA1 spliceforms, e.g. in the term "an OPA1 isoform having an apparent molecular weight calculated from amino acid sequences of the corresponding spliceform(s)", means that the respective OPA1 isoform can be related to or may be derived from said OPA1 spliceform(s). These spliceforms are also described herein below.

In context of the present invention, the term "spliceform" or "splice variant" of OPA1 means a form of OPA1 that emerges by alternative splicing of the primary transcript transcribed from the OPA1 gene. It is envisaged herein, that the term "spliceform" either refers to the mature transcript generated by alternative splicing, but also refers to the corresponding protein which has been translated from said mature transcript. Accordingly, the term "isoform being derived from (correponding) spliceform" means that an OPA1 isoform originates from a protein that has been translated from a mature (alternatively spliced) transcript of the OPA1 gene. Thereby, posttranslational processing (e. g. proteolytical processing) of said protein that has been translated from a mature (alternatively spliced) transcript of the OPA1 gene may occur. However, an OPA1 isoform may also directly originate from said protein, without further posttranslational processing. In such specific case, said protein then is said OPA1 isoform.

At present, 8 spliceforms of OPA1 are known in the art, which emerge by alternative splicing of exon 4, exon 4b and/or exon 5 (see Fig. 1). The corresponding amino acid sequences of these 8 spliceforms are given in SEQ ID No: 2, 4, 6, 8, 10, 12, 14 and 14 and are schown in Fig. 1. Their corresponding nucleotide acid sequences are given in SEQ ID No: 1, 3, 5, 7, 9, 11, 13 and 15.

Dependent whether exon 4, exon 4b and/or exon 5 is comprised, the OPA1 spliceforms can be defined by specific amino acid sequences, e. g. by one of the following amino acid sequences:
EYKWIVPDIVWEIDEYIDFGHKLVSEVIGASDLLLLL (SEQ ID NO: 31) corresponds to the amino acid sequences from exon 3 to exon 4b (lack of exon 4) and is comprised in spliceforms 3 and 6.
EYKWIVPDIVWEIDEYIDFGSPEETAFRATDRGSESDKHFRK (SEQ ID NO: 32) corresponds to the amino acid sequences from exon 3 to exon 5 (lack of exon 4 and 4b) and is comprised in spliceforms 2 and 4.
EKIRKALPNSEDLVKLAPDFDKIVESLSLLKDFFTSGSPEETAFRATDRGSESDKHFR K (SEQ ID NO: 33) corresponds to the amino acid sequences from exon 4 to exon 5 (lack of exon 4b) and is comprised in spliceforms 1 and 7.
GSPEETAFRATDRGSESDKHFRKVSDKEKIDQLQEELLHTQLKYQRILERLEKENKE LRK (SEQ ID NO: 34) corresponds to the amino acid sequences from exon 5 to exon 6 (lack of exon 5b) and is comprised in spliceforms 1, 2, 3 and 5.

Other amino acid sequences specific for a certain OPA1 spliceform can be derived from the amino acid sequences of the OPA1 spliceforms given herein below (e. g. Fig. 1).

Since an OPA1 isoform to be employed in context of the present invention may be derive from one particular OPA1 spliceform, the above mentioned amino acid sequences defining the different OPA1 spliceforms may also be used to determine the ratio of given OPAL1 isoforms as defined herein. For example, since the present invention provides evidence that OPA1-L1 be derived from spliceform 7 and OPA1-L2 be derived from spliceform 1, OPA1-L1 may, e. g. be characterized in that it comprises the amino acid sequence EKIRKALPNSEDLVKLAPDFDKIVESLSLLKDFFTSGSPEETAFRATD RGSESDKHFRK (SEQ ID NO: 33) and in that it not comprises the amino acid sequence GSPEETAFRATDRGSESDKHFRKVSDKEKIDQLQEELLHTQLKYQRILER LEKENKELRK (SEQ ID NO: 34) and OPA1-L2 may, e. g. be characterized in that it comprises the amino acid sequence EKIRKALPNSEDLVKLAPDFDKIVESLSLLKDFF TSGSPEETAFRATDRGSESDKHFRK (SEQ ID NO: 33) and GSPEETAFRATDRGS ESDKHFRKVSDKEKIDQLQEELLHTQLKYQRILERLEKENKELRK (SEQ ID NO: 34). However, since the OPA1 isoforms to be employed in context of the present invention may derive from the OPA1 splicefroms by (proteolytical) processing, not the complete amino acid sequences as given above, but fragments or derivatives thereof, may be used to determine a certain OPA1 isoform.

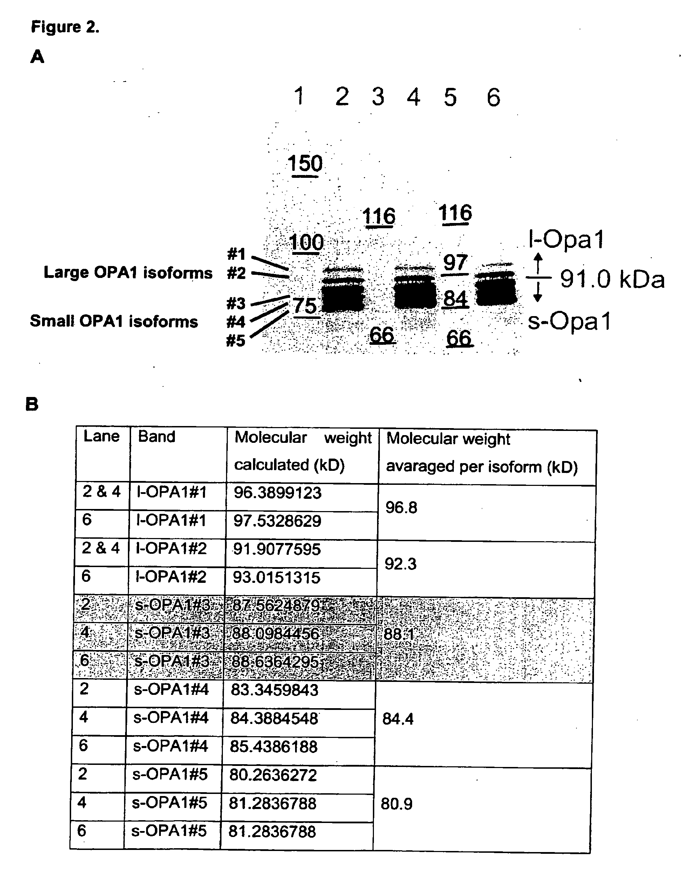

Particularly useful in context of the present invention, and exemplified in the appended examples (e. g. Fig.2), are SDS-PAGE-gels having a polyacrylamide concentration of 10%. However, it is also envisaged that SDS-PAGE-gels to be employed in context of the present invention have other polyacrylamide concentrations. E. g. said concentrations may be 1%, 2%, 3%, 4%, 5% or 10 % higher or lower than that of a 10% SDS-PAGE, e.g. than that of the 10% SDS-PAGE-gel as exemplified herein, but also other concentrations are envisaged. A person skilled in the art is readily in the position to transfer molecular weigth values deduced on the basis of an SDS-PAGE-gel having a certain polyacrylamide concentration, e. g. 10% as exemplified herein (e. g. Fig.2), to molecular weigth values deduced on the basis of an SDS-PAGE-gel having a different polyacrylamide concentration. As mentioned above, also other means than direct determination of a given molecular weight from an SDS-PAGE-gel may be used in context of this invention. For example, the appended experimental data provide for corresponding Western-blots.

The meaning of the term "Mass spectrometry" (MS) is and corresponding methods are known in the art. Particularly useful "mass spectrometry" methods to be employed in context of the present invention, and as exemplified herein (Example 3), are MALDI-MS or LC-MS/MS. Further "mass spectrometry" methods are known in the art and can easily be adapted to the specific needs of the present invention by a person skilled in the art.

The term "molecular weights being determined by mass spectrometry" means that the apparent molecular weight of a certain OPA1 isoform is determined by performing mass spectrometry analysis on said OPA1 isoform (e. g. as in example 3) and using the results of said mass spectrometry analysis to calculate said apparent molecular weight of said certain OPA1 isoform on the basis of the amino acid sequence of OPA1. Since eight alternative spliceforms exist of OPA1, having different amino acid sequences, the result of said calculation may vary, dependent on the spliceform, the amino acid sequence of which is used for said calculation. Examples of such determination of molecular weights by mass spectrometry are given herein (example 3). The principle of such determination is described in the following:

First, the amino acid sequences of certain peptide streches comprised in an OPA1 isoform to be analysed is determined by mass spectrometry (e. g. as in example 3). Second, the resulting amino acid sequences of said peptide streches are then compared with the amino acid sequences of the eight spliceforms of OPA1. Then, it is determined which certain OPA1 spliceforms comprise the amino acid sequences of said peptide streches and which do not. Subsequently, of the spliceforms that comprise the amino acid sequences of said peptide streches, that amino acid sequence it is estimated, which starts with the amino acid sequence of the most N-terminal peptide determined. Last, from said estimated amino acid sequence, the theoretical molecular weight of the respective OPA1 isoform to be analysed is calculated based on the known molecular weights of the amino acid residues comprised.

It is of note that the so determined theoretical molecular weight may be further increased by the presence of a few further N-terminally located amino acid residues present in the (proteolytically) processed mature OPA1 isoform. The person skilled in the art is readily in the position to determine said slightly increased molecular weight, by taking advantage of the teaching of the present invention. As a non limiting example, the determination of such slightly increased theoretical molecular weight of certain OPA1 isoforms is provided in the so called "refined analysis" of appended example 3

In context of the present invention, the large isoforms of OPA1 may comprise two isoforms (e. g. OPA1-L1 and OPA1-L2) and the small isoforms of OPA1 may comprise three isoforms (e. g. OPA1-S3, OPA1-S4 and OPA1-S5). However, it is also envisaged that further, possibly existing isoforms may assigned as large or small isoforms in context of the present invention. For instance, it is evident for a skilled person that, e. g., single bands of an SDS-PAGE/Western-blot as exemplified herein, may represent not only one, but several different isoforms and/or that further isoforms, larger or smaller than the particular isoforms defined herein may be present. For example, particularly the band corresponding to OPA1-S4 as defined herein may correspond to (a) further OPA1 isoform(s). Again, the gist of the present invention is based on the fact the determination of "small" versus "large" isoforms is illustrative for mitochondrial dysfunction and corresponding related disorders/diseases. Therefore, further, possibly existing isoforms may, e. g., be detectable by alternative comparable methods known in the art and may also be taken into consideration in the herein provided methods and means. For example, such methods may be SDS-PAGEs taking advantage of gels having very low polyacrylamide concentrations (e.g. 1%, 2%, 3% or 4%) and/or Western-blots taking advantage of radionuclide labelling, e. g. radionucleotide labelling of (secondary) antibodies used in said Western-blots, or other labelling approaches known in the art, e. g. other very sensitive labelling approaches being suitable for the detection of proteins being present in low amount(s)/concentration(s). Moreover, such methods may be a two dimensional gelelectrophoresis methods. These and orther alternative methods for detecting isoforms of certain proteins/genes, like OPA1, are known in the art. It is envisaged that such alternative methods may also be employed in context of the present invention.

However, it is preferred that each single band as evident from the SDS-PAGE analysis as employed and exemplified herein represents one single OPA1 isoform. Accordingly, in one embodiment of the present invention, the two large OPA1 isoforms as defined herein (e. g. OPA1-L1 and OPA1-L2) are represented by two single bands, and the three small OPA1 isoforms as defined herein (e. g. OPA1-S3, OPA1-S4 and OPA1-S5) are represented by three single bands occurring in an SDS-PAGE, e. g. an SDS-PAGE as exemplified herein.

In context of the present invention, the two large OPA1 isoforms are indicated by numbers 1 and 2, namely 1 for the largest and 2 for the second largest OPA1 isoform. The three small OPA1 isoforms are indicated by numbers 3, 4 and 5, namely 3 for the largest of the three small isoforms, 4 for the second largest of the three small isoforms and 5 for the smallest isoform. The numbering of the OPA1 isoforms to be employed in context of the present invention is also given in the appended examples and corresponding figures, e. g. Fig.2. In accordance thereto the OPA1 isoforms as employed in context of the present invention are termed as follows: OPA1-L/I1, L/I-OPA1#1, OPA1#1 or L/I1-OPA1 for the largest OPA1 isoform. OPA1-L/I2, L/I-OPA1#2, OPA1#2 or L/I2-OPA1 for the second largest OPA1 isoform. Large isoform(s) in context of the present invention is (are), e. g., OPA1-L1 and/or OPA1-L2. OPA1-S/s3, S/s-OPA1#3, OPA1#3 or S/s3-OPA1 for the largest of the three small OPA1 isoforms. OPA1-S/s4, S/s-OPA1#4, OPA1#4 or S/s4-OPA1 for the second largest of the three small isoforms. OPA1-S/s5, S/s-OPA1#5, OPA1#5 or S/s5-OPA1 for the smallest OPA1 isoform. Accordingly, small isoform(s) in context of the present invention is (are), e. g., OPA1-S3, OPA1-S4 and/or OPA1-S5.

It is of note that the specific numbering is indicative for the specific OPA1 isoform, and that the additional terming, like "I" for large; "s" for small or "OPA", "OPA1" or "OPA1#" for OPA1 may slightly vary. However the abbreviations "L" or "I" indicate large isoforms and "S" or "s" indicate small isoforms of OPA1.

In view of the teaching provided herein, also in the appended examples, the OPA1 isoforms employed in context of the present invention are defined as follows:

In one aspect of the present invention, the term "OPA1 isoform" means a protein encoded by the OPA1 gene, but particularly be derived from at least one of the different spliceforms of OPA1 (e. g. from at least one of spliceforms 1 to 8 as depicted in Figure 1), e. g. by posttranslational (e. g. proteolytical) processing, wherein said proteins are distinguishable by their molecular weight and/or (a) certain amino acid sequence(s). From the above, it is, inter alia, evident that an "OPA1 isoform" as employed in context of the present invention comprises (an) amino acid streche(s) which unambiguously characterize it as a polypeptide/protein derived from OPA1. In this context, "derived from OPA1" particularly means encoded by the OPA1 gene and/or generated from OPA1 by the herein described and defined OPA1 processing. Thus, an "OPA1 isoform" as employed can particularly be characterized by (a) certain amino acid strech(es) of any one od SEQ ID No: 2, 4, 6, 8, 10, 12, 14 or 16 or by (a) certain amino acid strech(es) encoded by any one od SEQ ID No: 1, 3, 5, 7, 9, 11, 13 or 15.

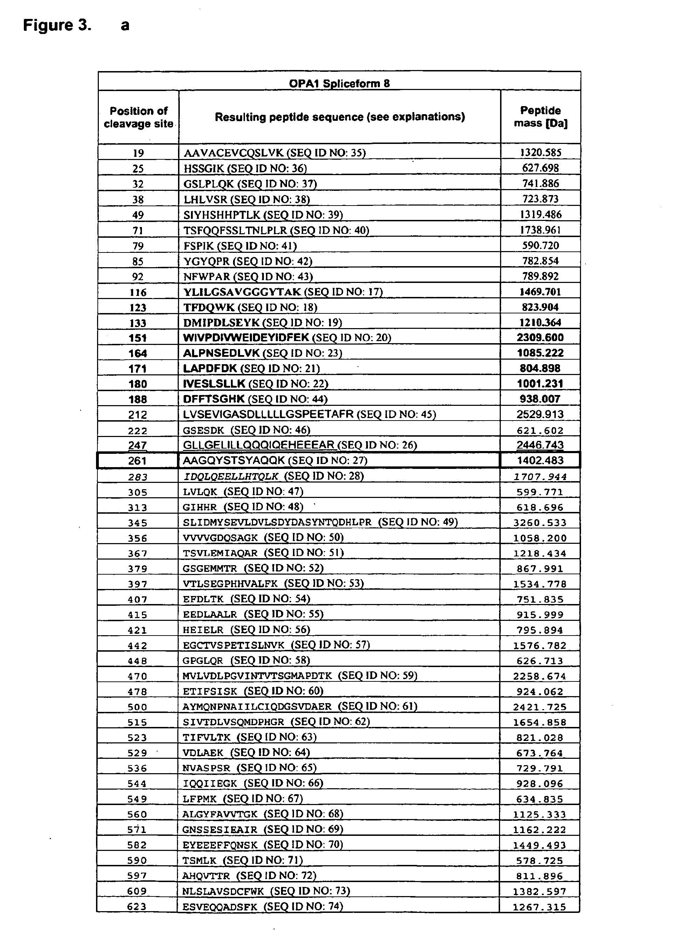

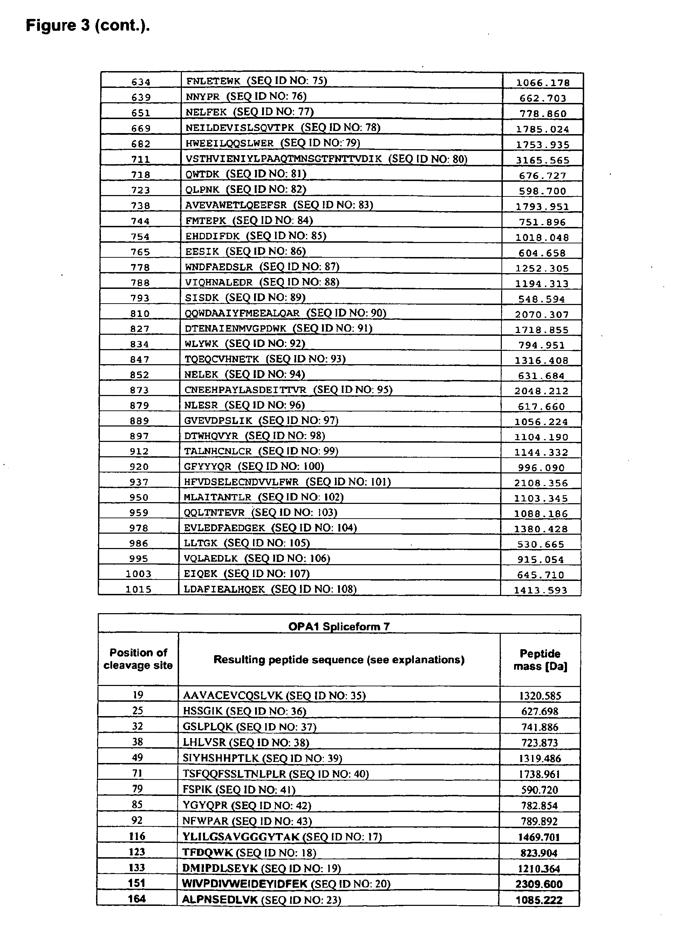

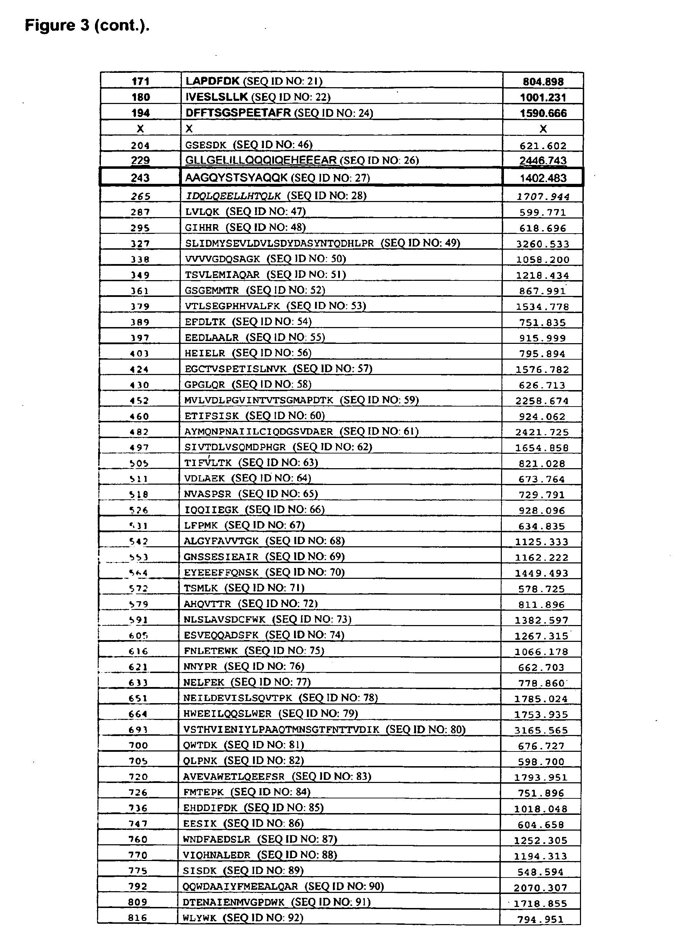

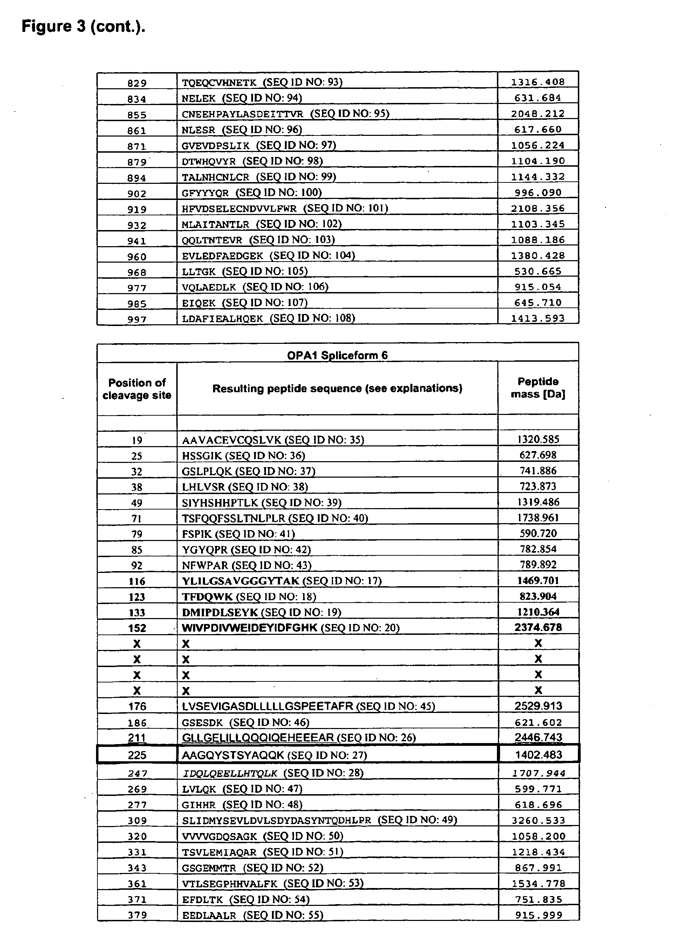

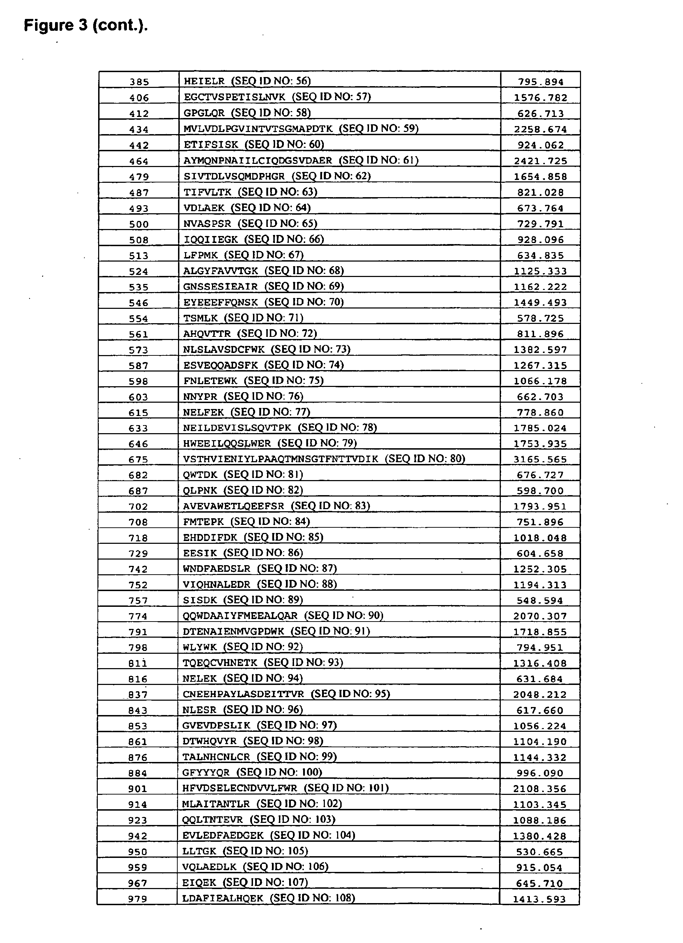

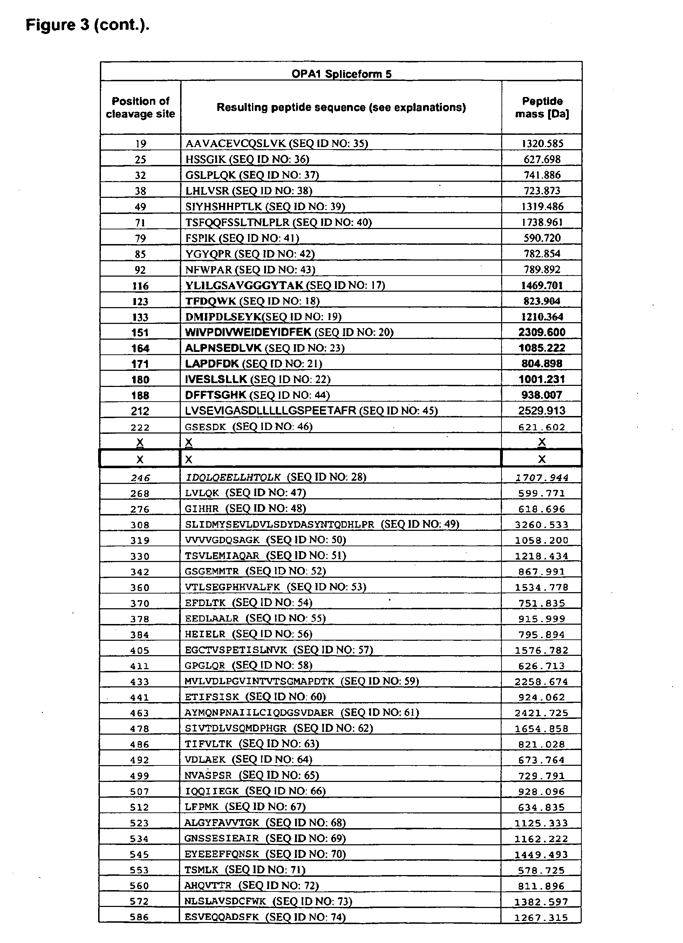

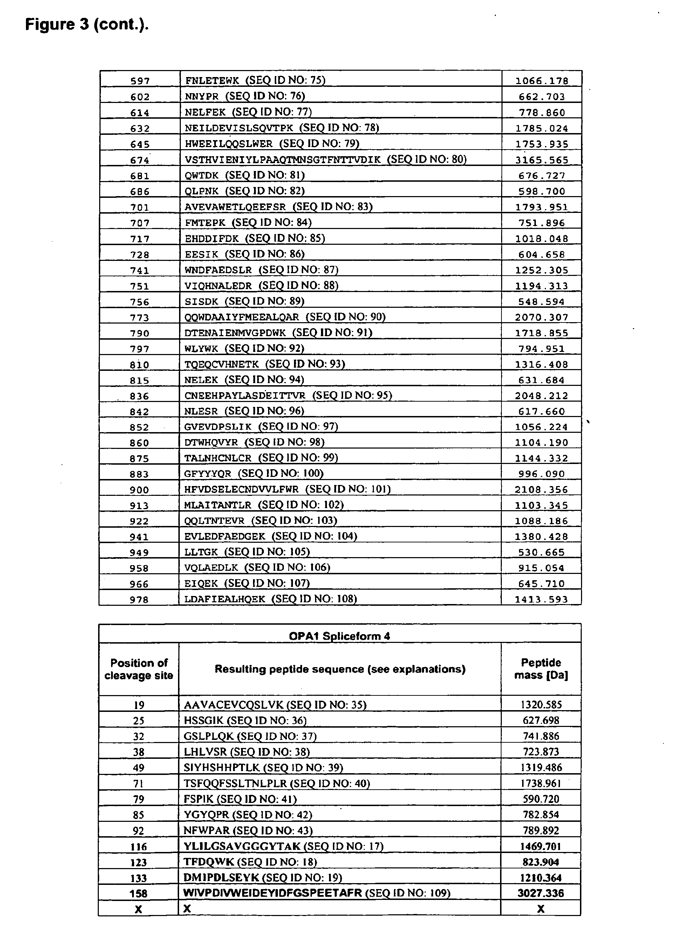

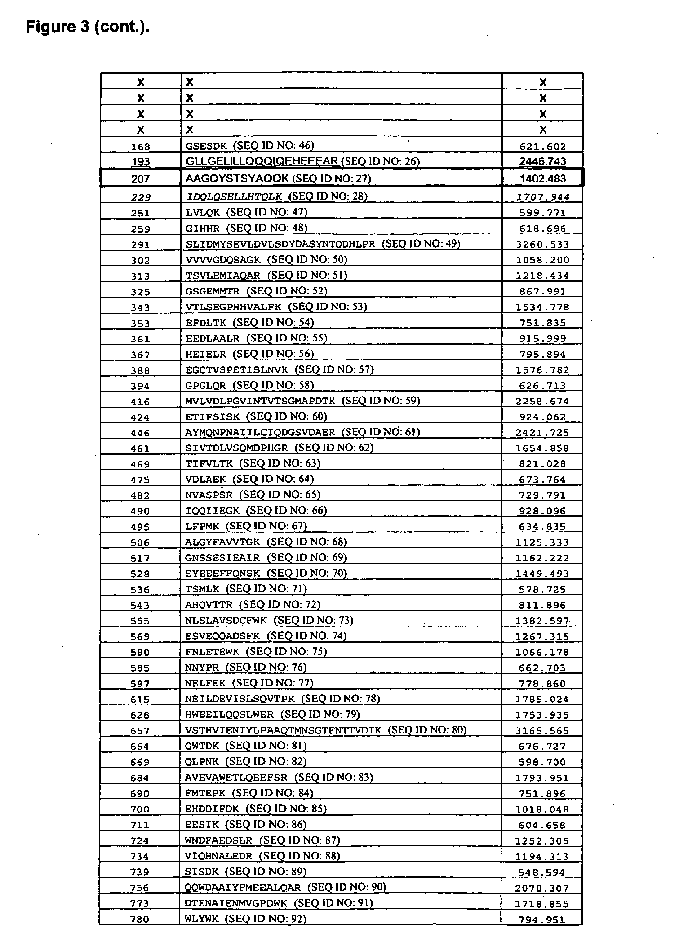

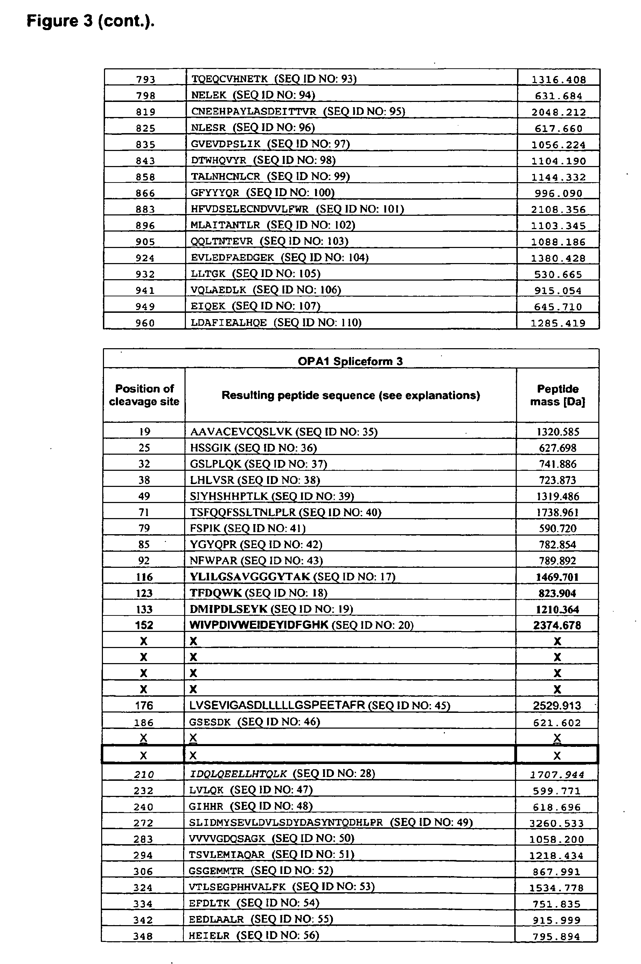

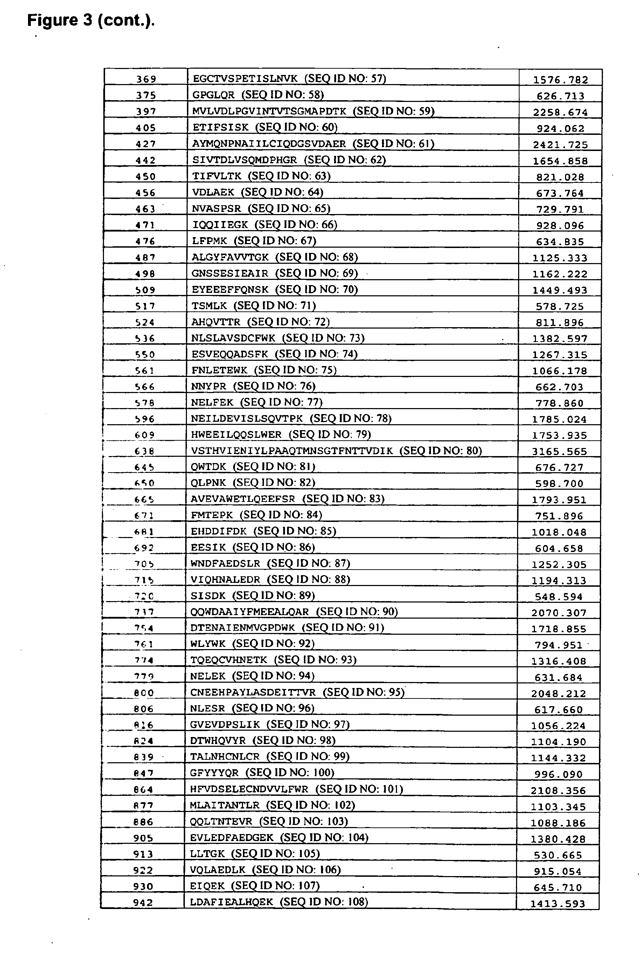

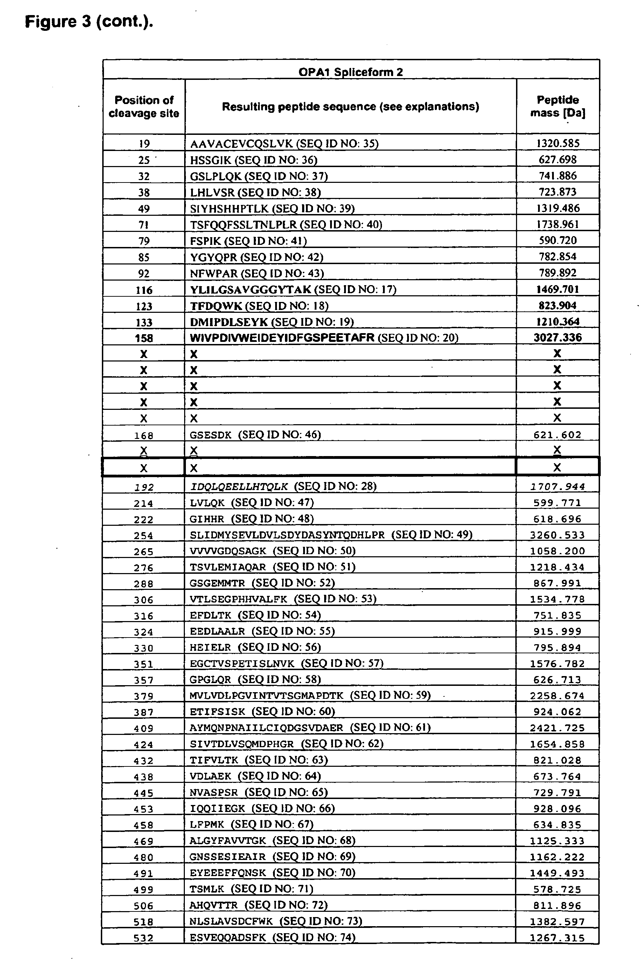

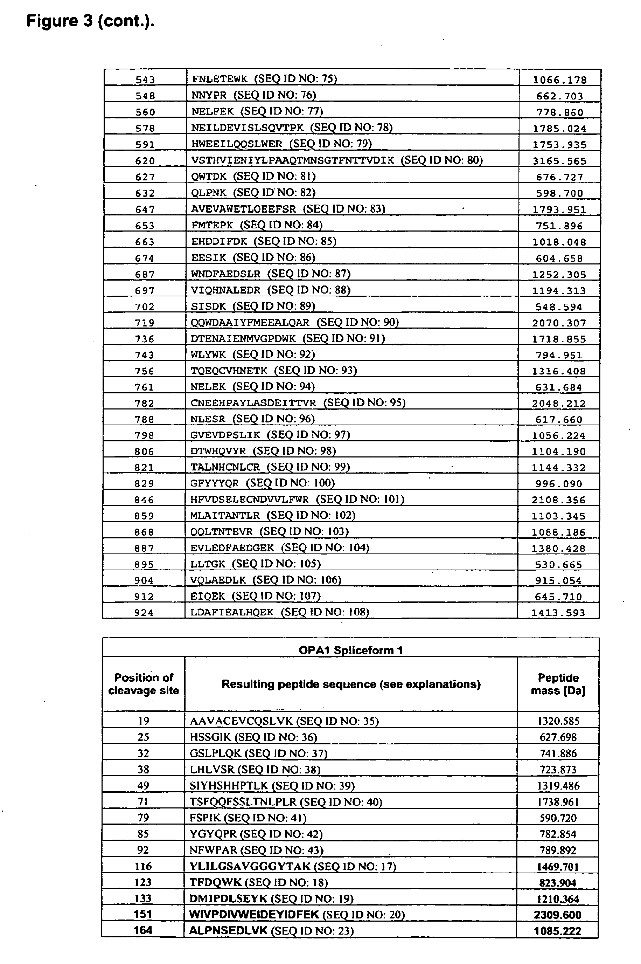

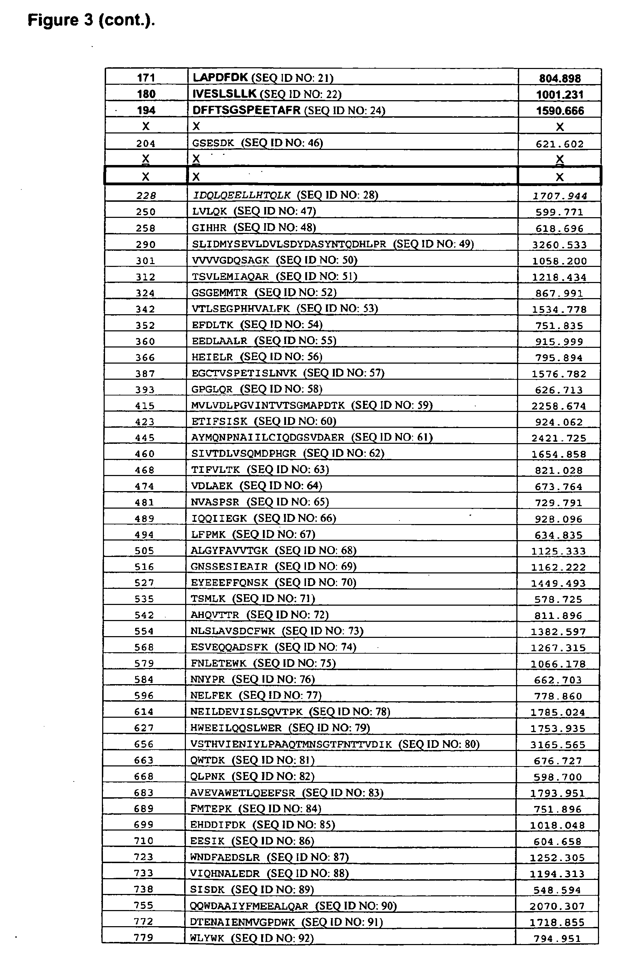

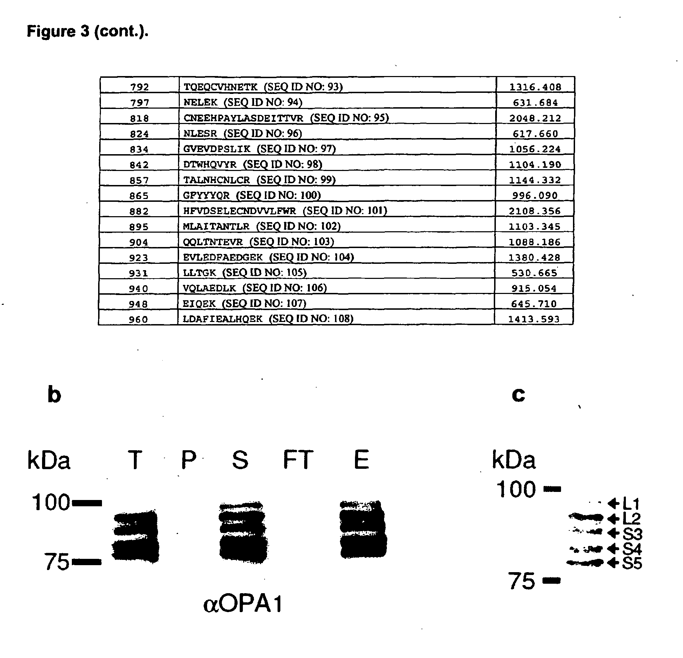

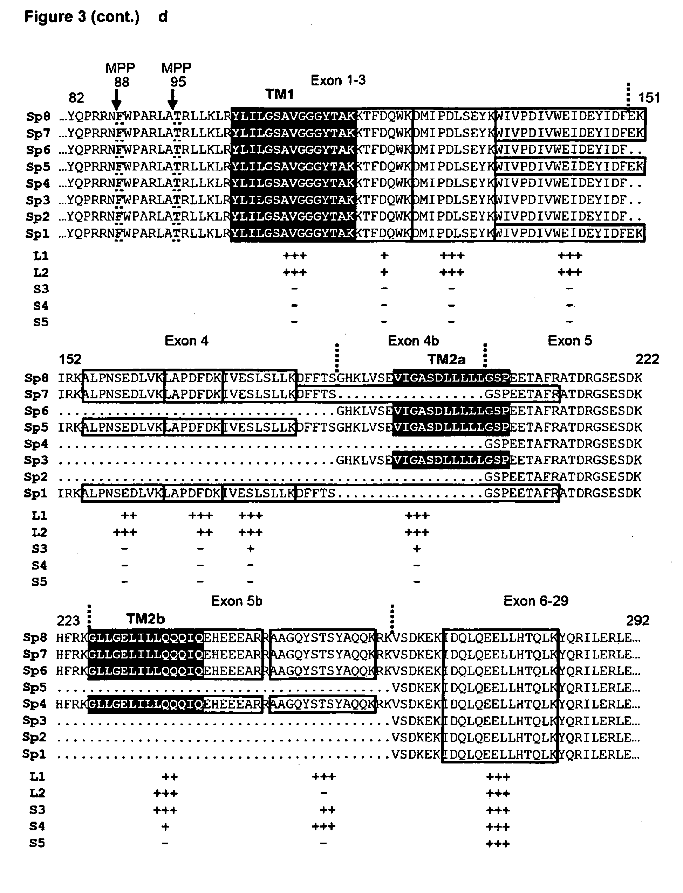

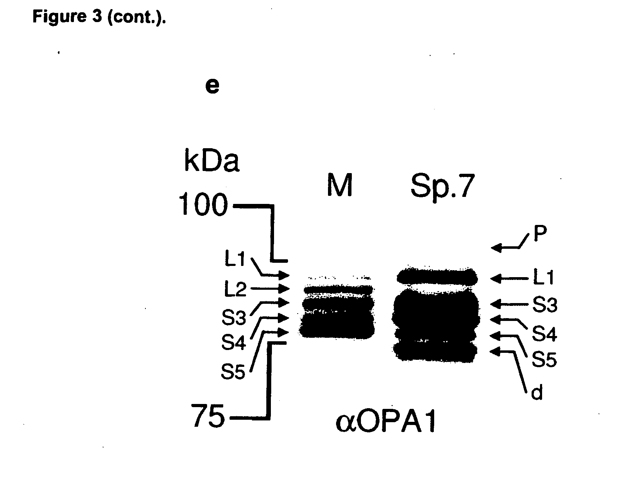

In context of the present invention, the term "molecular weight" may, inter alia, refer to the apparent molecular weight. Said apparent molecular weight can be determined by methods known in the art. E. g., said apparent molecular weight can be determined by SDS-PAGE, and, accordingly, also from Westem-blots, or can be calculated from the amino acid sequence of OPA1, particularly from the amino acid sequence(s) of the corresponding spliceform(s) by taking advantage of mass spectrometry methods. Examles of the determination of the OPA1 isoforms by using these techniques are given in the appended examples (e. g. example 2/3/11; Fig.2/3).

As already mentioned above, in context of the present invention, certain given molecular weigth values are apparent molecular weigth values. It is envisaged, that the certain molecular weigth values given herein may slightly vary, e. g. with respect to the molecular weigth of the protein present in vivo. Said variation may by in the range of 5 kD, 4 kD, 3 kD, 2 kD, 1 kD, 0.5 kD, 0.4 kD, 0.3 kD, 0.2 kD or 0.1 kD, whereby the smaller variations are preferred over the larger variations.

In context of the present invention, large isoforms comprise an isoform having an apparent molecular weight of about 97 kD (96.8 kD) (defined as OPA1-L1) or an isoform having an apparent molecular weight of about 92 kD (92.3 kD) (defined as OPA1-L2), said molecular weights being determined by SDS-PAGE analysis. Moreover, in context of the present invention large isoforms comprise an isoform having an apparent molecular weight of about 104 kD (104.0 kD) or, preferably, of about 105 kD (105.1 kD) (defined as OPA1-L1) or an isoform having an apparent molecular weight of about 99 kD (99.2 kD) or, preferably, of about 100 kD (100.0 kD) (defined as OPA1-L2), said molecular weights being determined by mass spectrometry.

The molecular weight values determined by mass spectrometry of OPA1-L1 and OPA1-L2 are given as averaged values of the four smallest (OPA1-L2) and the four largest (OPA1-L1) molecular weight values of the second column ("I-OPA1#1/#2") of the corresponding table in example 3.

In this context, it is of note that the present invention provides evidence that OPA1-L1 be derived from spliceform 7 and OPA1-L2 be derived from spliceform 1 or spliceform 4. Accordingly, it is particularly envisaged herein that OPA1-L has an apparent molecular weight determined by mass spectrometry of about 105 kD (104.9 kD) or, preferably, of about 106 kD (105.8 kD). OPA1-L2 is particularly envisaged to have an apparent molecular weight determined by mass spectrometry of about 101 kD (100.7 kD) or, preferably, of about 102 kD (101.5 kD), if determined on the basis of spliceform 1. If determined on the basis of spliceform 4, OPA1-L2 is particularly envisaged to have an apparent molecular weight determined by mass spectrometry of about 101 kD (100.8 kD) or, preferably, of about 102 kD (101.7 kD) (see second column ("I-OPA1#11#2") of the tables in example 3).

In addition to the above, the large OPA1 isoforms to be scrutinized in context of the present invention, namely OPA1-L1 and OPA1-L2, are characterized by comprising, e. g. as most N-terminal peptides, amino acid stretches or amino acid peptides comprising one or more of the following sequences: YLILGSAVGGGYTAK (SEQ ID No: 17), TFDQWK (SEQ ID No: 18), DMIPDLSEYK (SEQ ID No: 19), WIVPDIVWEIDEYIDFEK (SEQ ID No: 20), LAPDFDK (SEQ ID No: 21), IVESLSLLK (SEQ ID No: 22), ALPNSEDLVK (SEQ ID No: 23), DFFTSGSPEETAFR (SEQ ID No: 24) and TRLLKLRYLILGS (SEQ ID No: 25) and FWPARLATRLLKLRYLILGS (SEQ ID NO: 111).

It is evident from the teaching provided herein that the large OPA1 isoforms to be scrutinized in context of the present invention, namely OPA1-L and OPA1-L2, may (further) be characterized by further amino acid stretches or amino acid peptides of OPA1, or of particular OPA1 spliceforms, e. g. by further amino acid stretches or amino acid peptides of OPA1, or of particular the OPA1 spliceforms, lying more C-terminal to the above mentioned amino acid stretches or amino acid peptides. For instance, said further amino acid stretches or amino acid peptides of OPA1 may, in addition to or instead of the above mentioned sequences, comprise one or more of the following sequences: GLLGELILLQQQIQEHEEEAR (SEQ ID No: 26), AAGQYSTSYAQQK (SEQ ID No: 27) or IDQLQEELLHTQLK (SEQ ID No: 28)_{.}

However, it is of note that particularly OPA1-L2 does not comprise amino acid stretches or amino acid peptides comprising one or more of the following sequences: GLLGELILLQQQIQEHEEEAR (SEQ ID No: 26) or AAGQYSTSYAQQK (SEQ ID No: 27).

It is further evident from the teaching provided herein that, the large OPA1 isoforms to be scrutinized in context of the present invention, namely OPA1-L1 and OPA1-L2, may also be characterized by not comprising amino acid stretches or amino acid peptides comprising amino acid streches of OPA1, particularly of the OPA1 spliceforms, lying more N-terminal to those amino acid peptides comprising amino acid streches of OPA1, particularly of OPA1 spliceforms, that correspond to the most N-terminal peptides of the large OPA1 isoforms as defined herein, e. g. the peptide TRLLKLRYLILGS (SEQ ID No: 25) (see, e. g. Example 3; Fig. 1; Fig. 3).

Accordingly, the the large OPA1 isoforms to be scrutinized in context of the present invention, namely OPA1-L1 and OPA1-L2, are characterized by the feature that their apparent N-terminus correlates to amino acid position 102, preferably to amino acid position 95 of spliceforms 1 to 8 (SEQ ID No: 16, 14, 12, 10, 8, 6, 4 and 2, respectively).

As mentioned above, evidence is provided herein, that the OPA1 isoform OPA1-L1 be derived from spficeform 7 (SEQ ID Nos: 3/4) and the OPA1 isoform OPA1-L2 be derived from spliceform 1 (SEQ ID Nos: 15/16) or spliceform 4 (SEQ ID Nos: 9/10) of OPA1.

In context of the present invention, small isoforms comprise an isoform having an apparent molecular weight of about 88 kD (88.1 kD) (defined as OPA1-S3), an isoform having an apparent molecular weight of about 84 kD (84.4 kD) (defined as OPA1-S4) or an isoform having an apparent molecular weight of about 81 kD (80.9 kD) (defined as OPA1-S5), said molecular weights being determined by SDS-PAGE analysis. Moreover, in context of the present invention small isoforms comprise an isoform having an apparent molecular weight of about 92 kD (91.8 kD) or, preferably, of about 96 kD (95.9 kD) (defined as OPA1-S3), an isoform having an apparent molecular weight of about 89 kD (89.2 kD) or, preferably, of about 92 kD (91.8 kD) (defined as OPA1-S4) or an isoform having an apparent molecular weight of about 87 kD (86.8 kD) or, preferably, of about 87 kD (86.8 kD) (defined as OPA1-S5), said molecular weights being determined by mass spectrometry.

Based on the first estimate of appended example 3, the following applies with respect to the small OPA1 isoform employed herein:

OPA1-S3 is additionally characterized by comprising, e. g. as most N-terminal peptides, amino acid stretches or amino acid peptides comprising one or more of the following sequences: GLLGELILLQQQIQEHEEEAR (SEQ ID No: 26), AAGQYSTSYAQQK (SEQ ID No: 27) or IDQLQEELLHTQLK (SEQ ID No: 28).

OPA1-S4 is characterized by comprising, e. g. as most N-terminal peptides, amino acid stretches or amino acid peptides comprising one or more of the following sequences: AAGQYSTSYAQQK (SEQ ID No: 27) or IDQLQEELLHTQLK (SEQ ID No: 28) OPA1-S5 is characterized by comprising, e. g. as most N-terminal peptides, amino acid stretches or amino acid peptides comprising the following sequence: IDQLQEELLHTQLK (SEQ ID No: 28)

Moreover, the small OPA1 isoforms to be scrutinized in context of this invention, namely OPA1-S3, OPA1-S4 and OPA1-S5 are characterized by not comprising amino acid stretches or amino acid peptides comprising one or more of the following sequences: YLILGSAVGGGYTAK (SEQ ID No: 17), TFDQWK (SEQ ID No: 18), DMIPDLSEYK (SEQ ID No: 19), WIVPDIVWEIDEYIDFEK (SEQ ID No: 20), LAPDFDK (SEQ ID No: 21), IVESLSLLK (SEQ ID No: 22), ALPNSEDLVK (SEQ ID No: 23), DFFTSGSPEETAFR (SEQ ID No: 24) and TRLLKLRYLILGS (SEQ ID No: 25) It is of note that the small OPA1 isoforms to be scrutinized in context of this invention, namely OPA1-S3, OPA1-S4 and OPA1-S5 are further characterized by not comprising amino acid stretches or amino acid peptides of OPA1, particularly of one of the OPA1 spliceforms, lying more N-terminal to those amino acid peptides comprising amino acid streches of OPA1, particularly of OPA1 spliceforms, that correspond to the most N-terminal peptides of the small OPA1 isoforms as defined herein (see, e. g. Example 3; Fig. 1; Fig. 3).

Furthermore, OPA1-S4 is characterized by not comprising amino acid stretches or amino acid peptides comprising the following sequence: GLLGELILLQQQIQEHEEEAR (SEQ ID No: 26) and OPA1-S5 is characterized by not comprising amino acid stretches or amino acid peptides comprising one or more of the following sequences: GLLGELILLQQQIQEHEEEAR (SEQ ID No: 26) and AAGQYSTSYAQQK (SEQ ID No: 27).

Accordingly, OPA1-S3 is characterized by the feature that its apparent N-terminus correlates to amino acid position 227 of spliceform 8 (SEQ ID No: 2), to amino acid position 209 of spliceform 7 (SEQ ID No: 4), to amino acid position 191 of spliceform 6 (SEQ ID No: 6) or to amino acid position 173 of spliceform 4 (SEQ ID No: 10). Furthermore, OPA1-S4 is characterized by the feature that its apparent N-terminus correlates to amino acid position 249 of spliceform 8 (SEQ ID No: 2), to amino acid position 231 of spliceform 7 (SEQ ID No: 4), to amino acid position 213 of spliceform 6 (SEQ ID No: 6) or to amino acid position 195 of spliceform 4 (SEQ ID No: 10). Moreover, OPA1-S5 is characterized by the feature that its apparent N-terminus correlates to amino acid position 270 of spliceform 8 (SEQ ID No: 2), to amino acid position 252 of spliceform 7 (SEQ ID No: 4), to amino acid position 234 of spliceform 6 (SEQ ID No: 6), to amino acid position 233 of spliceform 5 (SEQ ID No: 8), to amino acid position 216 of spliceform 4 (SEQ ID No: 10), to amino acid position 197 of spliceform 3 (SEQ ID No: 12), to amino acid position 179 of spliceform 2 (SEQ ID No: 14) or to amino acid position 215 of spticeform 1 (SEQ ID No: 16).

Based on the refined analysis of appended example 3, the following preferred matter applies with respect to the small OPA1 isoform employed herein:

OPA1-S3 is additionally characterized by comprising, e. g. as most N-terminal peptides, amino acid stretches or amino acid peptides comprising one or more of the following sequences: IVESLSLLK (SEQ ID No: 22), DFFTSGSPEETAFR (SEQ ID No: 24), GLLGELILLQQQIQEHEEEAR (SEQ ID No: 26), AAGQYSTSYAQQK (SEQ ID No: 27) or IDQLQEELLHTQLK (SEQ ID No: 28). OPA1-S4 is characterized by comprising, e. g. as most N-terminal peptides, amino acid stretches or amino acid peptides comprising one or more of the following sequences: GLLGELILLQQQIQEHEEEAR (SEQ ID No: 26), AAGQYSTSYAQQK (SEQ ID No: 27) or IDQLQEELLHTQLK (SEQ ID No: 28). OPA1-S5 is characterized by comprising, e. g. as most N-terminal peptides, amino acid stretches or amino acid peptides comprising the following sequence: IDQLQEELLHTQLK (SEQ ID No: 28).

Moreover, the small OPA1 isoforms to be scrutinized in context of this invention, namely OPA1-S3, OPA1-S4 and OPA1-S5, are characterized by not comprising amino acid stretches or amino acid peptides comprising one or more of the following sequences: YLILGSAVGGGYTAK (SEQ ID No: 17), TFDQWK (SEQ ID No: 18), DMIPDLSEYK (SEQ 10 No: 19), WIVPDIVWEIDEYIDFEK (SEQ ID No: 20), LAPDFDK (SEQ ID No: 21), ALPNSEDLVK (SEQ ID No: 23) and TRLLKLRYLILGS (SEQ ID No: 25). It is of note that the small OPA1 isoforms to be scrutinized in context of this invention, namely OPA1-S3, OPA1-S4 and OPA1-S5, are further characterized by not comprising amino acid stretches or amino acid peptides of OPA1, particularly of one of the OPA1 spliceforms, lying more N-terminal to those amino acid peptides comprising amino acid streches of OPA1, particularly of OPA1 spliceforms, that correspond to the most N-terminal peptides of the small OPA1 isoforms as defined herein (see, e. g. Example 3; Fig. 1; Fig. 3). Furthermore, OPA1-S5 is characterized by not comprising amino acid stretches or amino acid peptides comprising one or more of the following sequences: GLLGELILLQQQIQEHEEEAR (SEQ ID No: 26) and AAGQYSTSYAQQK (SEQ ID No: 27).

Accordingly, OPA1-S3 is, inter alia, characterized by the feature that its apparent N-terminus correlates to amino acid position 172 of spliceform 7 (SEQ ID No: 4.

Furthermore, OPA1-S4 is characterized by the feature that its apparent N-terminus correlates to amino acid position 227 of spliceform 8 (SEQ ID No: 2), to amino acid position 209 of spticeform 7 (SEQ ID No: 4), to amino acid position 191 of spticeform 6 (SEQ ID No: 6) or to amino acid position 173 of spliceform 4 (SEQ ID No: 10).

Moreover, OPA1-S5 is characterized by the feature that its apparent N-terminus correlates to amino acid position 270 of spliceform 8 (SEQ ID No: 2), to amino acid position 252 of spliceform 7 (SEQ ID No: 4), to amino acid position 234 of spliceform 6 (SEQ ID No: 6), to amino acid position 233 of spliceform 5 (SEQ ID No: 8), to amino acid position 216 of spliceform 4 (SEQ ID No: 10), to amino acid position 197 of spticeform 3 (SEQ ID No: 12), to amino acid position 179 of spliceform 2 (SEQ ID No: 14) or to amino acid position 215 of spliceform 1 (SEQ ID No: 16).

It is of note that the molecular weight values of the OPA1 isoforms scrutinized herein which were determined by SDS-PAGE analysis, are given as averaged values corresponding to the molecular weight values of three different isoform bands within an SDS-PAGE/Westem-blot (see Fig.2).

In context of the present invention it is to be understood that the term "most N-terminal peptide(s)" means (a) peptide(s), e. g. (a) peptide(s) as defined above, that lies in the N-terminal region of the corresponding OPA1 isoform. Preferably, said peptide(s) is (are) the indeed most N-terminal peptide(s), what means that, e. g. when taking advantage of a particular protease during mass spectrometry analysis (e. g. see example 3), no other peptide can be found in the corresponding OPA1 isoform lying in a more N-terminal direction of said peptide. However, it is of note that the N-terminal amino acid(s) of said "most N-terminal peptide(s)" may not be the most N-terminal amino acid(s) of the corresponding OPA1 isoform(s). The most N-terminal amino acid(s) of the corresponding OPA1 isoform(s) may also correspond to a slightly more N-terminal amino acid position of OPA1, (particularly of the OPA1 spliceforms. For instance, said slightly more N-tenninal amino acid position may be a position lying 1 to 15, preferably 1 to 10, more preferably 1 to 5, more preferably 1 to 4, more preferably 1 to 3, more preferably 1 to 2 or 1 amino acid position(s) in the N-terminal direction of the amino acid sequence(s) corresponding to the "most N-terminal peptide(s)", e. g. the peptides as defined above.

Since, without being bound by theory, the particular amino acid sequence(s) of the "most N-terminal peptide(s)" as defined herein, e. g., depend(s) on the protease to be employed during mass spectrometry analysis (e. g. see example 3), the amino acid sequence(s) of the "most N-terminal peptide(s)" may slightly vary, e. g. in the above defined ranges in the N-terminal direction (and/or C-terminal direction). The person skilled in the art is readily in the position to figure out said slightly varied amino acid sequence(s) of the "most N-terminal peptide(s)", and accordingly the N-terminal amino acid of the OPA1 isoforms, by taking advantage of the teaching of the present invention, and, e. g., by applying mass spectrometry analysis taking advantage of different proteases, which, e. g. may cut the amino acid strand at different positions. Such mass spectrometry analysis are also envisaged to be employed in context of the present invention.

In view of the above, it is also of note that the amino acid positions of the different OPA1 spliceforms that correlates to the apparent N-termini of the corresponding OPA1 isoforms given herein above, may slightly vary. For instance, this variation may in the range of 1 to 15 amino acids in the N-terminal direction, wherein the smaller variations within this range are preferred.

The term "derivatives" or "derivatives thereof' as well as "homologous" as defined herein above, also apply, mutatis mutandis, in context of the peptides shown above, e. g. the peptides comprised in the OPA1 isoforms or the peptides that characterize the OPA1 spliceforms. Moreover, the term "derivatives" or "derivatives thereof" also refers to (a) fragment(s), e. g. (a) fragment(s) of the peptides shown above, e. g. the peptides comprised in the OPA1 isoforms or the peptides that characterize the OPA1 spliceforms. The term "fragment(s)" means amino acid streches of 3, 4, 5, 6, 7, 8, 9, 10, 11, 12, 13, 14, 15, 20, 30, 50, 100 or 150 amino acids. Also amino acid streches having other numbers of amino acids are envisaged.

In terms of the present invention the term "derivatives" or "derivatives thereof" also comprises homologies as well as conservative amino acid exchanges and further known modifications.

The term "determination of the identity, amount and/or ratio of large and/or small isoforms of the optic atrophy 1 protein (OPA1)" also refers to the determination of the identity, amount and/or ratio of N-terminal fragments being derived from posttranslational (e. g. proteolytical) processing of (an) OPA1 isoform(s)/spliceform(s) as defined herein, particularly of (an) large OPA1 isoform(s) as defined herein. Moreover, it is envisaged that further OPA1 fragments, e. g. C-terminal fragments of OPA1, being, without bound by theory, derived from posttranslational (e. g. proteolytical) processing of (an) OPA1 isoform(s)/spliceform(s) as defined herein, are scrutinized in context of the present invention, in order to diagnose mitochondrial disorders/dysfunctions, in particular the susceptibility for, predisposition for or the presence of a disorder correlated with mitochondrial dysfunction or mitochondrial disease.

Without being bound by theory, the (prolonged) posttranslational (e. g. proteolytical) processing of (an) OPA1 isoform(s)/spliceform(s) may result in short C-terminal fragments of OPA1, e. g. C-terminal fragments of OPA1 shorter than OPA1-S5 as defined herein. Again without being bound by theory, (an accumulation of) said short C-terminal fragments of OPA1 may be diagnostic for mitochondrial disorders/dysfunctions, in particular the susceptibility for, predisposition for or the presence of a disorder correlated with mitochondrial dysfunction or mitochondrial disease. Accordingly the determination of the identity, amount and/or ratio of such short C-terminal fragments of OPA1 is also disclosed herein, in order to diagnose mitochondrial disorders/dysfunctions, in particular the susceptibility for, predisposition for or the presence of a disorder correlated with mitochondrial dysfunction or mitochondrial disease.

The person skilled in the art is readily in a position to adapt the teaching provided herein to corresponding methods, kits and uses taking advantage of scrutinizing the above mentioned N-terminal and/or short C-terminal fragments of OPA1.

The term "determination of the identity, amount and/or ratio of large and/or small isoforms of the optic atrophy 1 protein (OPA1)" refers to the determination of the identity, amount and/or ratio of OPA1 isoforms via detection of any amino acid strech lying between the N-terminal amino acid of the "most N-terminal peptide(s)" defined herein of the OPA1 isoform(s) to be scrutinized, alternatively and preferred between the N-terminal amino acid of the OPA1 isoform to be scrutinized and the C-terminus of OPA1, particularly of the OPA1 spliceforms. Said C-terminus of OPA1, particularly of the OPA1 spliceforms, without being bound by theory, also corresponds to the C-terminus of the OPA1 isoforms defined herein.

For instance, said amino acid strech may be any epitope-bearing portion, or, e. g. any other portion to which a binding molecule as defined herein can bind, lying between the N-terminal amino acid of the "most N-terminal peptide(s)" defined herein of the OPA1 isoform(s) to be scrutinized, alternatively and preferred between the N-terminal amino acid of the OPA1 isoform to be scrutinized and the C-terminus of OPA1, particularly of the OPA1 spliceforms. For instance, said detection may be a detection method as defined and exemplified herein, e. g. a detection method taking advantage of the antibodies as defined and exemplified herein, or a detection method taking advantage of other binding molecules as defined herein.

In a non-limiting example, it is envisaged in context of the present invention that the ratio of the large OPA1 isoforms as defined herein, namely OPA1-L1 and OPA1-L2, can be determined via specific detection of any amino acid strech of the large OPA1 isoforms lying in N-terminal direction to the amino acid streches corresponding to the N-terminal amino acids of the "most N-terminal peptide(s)" defined herein of the small OPA1 isoform(s), alternatively and preferred lying in N-terminal direction to the amino acid streches corresponding to the N-terminal amino acid of the small OPA1 isoforms. In analogy to the above, said amino acid strech to be detected may be any epitope-bearing portion, or, e. g. any other portion to which a binding molecule as defined herein can bind and said detection may be a detection method as defined and exemplified herein, e. g. a detection method taking advantage of the antibodies as defined and exemplified herein, or a detection method taking advantage of other binding molecules as defined herein.

In another particular embodiments of this invention, it is envisaged to diagnostically distinguish between various types of mitochondrial dysfunction(s)/disease(s). In particular, it is envisaged to differentiate between mitochondrial dysfunction(s)/disease(s) dependent on depletion of mitochondrial DNA and other types of mitochondrial dysfunction(s)/disease(s). Moreover, a quantitative measure of mitochondrial dysfunction is also envisaged. These embodiments of this invention are also based on the findings provided herein and demonstrated in the appended examples (e.g. example 7), e. g. the findings that certain mitochondrial dysfunction(s)/disease(s) correlate with a particular pattern of large and/or small isoforms of the optic atrophy 1 protein (OPA1), e.g. with a certain ratio of OPA1 isoforms (e.g. a certain ratio of OPA1-S5 compared to all OPA1 isoforms (e.g. see example 7 and corresponding table)). The Person skilled in the art is, based on the teaching of the present invention, readily in the position to figure out (further) particular patterns or corresponding ratios of large and/or small isoforms of the optic atrophy 1 protein (OPA1) being specific for certain types of mitochondrial dysfunction(s)/disease(s).

In context of the present invention, it is intended that the ratio of large and/or small isoforms of OPA1 is determined by optical, spectrophotometric and/or densitometric measurements or analysis. Such determination methods are well known in the art. A particular choice of such methods is described in the appended examples. For instance, such methods comprise the SDS-PAGE analysis, Western blots, ELISA, RIA, CLIA, IRMA and/or EIA. These and further methods are known in the art and are, e. g., described in "Cell Biology: Laboratory manual 3rd edition" (2005, J. Celis, editor. Academic Press, New York).

It is also intended that the identity, amount or ratio of large and/or small isoforms of OPA1 is determined by peptide analysis. Again, such peptide analysis methods are well known in the art. For example such peptide analysis methods comprise mass spectrometry methods, like MALDI-MS or LC-MS/MS. The use of these particular mass spectrometry methods are described in the appended examples.

A person skilled in the art is able to figure out further methods for the determination of the identity, amount or ratio of large and/or small isoforms of OPA1. For examples such methods are array-based protein detection and quantification (e. g. as described in Nettikadan, 2006, Mol Cell Proteomics. 5:895-901), immuno-PCR (IPCR; e. g. as described in Adler, 2005, Adv Clin Chem. 39:239-92; Case, 1997, Biochem Soc Trans. 25:374S; Guo, 2006, Nucleic Acids Res. 34:e62; Imai, 1996, Tanpakushitsu Kakusan Koso. 41:614-7; Ruzicka, 1993, Science. 260:698-9; Sano, 1992, Science. 258:120-2; Zhou, 1993, Nucleic Acids Res. 21:6038-9), fluorescent immuno-PCR (e. g. as described in Niemeyer, 1997, Anal Biochem. 246:140-5), Immuno-detection amplified by T7 RNA polymerase (IDAT; e. g. as described in Zhang, 2001, Proc Natl Acad Sci U S A. 98:5497-502), and quantitative mass spectrometry (e.g. as described in Fomer, 2006, Mol Cell Proteomics, 5, 608-19; Zhou, 2004, Methods Mol Biol, 261,511-8).

It is envisaged in context of the present invention that the methods provided are carried out on (a) biological or medical sample(s). Said biological or medical sample(s) may comprise cell(s) or tissue(s). For example, said biological or medical sample(s) may comprise biological material of biopsies. The meaning of "biopsies" is known in the art. For instance, biopsies comprise cell(s) or tissue(s) taken, e. g. by the attending physician, from a patient, suffering from the herein defined mitochondrial disease(s), dysfunction(s) or disorder(s) or showing the herein defined clinical and/or pathological symptoms.

The method as described herein is to be carried out on biological/medical samples which comprise mitochondria, i.e. on tissues or cells or on biological fluids which comprise some cells comprising mitochondria. Yet, it is also envisaged that the method of the present invention is carried out on biological samples which do not comprise complete cells but cellular fragments or mitochondrial fragments. Accordingly, the method of the present invention is not limited to biological/medical samples which comprise or are cells or tissues.

As documented in the appended examples, in one embodiment, muscles cells are to be investigated in context of this invention, i.e. the non-limiting results provided herein document the usefulness of the present invention in particular in context of muscular disorders, like MERRF. As mentioned above, MERRF is myoclonic epilepsy with ragged-red fiber syndrome and MERRF patients suffering from severe myopathy. It was demonstrated herein (e. g. example 5), that in a cybrid cell line derived from a MERRF patient, cells showed highly fragmented mitochondria compared to control cells (Fig.6ab). The fragmentation staus of the mitochondria correlates with the pattern of the five detected OPA1 isoforms in a manner that in the MERRF cells large OPA1 isoforms (particularly OPA1-L1 and OPA1-L2) are reduced and the small OPA1 isoforms (particularly the smallest isoform of OPA1, OPA1-S5) are enhanced.

Examplarily, but not limiting, the biological or medical sample to be analysed in context of the present invention is or is derived from blood, plasma, white blood cells, urine, semen, sputum, cerebrospinal fluid, lymph or lymphatic tissues or cells, muscle cells, heart cells, nerve cells, cells from spinal cord, brain cells, liver cells, kidney cells, cells from the intestinal tract, colon cells skin, bone, bone marrow, placenta, amniotic fluid, hair, hair and/or follicles, stem cells (embryonal, neuronal, and/or others), primary or immortalized cell lines (lymphocytes, macrophages, or any cell lines derived from any of the aforementioned tissues), tumor-derived cell lines such as HeLa cell lines, neuroblastoma cell lines or insulinomo cell lines. The the biological or medical sample as defined herein may also be or be derived from biopsies (see also herein below).

It is envisaged that the determination of the identity of large and/or small OPA1 isoforms comprises the step of determining the identity of at least one OPA1 isoform as defined herein.

The determination of the "identity" of at least one OPA1 isoform relates to the determination of the presence of at least one of the herein defined OPA1 isoforms (OPA1-L1, -L2, -S3, -S4 and/or -S5). At least the presence of one, of two, of three, of four or of all five isoforms as defined herein is determined. The presence of the corresponding isoform may be determined by methods known in the art and as described herein, e.g. by determining the corresponding band(s) on SDS-PAGE gels, by Westem-Blot analysis, by other immunological / immunobiochemical / immunochemical tests (like ELISA-tests, RIA-tests, etc), by peptide analysis (for example by mass spectrometry, etc) and the like.

It may, inter alia, be deduced whether in a given sample, the OPA1 large isoforms are present and/or in what amount they are present. Similarly, the determination of the presence of a given (or all) small OPA1 isoforms is useful. Again, also the quantitative (as well as the qualitative) identity of the individual OPA1 isoforms as described herein may be deduced and measured. As detailed herein, the gist of the present invention is based on the surprising finding that a determined reduction of large OPA1 isoforms as described herein (OPA1-L1 and/or -L2) and/or a determined increase of small OPA1 isoforms as described herein (OPA1-S3, -S4 and/or -S5, in particular OPA1-S5) is indicative for the presence of or the susceptibility to a mitochondrial disease/disorder/dysfunction.

It is further envisaged that the determination of the amount of large and/or small OPA1 isoforms comprises the step of determining the amount of at least one OPA1 isoform as defined herein. As mentioned above, also comprised in the methods provided herein are tests for the presence or the absence of individual OPA1 isoforms as defined above. The amount of each individual OPA1 isoform as defined herein (OPA1-L1, -L2, -S3, - S4, -S5) may be deduced by methods known in the art, like quantitative measurements on Westem-blots or the quantification of the amount by known techniques directly form SDS-PAGEs, like the determination of quantities in stained gels (like silver-stained, Coommassie-stained gels, and the like). Further quantitative methods comprise but are not limited to methods like ELISA-tests and the like. The methods for quantification are well known on the art, see e.g. Cell Biology: Laboratory manual 3rd edition. J. Celis, editor. Academic Press, New York.

In addition, the quantitative amount of at least one isoform as defined herein is determined. It is also envisaged that at least two isoforms, at least three, at least four or all five isoforms of OPA1 as defined herein are determined. As documented in the appended examples, it is preferred that at least one isoform of the two large isoforms and/or at least one isoform of the small isoforms is to be determined. Most preferably, if the small isoform is to be determined, at least the OPA1-S5 isoform is to be deduced.

As mentioned above, the gist of the present invention lies in the surprising finding that in pathological samples (or samples which tend to become pathological), the amount of large isoforms of OPA1 is reduced and the amount of small isoforms is enhanced in said samples. The corresponding determination of the individual amount of each individual isoform may comprise a comparison to the corresponding standard amount (for example obtained from samples form healthy volunteers) or in direct comparison to samples obtained from healthy individuals.

A mitochondrial disorder (as well as a susceptibility to the same) may be diagnosed by determining the amount of the individual OPA1 isoforms described herein. The presence or the susceptibility to a mitochondrial dysfunction can be determined by evaluating the amount of "large" versus "small" OPA1 isoforms as described herein, wherein a higher amount of small isoforms, in particular isoform S5 is indicative for a mitochondrial dysfunction/disorder or a susceptibility thereto. However, it is of note that not only the amount of "small" versus "large" (and accordingly the ratio between these OPA1 isoforms) may be measured in a given sample in context of this invention, but it is also envisaged that the amount of an individual isoform is measured and compared to a corresponding standard, for example a standard derived from or obtained from samples form healthy individuals, in particular healthy humans.

In accordance with the present invention, it is envisaged that also the ratio of individual OPA1 isoforms in comparison to the other OPA1 isoforms as described herein are determined. As detailed below and as exemplified in the appended examples, also said ratio of individual OPA1 isoforms to the other four, three, two or single OPA1 isoforms, or to all five OPA1 isoforms, as described herein are of diagnostic relevance. Again, this diagnostic relevance for the elucidation whether a mitochondrial disorder is present or whether an individual has a prevalence for a mitochondrial dysfunction/disesase is based on the surprising finding that such affected individuals or individuals susceptible to such an disorder/dysfunction/disease are characterized by displaying in his/her biological samples a comparatively higher amount of smaller OPA1 isoforms than large OPA1 isoforms as described herein. Accordingly, also the ratio between small to large (or also large to small) OPA1 isoforms is of diagnostic relevance. In particular, a higher amount of OPA1 isoform S5 in comparison to the other isoforms, in particular the large isoform(s) L1 and/or L2 is diagnostic for the presence or the prevalence of a mitochondrial disorder/dysfunction/disease. Yet, as documented herein, also a higher amount of the OPA1 isoform S3 is of high diagnostic value for the determination of such a disorder.

Exemplarily, but not limiting, ratios between individual or combined OPA1 isoforms being of diagnostic relevance are given in the following. Such ratios can also be deduced from the appended examples (e.g. example 7 and 8).

The ratio between OPA1-S5 and all OPA1 isoforms may vary between 1% and 25%, preferably between 5% and 15%, for healthy patients and between 10% and 75%, preferably between 15% and 60%, for affected patients. For example, said ratio may be 10% +/- 1, 2, 3, 4, or 5% (particularly 9.9%) for healthy patients and 25% +/- 1, 2, 3, 4 or 5% (particularly 24.9%) for affected patients. Accordingly, the increase of said ratio of affected patients compared to healthy ones may be in the range of 1 to 12 fold, more preferably in the range of 2 to 8 fold, more preferably in the range of 2 to 6 fold and more preferably in the range of 2 to 3 fold or 4 to 5 fold. For example, said increase may be 2.5 fold or 4.4 fold.

In view of the above, it is envisaged in context of the present invention that, e. g. a ratio between OPA1-S5 and all OPA1 isoforms of more than 10 %, preferably of more than 15%, more preferably of more than 20 % and most preferably of more than 25% is diagnostic for the "affected patients". It is to be understood that, in context of the present invention, "affected patients" are patients suffering from a susceptibility for, a predisposition for or the presence of a disorder correlated with mitochondrial dysfunction or mitochondrial disease.

The ratio between OPA1-S4 and all OPA1 isoforms may be 27% +/- 1, 2, 3, 4, or 5% (e. g. 27.2%) for healthy patients and 28% +/- 1, 2, 3, 4, or 5% (e. g. 28.2%) for affected patients. Accordingly, the increase of said ratio of affected patients compared to healthy ones may be in the range of 1 to 1.5 fold. As mentioned herein above, a decrease of said ratio of affected patients compared to healthy ones, particularly in context of OPA1-S4, is also possible. For example, it is also possible that said ratio does not change between affected patients and healthy ones. The ratio between OPA1-S3 and all OPA1 isoforms may be 10% +/- 1, 2, 3, 4, or 5% (e. g. 9.9%) for healthy patients and 19% +/- 1, 2, 3, 4, or 5% (e. g. 19.2%) for affected patients. Accordingly, the increase of said ratio of affected patients compared to healthy ones may be in the range of 1 to 5 fold, more preferably in the range of 1.5 to 3 fold and more preferably in the range of 1.8 to 2.3 fold. For example, said increase may be particularly 1.9 fold.

The ratio between OPA1-S3, -S4 and -S5 and all OPA1 isoforms may be 47% +/- 1, 2, 3, 4, 5 or 10% (e. g. 47.4%) for healthy patients and be 93% +/- 1, 2, 3, 4, 5 or 10% (e. g. 93.0%) for affected patients. Accordingly, the increase of said ratio of affected patients compared to healthy ones may be in the range of 1.5 to 3 fold, more preferably in the range of 1.8 to 2.5 fold and more preferably in the range of 1.9 to 2.2 fold. For example, said increase may be particularly 2.0 fold.

The ratio between OPA1-S3 and -S5 and all OPA1 isoforms may be 20% +/- 1, 2, 3, 4, or 5% (e. g. 20.2%) for healthy patients and between be 65% +/- 1, 2, 3, 4, 5 or 10% (e. g. 64.8%) for affected patients. Accordingly, the increase of said ratio of affected patients compared to healthy ones may be in the range of 2 to 5 fold, more preferably in the range of 2.5 to 4 fold and more preferably in the range of 3 to 3.6 fold. For example, said increase may be particularly 3.2 fold.

In context of values of the ratio of OPA1 isoforms, e.g. the values given above, it is to be understood that within the ranges given for the values, the smaller variations are preferred.

It is intended that the ratios to be determined in context of the present invention may also differ from the ones exemplified above. As already mentioned above, examples that, in a non limiting manner, describe the evaluation of such ratios are given herein below (e. g. Example 7 and 8).

Inter alia, in context of the present invention, the term "ratio" or "density ratio", inter alia, refers to a comparison of density values of bands corresponding to OPA1 isoforms, as, e. g., derived from an SDS-PAGE/Western-blot. Methods how such density values can be obtained are known in the art and exemplified in the appended non limiting examples.

It is to be understood that not only the comparison of small (OPA1-S3, -S4 and/or - S5) versus large isoforms of OPA1 (OPA1-L1 and/or -L2) or small or large versus other or all OPA1 isoforms derived from an individual patient sample or sample to be tested is of diagnostic relevance, but that also a comparison to an healthy control or a corresponding standard is of relevance and can, in accordance with the teachings provided herein, be obtained. This applies, mutatis mutantis, for all diagnostic methods provided herein.

As mentioned above, the term "determination of the ratio of OPA1 isoforms" is not limited to the determination of the ratio between one individual OPA1 isoform and one other individual OPA1 isoform as defined herein, but comprises also the determination of (a) ratio(s) between one individual isoform and at least two other OPA1 isoforms or even all other four OPA1 isoforms or even all five OPA1 isoforms as described herein. Accordingly, the determining the susceptibility for, predisposition for or the presence of a disorder correlated with mitochondrial dysfunction, also comprises a method wherein said determation of the ratio comprises
(a) the determination of the ratio of at least one large OPA1 isoform to at least one small OPA1 isoform;
(b) the determination of the ratio of at least one small OPA1 isoform to at least one large OPA1 isoform;
(c) the determination of the ratio of one OPA1 isoform to all five OPA1 isoforms or to all five OPA1 isoforms plus further OPA1 isoforms;
(d) the determination of the ratio of one OPA1 isoform to four OPA1 isoforms;
(e) the determination of the ratio of two OPA1 isoforms to three OPA1 isoforms,;
(f) the determination of the ratio of three OPA1 isoforms to two OPA1 isoforms; or
(g) the determination of the ratio of four OPA1 isoforms to one OPA1 isoforms;
(h) the determination of the ratio of all five OPA1 isoforms or of all five OPA1 isoforms plus further OPA1 isoforms to one OPA1 isoforms.

Moreover, all other possible combinations are envisaged in context of the present invention.

In an particular embodiment of the present invention, the ratio between at least one large OPA1 isoform and at least one small isoform is determined.

It is preferred in context of the present invention that the ratio between at least one small OPA1 isoform (e. g. OPA1-S5) and (all) five or more OPA1 isoforms, at least four OPA1 isoforms, at least three OPA1 isoforms, at least two OPA1 isoforms or at least one OPA1 isoforms is determined. It is more preferred in context of the present invention that the ratio between the OPA1-S5 as defined herein and at least one other OPA1 isoform as defined herein is determined.

It is most preferred in context of the present invention that the ratio between the OPA1-S5 as defined herein and all five OPA1 isoforms as defined herein is determined.

The person skilled in the art is readily in a position to determine the ratio of individual (or more) OPA1 isoforms as described herein by methods known in the art, like for example densitometric, spectrophotometric, luminescent, autoradiographic or fluorescent quantification methods. Also in this context, methods comprising tests with specific anti-OPA1 isoform antibodies (also specific antibodies against individual OPA1-isoforrns as provided herein) are useful. Accordingly, methods, like Westem-blot analysis or ELISA/RIA-tests may be employed to determine the OPA1 isoform ratio(s). Corresponding non-limiting examples are illustrated in the appended experimental part.

As discussed herein above, the methods as provided herein are based on the determination of small OPA1 isoforms (S3, S4 and/or S5) as provided herein versus large OPA1 isoforms (L1 and/or L2). However, it is to be understood that the methods provided herein also comprise an evaluation whether in a given sample the amount of large OPA1 isoforms (L1 and/or L2) is lower, and/or the amount of small OPA1 isoforms (S3, S4 and/or S5; preferably S3 and/or S5; more preferably S5) is higher in comparison to an individual healthy control sample, said sample being derived from either another (non-affected) tissue or cell of the individual to be diagnosed and/or assessed or said sample being a control sample from another healthy individual or a pool of samples from healthy individuals. It is also envisaged that the samples to be assessed are compared to (a) corresponding standard, either a standard which is obtained from affected individuals and/or healthy individuals. It is within the routine skills of the person skilled in the art to obtain corresponding standards.

In context of the present invention it is also envisaged that the determination of the ratio of large and/or small OPA1 isoforms comprises the step of determining the ratio between at least one OPA1 isoform as defined herein and an external standard. However, it is also envisaged that for all other methods provided herein, internal standards may be employed. Internal standard are known in the art. As a non limiting example, β-actinmay be employed as an internal standard in context of the present invention.

In further embodiments of the present invention, the herein described method for determining the susceptibility, predisposition or the presence of a disorder correlated with mitochondrial dysfunction or disease, comprising the in vitro determination of the ratio of large and small isoforms of OPA1 in a given sample comprises a comparison of the obtained data/values to data/values obtained from a healthy control/standard and/or a comparison of the obtained data/values to data/values obtained from a known diseased control/standard and/or a comparison of the obtained data/values to data/values obtained for an internal standard, e. g. for β-actin.

It is further envisaged that the methods provided herein comprise the steps of determing in a sample the ratio of at least two OPA1 isoforms selected from the group consisting of OPA1-L1, -L2, -S3, -

S4 and -S5, wherein said ratio of said isoforms is compared to the corresponding isoforms of a healthy control or normal standard.

Herein disclosed is a kit useful for the detection and/or diagnosis of a mitochondrial dysfunction, of a disorder correlated with mitochondrial dysfunction or of a mitochondrial disease, comprising an assay for determining the presence, the absence, the identity, the amount or ratio of at least one OPA1 isoform selected from the group consisting of OPA1-L1, -L2,- S3, -S4 and -S5, as defined herein .

Herein disclosed is a kit for the detection and/or diagnosis of a mitochondrial dysfunction, of a disorder correlated with mitochondrial dysfunction or of a mitochondrial disease, comprising a binding molecule which specifically binds to/interacts with optic atrophy 1 protein (OPA1) or a a binding molecule which specifically binds to/interacts with an isoform of optic atrophy 1 protein (OPA1) as defined herein

Additionally, uses of a binding molecule which specifically binds to/interacts with optic atrophy 1 protein (OPA1) or a binding molecule which specifically binds to/interacts with an isoform of optic atrophy 1 protein (OPA1) as defined herein for the preparation of a diagnostic composition for the detection of a disorder correlated with mitochondrial dysfunction or mitochondrial disease are described herein.

The binding molecule may be selected form the group consisting of antibodies, affybodies, trinectins, anticalins, aptamers, RNAs, PNAs and the like.

The person skilled in the art is readily the position to use and to obtain specific binding molecules, useful in the methods, kits, assays provided herein. These molecules are directed and bind specifically to or specifically label OPA1 and/or the isoforms of OPA1 provided and described herein. Non-limiting examples of these binding molecules may be selected from aptamers (Gold, Ann. Rev. Biochem. 64 (1995), 763-797)), aptazymes, RNAi, shRNA, RNAzymes, ribozymes (see e.g., EP-B1 0 291 533, EP-A1 0 321 201, EP-B1 0 360 257), antisense DNA, antisense oligonucleotides, antisense RNA, si RNA, antibodies (Harlow and Lane "Antibodies, A Laboratory Manual", CSH Press, Cold Spring Harbor, 1988), affibodies (Hansson, Immunotechnology 4 (1999), 237-252; Henning, Hum Gene Ther. 13 (2000), 1427-1439), trinectins (Phylos Inc., Lexington, Massachusetts, USA; Xu, Chem. Biol. 9 (2002), 933), anticalins, or the like. These compounds are, for example, described in EP 1 017 814. Said European patent also describes the process of preparing such anticalins with the ability to bind a specific target.

The term "aptamer" means nucleic acid molecules that can bind to target molecules. Aptamers commonly comprise RNA, single stranded DNA, modified RNA or modified DNA molecules. The preparation of aptamers is well known in the art and may involve, inter alia, the use of combinatorial RNA libraries to identify binding sites (Gold (1995), Ann. Rev. Biochem 64 , 763-797).

A preferred binding molecule is an antibody specific for OPA1 or a particular isoform of OPA1 as defined herein or a group of isoforms of OPA1 as defined herein. Such an antibody, e. g., may bind to the amino acid streches or amino acid peptides defined herein of OPA1 or of a particular isoform of OPA1 as defined herein or of a group of isoforms of OPA1 as defined herein. However, it is to be understood that such an antibody may also bind to other amino acid streches or amino acid peptides of OPA1 or of a particular isoform of OPA1 as defined herein or of a group of isoforms of OPA1 as defined herein also.
It is envisaged that such antibody (but also the other binding molecules ) is specific for one single isoform of OPA1, preferably for OPA-L1, -L2, -S3, -S4 or -S5, more preferably for OPA1-S5. For example, such antibody (or other binding molecules) specific for one single isoform of OPA1 are envisaged to be particularly employed to determine in a sample the presence, the absence, the identity or the amount of at one single OPA1 isoform selected from the group consisting of OPA1-L1, -L2,- S3, -S4 and -S5. Said presence, absence, identity or amount of said isoform may then be compared to the corresponding isoform of a healthy control or an internal or normal standard.
In this context, it is to be understood that the person skilled in the art is, based on the teaching provided herein, readily in a position to deduce (further) amino acid streches/peptides being specific for a particular isoform of OPA1 as defined herein or of a group of isoforms of OPA1 as defined herein. Moreover, the teaching provided herein enables the person skilled in the art to figure out the entire amino acid sequences of every single OPA1 isoform as defined herein. For example, this can be done by starting from the disclosure content of this invention and the corresponding appended examples and applying further peptide analysis methods, like mass spectrometry (e. g. MALDI-MS and/or LC-MS/MS).
As evident form the disclosure provided herein and in particular from the appended examples, the methods, kits and uses provided herein are particularly useful in the in vitro diagnoses of mitochondrial diseases/dysfunctions/disorders. Particularly useful are in this context antibodies, antibody molecules, antibody derivatives, which specifically interact with OPA1 and/or the isoforms of OPA1 as described herein. The term "antibody/antibodies" as employed herein also comprise antibody derivatives, antibody fragments and the like.

The antibody described herein can be, for example, polyclonal or monoclonal. The term "antibody" also comprises derivatives or fragments thereof which still retain the binding specificity. Techniques for the production of antibodies are well known in the art and described, e.g. in Harlow and Lane "Antibodies, A Laboratory Manual", CSH Press, Cold Spring Harbor, 1988. These antibodies can be used for the monitoring of the presence, absence, amount, identiy and/or ratio of OPA1 and/or the herein defined OPA1 isoforms, in particular in diagnosis. Surface plasmon resonance as employed in the BIAcore system can be used to increase the efficiency of phage antibodies which bind to an epitope of the polypeptide of the invention (Schier, Human Antibodies Hybridomas 7 (1996), 97-105; Malmborg, J. Immunol. Methods 183 (1995), 7-13). Accordingly, also phage antibodies can be used.

Chimeric, single chain and humanized antibodies, as well as antibody fragments, like, inter alia, Fab fragments can also be used. Antibody fragments or derivatives further comprise F(ab')2, Fv or scFv fragments; see, for example, Harlow and Lane, loc. cit. Various procedures are known in the art and may be used for the production of such antibodies and/or fragments. Thus, the (antibody) derivatives can be produced by peptidomimetics. Further, techniques described for the production of single chain antibodies (see, inter alia, US Patent 4,946,778) can be adapted to produce single chain antibodies to polypeptide(s) as defined herein. Also, transgenic animals may be used to express humanized antibodies to OPA1 or OPA1 isoforms as described herein. Most preferably, the antibody to be employed is a monoclonal antibody. For the preparation of monoclonal antibodies, any technique which provides antibodies produced by continuous cell line cultures can be used. Examples for such techniques include the hybridoma technique (Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole, Monoclonal Antibodies and Cancer Therapy, Alan R. Liss, Inc. (1985), 77-96). Techniques describing the production of single chain antibodies (e.g., US Patent 4,946,778) can be adapted to produce single chain antibodies to OPA1 or OPA1 isoforms. Accordingly,

the term "antibody molecule" relates to full immunoglobulin molecules as well as to parts of such immunoglobulin molecules. Furthermore, the term relates, as discussed above, to modified and/or altered antibody molecules, like chimeric and humanized antibodies. The term also relates to monoclonal or polyclonal antibodies as well as to recombinantly or synthetically generated/synthesized antibodies. The term also relates to intact antibodies as well as to antibody fragments thereof, like, separated light and heavy chains, Fab, Fab/c, Fv, Fab', F(ab')2. The term "antibody molecule" also comprises bifunctional antibodies, trifunctional antibodies and antibody constructs, like single chain Fvs (scFv) or antibody-fusion proteins.

The binding molecules provided herein are to be used in the methods, and kits as well as assays provided in the context of the evaluation of OPA1 and OPA1 isoforms, in particular in the assay for OPA1 proteolytic isoforms (like OPA1-S3, -S4, -S5, in particular OPA1-S3 and OPA1-S5 and most preferably OPA1-S5) in a given sample to be tested or analyzed.

In accordance with the present invention by the term "sample" is intended any biological sample obtained from an individual, cell line, tissue culture, or other source containing polynucleotides or polypeptides or portions thereof. As indicated, biological samples include body fluids (such as blood, sera, plasma, urine, synovial fluid and spinal fluid) and tissue sources found to express the polynucleotides of the present invention. Methods for obtaining tissue biopsies and body fluids from mammals are well known in the art. A biological sample which includes mitochondria, mitochondrial DNA, mitochondrial proteins, mitochondrial fragments (e. g. fragments of the mitochondrial outer and/or, more preferred, inner membranes) is preferred as a source.

The diagnostic composition optionally comprises suitable means for detection. Binding molecule or detection means described above are, for example, suitable for use in immunoassays in which they can be utilized in liquid phase or bound to a solid phase carrier. Examples of well-known carriers include glass, polystyrene, polyvinyl ion, polypropylene, polyethylene, polycarbonate, dextran, nylon, amyloses, natural and modified celluloses, polyacrylamides, agaroses, and magnetite. The nature of the carrier can be either soluble or insoluble . As documented in the examples, a partiuclar preferred solid phase may also be the membranes used in Western blots. It is evident that not only the "binding molecule" but also the Target molecule (OPA1, OPA1 fragments or OPA1 isoforms as described herein) may be bound to the "solid phase" and may be tested with the "binding molecules" provided in "liquid phase".

Solid phase carriers are known to those in the art and may comprise polystyrene beads, latex beads, magnetic beads, colloid metal particles, glass and/or silicon chips and surfaces, nitrocellulose strips, membranes, sheets, duracytes and the walls of wells of a reaction tray, plastic tubes or other test tubes. Suitable methods of immobilizing of binding molecules like, anticalins, antibody(ies), aptamer(s), polypeptide(s), etc. on solid phases include but are not limited to ionic, hydrophobic, covalent interactions or (chemical) crosslinking and the like. Examples of immunoassays are competitive and non-competitive immunoassays in either a direct or indirect format. Commonly used detection assays can comprise radioisotopic or non-radioisotopic methods. Examples of such immunoassays are the radioimmunoassay (RIA), the sandwich (immunometric assay) and the Western blot assay. Furthermore, these detection methods comprise, inter alia, IRMA (Immune Radioimmunometric Assay), EIA (Enzyme Immuno Assay), ELISA (Enzyme Linked Immuno Assay), FIA (Fluorescent Immuno Assay), and CLIA (Chemioluminescent Immune Assay). Furthermore, the diagnostic compounds may be are employed in techniques like FRET (Fluorescence Resonance Energy Transfer) assays.

Appropriate labels and methods for labeling are known to those of ordinary skill in the art. Examples of the types of labels which can be used, include inter alia, fluorochromes (like fluorescein, rhodamine, Texas Red, etc.), enzymes (like horse radish peroxidase, β-galactosidase, alkaline phosphatase), radioactive isotopes (like 32P, 33P, 35S or 125I), biotin, digoxygenin, colloidal metals, chemi- or bioluminescent compounds (like dioxetanes, luminol or acridiniums). A variety of techniques are available for labeling biomolecules, are well known to the person skilled in the art
and comprise, inter alia, covalent coupling of enzymes or biotinyl groups, phosphorylations, biotinylations, random priming, nick-translations, tailing (using terminal transferases). Such techniques are, e.g., described in Tijssen, "Practice and theory of enzyme immunoassays", Burden and von Knippenburg (Eds), Volume 15 (1985); "Basic methods in molecular biology", Davis LG, Dibmer MD, Battey Elsevier (1990); Mayer, (Eds) "Immunochemical methods in cell and molecular biology" Academic Press, London (1987); or in the series "Methods in Enzymology", Academic Press, Inc. Detection methods comprise, but are not limited to, autoradiography, fluorescence microscopy, direct and indirect enzymatic reactions, etc.

The gist of this invention is based on the provision of a distinct composition of molecular markers derived from OPA1, i.e. OPA1 isoforms as defined herein, whereby these molecular markers (namely OPA1-L1, -L2, -S3, -S4, -S5) can be measured and analyzed by means and methods provided herein and wherein the composition of the totality of these molecular markers differ between samples obtained from healthy individuals and samples obtained from individuals suffering from or prone to be suffering from a mitochondrial dysfunction or a disorder correlated with mitochondrial dysfunction(s). The composition of molecular markers provided in context of this invention comprises, inter alia, two large isoforms of OPA1 (OPA1-L1, -L2) and three small isoforms of OPA1 (OPA1-S3, -S4, -S5), whereby in particular the ratio between large and small isoforms differs in samples derived from healthy individuals or controls in comparison to samples derived form patients suffering form the mitochondrial dysfunction/disease or patients susceptible to such a disease ("affected patients"). The corresponding novel and inventive finding relates to the fact that in samples derived from individuals who are susceptible for, have predisposition for or have a disorder correlated with mitochondrial dysfunction or mitochondrial disease, the amount of "large isoforms" (OPA1-L1/-L2) is reduced whereas the amount of "small isoforms" (OPA1- S3,-S4, -S5, in particular OPA1-S3 and -S5 and more particular OPA1-S5 as defined herein) is increased. As documented, herein one measure for the determination the susceptibility for, predisposition for or the presence of a disorder correlated with mitochondrial dysfunction or mitochondrial disease is the amount or ratio of the small isoforms versus the large isoforms. Accordingly, the composition of molecular weight markers as provided herein preferably relates to the OPA1 markers in form of OPA1 isoforms (OPA1-L1, -L2, -S3, -S4, -S5 as defined herein), whereby in particular a ratio of more than 10 %, preferably of more than 15%, more preferably of more than 20 % and most preferably of more than 25% of OPA1-S5 in the total amount of all isoforms (OPA1-L1, -L2, -S3, -S4 and -S5) (as evalutated in e.g. SDS-gels or Western-blots) is diagnostic for the susceptibility for, predisposition for or the presence of a disorder correlated with mitochondrial dysfunction or mitochondrial disease.

Accordingly, as a cut-off and-read-out for the evaluation of normal/healthy versus diseased status or susceptibility, the following values can be taken (as non-limiting examples) when for example the five molecular markers as provided herein are analysed in gels (SDS-gels or Western blots derived from said SDS-gels):

For the ratio between OPA1-S5 and all OPA1 isoforms: More than 10%, preferably more than 15%, more preferably more than 20% and more preferably more than 25%. For the ratio between OPA1-S4 and all OPA1 isoforms: More than 25%, preferably more than 27.5% and more preferably more than 30%. For the ratio between OPA1-S3 and all OPA1 isoforms: More than 10%, preferably more than 15% and more preferably more than 20%. For the ratio between OPA1-S3, -S4 and - S5 and all OPA1 isoforms: More than 50%, preferably more than 70% and more preferably more than 90%. For the ratio between OPA1-S3 and -S5 and all OPA1 isoforms: More than 20%, preferably more than 40% and more preferably more than 60%.

Therefore, the present invention also relates, in one embodiment, to a distinct composition of molecular markers, e.g. on a Western blot or in an SDS-PAGE, which comprise the following bands on said SDS-PAGE or Western blot or proteins corresponding to said bands:

An OPA1-L1 band that has an apparent molecular weight of about 97kD (96.8 kD), an OPA1-L2 band that has an apparent molecular weight of about 92 kD (92.3 kD), an OPA1-S3 band that has an apparent molecular weight of about 88 kD (88.1 kD), an OPA1-S4 band that has an apparent molecular weight of about 84 kD (84.4 kD), and/or an OPA1-S5 band that has an apparent molecular weight of about 81 kD (80.9 kD), and wherein the OPA1-S5 band comprises more than 15% +/- 3%, preferably +/- 2%, more preferably +/- 1 % of the density ratio/amount of the all herein defined bands (OPA1-L1, -L2, -S3, -S4 and -S5). In a sample derived from an healthy individual said density ratio/amount is lower than 15% +/- 3%, preferably +/-2%, more preferably +/- 1 %.

In context of this invention, in particular this embodiment, the term "density ratio" or "ratio" means the value of measured amount, e. g. of at least one OPA1 isoform compared to at least one other (or also the same, see above) OPA1 isoform, which can be deduced by standard methods known in the art and which are normally based on optical (also computer-assisted, like image analysis software) measurements. These measurements are also described in the appended examples and comprise scanning of westem-blots and densitometric analysis using standard image software, densitometry and spectrometry.

Therefore, it is within the routine skills of the artisan to detect and deduce the individual OPA1 isoforms protein levels (e.g. the optical density ratio), through densitometry, spectrophotometry and the like.

Exemplarily, the present invention is advantageous in context of high throughput approaches (HTS-methods), such as high throughput assays for determining the ratio of at least one OPA1 isoform as defined herein. Said methods are useful for the determiantion and /or evaluation of mitochondrial dysfunctions and may, therefore, be employed to detect and/or diagnose a mitochondrial dysfunction, a disorder correlated with mitochondrial dysfunction or a mitochondrial disease. This particular advantage is, inter alia, due to the fact that the methods provided herein are fast, robust and easy to perform. The skilled person is readily in a position to apply the experimental approaches performed and discosed herein in the appended examples to corresponding high throughput approaches. "High throughput approaches", which can be employed in context of the present invention are known in the art.

A further aspect disclosed herein relates to a method of screening for a compound capable of (specifically) modulating the activity of proteolytic processing of OPA1. Said method comprises the steps of
(a) contacting OPA1 or a biological system comprising OPA1 with said compound to be screened under conditions allowing proteolytic processing of OPA1 to occur, and
(b) evaluation whether proteolytic processing of OPA1 is altered compared to a control, wherein OPA1 is present and said compound to be screened is absent under conditions allowing proteolytic processing of OPA1 to occur.

In context of this method, the definitions given herein above with respect to terms like "OPA1 processing" or "processing of OPA1" apply here, mutatis mutandis, with respect to the term "proteolytic processing of OPA1". As mentioned above, the process of "OPA1 processing", "processing of OPA1" or "proteolytic processing of OPA1" is characterized by a decrease of the amount of large isoforms of OPA1, an increase of the amount of small isoforms of OPA1 and/or a decrease of the ratio of large versus small isoforms of OPA1 compared to the control. An "altered proteolytic processing of OPA1" as used in context of the above method of screening is, for example, characterized by an altered amount of large and/or small isoforms of OPA1 and/or an altered ratio of large versus small isoforms of OPA1 compared to the control. All corresponding definitions given herein above with respect to the diagnostic methods apply here, mutatis mutandis. Particularly, the large and small isoforms to be employed in context of the method for screening are defined as mentioned herein above. The biological system as employed in context of the herein disclosed screening method can be established by the skilled person. For example, such system may be a cellular system, like those disclosed herein (for example HeLa cells), where OPA1 processing occurs endogenously, or a cellular system, like those disclosed herein (for example yeast cells), where certain components to obtain a condition allowing proteolytic processing of OPA1 to occur are expressed; at least partially, heterologuously.

Based on the teaching provided herein, the skilled person is readily in the position to establish particular methods of screening. For this purpose, the use of a yeast expression system, for example a yeast expression system like the one exemplarily disclosed herein and described in the appended examples, can be of particular benefit. However, the method of screening, is not limited to the use of such an expression system. The skilled person is able to find further expression systems to be employed in context of the method of screening provided herein.

The present invention is further described by reference to the following non-limiting figures and examples.

The Figures show:

### Figure 1. Protein sequence alignment of OPA1 spliceforms.

Amino acids encoded by alternative exons 4 (bold), 4b (bold/cursive), and 5b (bold/underlined) are indicated. The amino acid position of each isoform is indicated on the right.

### Figure 2. Determination of the apparent molecular weight of OPA1 isoforms by 10% SDS-PAGE.

**A,** Lane 2, 4, 6: Protein extract from isolated HeLa mitochondria; lane 1: Biorad prestained molecular weight marker; lane 3 PeqLab LMW molecular weight marker; lane 5: Sigma HMW molecular weight marker. Molecular weight of marker proteins (lane 1, 3, 5) is given in kD. OPA1 isoforms with apparent molecular weight larger than 91 kD are defined as large OPA1 isoforms (I-OPA1#1 and I-OPA1#2). OPA1 isoforms with apparent molecular weight smaller or equal than 91 kD are defined as small OPA1 isoforms (s-OPA1#3, s-OPA1#4 and s-OPA1#5). The dotted white line indicates the 91.0 kD boundary.

**B,** Molecular weight of all bands representing OPA1 isoforms (lanes 2, 4 and 6 of A) were calculated from logarithmic regression analysis of migration length versus molecular weight for all shown marker bands (lanes 1, 3 and 5 of A).

### Figure 3. Identification of OPA1 isoforms by mass spectrometry.

**a,** Critical peptides for assigning the OPA1 spliceform 1 to 8 and the OPA1 isoforms (I-OPA1#1, I-OPA1#2, s-OPA1#3, s-OPA1#4-, s-OPA1#5) according to a first estimation are indicated. Predicted peptides for the eight OPA1 spliceforms shown, were generated by the program Peptidecutter (http://www.expasy.org/tools/peptidecutter; more sophisticated model; Trypsin, lowest cleavage probability 10%). Masses given are M, not M+1 or M-1. In 'times new roman': peptides important to determine the starting point of the mature OPA1 proteins; In 'arial': peptides important to determine the spliceform; in 'courier' : peptides common to all forms of OPA1; **In bold: Most N-terminal peptides found in I-OPA1 #1 and in I-OPA1 #2 but not in any s-OPA1 isoform;** Underlined: Most N-terminal peptide found in s-OPA1 #3; Bold framed: Most N-terminal peptide found in] s-OPA1 #4; *In italic: Most N-terminal peptide found in s-OPA1* #5. Position of cleavage site gives most C-terminal amino acid of respective peptide. Peptides comprising amino acids before position 102 were not detected in this experiment.
**b,** Immunoprecipitation of OPA1 from isolated HeLa mitochondria (15 mg) using anti-OPA1 antibodies. Equal fractions (0.25%) of total mitochondria (T), supernatant (S) and pellet (P) of solubilized mitochondria after clarifying spin, flow through (FT) and 1% of the elution fraction (E) were analyzed by SDS-PAGE and immunoblotting with anti-OPA1 antibodies.
**c,** Coomassie stained bands of immunoprecipitated OPA1 isoforms. Each band was separately cut out and used for ESI-LC-MS/MS.
**d,** Alignment of the N-termini of the eight OPA1 splice variants. The predicted MPP cleavage sites N-terminal to T95 or, alternatively, F88 are indicated. Vertical dotted lines indicate the exon boundaries. Grey highlighted areas represent hydrophobic stretches called TM1, TM2a and TM2b (Herlan 2004, J Cell Biol 165, 167-173). Boxes represent peptides found by ESI-LC-MS/MS analysis of any of the different immunoprecipitated OPA1 isoforms according to the herein disclosed refined MS analysis. The absence (-) or presence (+) of each peptide in different OPA1 isoforms (L1 to S5) is indicated below the alignment. The confidence of peptide identification is indicated by '+++' (excellent), '++' (very good), or '+' (good) based on the number of identifications in separate ESI-LC-MS/MS runs and on the ion score.
**e,** OPA1 splice variant 7 (Sp.7) was expressed in wild type yeast cells. Total yeast extracts and mitochondria isolated from HeLa cells (M) were analyzed by western blotting with anti-OPA1 antibodies. Bands are labelled according to apparent corresponding size of OPA1 isoforms in HeLa mitochondria (L1, L2, S3, S4, and S5). Precursor protein (P) and a degradation product (d) are indicated.

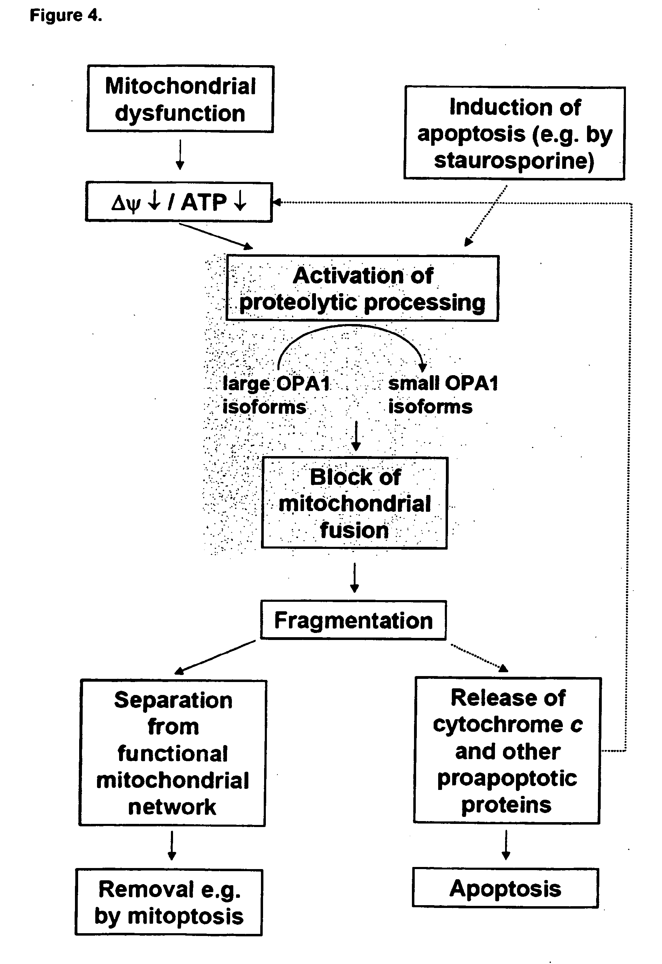

### Figure 4. Model of OPA1-mediated fragmentation of mitochondria in mitochondrial dysfunction and apoptosis.

Mitochondrial dysfunction as observed in a number of human diseases leads to impairment of bioenergetic competence of mitochondria. The reduction of the membrane potential and/or ATP level in mitochondria leads to a proteolytic breakdown of large isoforms of OPA1 to short isoforms. As a consequence, fusion of mitochondria is blocked, and in the presence of ongoing fission, such mitochondria become fragmented. In this way, dysfunctional mitochondria become segregated from the intact network. This may serve two purposes. First, such dysfunctional mitochondria may be removed from cells by autophagy, a process also termed mitoptosis (Skulachev, 2004, Mol Cell Biochem, 256-257, 341-358). Second, a spatial separation as well as a removal would reduce further damage to non-mutated mtDNA caused by ROS produced by a damaged respiratory chain. This mechanism proposes inactivation of OPA1 as a key regulatory step (grey) in counterselecting against damaged mitochondria within cells. The same regulatory step appears to have an early and essential role in apoptosis (dotted arrow lines). Induction of apoptosis, e.g. by staurosporine, activates the proteolytic cleavage of large isoforms of OPA1 to short isoforms. This leads to a block of mitochondrial fusion and fragmentation prior to cytochrome c release. Later in apoptosis cytochrome c and other proapoptotic factors are released from mitochondria and the membrane potential is reduced, leading to further increase of OPA1 processing and mitochondrial fragmentation before cells finally undergo apoptosis.

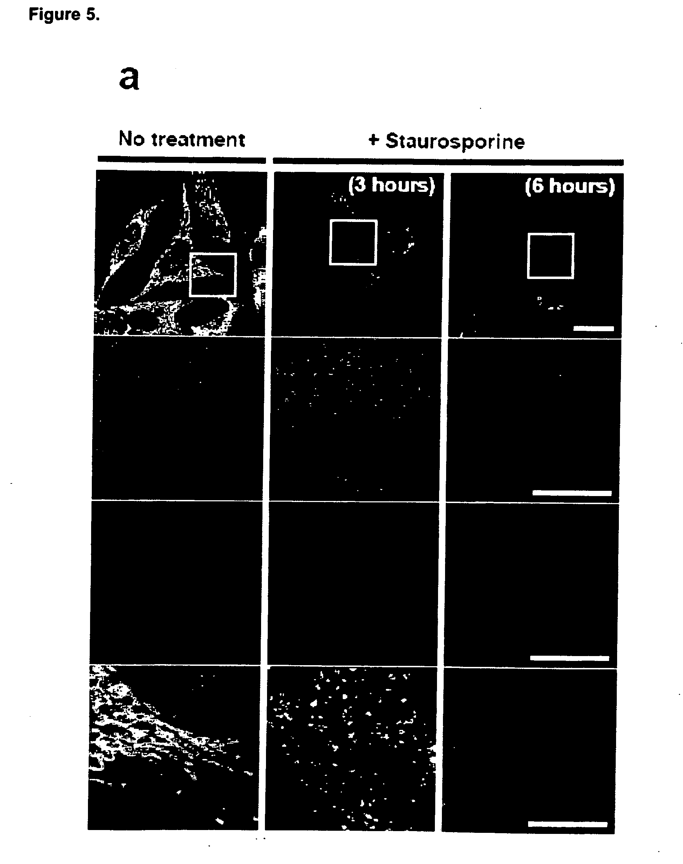

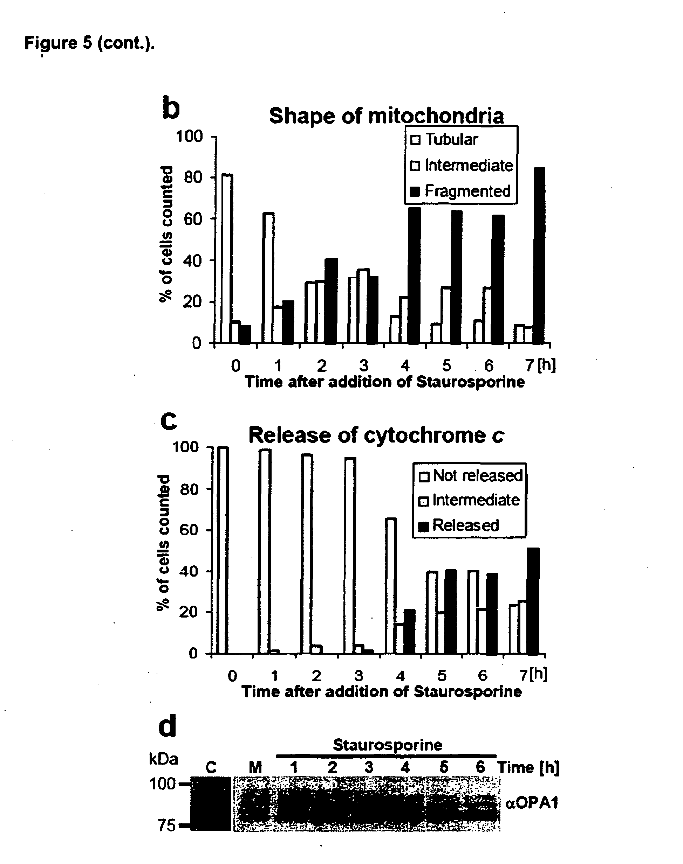

### Figure 5. Mitochondrial fragmentation and alteration of OPA1 protein isoforms precede cytochrome c release in apoptosis.

HeLa cells were treated with 1 µM staurosporine for the indicated time periods, stained with MitoTracker (red) and with cytochrome c antibodies (green).
a, Merged (top row; bottom row) or separately green (2^{nd} row) and red (3^{rd} row) confocal fluorescence images. Top, overview (scale bar 20 µm); bottom, indicated box, (scale bar 10 µm).
**b,** Cells were classified. Tubular, at least one mitochondrial tubule of 5 µm or more; intermediate, at least one between 0.5 and 5 µm but none more than 5 µm; fragmented, none of more than 0.5 µm in length. A representative experiment out of three is shown.
**c,** Cells were classified for cytochrome c release by immunostaining. Not released, completely colocalised with MitoTracker; intermediate, partly co-localised with MitoTracker; and released, not co-localised with MitoTracker.
**d,** OPA1 protein isoforms were determined by western blot analysis of total cell extracts. Total cell extracts (C) and isolated mitochondria (M) from untreated HeLa cells were used as controls.

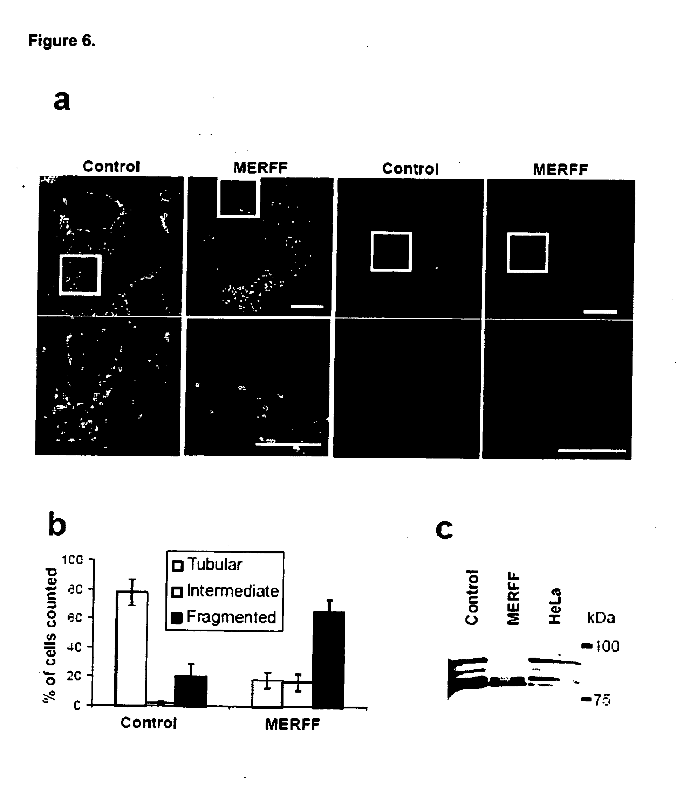

### Figure 6. MERRF cybrid cells show fragmentation of mitochondria and alterations in OPA1 isoforms.

MERRF and control cybrid cell lines were cultured, stained either with MitoTracker (red) and immunostained against cytochrome *c* (green) or with MitoTracker (red) and DAPI (blue).
**a,** Merged confocal fluorescence images are shown (1^{st} column: red and green; 2^{nd} column: red and green; 3^{rd} column: red and blue; 4^{th} column: red and blue. Top, overview (scale bar 20 µm); bottom, indicated box, (scale bar 10 µm).
**b,** Quantification of mitochondrial morphology in cells: Tubular, at least one mitochondrial tubule of 5 µm or more; intermediate, at least one between 0.5 and 5 µm but none more than 5 µm; fragmented, none of more than 0.5 µm in length. Error bars represent standard deviation of five slides evaluated.
**c,** OPA1 isoforms by western blot analysis of total cell extracts. In c, HeLa cell extracts are shown for comparison.

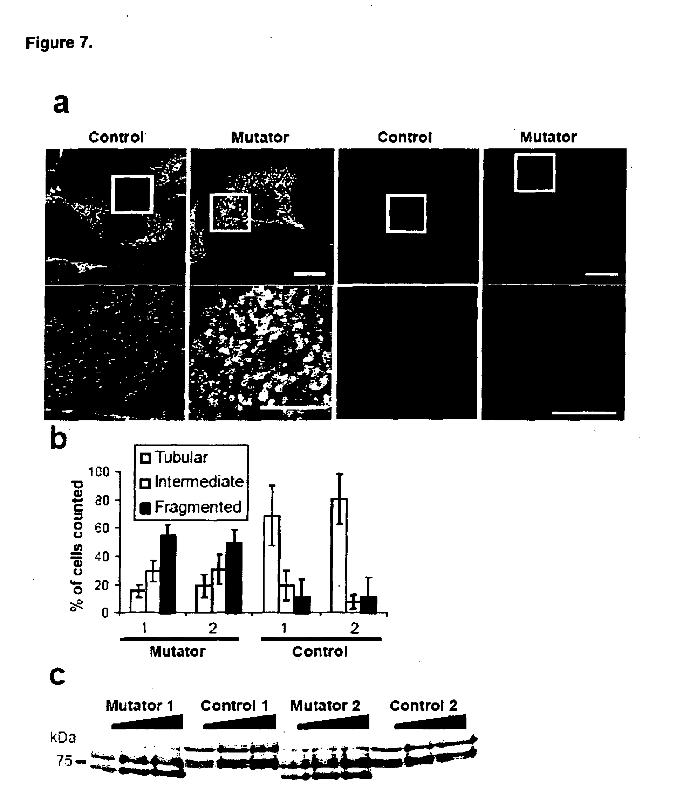

### Figure 7. Mutator mouse embryonic fibroblasts show fragmentation of mitochondria and alterations in OPA1 isoforms.

Immortalized mouse embryonic fibroblasts from two control mice and two mutator mice were cultured, stained either with MitoTracker (red) and immunostained against cytochrome c (green) or with MitoTracker (red) and DAPI (blue).
**a,** Merged confocal fluorescence images are shown (1^{st} column: red and green; 2^{nd} column: red and green; 3^{rd} column: red and blue; 4^{th} column: red and blue. Top, overview (scale bar 20 µm); bottom, indicated box, (scale bar 10 µm).
**b,** Quantification of mitochondrial morphology as in Fig.6b. Error bars represent standard deviation of five slides evaluated.
**c,** OPA1 isoforms by western blot analysis of total cell extracts.

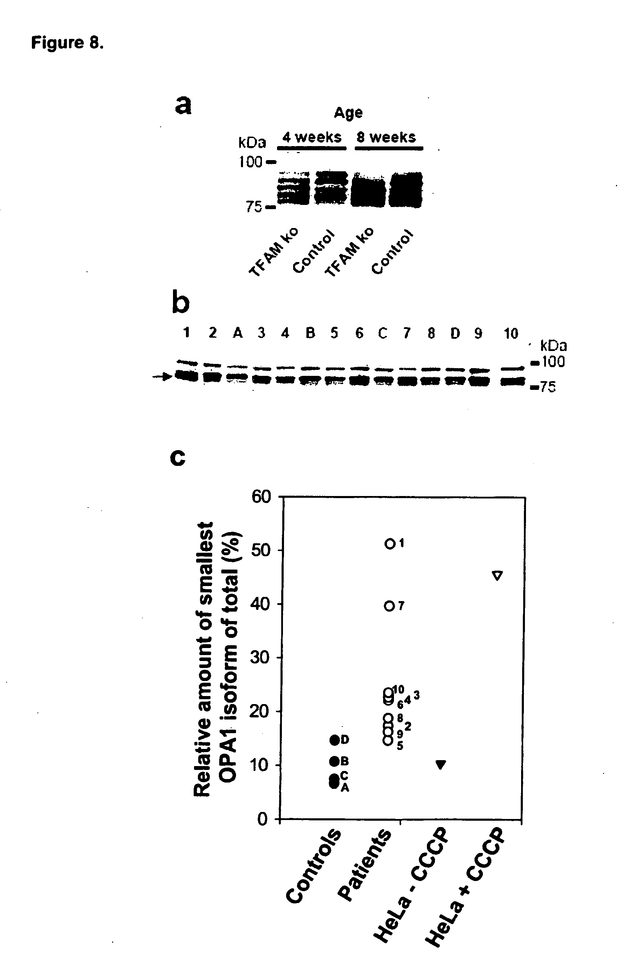

### Figure 8. Patterns of OPA1 isoforms are altered in tissue samples exemplary of mitochondrial dysfunction.

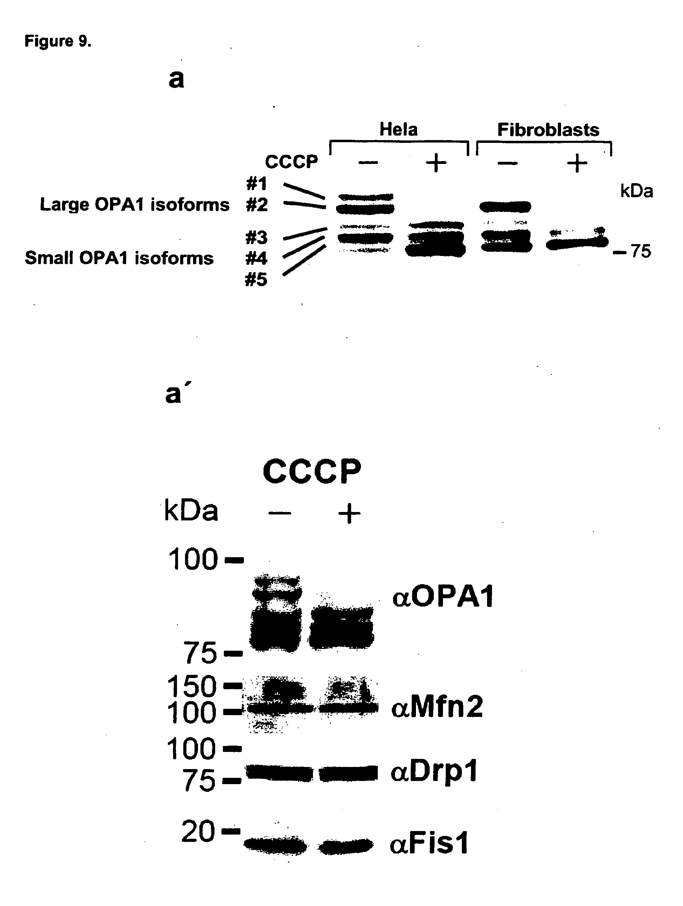

**a,** Western blot analysis of OPA1 isoforms of heart tissue from mice with a heart-specific knock-out of TFAM (TFAM ko) at 4 or 8 weeks age and of control mice.
**b,** Homogenates from skeletal muscle biopsies from control individuals (A-D, see Tab.2) and from patients suffering from respiratory disorders (1-10, see Tab.2) analyzed by western blotting for OPA1. The smallest form of OPA1, OPA1-S5 ("s-OPA1"), detected is indicated (arrow).
**c,** Relative amounts of the smallest OPA1 isoform OPA1-S5 of all OPA1 isoforms determined densitometrically from panel b and from HeLa cells treated or not for 6 h with CCCP (see Fig.9a, two left lanes).

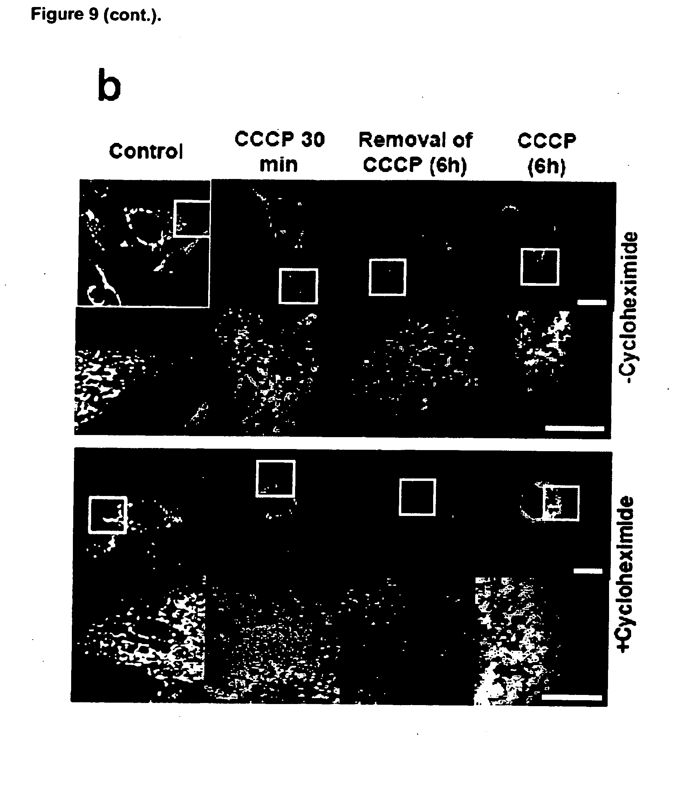

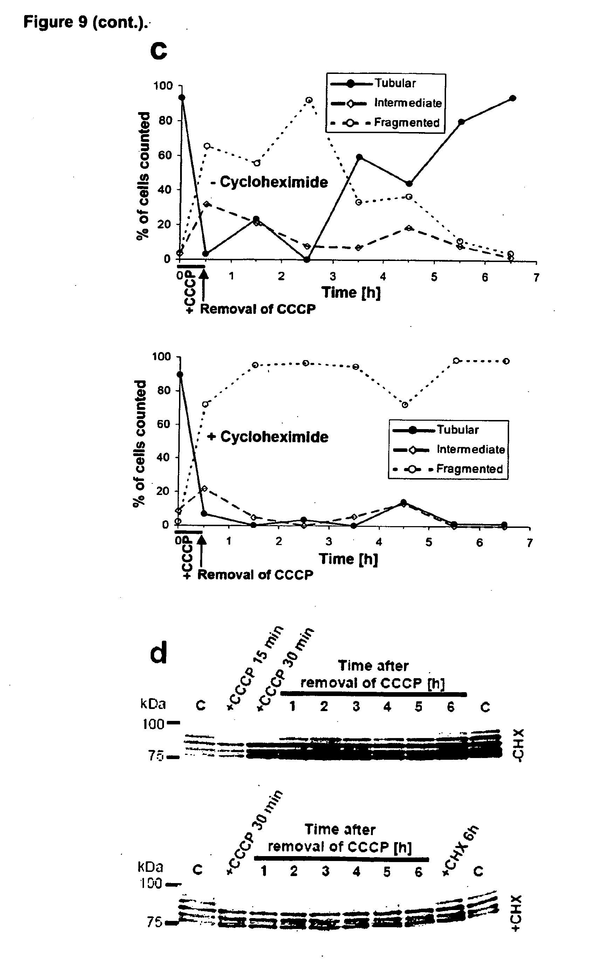

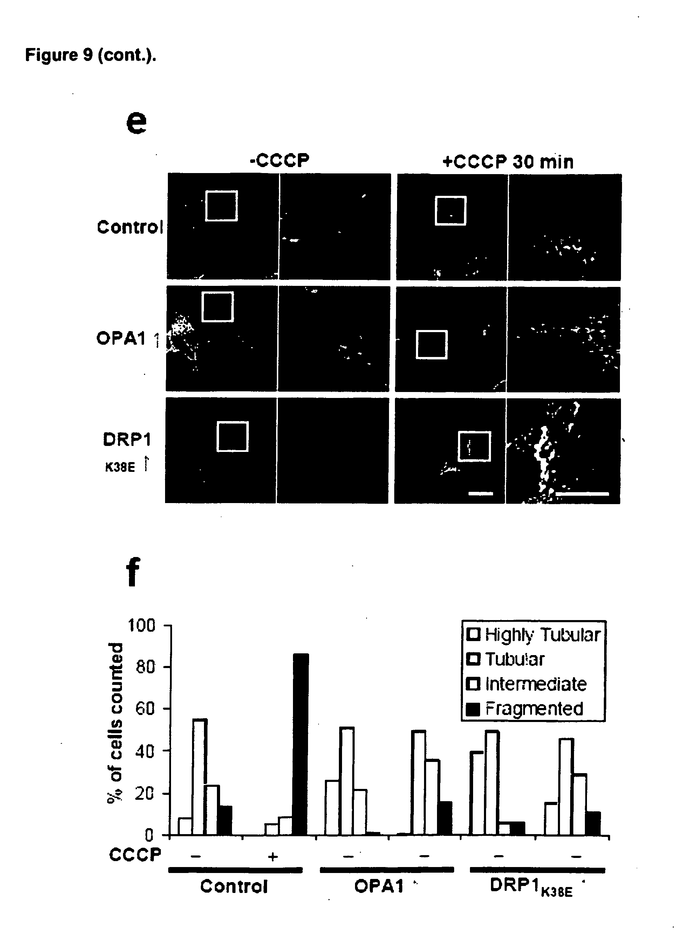

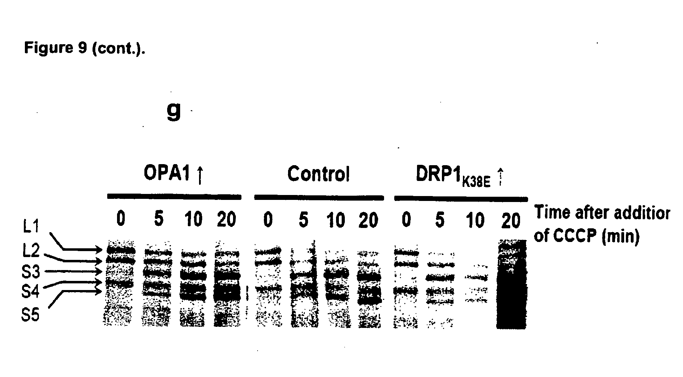

### Figure 9. Mitochondrial fragmentation and OPA1 processing after dissipation of the membrane potential are causally linked.

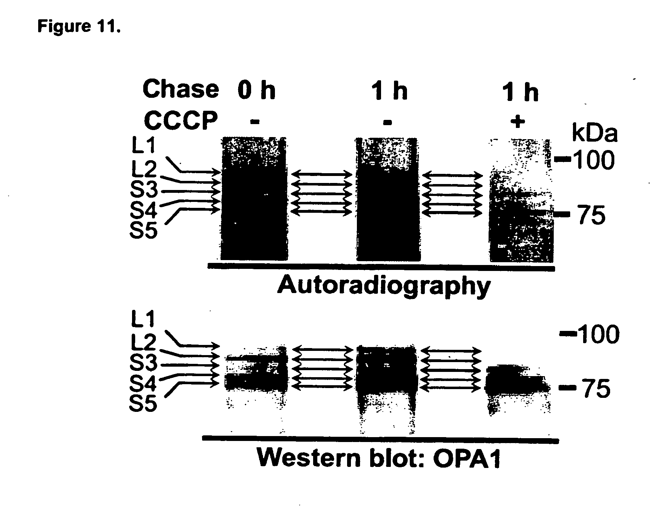

**a,** HeLa cells and human fibroblasts were treated with CCCP (20 µM) for 6 h and total cell extracts were subjected to western blotting with OPA1 antibodies **b-d**, Cells were treated or not (Control) with CCCP (20 µM) for 30 min, washed, and incubated further with medium lacking CCCP for the indicated time periods or CCCP (20 µM) was left for 6 h. In parallel, these experiments were performed in the presence of cycloheximide (CHX; 175 µg/ml). Cells were stained with MitoTracker (red) and with cytochrome c antibodies (green).
**a',** HeLa cells were treated or not with CCCP (20 µM) for 30 min and total cell lysates were subjected to western blotting with the indicated antibodies.
**b,** Merged confocal fluorescence images are shown. Top, overview (scale bar 20 µm); bottom, indicated box, (scale bar 10 µm).
**c,** Quantification of mitochondrial morphology as in Fig.6b.
**d,** OPA1 isoforms by western blot analysis. Extracts of untreated cells (C) and of cells treated for 6 h with CHX alone (+CHX 6 h) were used as controls.
**e,** HeLa cells were transfected with a plasmid expressing mitochondrial GFP (control), or co-transfected with this plasmid and a plasmid expressing either a mouse isoform of OPA1 corresponding to spliceform 1 (OPA1 ↑), or a dominant negative variant of DRP1 (DRP1_{K38E} ↑). 36 h after transfection, cells were treated with CCCP for 30 min or not, fixed, and immunostained with GFP (green) and cytochrome c antibodies (red). Merged confocal fluorescence images. Left, overview (scale bar 20 µm); right, indicated box (scale bar 10 µm).
**f,** Mitochondrial morphology of mitochondrial GFP positive cells was quantified. Cells with at least one highly elongated mitochondrion of more than 10 µm (highly tubular) were quantified in addition to those classes described in Fig.6b.
g, Pulse-chase experiment; HeLa cells were transfected as described herein. 24 h after transfection, cells were subjected to radioactive labeling and subsequent CCCP treatment during the chase period as described for Fig. 11. At indicated times after addition of CCCP, cells were washed, harvested, lysed and subjected to immunoprecipitation with antibodies raised against OPA1 in the presence of 5 mM o-phenathroline / 10 mM EDTA. OPA1 isoformsin the elution fractions were separated by SDS-PAGE and analyzed by digital autoradiography.

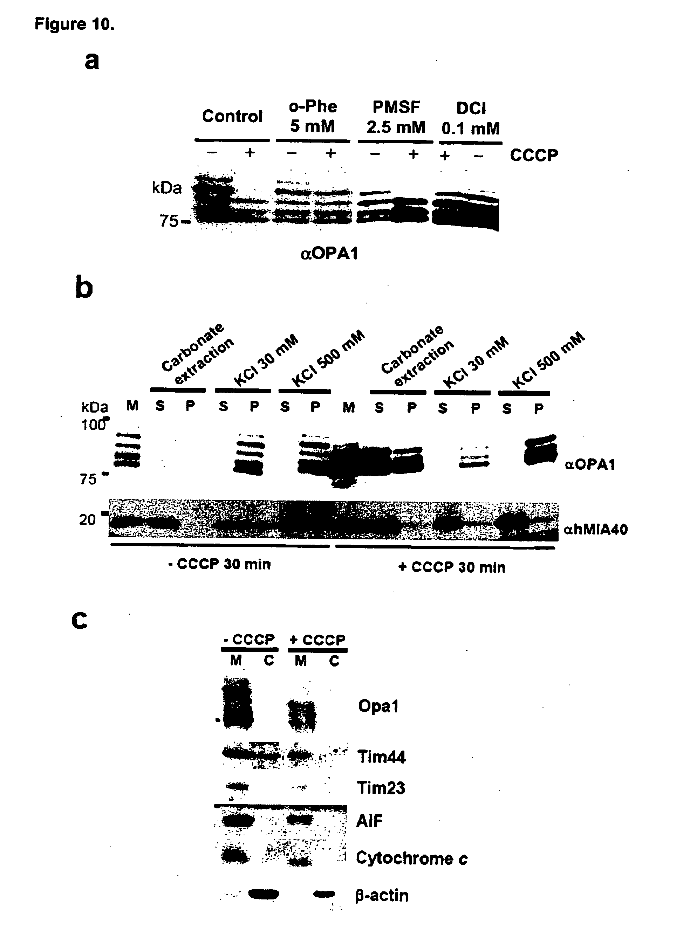

### Figure 10. Processing and membrane association of OPA1 isoforms.

**a,** The protease inhibitor o-phenanthroline (o-Phe) and partially DCI (3,4-Dichloroisocoumarin), but not phenylmethylsulphonylfluoride (PMSF) block uncoupler induced conversion of larger OPA1 isoforms to smaller isoforms. HeLa cells were preincubated for 10 min with or without the protease inhibitors at the indicated concentration before CCCP (20 µM) was added. Cells were further incubated for 25 min before they were harvested, lysed in loading buffer, loaded on a SDS-PAGE, and immunoblotted with OPA1 antibodies.
**b,** The larger isoforms of OPA1 behave in salt and carbonate extraction experiments as integral inner membrane proteins, whereas the smaller isoforms behave like peripherally attached proteins. Shorter isoforms remain peripherally attached to mitochondria even after cells were pretreated with CCCP for 30 minutes (right part). Isolated mitochondria from HeLa cells treated with CCCP for 30 minutes (right) or untreated (left) were extracted either with 30 mM or 500 mM KCl after sonication or with 0.1 M sodium carbonate (pH 11). Pellets were recovered by centrifugation (130,000g, 30 min, 4°C). 100 µg of mitochondrial proteins were used for extraction and 25 % of each pellet (P) and supernatant (S) was analyzed by SDS-PAGE and immunoblotting for the indicated proteins.
**c,** OPA1 isoforms remain in the mitochondrial fraction independent of pretreatment of cells with CCCP. In addition, neither degradation nor release of other mitochondrial proteins (Tim44, Tim23, AIF, cytochrome c) was observed. As cytosolic marker protein β-actin was used. Hela cells were treated with CCCP as described in b, and subjected to subcellular fractionation. Equal proportions of the mitochondrial fraction (M) and of the cytosolic fraction (C) are analyzed by SDS-PAGE and immunoblotted with indicated antibodies. In total less material was loaded from the CCCP treated derived fractions.

### Figure 11. Pulse-chase experiment.

HeLa cells were subjected to radioactive labeling of newly synthesized proteins. After labeling cells were washed, incubated for the indicated time in the absence of radiolabeled amino acids (chase) either in the presence or the absence of CCCP (20 µM). Cells were lysed at indicated times of chase and subjected to immunoprecipitation with antibodies raised against OPA1. Elution fractions were analyzed by digital autoradiography (top panel) and the same membrane was subjected to western blot analysis using OPA1 antibodies (bottom panel). The different OPA1 isoforms are indicated by arrows and named L1-, L2-, S3-, S4- and S5-OPA1.

The Examples illustrate the invention.

### Example 1: Material and Methods

### Cell culture and reagents:

If not described otherwise, HeLa cells, human fibroblasts, immortalised mouse embryonic fibroblast from control (MEF 13, 14) and mutator mice (MEF 2, 7) (Trifunovic, 2004, Nature 429, 417; Trifunovic, 2005, Proc Natl Acad Sci USA 102, 17993), and cybrid cell lines (pT1, pT3) (Chomyn, 1991, Mol Cell Biol 11, 2236) were grown under standard conditions in Dulbecco modified Eagle's medium (DMEM) containing 4.5 g/l glucose and 2 mM L-glutamine supplemented with 10% fetal bovine serum, 50 U/ml penicillin and 50 µg/ml streptomycin. Cell culture reagents were obtained from PAA laboratories (Cölbe, Germany), CCCP, o-phenanthroline and cycloheximide were purchased from Sigma (Germany), DCl from Sigma (Germany), and PMSF from Serva (Germany). The OPA1 plasmid (pMSCV-OPA1) was a kind gift of Luca Scorrano (Padova, Italy) (Cipolat, 2004, Proc Natl Acad Sci USA). The DRP1_{K38E} N-terminally fused to CFP (pECFP-C1-DVLP_{K38E}) and mitochondrially targeted GFP (pcDNA3-pOCT:GFP) plasmids were kind gifts of Heidi McBride (Ottawa Heart institute, Canada; Neuspiel, 2005, J Biol Chem, 280, 25060-70; Harder, 2004, Curr Biol, 14, 340-5). Transient transfections of HeLa cells were performed using Metafectene (Biontex Laboratories, Germany). Mitochondria were prepared from HeLa cells by differential centrifugation as described herein.

### Yeast plasmids, strains and growth conditions:

OPA1 splice variant 7 was amplified from human cDNA and cloned into pYES2 (Invitrogen, USA). All sequences were verified by DNA sequencing. Haploid W303 was used as wild type control elsewhere. Cells were grown under standard conditions (Guthrie, Methods Enzymol 194, 1-270, 1991). The strain expressing OPA1 was cultured using 2% galactose and 0.5% glucose.

### Tissue samples:

Heart tissue was obtained from heart-specific TFAM knockout mice as described earlier (Hansson, 2004, Proc Natl Acad Sci USA 101, 3136). Skeletal muscle biopsies were derived from patients diagnosed with respiratory chain disorders or control patients with no such defects (see Tab.2). Informed consent was given by all patients.

### Antibodies for immunoblotting:

Anti-OPA1 antibody was affinity purified from a rabbit polyclonal serum raised against the C-terminus of human OPA1 using synthetic peptide: CDLKKVREIQEKLDAFIEALHQEK (SEQ ID No: 29) (Pineda Antikörper-Service, Berlin; BD Biosciences). Antibodies against human MIA40 were raised in rabbits using purified MIA40 fused to MBP. Polyclonal rabbit sera against hTim44, and hTim23 were raised in rabbits as described Bauer (1999, J Mol Biol 289(1), 69-82). Antibodies against AIF (goat anti-AIF, D-20:sc-9416, Santa Cruz Biotechnology, USA), rabbit anti-Fis1 sera (IMGENEX), cytochrome c (mouse, clone 7H8.2C12, BD Biosciences), anti-DRP1 (DLP1 clone 8; BD Biosciences) and β-actin (clone AC-15, Sigma, Germany) were used according to the manufacturer's instructions. The anti-Mfn2 serum was a kind gift of Antonio Zorzano (University of Barcelona, Spain; Pich Hum Mol Genet 2005 14(11):1405-1415).

### Fluorescence microscopy:

Live cells were fluorescently labeled with MitoTracker® Red CMXRos (Molecular probes, USA) for mitochondria or with 4',6-diamidino-2-phenylindole dihydrochloride (DAPI; Molecular probes, USA) for the nucleus, subsequently fixed, and permeabilised. Immunostaining was carried out with mouse anti-cytochrome c monoclonal antibody (clone 6H2.B4; BD Biosciences) and chicken anti-GFP antibody (Aves Lab Inc., USA). The following secondary antibodies conjugated to fluorescent dyes were used: Alexa Fluor^{®} 488 anti-mouse IgG (H+L) (Molecular probes, USA), Cy3-conjugated anti-mouse IgG and Fluorescein(FITC)-conjugated anti-chicken IgG (Jackson Immunoresearch, USA). Cells were mounted with Prolong® Gold antifade reagent (Molecular probes, USA). All treatments were done according to the manufacturers' instructions. Mitochondrial morphology was analysed by confocal microscopy using a Zeiss LSM 510 (Carl Zeiss Microscopy, Jena, Germany) equipped with a 63x objective. For all imaging, 512x512 pixel images of single confocal planes were acquired and processed with the Bitplane Imaris 4 software.

### Example 2: Detection of OPA1 isoforms by SDS-PAGE

Cell pellets were lysed in Lämmli-buffer 1x and heated for 5 min at 95°C. Samples equivalent to ~10⁶ cells were loaded on a 10% acrylamid gel (12 cm high x 16 cm wide plates separated by 1.5 mm thick spacers). Gels were run for 3.5 hours at constant 30 mA or until the 37 kD band of the BioRad Precision Plus Protein standard reaches the bottom of the gel. The proteins were transferred to a nitrocellulose membrane by semi-dry blotting for 1.5 hours at 200 mA using Tris/glycine buffer. The membrane was incubated in TBS buffer containing 5% non fat milk powder for 30 min. The membrane was then incubated in TBS buffer containing 5% non fat milk powder and affinity purified rabbit OPA1 antibodies (raised against the C-terminal peptide of OPA1: DLKKVREIQEKLDAFIEALHQEK (SEQ ID No: 30)) diluted 1:500 for 1 hour. After three rapid washes to remove the primary antibody in excess, the membrane was further incubated for 1 hour in TBS containing 5% non fat milk powder and secondary antibodies anti rabbit conjugated to HRP (horseradish peroxidase) diluted 1:10,000 (BioRad). The membrane was then washed in TBS, once rapidly, and four more times for 5 min each. ECL reagent (enhanced chemoluminescence reagent) was used to reveal the different OPA1 isoform bands on the membrane. Expositions of 1 to 2 min were usually sufficient to detect all OPA1 forms. The compositions of the buffers and gels to be employed are as follows.

### 4X Lämmli buffer 100ml:

8g SDS
40ml Glycerin
40 min 0.6M Tris pH 6.8
80mg Bromophenol blue
Adjust the volume to 80ml with distilled water and then add
β-Mercaptoethanol 20ml

### SDS-PAGE running buffer (electrophoresis buffer) 10X for 5I:

50g SDS
150g Tris
720g Glycine
Add distilled water to reach 5 I

### 10% Separating gel (10%):

| | |
|---|---|
| Acrylamide 30% / N, N'-Methylene-bis-acrylamide 0.5 % | 5.7 ml |
| 1M Tris pH 8.8 | 6.5 ml |
| Distilled water | 4.5 ml |
| TEMED | 10 µl |
| 10%APS | 100 µl |
| Total (for one gel) | 17 ml |

### Stacking gel:

| | |
|---|---|
| Acrylamide 30% / N, N'-Methylene-bis-acrylamide 0.5 % | 17 ml |
| 1 M Tris-HCl pH 8.8 | 6 ml |
| 10% SDS | 1 ml |
| Distilled water | 76 ml |
| Total (for 20gels) | 100 ml |

Store at 4°C. Take 5ml for one gel.
Add 25µl 10%APS
5µl TEMED

### 1X blotting buffer:

| | |
|---|---|
| 20mM Tris | 4.84g |
| 150mM Glycine | 22.52g |
| 20% Methanol | 400ml |
| 0.08% SDS | 1.6g |
| Total | 2l (adjusted with distilled water) |

### 10X TBS:

| | |
|---|---|
| 100mM Tris | 60.57g |
| 9% NaCl | 450g |
| Total | 5l (adjusted with distilled water) |

Adjusted to pH 7.4 with HCI.

### Example 3: Identification of OPA1 peptides by mass spectrometry

Cells were lysed in lysis buffer (0.5% TritonX100, 150 mM NaCl, 10 mM Tris/HCl pH 7.5, 5 mM EDTA, supplemented with complete protease inhibitor cocktail; Roche, Switzerland) and subjected to standard immunoprecipitation. Thereby, OPA1 isoforms from isolated HeLa mitochondria were immunoprecipitated using an antibody raised against the C-terminal peptide of OPA1 covalently coupled to Sulfo-Link sepharose beads (Pierce, USA) or, alternatively, coupled to Protein A Sepharose CL-4B beads (Amersham Biosciences). If not described otherwise, elution was performed with 1x Lämmli-buffer, separated by SDS-PAGE, stained with Coomassie Blue and bands were cut after in-gel digest with trypsin. All bands were clearly identified as derived from OPA1. Cut gel slices from SDS-PAGE were washed 2x with water and 2x with 40 mM ammonium bicarbonate for 30 min each. After 2x 5 min treatment with 50% acetonitrile, trypsin (Sequencing Grade Modified, Promega) was added and proteins were digested over-night in 40 mM ammonium bicarbonate at 37°C while shaking (650 rpm). Peptides were directly analyzed by MALDI-MS or LC-MS/MS. For example, if not described otherwise, peptides were directly analyzed by nano-ESI-LC-MS/MS for which they were separated on a C18 reversed phase column (75 µm i.d. x 15 cm, packed with C18 PepMap™, 3 µm, 100 A by LC Packings) via a linear acetonitrile gradient, MS and MS/MS spectra were recorded on a QSTAR XL mass spectrometer (Applied Biosystems), and analyzed via the Mascot™ Software (Matrix Science) using the NCBl^{nr} Protein Database.

The theoretical molecular weight for a corresponding amino acid sequence was calculated using the program "pi_tool" (http://www.expasy.ch/tools/pi tool.html; Bjellqvist, Electrophoresis 1993, 14, 1023-1031; Bjellqvist, Electrophoresis 1994, 15, 529-539; Gasteiger, 2005, Protein Identification and Analysis Tools on the ExPASy Server, (In) John M. Walker (ed): The Proteomics Protocols Handbook, Humana Press). Thereby, the option "average" regarding the resolution was used.

The corresponding results from a first estimation are as follows:

In the protein bands corresponding to I-OPA1#1 and #2, as most N-terminal peptides, the following peptides were found, besides various peptides present in all OPA1 spliceforms:
- YLILGSAVGGGYTAK (SEQ ID NO: 17)
- TFDQWK (SEQ ID NO: 18)
- DMIPDLSEYK (SEQ ID NO: 19)
- WIVPDIVWEIDEYIDFEK (SEQ ID NO: 20)
- LAPDFDK (SEQ ID NO: 21)
- IVESLSLLK (SEQ ID NO: 22)
- ALPNSEDLVK (SEQ ID NO: 23)
- DFFTSGSPEETAFR (SEQ ID NO: 24)
(e. g. see Figure 1 and 3a)

Accordingly, in large OPA1 isoforms at least the amino acids from position 102 to the most C-terminal amino acid of all OPA1 spliceforms (SEQ ID No:2, 4, 6, 8, 10, 12, 14, 16) are comprised and the theoretical molecular weights vary between 89 und 99 kD, depending of the corresponding spliceform to be used for molecular weight calculation.

Based on *in silico* predictions (prediction of cleavage of the leader sequence by mitochondrial processing peptidase (MPP) using the computer program Mitoprot (Claros, 1996, Eur J Biochem, 241, 779-86)), it was deduced that large OPA1 isoforms already start with amino acid position 95 of all OPA1 spliceforms (SEQ ID No:2, 4, 6, 8, 10, 12, 14, 16), and hence, start with the peptide TRLLKLRYLILGS (SEQ ID NO: 25) .... According to this finding, the above mentioned theoretical molecular weights are increased by 0.9 kD.

None of the above mentioned peptides were detected in protein bands corresponding to small isoforms of OPA1.

Further, in I-OPA1#1 but not in I-OPA1#2 the following peptides were found: GLLGELILLQQQIQEHEEEAR (SEQ ID NO: 26) and AAGQYSTSYAQQK (SEQ ID NO: 27).

From the data provided in the first estimation, it can be deduced that I-OPA1#1 be derived from spliceform 7 and I-OPA1 #2 be derived from spliceform 1.

In the protein band corresponding to s-OPA1#3, as most N-terminal peptide, the peptide GLLGELILLQQQIQEHEEEAR (SEQ ID NO: 26) was found. Moreover and inter alia, the peptides AAGQYSTSYAQQK (SEQ ID NO: 27) and IDQLQEELLHTQLK (SEQ ID NO: 28) were found. It was deduced that s-OPA1#3 could be derived from spliceforms 4, 6, 7, and/or 8 and that the corresponding most N-terminal amino acid is, dependent on the amino acid sequence of the mentioned spliceforms, at position 173 (SEQ ID No: 10), 191 (SEQ ID No: 6), 209 (SEQ ID No: 4) and 227 (SEQ ID No: 2), respectively. The theoretically predicted molecular weight of the s-OPA1#3 is about 91.8 kD.

In the protein band corresponding to s-OPA1#4, as most N-terminal peptide, the peptide AAGQYSTSYAQQK (SEQ ID NO: 27) was found. Moreover and inter alia, the peptide IDQLQEELLHTQLK (SEQ ID NO: 28) was found. It was deduced that s-OPA1#4 could derive from spliceforms 4, 6, 7, and/or 8 and that the corresponding most N-terminal amino acid is, dependent on the amino acid sequence of the mentioned spliceforms, at position 195 (SEQ ID No: 10), 213 (SEQ ID No: 6), 231 (SEQ ID No: 4) and 249 (SEQ ID No: 2), respectively. The theoretically predicted molecular weight of the s-OPA1#4 is about 89.2 kD.

In the protein band corresponding to s-OPA1#5; as most N-terminal peptide, the peptide IDOLOEELLHTQLK (SEQ ID NO: 28) was found. It was deduced that s-OPA1#5 could derive from one or more of spliceforms 1 to 8 and that the corresponding most N-terminal amino acid is, dependent on the amino acid sequence of the mentioned spliceforms, at position 215 (SEQ ID No: 16), 179 (SEQ ID No: 14), 197 (SEQ ID No: 12), 216 (SEQ ID No: 10), 233 (SEQ ID No: 8), 234 (SEQ ID No: 6), 252 (SEQ ID No: 4) and 270 (SEQ ID No: 2), respectively.

The theoretically predicted molecular weight of the s-OPA1#5 is about 86.8 kD.

Moreover, in none of the small OPA1 isoforms, the peptide DFFTSGSPEETAFR (SEQ ID NO: 24) was found. Accordingly, is can be deduced that the N-terminal amino acid of each s-OPA1 lies within or shortly C-terminal to this sequence. In either case, the N-terminal amino acid of each small OPA1 lies N-terminal to the above described found most N-terminal peptides of each small OPA1 (see above).

Based on the results from the first estimation with respect to the OPA1 peptides determined by the mass spectrometry experiments, for each OPA1 isoform the following theoretically calculated molecular weights and the apparent starting position in the corresponding spliceform of OPA1 (see also the corresponding SEQ ID No) were deduced:

| **Isoform (amino acid position start / MW in kD)** | | | | |
|---|---|---|---|---|
| **Spliceform** | I-OPA1#1/#2 | s-OPA1#3 | s-OPA1#4 | s-OPA1#5 |
| **8** | 95 / 105.9 (106,8*) | 227 / 91.8 | 249 / 89.2 | 270 / 86.8 |
| **7** | 95 / 104.9 | 209 / 91.8 | 231 / 89.2 | 252 / 86.8 |
| **6** | 95 / 102.7 | 191 / 91.8 | 213 / 89.2 | 234 / 86.8 |
| **5** | 95 / 102.5 | x | x | 233 / 86.8 |
| **4** | 95/100.8 | 173/91.8 | 195/89.2 | 216 / 86.8 |
| **3** | 95 / 98.5 | x | x | 197 / 86.8 |
| **2** | 95/96.6 | x | x | 179 / 86.8 |
| **1** | 95 / 100.7 | x | x | 215 / 86.8 |

To obtain an averaged apparent molecular weight value for either I-OPA1#1 or I-OPA1#2, the four smallest and the four largest molecular weight values of the second column of the above table ("I-OPA1#1/#2") where averaged. Accordingly, for I-OPA1#1 an averaged molecular weight value of 104.0 kD (104,2*) and for I-OPA1#2 an averaged molecular weight value of 99.2 kD was derived. The values indicated by an asterix (*) are based on a repeated estimation.

Alternatively, based the herein provided evindence that I-OPA1#1 is derived from spliceform 7 and that I-OPA1#2 is derived from spliceform 1, for I-OPA1#1 a theoretic molecular weight value of 104.9 kD and for I-OPA1#2 a theoretic molecular weight value of 100.7 kD was calculated.

In the following, the corresponding results from a second, refined analysis are given:

In the protein bands corresponding to I-OPA1#1 and #2, as most N-terminal peptides, the following peptides were found, besides various peptides present in all OPA1 spliceforms:
- YLILGSAVGGGYTAK (SEQ ID NO: 17)
- TFDQWK (SEQ ID NO: 18)
- DMIPDLSEYK (SEQ ID NO: 19)
- WIVPDIVWEIDEYIDFEK (SEQ ID NO: 20)
- LAPDFDK (SEQ ID NO: 21)
- IVESLSLLK (SEQ ID NO: 22)
- ALPNSEDLVK (SEQ ID NO: 23)
- DFFTSGSPEETAFR (SEQ ID NO: 24)
(e. g. see Figure 1 and 3d)

Accordingly, in large OPA1 isoforms at least the amino acids from position 102 to the most C-terminal amino acid of all OPA1 spliceforms (SEQ ID No:2, 4, 6, 8, 10, 12, 14, 16) are comprised and the theoretical molecular weights vary between 95.7 und 105.9 kD, depending of the corresponding spliceform to be used for molecular weight calculation (see below).

Based on *in silico* predictions (prediction of cleavage of the leader sequence by mitochondrial processing peptidase (MPP) using the computer program Mitoprot (Claros, 1996, Eur J Biochem, 241, 779-86)), it was deduced in a first estimation that large OPA1 isoforms already start with amino acid position 95 of all OPA1 spliceforms (SEQ ID No:2, 4, 6, 8, 10, 12, 14, 16), and hence, start with the peptide TRLLKLRYLILGS (SEQ ID NO: 25). According to this finding, the above mentioned theoretical molecular weights are increased by 0.9 kD. However, based on an alternative MPP cleavage site present from amino acid 86 to 88 (Taylor Structure. 2001 Jul 3;9(7):615-25), it was deduced in a refined analysis that large OPA1 isoforms already start with amino acid position 88 of all OPA1 spliceforms (SEQ ID No:2, 4, 6, 8, 10, 12, 14, 16), and hence, start with the peptide FWPARLATRLLKLRYLILGS (SEQ ID NO: 111). According to this finding, the above mentioned theoretical molecular weights are increased by 1.7 kD instead of 0.9 kD. None of the above mentioned peptides were detected in protein bands corresponding to small isoforms of OPA1.

Further, in I-OPA1#1 but not in I-OPA1#2 the following peptide was found:
AAGQYSTSYAQQK (SEQ ID NO: 27).

From the data provided in the refined analyses, it can be deduced that I-OPA1#1 be derived from spliceform 7 and I-OPA1 #2 represents a mixture of two isoforms derived from splice variants 1 and 4.

In the protein band corresponding to s-OPA1#3, as most N-terminal peptides, the peptides IVESLSLLK (SEQ ID No: 22), DFFTSGSPEETAFR (SEQ ID No: 24), and GLLGELILLQQQIQEHEEEAR (SEQ ID NO: 26) were found. Moreover and inter alia, the peptides AAGQYSTSYAQQK (SEQ ID NO: 27) and IDQLQEELLHTQLK (SEQ ID NO: 28) were found. It was deduced that s-OPA1#3 is derived from spliceform 7 and that the corresponding most N-terminal amino acid is 172 (SEQ ID No: 4), or close-by more N-terminal to this position. The theoretically predicted molecular weight of the s-OPA1#3 is about 95.9 kD.

In the protein band corresponding to s-OPA1#4, as most N-terminal peptides, the peptides .GLLGELILLQQQIQEHEEEAR (SEQ ID NO: 26) and AAGQYSTSYAQQK (SEQ ID NO: 27) were found. Moreover and inter alia, the peptide IDQLQEELLHTQLK (SEQ ID NO: 28) was found. It was deduced that s-OPA1#4 could derive from spliceforms 4, 6, 7, and/or 8 and that the corresponding most N-terminal amino acid is, dependent on the amino acid sequence of the mentioned spliceforms, at position 173 (SEQ ID No: 10), 191 (SEQ ID No: 6), 209 (SEQ ID No: 4) and 227 (SEQ ID No: 2), respectively. The theoretically predicted molecular weight of the s-OPA1#3 is about 91.8 kD.

In the protein band corresponding to s-OPA1#5, as most N-terminal peptide, the peptide IDQLQEELLHTQLK (SEQ ID NO: 28) was found. It was deduced that s-OPA1#5 could derive from one or more of spliceforms 1 to 8 and that the corresponding most N-terminal amino acid is, dependent on the amino acid sequence of the mentioned spliceforms, at position 215 (SEQ ID No: 16), 179 (SEQ ID No: 14), 197 (SEQ ID No: 12), 216 (SEQ ID No: 10), 233 (SEQ ID No: 8), 234 (SEQ ID No: 6), 252 (SEQ ID No: 4) and 270 (SEQ ID No: 2), respectively.

The theoretically predicted molecular weight of the s-OPA1#5 is about 86.8 kD.

Moreover, in the refined analyses, in none of the small OPA1 isoforms, the peptide LAPDFDK (SEQ ID NO: 21) was found. Accordingly, is can be deduced that the N-terminal amino acid of each s-OPA1 lies within or shortly C-terminal to this sequence. In either case, the N-terminal amino acid of each small OPA1 lies N-terminal to the above described found most N-terminal peptides of each small OPA1 (see above).

Based on the results from the refinded analyses with respect to the OPA1 peptides determined by the mass spectrometry experiments, for each OPA1 1 isoform the following theoretically calculated molecular weights and the apparent starting position in the corresponding spliceform of OPA1 (see also the corresponding SEQ ID No) were deduced:

| **Isoform (amino acid position start / MW in kD)** | | | | |
|---|---|---|---|---|
| **Spliceform** | I-OPA1#1/#2 | s-OPA1#3 | s-OPA1#4 | s-OPA1#5 |
| **8** | 88 / 107.6 | x | 227 / 91.8 | 270 / 86.8 |
| **7** | 88 / 105.8 | 172 / 95.9 | 209 / 91.8 | 252 / 86.8 |
| **6** | 88 / 103.5 | x | 191 / 91.8 | 234 / 86.8 |
| **5** | 88 / 103.4 | x | x | 233 / 86.8 |
| **4** | 88/101.7 | x | 173/91.8 | 216 / 86.8 |
| **3** | 88/99.3 | x | x | 197 / 86.8 |
| **2** | 88/97.4 | x | x | 179 / 86.8 |
| **1** | 88/ 101.5 | x | x | 215 / 86.8 |

To obtain an averaged apparent molecular weight value for either I-OPA1#1 or I-OPA1#2, the four smallest and the four largest molecular weight values of the second column of the above table ("I-OPA1#1/#2") where averaged. Accordingly, for I-OPA1#1 an averaged molecular weight value of 105.1 kD and for I-OPA1#2 an averaged molecular weight value of 100.0 kD was derived.

Altematively, based the herein provided evindence that I-OPA1#1 is derived from spliceform 7 and that I-OPA1#2 is derived from spliceform 1 or spliceform 4, for I-OPA1#1 a theoretic molecular weight value of 105.8 kD and for I-OPA1#2 a theoretic molecular weight value of 101.5 kD or 101.7 kD, respectively, was calculated.

To examine to which extent the large and/or small OPA1 isoforms are generated by alternative splicing or limited proteolysis, mitochondria from HeLa cells were isolated and the different OPA1 species were purified by immunoprecipitation (Fig. 3bc). The antibodies used were directed against a C-terminal peptide of OPA1 present in all OPA1 isoforms. The various species were resolved by SDS-PAGE and analyzed by LC-MS/MS spectrometry (Figure 3d). The most N-terminal tryptic peptide found in L1 and L2 was located a few amino acid residues C-terminal to the cleavage site of the mitochondrial processing peptidase (MPP) between A94 and T95 or, alternatively, N87 and F88 (see above). Specifically for L1 and L2 a number of peptides were found located C-terminally to the MPP cleavage site (Figure 3d). These peptides were derived from exon 3 and from alternatively spliced exons 4 and 5b but not from exon 4b. Small OPA1 isoforms (S3 to S5) were lacking increasingly more of these peptides from the N-terminus (Figure 3d). All S-forms lacked the transmembrane segment TM1. Besides that, numerous peptides corresponding to exon 6-29, which are common to all splice variants, were identified in all isoforms (Figure 3d and data not shown).

In order to assign the isoforms to individual splice variants the presence or absence of peptides in the purified isoforms that cover or overlap exons affected by alternative splicing were taken into account. For L1 peptides that all could be derived from splice variant 7 but not from a different splice variant alone were obtained (Figure 3). The calculated molecular weights of each splice variant when cleaved by MPP are approximately as follows: 101.5 kDa (Sp.1), 97.4 kDa (Sp.2), 99.3 kDa (Sp.3), 101.7 kDa (Sp.4), 103.4 kDa (Sp.5), 103.5 kDa (Sp.6), 105.8 kDa (Sp.7), and 107.6 kDa (Sp.8). Thus, as L2 contained the same nine N-terminal peptides as L1 but was smaller in size (Fig. 3bc) it is unlikely to be derived from splice variant 7 or 8. Further, the peptide pattern found for L2 was consistent with splice variant 1 except for one peptide (GLLGELILLQQQIQEHEEEAR (SEQ ID NO: 26)) which could theoretically be derived from splice variants 4, 6, 7 or 8 (Figure 3d). However, only the predicted size of MPP cleaved splice variant 4 is nearly identical to the one of splice variant 1. Therefore, in HeLa cells L2 most likely represents a mixture of two isoforms derived from splice variants 1 and 4 whereas L1 is derived from splice variant 7 (Figure 3d). These results are consistent with the high level of expression of these variants in HeLa cells (Satoh, Biochem Biophys Res Commun 300, 482-493, 2003). S3 contains peptides representing splice variant 7 while S4 contains peptides derived from splice variants 4, 6, 7, or 8, and S5 contains only peptides common to all eight splice variants. In conclusion, these results suggest that S-forms are generated by proteolysis of larger OPA1 isoforms possibly derived from different splice variants.

### Example 4: The role of OPA1 during apoptosis

In a first approach to study the molecular mechanism of fragmentation of mitochondria in higher organisms, the role of fusion-promoting OPA1 during apoptosis was investigated. The levels of OPA1 isoforms in HeLa cells after induction of apoptosis were determined. Treatment of cells with staurosporine resulted in rapid fragmentation of mitochondria within 2 - 3 h (Fig. 5ab). This coincided with the disappearance of the two largest OPA1 isoforms and a concomitant increase of small isoforms of OPA1 (Fig.5d). Release of cytochrome c occurred at markedly later time; only after 5 - 6 h had more than 50% of cells released cytochrome c (Fig.5c). Thus, fragmentation during apoptosis occurs earlier than release of cytochrome c and concomitantly with disappearance of larger OPA1 isoforms.

### Example 5: Determination of OPA1 isoforms in MERRF cells

In a further approach to study the molecular mechanism of fragmentation of mitochondria in human diseases, the role of fusion-promoting OPA1 in a cybrid cell line derived from a MERRF patient suffering from severe myopathy (Chomyn, 1991, Mol Cell Biol 11, 2236) was investigated. The mtDNA in the cell line contained the A8344G mutation in the tRNA^{Lys} in a nearly homoplasmic (∼98%) manner leading to a substantial impairment of mitochondrial function (Chomyn, 1991, Mol Cell Biol 11, 2236). The cells from the MERRF patient, but not control cells, showed highly fragmented mitochondria (Fig. 6ab). The pattern of the five detected OPA1 protein isoforms was altered in the MERRF cells compared to control cells (Fig.6c). It appears that in the MERRF cells the larger isoforms are reduced compared to, at least, the smallest isoform of OPA1 (OPA1-S5). As OPA1 is required for mitochondrial fusion the observed loss of large OPA1 isoforms may explain the fragmentation of mitochondria in this model system of mitochondrial dysfunction.

### Example 6: Determination of OPA1 isoforms in fibroblasts of the 'mutator mouse'

To further substantiate the findings of Example 5, immortalized mouse embryonic fibroblasts derived from the so-called 'mutator mouse' (Trifunovic, 2004, Nature 429, 417; Trifunovic, 2005, Proc Natl Acad Sci USA 102, 17993) were analyzed. This mouse was generated by a homozygous knock-in of a variant of mtDNA polymerase γ resulting in a phenotype of premature aging. The variant enzyme has much reduced proofreading activity and mtDNA accumulates random point mutations at a 3-5 fold higher rate than normal leading to severe mitochondrial dysfunction. Mitochondria were extensively fragmented in mutator but not in control cell lines (Fig. 7ab). The levels of the larger OPA1 isoforms were strongly reduced whereas the levels of the smaller forms were increased in the mutant cells (Fig.7c). These findings suggest that mutations in mtDNA are causative to changes in OPA1 isoform levels and mitochondrial fragmentation.

### Example 7: Determination of OPA1 isoforms in heart tissue of TFAM knockout mice

TFAM is an essential mitochondrial transcription factor also required for mtDNA maintenance. A heart-specific knockout of TFAM in mice led to a severe depletion of mtDNA in the heart, resulting in cardiomyopathy and altered mitochondrial morphology (Hansson, 2004, Proc Natl Acad Sci USA 101, 3136). The pattern of OPA1 isoforms in heart tissue of these mice was changed as compared to controls; the abundance of large OPA1 isoforms was reduced whereas that of small isoforms was increased (Fig.8a). This was even more pronounced at eight weeks compared to four weeks of age (Fig.8a), consistent with the progression of the cardiomyopathy in those mice (Hansson, 2004, Proc Natl Acad Sci USA 101, 3136). This shows that mitochondrial dysfunction resulting from mtDNA depletion is linked to the reduction of large OPA1 isoforms in the affected tissue. Moreover, the alterations of mitochondrial morphology observed earlier by electron microscopy (Hansson, 2004, Proc Natl Acad Sci USA 101, 3136) are likely due to the shift in the levels of OPA1 isoforms. In addition, it was observed whether alterations in the OPA1 isoform pattern are detectable in patients diagnosed with respiratory chain defects (Tab.2). Patients 1 to 10 included in this study were previously diagnosed with respiratory chain disorders. All patients suffered from mitochondrial encephalomyopathies or isolated myopathies on the basis of clinical, biochemical, morphological and, in some cases, genetic findings. Skeletal muscle biopsies from patients A to D representing non-mitochondrial disorders served as controls. The activities of the respiratory chain complexes of all control patients were within the normal range. Measurements of rotenone sensitive NADH-ubiquinone oxidoreductase (complex I), succinate-cytochrome c oxidoreductase (complexes II and III) and cytochrome c oxidase (complex IV) were determined spectrophotometrically in skeletal muscle homogenates according to Fischer, 1986, Eur J Pediatr, 144, 441-444, after informed consent was given by the patient. Activities were expressed as units per gram of non-collagenous protein and related to the mitochondrial marker enzyme citrate synthase. A mitochondrial DNA depletion syndrome was diagnosed in patients 1, 4 and 10. In patient 8, a homozygous mutation (G1541A) was identified in the SCO2 gene and in patient 7, mtDNA analysis led to the identification of a heteroplasmic A3243G mutation in the mitochondrial tRNA-Leu_{(UUR)} gene, the MELAS mutation. Homogenates from skeletal muscle biopsies were analysed by western blotting for OPA1 pattern (Fig.8b). The relative amount of the smallest detected form of OPA1 ("s-Opa1", which is isoform s-OPA1#5) of all OPA1 isoforms was analyzed densitometrically from the immunoblot (Fig. 8bc).

Indeed, an increase of small OPA1 isoforms was observed in skeletal muscle from these patients but not from controls (Fig.8b). The ratios of the levels of the smallest OPA1 isoform ("s-Opa1", which is isoform s-OPA1#5) to the total of all OPA1 isoforms exhibited a much broader variation in patients compared to controls. The patients with mitochondrial DNA depletion syndromes or harboring a MELAS mutation were among those with the highest ratios (Fig.8c, Table below; patients 1 and 7). In none of the controls, such a shift was observed. This demonstrates that OPA1 isoforms are altered in patients with mitochondrial disorders, in particular those with depletion or mutation of the mtDNA.

| **Samples** | | **Respiratory chain enzyme activities¹** | | **Genetics** | **% s-OPA1 of total²** |
|---|---|---|---|---|---|
| A | | normal | | - | 6.7 |
| C | | normal | | - | 7.5 |
| B | | normal | | - | 10.7 |
| D | | normal | | - | 14.7 |
| 5 | I: 10% | | | unknown | 14.7 |
| 9 | I: 80% | II/III: 40 %; | IV: 20% | unknown | 16.3 |
| 2 | | | IV: 20 % | unknown | 17.3 |
| 8 | 1: 70% | II/III: 60% | IV: 10% | A1541G mutation in SCO2 | 18.7 |
| 6 | 1: 30% | | | unknown | 22.1 |
| 4 | I: 30% | | IV: 30% | mtDNA depletion | 22.7 |
| 3 | I: 30% | II/III: 80% | IV: 30% | unknown | 22.9 |
| 10 | I: 50% | II/III: 50% | IV: 20% | mtDNA depletion | 23.5 |
| 7 | I: 50 % I: 50 % | II/III: 50% | IV: 80% IV: 80% | MELAS A3243G mutation in tRNA-Leu^{(UUR)} | 39.7 |
| 1 | I: 50% | II/III: 50% | IV: 20% | mtDNA depletion | 51.2 |

| | | | | | |
|---|---|---|---|---|---|
| ¹Activities of the respiratory chain complexes I, II/III and IV are expressed in % of the lowest limit of the respective reference range. ²The term "s-OPA1" used in this table denotes the herein definded s-OPA1#5. | | | | | |

### Example 8: Determination of ratios of OPA1 isoforms

From the results of the below described uncoupler experiments using HeLa cells (Example 9; Fig.9a), the ratio of the relative amount of various OPA1 isoforms was determined. For this purpose, total protein extracts were separated by SDS-PAGE and immunoblotted using an antibody raised against the C-terminus of OPA1. The western blot was scanned and densitometrically analysed using standard imaging software. The intensity of each band was determined and the background intensity was subtracted. The results so generated are listed in the following table:

| **Experiment** | **OPA1#3+#5 / all bands %** | **OPA1#3+#4+#5/ / all bands %** | **OPA1#3/ all bands %** | **OPA1#4 / all bands %** | **OPA1#5 / all bands %** |
|---|---|---|---|---|---|
| **HeLa - CCCP** | 20.2 | 47.4 | 9.9 | 27.2 | 10.3 |
| **HeLa + CCCP** | 64.8 | 93.0 | 19.2 | 28.2 | 45.6 |
| **fold increase** | 3.2 | 2.0 | 1.9 | 1.0 | 4.4 |

### Example 9: Uncoupler Experiments

It was further observed whether dissipation of the membrane potential is sufficient to trigger changes in abundance of OPA1 isoforms and fragmentation of mitochondria. Indeed, treatment of HeLa cells or fibroblasts with the uncoupler CCCP led to a dramatic shift of OPA1 isoforms towards the smaller isoforms (Fig.8c, 9a). This is apparently due to proteolytic cleavage of the large forms since this process was (at least partially) blocked in the presence of the protease inhibitors o-phenanthroline and DCI (Fig.10a). Further, the small isoforms produced could be extracted from mitochondria with detergent-free buffer with a higher efficiency than the large forms suggesting that the large forms are integral membrane protein isoforms whereas the small ones are only peripherally attached to the membrane (Fig.10b). Proteolytic processing occurs within mitochondria as shown by cellular fractionation experiments (Fig.10c). This is specific for OPA1 since degradation of other mitochondrial proteins is not observed (Fig.10c). Fragmentation of mitochondria occurred rapidly within 15 to 30 min after addition of CCCP (Fig. 9bc). Processing of OPA1 took place within the same narrow time frame (Fig.9d). Impairment of fusion of mitochondria may therefore be due to rapid inactivation of OPA1 by proteolysis of large isoforms. Moreover, upon removal of CCCP normal mitochondrial morphology was recovered and this coincided with the reappearance of large isoforms of OPA1 (Fig. 9cd). Mitochondrial fragmentation and OPA1 processing are not accompanied by cytochrome c release in this or in any of the investigated models of mitochondrial dysfunction (Fig.6a; 7a; 9b; 10c and data not shown). This suggests that mitochondrial fragmentation per se does not result in apoptosis. It was tested whether uncoupler induced fragmentation and reversal of fragmentation require protein synthesis. The protein synthesis inhibitor cycloheximide (CHX) did not interfere with fragmentation and OPA1 processing (Fig. 9cd). This indicates that fragmentation of mitochondria and activation of OPA1 cleavage are independent of protein synthesis. However, recovery of a tubular mitochondrial network as well as the reappearance of large OPA1 isoforms, was impaired in the presence of CHX (Fig. 9cd). Therefore, mitochondrial fragmentation and the shift of OPA1 isoforms from large to small are not reversible without ongoing protein synthesis, consistent with the explanation that a proteolytic inactivation of OPA1 causes mitochondrial fragmentation. In order to show the causal and specific role of OPA1 in this process it was investigated whether OPA1 overexpression can block uncoupler-induced fragmentation. Indeed, fragmentation of mitochondria after CCCP treatment was largely prevented upon overexpression of OPA1 (Fig. 9ef). This suggests that OPA1 is directly involved in the fragmentation of mitochondria induced after loss of the mitochondrial membrane potential. A similar effect was observed after the expression of a dominant-negative variant of DRP1

(DRP1_{K38E}) that prevents fission of mitochondria (Fig. 9ef). Thus, the fragmentation that occurs by a block of mitochondrial fusion depends on the normal DRP1-dependent fission pathway.

In order to check whether overexpression of OPA1 or of a dominant negative variant of DRP1 (DRP1_{K38E}) affects the rate of uncoupler-induced conversion of large OPA1 isoforms into smaller OPA1 isoforms a pulse-chase experiment was performed. Indeed, overexpression of OPA1 slowed down processing of OPA1 as indicated by the prolonged presence of L1- and L2-OPA1 when compared to the control overexpression of GFP or of DRP1K38E (Fig. 9g). This effect may even be underestimated since also endogenous OPA1 from about 50 % of nontransfected cells contributes to the signal. Thus, additional expression of large isoforms of OPA1 blocks CCCP-induced mitochondrial fragmentation. A dominant-negative variant of DRP1 (DRP1K38E) does not affect the rate of OPA1 processing but blocks mitochondrial fragmentation (Fig. 9efg). Thus, the fragmentation that occurs by a block of mitochondrial fusion depends on the normal DRP1-dependent fission pathway.

### Example 10: Pulse-chase experiments

The large OPA1 isoforms I-OPA1#1 and I-OPA1#2 are converted to the small OPA1 isoforms s-OPA1#3, s-OPA1#4 and/or s-OPA1#5. These findings were demonstrated by a pulse-chase experiment in which HeLa cells were grown under standard growth conditions in 500 µl DMEM w/o methionine/cysteine for 30 min (starvation), then pulse-labelled by the addition of 50 µCi ³⁵[S]-methionine/cysteine (5 µl) for 2.5 hours. Then cells were washed 3 times with PBS and 1 ml fresh DMEM medium + 10 %FCS without radioactive methione (chase) was added. Cells were either treated with 20 µM CCCP for 1 hour or not. OPA1 was immunoprecipitated using an antibody raised against the C-terminus of OPA1 and OPA1 was detected by autoradiography and by western-blotting after SDS-PAGE. By both methods a shift from the large isoforms to the short isoforms was detected upon treatment with CCCP (Fig. 11). Accordingly, it can be concluded that I-OPA1 isoforms are actually converted to s-OPA1 isofoms, in particular towards s-OPA1#5.

**Example 11: Determination of the apparent molecular weight of OPA1 isoforms by 10% SDS-PAGE**

Mitochondria were prepared from HeLa cells by standard methods using differential centrifugation. For this purpose, cells were, for example, harvested, washed in phosphate-buffered saline supplemented with 5 mM EDTA, and resuspended in 1 ml of RSB buffer (10 mM HEPES (pH 7.5), 1 mM EDTA, 210 mM mannitol, 70 mM sucrose, supplemented with complete protease inhibitor mixture). Mitochondria were prepared after disruption of HeLa cells by passing 6 times through a 26G needle fitted to a 5 ml syringe applying a method adapted from Arnoult, 2005, J Biol Chem, 280(42), 35742-50. Cell pellets from low speed centrifugations (2,000 X g, 10 min, 4 °C) were resuspended in RSB buffer and passaged again through a needle as described. This step was repeated 3 to 4 times. The supernatants from low speed centrifugations were pooled and centrifuged again (13,000 X g, 10 min, 4 °C) to obtain a crude mitochondrial pellet and a cytosolic supernatant. Three aliquots of a protein extract (24 µg each) from isolated HeLa mitochondria were separated by 10% SDS-PAGE as described above (see example 2 and Fig.2). Three different molecular weight markers were loaded next to these (BioRad, Germany, Precision Plus Protein Standard All Blue, #1610373; PeqLab, Germany, Protein MW marker, #27-1010; Sigma, Germany, HMW marker, #M3788). Five OPA1 isoforms with apparent average molecular weights of 96.8 kD, 92.3 kD, 88.1 kD, 84.4 kD, and 80.9 kD were observed (Fig.2). The molecular weight was determined by logarithmic regressions analysis of the migration length all marker bands with known molecular weights, determining the migration length of all OPA1 isoforms, and subsequently calculating the apparent molecular weight in kD. The bands larger than 91 kD are defined as large OPA1 isoforms ((I-)OPA1#1 and (I-)OPA1#2). OPA1 isoforms with apparent molecular weight smaller or equal than 91 kD are defined as small OPA1 isoforms ((s-)OPA1#3, (s-)OPA1#4 and (s-)OPA1#5).

The present invention refers to the following nucleotide and amino acid sequences:
SEQ ID No. 1:
   Nucleotide sequence encoding OPA1 spliceform 8 (NM_130837; CDS 56-3103)
SEQ ID No. 2:
   Amino acid sequence of OPA1 spliceform 8 (NP_570850; 1015 aa)
SEQ ID No. 3:
   Nucleotide sequence encoding OPA1 spliceform 7 (NM_130836; CDS 56-3049)
SEQ ID No. 4:
   Amino acid sequence of OPA1 spliceform 7 (NP_570849; 997 aa)
SEQ ID No. 5:
   Nucleotide sequence encoding OPA1 spliceform 6 (NM_130835; CDS 56-2995)
SEQ ID No. 6:
   Amino acid sequence of OPA1 spliceform 6 (NP_570848; 979 aa)
SEQ ID No. 7:
   Nucleotide sequence encoding OPA1 spliceform 5 (NM_130834; CDS 56-2992)
SEQ ID No. 8:
   Amino acid sequence of OPA1 spliceform 5 (NP_570847; 978 aa)
SEQ ID No. 9:
   Nucleotide sequence encoding OPA1 spliceform 4 (NM_130833; CDS 56-2941)
SEQ ID No. 10:
   Amino acid sequence of OPA1 spliceform 4 (NP_570846; 961 aa)
SEQ ID No. 11:
   Nucleotide sequence encoding OPA1 spliceform 3 (NM_130832; CDS 56-2884)
SEQ ID No. 12:
   Amino acid sequence of OPA1 spliceform 3 (NP_570845; 942 aa)
SEQ ID No. 13:
   Nucleotide sequence encoding OPA1 spliceform 2 (NM_130831; CDS 56-2830)
SEQ ID No. 14:
   Amino acid sequence of OPA1 spliceform 2 (NP_570844; 924 aa)
SEQ ID No. 15:
   Nucleotide sequence encoding OPA1 spliceform 1 (BC075805; CDS 155-3037)
SEQ ID No. 16:
   Amino acid sequence of OPA1 spliceform 1 (AAH75805; 960 aa)

## Claims

1. A method for determining the susceptibility for, predisposition for or the presence of a disorder correlated with mitochondrial dysfunction or mitochondrial disease, comprising the in vitro determination selected from the group consisting of
(a) the in vitro determination of the ratio of at least one large and at least one small isoform of the optic atrophy 1 protein (OPA1), whereby said large isoform has an apparent molecular weight of more than 91 kD and whereby said small isoform has an apparent molecular weight of less than 91 kD, said molecular weights being determined by SDS-PAGE analysis; and/or
whereby said large isoform has an apparent molecular weight of more than 95 kD and whereby said small isoform has an apparent molecular weight of less than 95 kD, said molecular weights being determined by mass spectrometry;
(b) the in vitro determination of (a) wherein the ratio of at least two OPA1 isoforms is determined; and
(c) the in vitro determination of (a) comprising the step of determining the ratio between at least one large OPA1 isoform and an internal standard and between at least one small OPA1 isoform and an internal standard, wherein a decrease of the large OPA1 isoforms accompanied by an increase of the small OPA1 isoforms is diagnostic for said disorder or disease.

2. The method of claim 1, wherein said large isoforms comprise two isoforms (OPA1-L1 and OPA1-L2) and wherein said small isoforms comprise three isoforms (OPA1-S3, OPA1-S4 and OPA1-S5).

3. The method of claim 2, wherein said large isoforms comprise an isoform having an apparent molecular weight of 97 kD (OPA1-L1) or an isoform having an apparent molecular weight of 92 kD (OPA1-L2), said molecular weights being determined by SDS-PAGE analysis.

4. The method of claim 2, wherein said small isoforms comprise an isoform having an apparent molecular weight of 88 kD (OPA1-S3), an isoform having an apparent molecular weight of 84 kD (OPA1-S4) or an isoform having an apparent molecular weight of 81 kD (OPA1-S5), said molecular weights being determined by SDS-PAGE analysis.

5. The method of claim 2, wherein said large isoforms comprise an isoform having an apparent molecular weight of 104 kD (OPA1-L1) or an isoform having an apparent molecular weight of 99 kD (OPA1-L2), said molecular weights being determined by mass spectrometry.

6. The method of claim 2, wherein said small isoforms comprise an isoform having an apparent molecular weight of 92 kD (OPA1-S3), an isoform having an apparent molecular weight of 89 kD (OPA1-S4) or an isoform having an apparent molecular weight of 87 kD (OPA1-S5), said molecular weights being determined by mass spectrometry.

7. The method of anyone of claims 2 to 6, wherein
said OPA1-L1 has an apparent molecular weight of 97 kD,
said OPA1-L2 has an apparent molecular weight of 92 kD,
said OPA1-S3 has an apparent molecular weight of 88 kD,
said OPA1-S4 has an apparent molecular weight of 84 kD, and/or
said OPA1-S5 has an apparent molecular weight of 81 kD,
said molecular weights being determined by SDS-PAGE analysis; or
wherein
said OPA1-L1 has an apparent molecular weight of 104 kD,
said OPA1-L2 has an apparent molecular weight of 99 kD,
said OPA1-S3 has an apparent molecular weight of 92 kD,
said OPA1-S4 has an apparent molecular weight of 89 kD, and/or
said OPA1-S5 has an apparent molecular weight of 87 kD,
said molecular weights being determined by mass spectrometry.

8. The method of any one of claims 1 to 7, wherein said large isoform(s) is (are) OPA1-L1 and/or OPA1-L2.

9. The method of any one of claims 1 to 7, wherein said small isoform(s) is (are) OPA1-S3, OPA1-S4 and/or OPA1-S5.

10. The method of any one of claims 2 to 9, wherein
(a) OPA1-L1 and OPA1-L2 are **characterized by** comprising amino acid stretches or amino acid peptides comprising one or more of the following sequences:
YLILGSAVGGGYTAK (SEQ ID No: 17);
TFDQWK (SEQ ID No: 18);
DMIPDLSEYK (SEQ ID No: 19);
WIVPDIVWEIDEYIDFEK (SEQ ID No: 20);
LAPDFDK (SEQ ID No: 21);
IVESLSLLK (SEQ ID No: 22);
ALPNSEDLVK (SEQ ID No: 23);
DFFTSGSPEETAFR (SEQ ID No: 24);
TRLLKLRYLILGS (SEQ ID No: 25); and
FWPARLATRLLKLRYLILGS (SEQ ID NO: 111).
(b) OPA1-S3 is **characterized by** comprising amino acid stretches or amino acid peptides comprising one or more of the following sequences:
IVESLSLLK (SEQ ID No: 22);
DFFTSGSPEETAFR (SEQ ID No: 24);
GLLGELILLQQQIQEHEEEAR (SEQ ID No: 26);
AAGQYSTSYAQQK (SEQ ID No: 27); and
IDQLQEELLHTQLK (SEQ ID No: 28).
(c) OPA1-S4 is **characterized by** comprising amino acid stretches or amino acid peptides comprising one or more of the following sequences:
GLLGELILLQQQIQEHEEEAR (SEQ ID No: 26);
AAGQYSTSYAQQK (SEQ ID No: 27); and
IDQLQEELLHTQLK (SEQ ID No: 28),
and/or
(d) OPA1-S5 is **characterized by** comprising amino acid stretches or amino acid peptides comprising the following sequence:
IDQLQEELLHTQLK (SEQ ID No: 28).

11. The method of any one of claims 2 to 10, wherein
(a) OPA1-L2 is **characterized by** not comprising amino acid stretches or amino acid peptides comprising one or more of the following sequences:
GLLGELILLQQQIQEHEEEAR (SEQ ID No: 26); and
AAGQYSTSYAQQK (SEQ ID No: 27),
and/or
(b) OPA1-S3 is **characterized by** not comprising amino acid stretches or amino acid peptides comprising one or more of the following sequences:
YLILGSAVGGGYTAK (SEQ ID No: 17);
TFDQWK (SEQ ID No: 18);
DMIPDLSEYK (SEQ ID No: 19);
WIVPDIVWEIDEYIDFEK (SEQ ID No: 20);
LAPDFDK (SEQ ID No: 21);
ALPNSEDLVK (SEQ ID No: 23);
FWPARLATRLLKLRYLILGS (SEQ ID NO: 111); and
TRLLKLRYLILGS (SEQ ID No: 25),
(c) OPA1-S4 is **characterized by** not comprising amino acid stretches or amino acid peptides comprising one or more of the following sequences:
YLILGSAVGGGYTAK (SEQ ID No: 17);
TFDQWK (SEQ ID No: 18);
DMIPDLSEYK (SEQ ID No: 19);
WIVPDIVWEIDEYIDFEK (SEQ ID No: 20);
LAPDFDK (SEQ ID No: 21);
IVESLSLLK (SEQ ID No: 22);
ALPNSEDLVK (SEQ ID No: 23);
DFFTSGSPEETAFR (SEQ ID No: 24);
FWPARLATRLLKLRYLILGS (SEQ ID NO: 111); and
TRLLKLRYLILGS (SEQ ID No: 25),
and/or
(d) OPA1-S5 is **characterized by** not comprising amino acid stretches or amino acid peptides comprising one or more of the following sequences:
YLILGSAVGGGYTAK (SEQ ID No: 17);
TFDQWK (SEQ ID No: 18);
DMIPDLSEYK (SEQ ID No: 19);
WIVPDIVWEIDEYIDFEK (SEQ ID No: 20);
LAPDFDK (SEQ ID No: 21);
IVESLSLLK (SEQ ID No: 22);
ALPNSEDLVK (SEQ ID No: 23);
DFFTSGSPEETAFR (SEQ ID No: 24);
TRLLKLRYLILGS (SEQ ID No: 25);
GLLGELILLQQQIQEHEEEAR (SEQ ID No: 26); and
AAGQYSTSYAQQK (SEQ ID No: 27).

12. The method of claims 1 to 11, wherein said determination of the ratio of OPA1 isoforms comprises
(a) the determination of the ratio of at least one large OPA1 isoform to at least one small OPA1 isoform;
(b) the determination of the ratio of at least one small OPA1 isoform to at least one large OPA1 isoform;
(c) the determination of the ratio of one OPA1 isoform to five or more OPA1 isoforms;
(d) the determination of the ratio of one OPA1 isoform to four OPA1 isoforms;
(e) the determination of the ratio of two OPA1 isoforms to three OPA1 isoforms;
(f) the determination of the ratio of three OPA1 isoforms to two OPA1 isoforms;
(g) the determination of the ratio of four OPA1 isoforms to one OPA1 isoforms; or
(h) the determination of the ratio of five or more OPA1 isoforms to one OPA1 isoforms.

13. The method of claims 1 to 12, whereby the ratio between at least one large OPA1 isoform and at least one small isoform is determined.

14. The method of claims 1 to 13, wherein the ratio between at least one small OPA1 isoform and
(a) five or more OPA1 isoforms;
(b) at least four OPA1 isoforms;
(c) at least three OPA1 isoforms;
(d) at least two OPA1 isoforms; or
(e) at least one OPA1 isoforms is determined.

15. The method of claim 14, wherein the ratio between the small OPA1-S5 as defined in any one of claims 4, 6, 7, 10 or 11 and at least one other OPA1 isoform as defined in any one of claims 3 to 7 and 10 or 11 is determined.

16. The method of claim 14 or 15, wherein the ratio between the small OPA1-S5 as defined in any one of claims 4, 6, 7, 10 or 11 and all five OPA1 isoforms as defined in any one of claims 3 to 7 and 10 or 11 is determined.

17. The method of any one of claims 1 to 16, wherein said method for determining the susceptibility, predisposition or the presence of a disorder correlated with mitochondrial dysfunction or disease, comprising the in vitro determination of the ratio of large and small isoforms of OPA1 in a given sample comprises a comparison of the obtained data/values to data/values obtained from a healthy control/standard and/or a comparison of the obtained data/values to data/values obtained from a known diseased control/standard and/or a comparison of the obtained data/values to data/values obtained for an internal standard.

18. The method of claims 1 to 11 and 17, wherein said internal standard is β-actin.

19. The method of any one of claims 2 to 18, said method comprising the steps of determing in a sample the ratio of at least two OPA1 isoforms selected from the group consisting of OPA1-L1, -L2,- S3, -S4 and -S5,
wherein said ratio of said isoforms is compared to the corresponding isoforms of a healthy control or normal standard.

20. The method of any one of claims 1 to 19, wherein said disorder is selected from the group consisting of premature ageing, cardiomyopathy, a respiratory chain disorder, mtDNA depletion syndrome, myoclonus epilepsy, ragged-red fibers syndrome (MERRF), myopathy encephalopathy lactic acidosis, stroke-like episodes (MELAS) and optic atrophy.

## Patentansprüche

1. Verfahren zur Bestimmung der Empfänglichkeit für, Prädisposition für oder des Vorhandenseins eine(r) Störung, die mit einer mitochondrialen Dysfunktion oder einer mitochondrialen Erkrankung korreliert, umfassend die in vitro-Bestimmung, die ausgewählt ist aus der Gruppe bestehend aus
(a) der in vitro-Bestimmung des Verhältnisses von mindestens einer großen und mindestens einer kleinen Isoform des Optikusatrophie 1-Proteins (OPA1), wobei die große Isoform ein scheinbares Molekulargewicht von mehr als 91 kD hat und wobei die kleine Isoform ein scheinbares Molekulargewicht von weniger als 91 kD hat, wobei die Molekulargewichte durch SDS-PAGE-Analyse bestimmt werden; und/oder wobei die große Isoform ein scheinbares Molekulargewicht von mehr als 95 kD hat und wobei die kleine Isoform ein scheinbares Molekulargewicht von weniger als 95 kD hat, wobei die Molekulargewichte durch Massenspektrometrie bestimmt werden;
(b) der in vitro-Bestimmung von (a) wobei das Verhältnis von mindestens zwei OPA1-Isoformen bestimmt wird; und
(c) der in vitro-Bestimmung von (a), umfassend den Schritt der Bestimmung des Verhältnisses zwischen mindestens einer großen OPA1-Isoform und einem internen Standard und zwischen mindestens einer kleinen OPA1-Isoform und einem internen Standard,
wobei eine Abnahme der großen OPA1-Isoformen begleitet von einer Zunahme der kleinen OPA1-Isoformen diagnostisch für die Störung oder Erkrankung ist.

2. Verfahren nach Anspruch 1, wobei die großen Isoformen zwei Isoformen (OPA1-L1 und OPA1-L2) umfassen und wobei die kleinen Isoformen drei Isoformen (OPA1-S3, OPA1-S4 und OPA1-S5) umfassen.

3. Verfahren nach Anspruch 2, wobei die großen Isoformen eine Isoform mit einem scheinbaren Molekulargewicht von 97 kD (OPA1-L1) oder eine Isoform mit einem scheinbaren Molekulargewicht von 92 kD (OPA1-L2) umfassen, wobei die Molekulargewichte durch SDS-PAGE-Analyse bestimmt werden.

4. Verfahren nach Anspruch 2, wobei die kleinen Isoformen eine Isoform mit einem scheinbaren Molekulargewicht von 88 kD (OPA1-S3), eine Isoform mit einem scheinbaren Molekulargewicht von 84 kD (OPA1-S4) oder eine Isoform mit einem scheinbaren Molekulargewicht von 81 kD (OPA1-S5) umfassen, wobei die Molekulargewichte durch SDS-PAGE-Analyse bestimmt werden.

5. Verfahren nach Anspruch 2, wobei die großen Isoformen eine Isoform mit einem scheinbaren Molekulargewicht von 104 kD (OPA1-L1) oder eine Isoform mit einem scheinbaren Molekulargewicht von 99 kD (OPA1-L2) umfassen, wobei die Molekulargewichte durch Massenspektrometrie bestimmt werden.

6. Verfahren nach Anspruch 2, wobei die kleinen Isoformen eine Isoform mit einem scheinbaren Molekulargewicht von 92 kD (OPA1-S3), eine Isoform mit einem scheinbaren Molekulargewicht von 89 kD (OPA1-S4) oder eine Isoform mit einem scheinbaren Molekulargewicht von 87 kD (OPA1-S5) umfassen, wobei die Molekulargewichte durch Massenspektrometrie bestimmt werden.

7. Verfahren nach einem der Ansprüche 2 bis 6, wobei
das OPA1-L1 ein scheinbares Molekulargewicht von 97 kD hat,
das OPA1-L2 ein scheinbares Molekulargewicht von 92 kD hat,
das OPA1-S3 ein scheinbares Molekulargewicht von 88 kD hat,
das OPA1-S4 hat ein scheinbares Molekulargewicht von 84 kD hat, und/oder das OPA1-S5 ein scheinbares Molekulargewicht von 81 kD hat,
wobei die Molekulargewichte durch SDS-PAGE-Analyse bestimmt werden; oder
wobei
das OPA1-L1 ein scheinbares Molekulargewicht von 104 kD hat,
das OPA1-L2 ein scheinbares Molekulargewicht von 99 kD hat,
das OPA1-S3 ein scheinbares Molekulargewicht von 92 kD hat,
das OPA1-S4 ein scheinbares Molekulargewicht von 89 kD hat, und/oder das OPA1-S5 ein scheinbares Molekulargewicht von 87 kD hat,
wobei die Molekulargewichte durch Massenspektrometrie bestimmt werden.

8. Verfahren nach einem der Ansprüche 1 bis 7, wobei die große(n) Isoform(en) OPA1-L1 und/oder OPA1-L2 ist (sind).

9. Verfahren nach einem der Ansprüche 1 bis 7, wobei die kleinen Isoform(en) OPA1-S3, OPA1-S4 und/oder OPA1-S5 ist (sind).

10. Verfahren nach einem der Ansprüche 2 bis 9, wobei
(a) OPA1-L1 und OPA1-L2 **dadurch gekennzeichnet sind, dass** sie Aminosäureabschnitte oder Aminosäurepeptide umfassen, welche eine oder mehrere der folgenden Sequenzen umfassen:
YLILGSAVGGGYTAK (SEQ ID No: 17);
TFDQWK (SEQ ID No: 18);
DMIPDLSEYK (SEQ ID No: 19);
WIVPDIVWEIDEYIDFEK (SEQ ID No: 20);
LAPDFDK (SEQ ID No: 21);
IVESLSLLK (SEQ ID No: 22);
ALPNSEDLVK (SEQ ID No: 23);
DFFTSGSPEETAFR (SEQ ID No: 24);
TRLLKLRYLILGS (SEQ ID No: 25); und
FWPARLATRLLKLRYLILGS (SEQ ID NO: 111),
(b) OPA1-S3 **dadurch gekennzeichnet ist, dass** es Aminosäureabschnitte oder Aminosäurepeptide umfasst, welche eine oder mehrere der folgenden Sequenzen umfassen:
IVESLSLLK (SEQ ID No: 22);
DFFTSGSPEETAFR (SEQ ID No: 24);
GLLGELILLQQQIQEHEEEAR (SEQ ID No: 26);
AAGQYSTSYAQQK (SEQ ID No: 27); und
IDQLQEELLHTQLK (SEQ ID No: 28),
(c) OPA1-S4 **dadurch gekennzeichnet ist, dass** es Aminosäureabschnitte oder Aminosäurepeptide umfasst, welche eine oder mehrere der folgenden Sequenzen umfassen:
GLLGELILLQQQIQEHEEEAR (SEQ ID No: 26);
AAGQYSTSYAQQK (SEQ ID No: 27); und
IDQLQEELLHTQLK (SEQ ID No: 28),
und/oder
(d) OPA1-S5 **dadurch gekennzeichnet ist, dass** es Aminosäureabschnitte oder Aminosäurepeptide umfasst, welche die folgende Sequenz umfassen:
IDQLQEELLHTQLK (SEQ ID No: 28).

11. Verfahren nach einem der Ansprüche 2 bis 10, wobei
(a) OPA1-L2 **dadurch gekennzeichnet ist, dass** es keine Aminosäureabschnitte oder Aminosäurepeptide umfasst, welche eine oder mehrere der folgenden Sequenzen umfassen:
GLLGELILLQQQIQEHEEEAR (SEQ ID No: 26); und
AAGQYSTSYAQQK (SEQ ID No: 27);
und/oder
(b) OPA1-S3 **dadurch gekennzeichnet ist, dass** es keine Aminosäureabschnitte oder Aminosäurepeptide umfasst, welche eine oder mehrere der folgenden Sequenzen umfassen:
YLILGSAVGGGYTAK (SEQ ID No: 17);
TFDQWK (SEQ ID No: 18);
DMIPDLSEYK (SEQ ID No: 19);
WIVPDIVWEIDEYIDFEK (SEQ ID No: 20);
LAPDFDK (SEQ ID No: 21);
ALPNSEDLVK (SEQ ID No: 23);
FWPARLATRLLKLRYLILGS (SEQ ID NO: 111); und
TRLLKLRYLILGS (SEQ ID No: 25),
(c) OPA1-S4 **dadurch gekennzeichnet ist, dass** es keine Aminosäureabschnitte oder Aminosäurepeptide umfasst, welche eine oder mehrere der folgenden Sequenzen umfassen:
YLILGSAVGGGYTAK (SEQ ID No: 17);
TFDQWK (SEQ ID No: 18);
DMIPDLSEYK (SEQ ID No: 19);
WIVPDIVWEIDEYIDFEK (SEQ ID No: 20);
LAPDFDK (SEQ ID No: 21);
IVESLSLLK (SEQ ID No: 22);
ALPNSEDLVK (SEQ ID No: 23);
DFFTSGSPEETAFR (SEQ ID No: 24);
FWPARLATRLLKLRYLILGS (SEQ ID NO: 111); und
TRLLKLRYLILGS (SEQ ID No: 25),
und/oder
(d) OPA1-S5 **dadurch gekennzeichnet ist, dass** es keine Aminosäureabschnitte oder Aminosäurepeptide umfasst, welche eine oder mehrere der folgenden Sequenzen umfassen:
YLILGSAVGGGYTAK (SEQ ID No: 17);
TFDQWK (SEQ ID No: 18);
DMIPDLSEYK (SEQ ID No: 19);
WIVPDIVWEIDEYIDFEK (SEQ ID No: 20);
LAPDFDK (SEQ ID No: 21);
IVESLSLLK (SEQ ID No: 22);
ALPNSEDLVK (SEQ ID No: 23);
DFFTSGSPEETAFR (SEQ ID No: 24);
TRLLKLRYLILGS (SEQ ID No: 25);
GLLGELILLQQQIQEHEEEAR (SEQ ID No: 26); und
AAGQYSTSYAQQK (SEQ ID No: 27).

12. Verfahren nach den Ansprüchen 1 bis 11, wobei die Bestimmung des Verhältnisses von OPA1-Isoformen umfasst
(a) die Bestimmung des Verhältnisses von mindestens einer großen OPA1-Isoform zu mindestens einer kleinen OPA1-Isoform;
(b) die Bestimmung des Verhältnisses von mindestens einer kleinen OPA1-Isoform zu mindestens einer großen OPA1-Isoform;
(c) die Bestimmung des Verhältnisses von einer OPA1-Isoform zu fünf oder mehr OPA1-Isoformen;
(d) die Bestimmung des Verhältnisses von einer OPA1-Isoform zu vier OPA1-Isoformen;
(e) die Bestimmung des Verhältnisses von zwei OPA1-Isoformen zu drei OPA1-Isoformen;
(f) die Bestimmung des Verhältnisses von drei OPA1-Isoformen zu zwei OPA1-Isoformen;
(g) die Bestimmung des Verhältnisses von vier OPA1-Isoformen zu einer OPA1-Isoform; oder
(h) die Bestimmung des Verhältnisses von fünf oder mehr OPA1-Isoformen zu einer OPA1-Isoform.

13. Verfahren nach den Ansprüchen 1 bis 12, wobei das Verhältnis zwischen mindestens einer großen OPA1-Isoform und mindestens einer kleinen OPA1-Isoform bestimmt wird.

14. Verfahren nach den Ansprüchen 1 bis 13, wobei das Verhältnis zwischen mindestens einer kleinen OPA1-Isoform und
(a) fünf oder mehr OPA1-Isoformen;
(b) mindestens vier OPA1-Isoformen;
(c) mindestens drei OPA1-Isoformen;
(d) mindestens zwei OPA1-Isoformen; oder
(e) mindestens einer OPA1-Isoform bestimmt wird.

15. Verfahren nach Anspruch 14, wobei das Verhältnis zwischen der kleinen OPA1-S5, wie in einem der Ansprüche 4, 6, 7, 10 oder 11 definiert, und mindestens einer anderen OPA1-Isoform, wie in einem der Ansprüche 3 bis 7 und 10 oder 11 definiert, bestimmt wird.

16. Verfahren nach Anspruch 14 oder 15, wobei das Verhältnis zwischen der kleinen OPA1-S5, wie in einem der Ansprüche 4, 6, 7, 10 oder 11 definiert, und allen fünf OPA1-Isoformen, wie in einem der Ansprüche 3 bis 7 und 10 oder 11 definiert, bestimmt wird.

17. Verfahren nach einem der Ansprüche 1 bis 16, wobei das Verfahren zur Bestimmung der Empfänglichkeit für, Prädisposition für oder des Vorhandenseins eine(r) Störung, die mit mitochondrialer Dysfunktion oder Erkrankung korreliert, umfassend die in vitro-Bestimmung des Verhältnisses von großen und kleinen OPA1-Isoformen in einer gegebenen Probe, einen Vergleich der erhaltenen Daten/Werte, mit Daten/Werten, die von einer/einem gesunden Kontrolle/Standard erhalten wurden, und/oder einen Vergleich der erhaltenen Daten/Werte mit Daten/Werten, welche von einer/einem bekannten erkrankten Kontrolle/Standard erhalten wurden, und/oder einen Vergleich der erhaltenen Daten/Werte mit Daten/Werten, welche für einen internen Standard erhalten wurden, umfasst.

18. Verfahren nach den Ansprüchen 1 bis 11 und 17, wobei der interne Standard β-Aktin ist.

19. Verfahren nach einem der Ansprüche 2 bis 18, wobei das Verfahren die Schritte des Bestimmens in einer Probe des Verhältnisses von mindestens zwei OPA1-Isoformen umfasst, die ausgewählt sind aus der Gruppe bestehend aus OPA1-L1, -L2, -S3, -S4 und -S5,
wobei das Verhältnis der Isoformen mit den entsprechenden Isoformen einer gesunden Kontrolle oder eines normalen Standards verglichen wird.

20. Verfahren nach einem der Ansprüche 1 bis 19, wobei die Störung ausgewählt ist aus der Gruppe bestehend aus vorzeitiger Alterung, Kardiomyopathie, einer Störung der Atmungskette, mtDNA-Depletionssyndrom, Myoklonusepilepsie, Ragged-Red-Fasern-Syndrom (MERRF), Myopathie-Enzephalopathie-Laktatazidose, Schlaganfall-ähnliche Episoden (MELAS) und Optikusatrophie.

## Revendications

1. Procédé de détermination de la sensibilité à, de la prédisposition à ou de la présence d'un trouble corrélé avec un dysfonctionnement mitochondrial ou une maladie mitochondriale, comprenant la détermination *in vitro* choisie dans le groupe constitué par
(a) la détermination *in vitro* du rapport d'au moins une grande et d'au moins une petite isoforme de la protéine de l'atrophie optique 1 (OPA1), moyennant quoi ladite grande isoforme a une masse moléculaire apparente supérieure à 91 kD et moyennant quoi ladite petite isoforme a une masse moléculaire apparente inférieure à 91 kD, lesdites masses moléculaires étant déterminées par analyse SDS-PAGE ; et/ou moyennant quoi ladite grande isoforme a une masse moléculaire apparente supérieure à 95 kD et moyennant quoi ladite petite isoforme a une masse moléculaire apparente inférieure à 95 kD, lesdites masses moléculaires étant déterminées par spectrométrie de masse ;
(b) la détermination *in vitro* de (a) où le rapport d'au moins deux isoformes de l'OPA1 est déterminé ; et
(c) la détermination *in vitro* de (a) comprenant l'étape consistant à déterminer le rapport entre au moins une grande isoforme de l'OPA1 et un étalon interne et entre au moins une petite isoforme de l'OPA1 et un étalon interne,
dans lequel une diminution des grandes isoformes de l'OPA1 accompagnée d'une augmentation des petites isoformes de l'OPA1 représente un diagnostic pour ledit trouble ou ladite maladie.

2. Procédé selon la revendication 1, dans lequel lesdites grandes isoformes comprennent deux isoformes (OPA1-L1 et OPA1-L2) et dans lequel lesdites petites isoformes comprennent trois isoformes (OPA1-S3, OPA1-S4 et OPA1-S5).

3. Procédé selon la revendication 2, dans lequel lesdites grandes isoformes comprennent une isoforme ayant une masse moléculaire apparente de 97 kD (OPA1-L1) ou une isoforme ayant une masse moléculaire apparente de 92 kD (OPA1-L2), lesdites masses moléculaires étant déterminées par analyse SDS-PAGE.

4. Procédé selon la revendication 2, dans lequel lesdites petites isoformes comprennent une isoforme ayant une masse moléculaire apparente de 88 kD (OPA1-S3), une isoforme ayant une masse moléculaire apparente de 84 kD (OPA1-S4) ou une isoforme ayant une masse moléculaire apparente de 81 kD (OPA1-S5), lesdites masses moléculaires étant déterminées par analyse SDS-PAGE.

5. Procédé selon la revendication 2, dans lequel lesdites grandes isoformes comprennent une isoforme ayant une masse moléculaire apparente de 104 kD (OPA1-L1) ou une isoforme ayant une masse moléculaire apparente de 99 kD (OPA1-L2), lesdites masses moléculaires étant déterminées par spectrométrie de masse.

6. Procédé selon la revendication 2, dans lequel lesdites petites isoformes comprennent une isoforme ayant une masse moléculaire apparente de 92 kD (OPA1-S3), une isoforme ayant une masse moléculaire apparente de 89 kD (OPA1-S4) ou une isoforme ayant une masse moléculaire apparente de 87 kD (OPA1-S5), lesdites masses moléculaires étant déterminées par spectrométrie de masse.

7. Procédé selon l'une quelconque des revendications 2 à 6, dans lequel ladite OPA1-L1 a une masse moléculaire apparente de 97 kD,
ladite OPA1-L2 a une masse moléculaire apparente de 92 kD,
ladite OPA1-S3 a une masse moléculaire apparente de 88 kD,
ladite OPA1-S4 a une masse moléculaire apparente de 84 kD, et/ou ladite OPA1-S5 a une masse moléculaire apparente de 81 kD,
lesdites masses moléculaires étant déterminées par analyse SDS-PAGE ; ou dans lequel
ladite OPA1-L1 a une masse moléculaire apparente de 104 kD,
ladite OPA1-L2 a une masse moléculaire apparente de 99 kD,
ladite OPA1-S3 a une masse moléculaire apparente de 92 kD,
ladite OPA1-S4 a une masse moléculaire apparente de 89 kD, et/ou ladite OPA1-S5 a une masse moléculaire apparente de 87 kD,
lesdites masses moléculaires étant déterminées par spectrométrie de masse.

8. Procédé selon l'une quelconque des revendications 1 à 7, dans lequel ladite/lesdites grande(s) isoforme(s) est/sont l'OPA1-L1 et/ou l'OPA1-L2.

9. Procédé selon l'une quelconque des revendications 1 à 7, dans lequel ladite/lesdites petite(s) isoforme(s) est/sont l'OPA1-S3, l'OPA1-S4 et/ou l'OPA1-S5.

10. Procédé selon l'une quelconque des revendications 2 à 9, dans lequel
(a) l'OPA1-L1 et l'OPA1-L2 sont **caractérisées en ce qu'**elles comprennent des suites d'acides aminés ou des peptides d'acides aminés comprenant une ou plusieurs des séquences suivantes :
YLILGSAVGGGYTAK (SEQ ID NO : 17) ;
TFDQWK (SEQ ID NO : 18) ;
DMIPDLSEYK (SEQ ID NO : 19) ;
WIVPDIVWEIDEYIDFEK (SEQ ID NO : 20) ;
LAPDFDK (SEQ ID NO : 21) ;
IVESLSLLK (SEQ ID NO : 22) ;
ALPNSEDLVK (SEQ ID NO : 23) ;
DFFTSGSPEETAFR (SEQ ID NO : 24) ;
TRLLKLRYLILGS (SEQ ID NO : 25) ; et
FWPARLATRLLKLRYLILGS (SEQ ID NO : 111),
(b) l'OPA1-S3 est **caractérisée en ce qu'**elle comprend des suites d'acides aminés ou des peptides d'acides aminés comprenant une ou plusieurs des séquences suivantes :
IVESLSLLK (SEQ ID NO : 22) ;
DFFTSGSPEETAFR (SEQ ID NO : 24) ;
GLLGELILLQQQIQEHEEEAR (SEQ ID NO : 26) ;
AAGQYSTSYAQQK (SEQ ID NO : 27) ; et
IDQLQEELLHTQLK (SEQ ID NO : 28),
(c) l'OPA1-S4 est **caractérisée en ce qu'**elle comprend des suites d'acides aminés ou des peptides d'acides aminés comprenant une ou plusieurs des séquences suivantes :
GLLGELILLQQQIQEHEEEAR (SEQ ID NO : 26) ;
AAGQYSTSYAQQK (SEQ ID NO : 27) ; et
IDQLQEELLHTQLK (SEQ ID NO : 28),
et/ou
(d) l'OPA1-S5 est **caractérisée en ce qu'**elle comprend des suites d'acides aminés ou des peptides d'acides aminés comprenant la séquence suivante :
IDQLQEELLHTQLK (SEQ ID NO : 28).

11. Procédé selon l'une quelconque des revendications 2 à 10, dans lequel
(a) l'OPA1-L2 est **caractérisée en ce qu'**elle ne comprend pas des suites d'acides aminés ou des peptides d'acides aminés comprenant une ou plusieurs des séquences suivantes :
GLLGELILLQQQIQEHEEEAR (SEQ ID NO : 26) ; et
AAGQYSTSYAQQK (SEQ ID NO : 27),
et/ou
(b) l'OPA1-S3 est **caractérisée en ce qu'**elle ne comprend pas des suites d'acides aminés ou des peptides d'acides aminés comprenant une ou plusieurs des séquences suivantes :
YLILGSAVGGGYTAK (SEQ ID NO : 17) ;
TFDQWK (SEQ ID NO : 18) ;
DMIPDLSEYK (SEQ ID NO : 19) ;
WIVPDIVWEIDEYIDFEK (SEQ ID NO : 20) ;
LAPDFDK (SEQ ID NO : 21) ;
ALPNSEDLVK (SEQ ID NO : 23) ;
FWPARLATRLLKLRYLILGS (SEQ ID NO : 111) ; et
TRLLKLRYLILGS (SEQ ID NO : 25),
(c) l'OPA1-S4 est **caractérisée en ce qu'**elle ne comprend pas des suites d'acides aminés ou des peptides d'acides aminés comprenant une ou plusieurs des séquences suivantes :
YLILGSAVGGGYTAK (SEQ ID NO : 17) ;
TFDQWK (SEQ ID NO : 18) ;
DMIPDLSEYK (SEQ ID NO : 19) ;
WIVPDIVWEIDEYIDFEK (SEQ ID NO : 20) ;
LAPDFDK (SEQ ID NO : 21);
IVESLSLLK (SEQ ID NO : 22) ;
ALPNSEDLVK (SEQ ID NO : 23) ;
DFFTSGSPEETAFR (SEQ ID NO : 24) ;
FWPARLATRLLKLRYLILGS (SEQ ID NO : 111) ; et
TRLLKLRYLILGS (SEQ ID NO : 25),
et/ou
(d) l'OPA1-S5 est **caractérisée en ce qu'**elle ne comprend pas des suites d'acides aminés ou des peptides d'acides aminés comprenant une ou plusieurs des séquences suivantes :
YLILGSAVGGGYTAK (SEQ ID NO : 17) ;
TFDQWK (SEQ ID NO : 18) ;
DMIPDLSEYK (SEQ ID NO : 19) ;
WIVPDIVWEIDEYIDFEK (SEQ ID NO : 20) ;
LAPDFDK (SEQ ID NO : 21);
IVESLSLLK (SEQ ID NO : 22) ;
ALPNSEDLVK (SEQ ID NO : 23) ;
DFFTSGSPEETAFR (SEQ ID NO : 24) ;
TRLLKLRYLILGS (SEQ ID NO : 25) ;
GLLGELILLQQQIQEHEEEAR (SEQ ID NO : 26) ; et
AAGQYSTSYAQQK (SEQ ID NO : 27).

12. Procédé selon les revendications 1 à 11, dans lequel ladite détermination du rapport des isoformes de l'OPA1 comprend
(a) la détermination du rapport d'au moins une grande isoforme de l'OPA1 sur au moins une petite isoforme de l'OPA1 ;
(b) la détermination du rapport d'au moins une petite isoforme de l'OPA1 sur au moins une grande isoforme de l'OPA1 ;
(c) la détermination du rapport d'une isoforme de l'OPA1 sur cinq isoformes de l'OPA1 ou plus;
(d) la détermination du rapport d'une isoforme de l'OPA1 sur quatre isoformes de l'OPA1 ;
(e) la détermination du rapport de deux isoforms de l'OPA1 sur trois isoformes de l'OPA1 ;
(f) la détermination du rapport de trois isoformes de l'OPA1 sur deux isoformes de l'OPA1 ;
(g) la détermination du rapport de quatre isoformes de l'OPA1 sur une isoforme de l'OPA1 ; ou
(h) la détermination du rapport de cinq isoformes de l'OPA1 ou plus sur une isoforme de l'OPA1.

13. Procédé selon les revendications 1 à 12, par lequel le rapport entre au moins une grande isoforme de l'OPA1 et au moins une petite isoforme est déterminé.

14. Procédé selon les revendications 1 à 13, dans lequel le rapport entre au moins une petite isoforme de l'OPA1 et
(a) cinq isoformes de l'OPA1 ou plus ;
(b) au moins quatre isoformes de l'OPA1 ;
(c) au moins trois isoformes de l'OPA1 ;
(d) au moins deux isoformes de l'OPA1 ; ou
(e) au moins une isoforme de l'OPA1, est déterminé.

15. Procédé selon la revendication 14, dans lequel le rapport entre la petite isoforme OPA1-S5 telle que définie dans l'une quelconque des revendications 4, 6, 7, 10 ou 11 et au moins une autre isoforme de l'OPA1 telle que définie dans l'une quelconque des revendications 3 à 7 et 10 ou 11 est déterminé.

16. Procédé selon la revendication 14 ou 15, dans lequel le rapport entre la petite isoforme OPA1-S5 telle que définie dans l'une quelconque des revendications 4, 6, 7, 10 ou 11 et la totalité des cinq isoformes de l'OPA1 telles que définies dans l'une quelconque des revendications 3 à 7 et 10 ou 11 est déterminé.

17. Procédé selon l'une quelconque des revendications 1 à 16, dans lequel ledit procédé de détermination de la sensibilité à, de la prédisposition à ou de la présence d'un trouble corrélé avec un dysfonctionnement ou une maladie mitochondriale, comprenant la détermination *in vitro* du rapport des grandes et des petites isoformes de l'OPA1 dans un échantillon donné comprend une comparaison des données/valeurs obtenues aux données/valeurs obtenues à partir d'un contrôle en bonne santé/étalon et/ou une comparaison des données/valeurs obtenues aux données/valeurs obtenues à partir d'un contrôle d'une maladie connue/étalon et/ou une comparaison des données/valeurs obtenues aux données/valeurs obtenues à partir d'un étalon interne.

18. Procédé selon les revendications 1 à 11 et 17, dans lequel ledit étalon interne est la
β-actine.

19. Procédé selon l'une quelconque des revendications 2 à 18, ledit procédé comprenant les étapes consistant à déterminer dans un échantillon le rapport d'au moins deux isoformes de l'OPA1 choisies dans le groupe constitué des OPA-L1, -L2, -S3, -S4 et -S5,
dans lequel ledit rapport desdites isoformes est comparé aux isoformes correspondantes d'un contrôle en bonne santé ou d'un étalon normal.

20. Procédé selon l'une quelconque des revendications 1 à 19, dans lequel ledit trouble est choisi dans le groupe constitué par un vieillissement prématuré, une cardiomyopathie, un trouble de la chaîne respiratoire, le syndrome de déplétion en ADNmt, l'épilepsie myoclonique, le syndrome des fibres rouges déchiquetées (MERRF), le syndrome MELAS (myopathy-encephalopathy-lactic acidosis- and stroke-like episodes) et l'atrophie optique.


### SEQUENCE LISTING

<110> Ludwig-Maximilians-Universität München
<120> Method for diagnosing mitochondrial dysfunction
<130> M1590 PCT S3
<160> 111
<170> PatentIn version 3.3
<210> 1
   <211> 3048
   <212> DNA
   <213> Homo sapiens
<220>
   <221> CDS
   <222> (1)..(3048)
<400> 1
   atg tgg cga cta cgt cgg gcc gct gtg gcc tgt gag gtc tgc cag tct      48
   Met Trp Arg Leu Arg Arg Ala Ala Val Ala Cys Glu Val Cys Gln Ser
   1               5                   10                  15
   tta gtg aaa cac agc tct gga ata aaa gga agt tta cca cta caa aaa      96
   Leu Val Lys His Ser Ser Gly Ile Lys Gly Ser Leu Pro Leu Gln Lys
               20                  25                  30
   cta cat ctg gtt tca cga agc att tat cat tca cat cat cct acc tta     144
   Leu His Leu Val Ser Arg Ser Ile Tyr His Ser His His Pro Thr Leu
           35                  40                  45
   aag ctt caa cga ccc caa tta agg aca tcc ttt cag cag ttc tct tct     192
   Lys Leu Gln Arg Pro Gln Leu Arg Thr Ser Phe Gln Gln Phe Ser Ser
       50                  55                  60
   ctg aca aac ctt cct tta cgt aaa ctg aaa ttc tct cca att aaa tat     240
   Leu Thr Asn Leu Pro Leu Arg Lys Leu Lys Phe Ser Pro Ile Lys Tyr
   65                  70                  75                  80
   ggc tac cag cct cgc agg aat ttt tgg cca gca aga tta gct acg aga     288
   Gly Tyr Gln Pro Arg Arg Asn Phe Trp Pro Ala Arg Leu Ala Thr Arg
                   85                  90                  95
   ctc tta aaa ctt cgc tat ctc ata cta gga tcg gct gtt ggg ggt ggc     336
   Leu Leu Lys Leu Arg Tyr Leu Ile Leu Gly Ser Ala Val Gly Gly Gly
               100                 105                 110
   tac aca gcc aaa aag act ttt gat cag tgg aaa gat atg ata ccg gac     384
   Tyr Thr Ala Lys Lys Thr Phe Asp Gln Trp Lys Asp Met Ile Pro Asp
           115                 120                 125
   ctt agt gaa tat aaa tgg att gtg cct gac att gtg tgg gaa att gat     432
   Leu Ser Glu Tyr Lys Trp Ile Val Pro Asp Ile Val Trp Glu Ile Asp
       130                 135                 140
   gag tat atc gat ttt gag aaa att aga aaa gcc ctt cct aat tca gaa     480
   Glu Tyr Ile Asp Phe Glu Lys Ile Arg Lys Ala Leu Pro Asn Ser Glu
   145                 150                 155                 160
   gac ctt gta aag tta gca cca gac ttt gac aag att gtt gaa agc ctt      528
   Asp Leu Val Lys Leu Ala Pro Asp Phe Asp Lys Ile Val Glu Ser Leu
                   165                 170                 175
   agc tta ttg aag gac ttt ttt acc tca ggt cac aaa ttg gtt agt gaa      576
   Ser Leu Leu Lys Asp Phe Phe Thr Ser Gly His Lys Leu Val Ser Glu
               180                 185                 190
   gtc ata gga gct tct gac cta ctt ctc ttg tta ggt tct ccg gaa gaa      624
   Val Ile Gly Ala Ser Asp Leu Leu Leu Leu Leu Gly Ser Pro Glu Glu
           195                 200                 205
   acg gcg ttt aga gca aca gat cgt gga tct gaa agt gac aag cat ttt      672
   Thr Ala Phe Arg Ala Thr Asp Arg Gly Ser Glu Ser Asp Lys His Phe
       210                 215                 220
   aga aag ggt ctg ctt ggt gag ctc att ctc tta caa caa caa att caa      720
   Arg Lys Gly Leu Leu Gly Glu Leu Ile Leu Leu Gln Gln Gln Ile Gln
   225                 230                 235                 240
   gag cat gaa gag gaa gcg cgc aga gcc gct ggc caa tat agc acg agc      768
   Glu His Glu Glu Glu Ala Arg Arg Ala Ala Gly Gln Tyr Ser Thr Ser
                   245                 250                 255
   tat gcc caa cag aag cgc aag gtg tca gac aaa gag aaa att gac caa      816
   Tyr Ala Gln Gln Lys Arg Lys Val Ser Asp Lys Glu Lys Ile Asp Gln
               260                 265                 270
   ctt cag gaa gaa ctt ctg cac act cag ttg aag tat cag aga atc ttg      864
   Leu Gln Glu Glu Leu Leu His Thr Gln Leu Lys Tyr Gln Arg Ile Leu
           275                 280                 285
   gaa cga tta gaa aag gag aac aaa gaa ttg aga aaa tta gta ttg cag      912
   Glu Arg Leu Glu Lys Glu Asn Lys Glu Leu Arg Lys Leu Val Leu Gln
       290                 295                 300
   aaa gat gac aaa ggc att cat cat aga aag ctt aag aaa tct ttg att      960
   Lys Asp Asp Lys Gly Ile His His Arg Lys Leu Lys Lys Ser Leu Ile
   305                 310                 315                 320
   gac atg tat tct gaa gtt ctt gat gtt ctc tct gat tat gat gcc agt     1008
   Asp Met Tyr Ser Glu Val Leu Asp Val Leu Ser Asp Tyr Asp Ala Ser
                   325                 330                 335
   tat aat acg caa gat cat ctg cca cgg gtt gtt gtg gtt gga gat cag     1056
   Tyr Asn Thr Gln Asp His Leu Pro Arg Val Val Val Val Gly Asp Gln
               340                 345                 350
   agt gct gga aag act agt gtg ttg gaa atg att gcc caa gct cga ata     1104
   Ser Ala Gly Lys Thr Ser Val Leu Glu Met Ile Ala Gln Ala Arg Ile
           355                 360                 365
   ttc cca aga gga tct ggg gag atg atg aca cgt tct cca gtt aag gtg     1152
   Phe Pro Arg Gly Ser Gly Glu Met Met Thr Arg Ser Pro Val Lys Val
       370                 375                 380
   act ctg agt gaa ggt cct cac cat gtg gcc cta ttt aaa gat agt tct     1200
   Thr Leu Ser Glu Gly Pro His His Val Ala Leu Phe Lys Asp Ser Ser
   385                 390                 395                 400
   cgg gag ttt gat ctt acc aaa gaa gaa gat ctt gca gca tta aga cat      1248
   Arg Glu Phe Asp Leu Thr Lys Glu Glu Asp Leu Ala Ala Leu Arg His
                   405                 410                 415
   gaa ata gaa ctt cga atg agg aaa aat gtg aaa gaa ggc tgt acc gtt      1296
   Glu Ile Glu Leu Arg Met Arg Lys Asn Val Lys Glu Gly Cys Thr Val
               420                 425                 430
   agc cct gag acc ata tcc tta aat gta aaa ggc cct gga cta cag agg      1344
   Ser Pro Glu Thr Ile Ser Leu Asn Val Lys Gly Pro Gly Leu Gln Arg
           435                 440                 445
   atg gtg ctt gtt gac tta cca ggt gtg att aat act gtg aca tca ggc      1392
   Met Val Leu Val Asp Leu Pro Gly Val Ile Asn Thr Val Thr Ser Gly
       450                 455                 460
   atg gct cct gac aca aag gaa act att ttc agt atc agc aaa gct tac      1440
   Met Ala Pro Asp Thr Lys Glu Thr Ile Phe Ser Ile Ser Lys Ala Tyr
   465                 470                 475                 480
   atg cag aat cct aat gcc atc ata ctg tgt att caa gat gga tct gtg      1488
   Met Gln Asn Pro Asn Ala Ile Ile Leu Cys Ile Gln Asp Gly Ser Val
                   485                 490                 495
   gat gct gaa cgc agt att gtt aca gac ttg gtc agt caa atg gac cct      1536
   Asp Ala Glu Arg Ser Ile Val Thr Asp Leu Val Ser Gln Met Asp Pro
               500                 505                 510
   cat gga agg aga acc ata ttc gtt ttg acc aaa gta gac ctg gca gag      1584
   His Gly Arg Arg Thr Ile Phe Val Leu Thr Lys Val Asp Leu Ala Glu
           515                 520                 525
   aaa aat gta gcc agt cca agc agg att cag cag ata att gaa gga aag      1632
   Lys Asn Val Ala Ser Pro Ser Arg Ile Gln Gln Ile Ile Glu Gly Lys
       530                 535                 540
   ctc ttc cca atg aaa gct tta ggt tat ttt gct gtt gta aca gga aaa      1680
   Leu Phe Pro Met Lys Ala Leu Gly Tyr Phe Ala Val Val Thr Gly Lys
   545                 550                 555                 560
   ggg aac agc tct gaa agc att gaa gct ata aga gaa tat gaa gaa gag      1728
   Gly Asn Ser Ser Glu Ser Ile Glu Ala Ile Arg Glu Tyr Glu Glu Glu
                   565                 570                 575
   ttt ttt cag aat tca aag ctc cta aag aca agc atg cta aag gca cac      1776
   Phe Phe Gln Asn Ser Lys Leu Leu Lys Thr Ser Met Leu Lys Ala His
               580                 585                 590
   caa gtg act aca aga aat tta agc ctt gca gta tca gac tgc ttt tgg      1824
   Gln Val Thr Thr Arg Asn Leu Ser Leu Ala Val Ser Asp Cys Phe Trp
           595                 600                 605
   aaa atg gta cga gag tct gtt gaa caa cag gct gat agt ttc aaa gca      1872
   Lys Met Val Arg Glu Ser Val Glu Gln Gln Ala Asp Ser Phe Lys Ala
       610                 615                 620
   aca cgt ttt aac ctt gaa act gaa tgg aag aat aac tat cct cgc ctg      1920
   Thr Arg Phe Asn Leu Glu Thr Glu Trp Lys Asn Asn Tyr Pro Arg Leu
   625                 630                 635                 640
   cgg gaa ctt gac cgg aat gaa cta ttt gaa aaa gct aaa aat gaa atc     1968
   Arg Glu Leu Asp Arg Asn Glu Leu Phe Glu Lys Ala Lys Asn Glu Ile
                   645                 650                 655
   ctt gat gaa gtt atc agt ctg agc cag gtt aca cca aaa cat tgg gag     2016
   Leu Asp Glu Val Ile Ser Leu Ser Gln Val Thr Pro Lys His Trp Glu
               660                 665                 670
   gaa atc ctt caa caa tct ttg tgg gaa aga gta tca act cat gtg att     2064
   Glu Ile Leu Gln Gln Ser Leu Trp Glu Arg Val Ser Thr His Val Ile
           675                 680                 685
   gaa aac atc tac ctt cca gct gcg cag acc atg aat tca gga act ttt     2112
   Glu Asn Ile Tyr Leu Pro Ala Ala Gln Thr Met Asn Ser Gly Thr Phe
       690                 695                 700
   aac acc aca gtg gat atc aag ctt aaa cag tgg act gat aaa caa ctt     2160
   Asn Thr Thr Val Asp Ile Lys Leu Lys Gln Trp Thr Asp Lys Gln Leu
   705                 710                 715                 720
   cct aat aaa gca gta gag gtt gct tgg gag acc cta caa gaa gaa ttt     2208
   Pro Asn Lys Ala Val Glu Val Ala Trp Glu Thr Leu Gln Glu Glu Phe
                   725                 730                 735
   tcc cgc ttt atg aca gaa ccg aaa ggg aaa gag cat gat gac ata ttt     2256
   Ser Arg Phe Met Thr Glu Pro Lys Gly Lys Glu His Asp Asp Ile Phe
               740                 745                 750
   gat aaa ctt aaa gag gcc gtt aag gaa gaa agt att aaa cga cac aag     2304
   Asp Lys Leu Lys Glu Ala Val Lys Glu Glu Ser Ile Lys Arg His Lys
           755                 760                 765
   tgg aat gac ttt gcg gag gac agc ttg agg gtt att caa cac aat gct     2352
   Trp Asn Asp Phe Ala Glu Asp Ser Leu Arg Val Ile Gln His Asn Ala
       770                 775                 780
   ttg gaa gac cga tcc ata tct gat aaa cag caa tgg gat gca gct att     2400
   Leu Glu Asp Arg Ser Ile Ser Asp Lys Gln Gln Trp Asp Ala Ala Ile
   785                 790                 795                 800
   tat ttt atg gaa gag gct ctg cag gct cgt ctc aag gat act gaa aat     2448
   Tyr Phe Met Glu Glu Ala Leu Gln Ala Arg Leu Lys Asp Thr Glu Asn
                   805                 810                 815
   gca att gaa aac atg gtg ggt cca gac tgg aaa aag agg tgg tta tac     2496
   Ala Ile Glu Asn Met Val Gly Pro Asp Trp Lys Lys Arg Trp Leu Tyr
               820                 825                 830
   tgg aag aat cgg acc caa gaa cag tgt gtt cac aat gaa acc aag aat     2544
   Trp Lys Asn Arg Thr Gln Glu Gln Cys Val His Asn Glu Thr Lys Asn
           835                 840                 845
   gaa ttg gag aag atg ttg aaa tgt aat gag gag cac cca gct tat ctt     2592
   Glu Leu Glu Lys Met Leu Lys Cys Asn Glu Glu His Pro Ala Tyr Leu
       850                 855                 860
   gca agt gat gaa ata acc aca gtc cgg aag aac ctt gaa tcc cga gga     2640
   Ala Ser Asp Glu Ile Thr Thr Val Arg Lys Asn Leu Glu Ser Arg Gly
   865                 870                 875                 880
   gta gaa gta gat cca agc ttg att aag gat act tgg cat caa gtt tat      2688
   Val Glu Val Asp Pro Ser Leu Ile Lys Asp Thr Trp His Gln Val Tyr
                   885                 890                 895
   aga aga cat ttt tta aaa aca gct cta aac cat tgt aac ctt tgt cga      2736
   Arg Arg His Phe Leu Lys Thr Ala Leu Asn His Cys Asn Leu Cys Arg
               900                 905                 910
   aga ggt ttt tat tac tac caa agg cat ttt gta gat tct gag ttg gaa      2784
   Arg Gly Phe Tyr Tyr Tyr Gln Arg His Phe Val Asp Ser Glu Leu Glu
           915                 920                 925
   tgc aat gat gtg gtc ttg ttt tgg cgt ata cag cgc atg ctt gct atc      2832
   Cys Asn Asp Val Val Leu Phe Trp Arg Ile Gln Arg Met Leu Ala Ile
       930                 935                 940
   acc gca aat act tta agg caa caa ctt aca aat act gaa gtt agg cga      2880
   Thr Ala Asn Thr Leu Arg Gln Gln Leu Thr Asn Thr Glu Val Arg Arg
   945                 950                 955                 960
   tta gag aaa aat gtt aaa gag gta ttg gaa gat ttt gct gaa gat ggt      2928
   Leu Glu Lys Asn Val Lys Glu Val Leu Glu Asp Phe Ala Glu Asp Gly
                   965                 970                 975
   gag aag aag att aaa ttg ctt act ggt aaa cgc gtt caa ctg gcg gaa      2976
   Glu Lys Lys Ile Lys Leu Leu Thr Gly Lys Arg Val Gln Leu Ala Glu
               980                 985                 990
   gac ctc aag aaa gtt aga gaa att caa gaa aaa ctt gat gct ttc att      3024
   Asp Leu Lys Lys Val Arg Glu Ile Gln Glu Lys Leu Asp Ala Phe Ile
           995                 1000                1005
   gaa gct ctt cat cag gag aaa taa                                      3048
   Glu Ala Leu His Gln Glu Lys
       1010                1015
<210> 2
   <211> 1015
   <212> PRT
   <213> Homo sapiens
<400> 2
   Met Trp Arg Leu Arg Arg Ala Ala Val Ala Cys Glu Val Cys Gln Ser
   1               5                   10                  15
   Leu Val Lys His Ser Ser Gly Ile Lys Gly Ser Leu Pro Leu Gln Lys
               20                  25                  30
   Leu His Leu Val Ser Arg Ser Ile Tyr His Ser His His Pro Thr Leu
           35                  40                  45
   Lys Leu Gln Arg Pro Gln Leu Arg Thr Ser Phe Gln Gln Phe Ser Ser
   50 55 60
   Leu Thr Asn Leu Pro Leu Arg Lys Leu Lys Phe Ser Pro Ile Lys Tyr
   65 70 75 80
   Gly Tyr Gln Pro Arg Arg Asn Phe Trp Pro Ala Arg Leu Ala Thr Arg
   85 90 95
   Leu Leu Lys Leu Arg Tyr Leu Ile Leu Gly Ser Ala Val Gly Gly Gly
   100 105 110
   Tyr Thr Ala Lys Lys Thr Phe Asp Gln Trp Lys Asp Met Ile Pro Asp
   115 120 125
   Leu Ser Glu Tyr Lys Trp Ile Val Pro Asp Ile Val Trp Glu Ile Asp
   130 135 140
   Glu Tyr Ile Asp Phe Glu Lys Ile Arg Lys Ala Leu Pro Asn Ser Glu
   145 150 155 160
   Asp Leu Val Lys Leu Ala Pro Asp Phe Asp Lys Ile Val Glu Ser Leu
   165 170 175
   Ser Leu Leu Lys Asp Phe Phe Thr Ser Gly His Lys Leu Val Ser Glu
   180 185 190
   Val Ile Gly Ala Ser Asp Leu Leu Leu Leu Leu Gly Ser Pro Glu Glu
   195 200 205
   Thr Ala Phe Arg Ala Thr Asp Arg Gly Ser Glu Ser Asp Lys His Phe
   210 215 220
   Arg Lys Gly Leu Leu Gly Glu Leu Ile Leu Leu Gln Gln Gln Ile Gln
   225 230 235 240
   Glu His Glu Glu Glu Ala Arg Arg Ala Ala Gly Gln Tyr Ser Thr Ser
   245 250 255
   Tyr Ala Gln Gln Lys Arg Lys Val Ser Asp Lys Glu Lys Ile Asp Gln
   260 265 270
   Leu Gln Glu Glu Leu Leu His Thr Gln Leu Lys Tyr Gln Arg Ile Leu
   275 280 285
   Glu Arg Leu Glu Lys Glu Asn Lys Glu Leu Arg Lys Leu Val Leu Gln
   290 295 300
   Lys Asp Asp Lys Gly Ile His His Arg Lys Leu Lys Lys Ser Leu Ile
   305 310 315 320
   Asp Met Tyr Ser Glu Val Leu Asp Val Leu Ser Asp Tyr Asp Ala Ser
   325 330 335
   Tyr Asn Thr Gln Asp His Leu Pro Arg Val Val Val Val Gly Asp Gln
   340 345 350
   Ser Ala Gly Lys Thr Ser Val Leu Glu Met Ile Ala Gln Ala Arg Ile
   355 360 365
   Phe Pro Arg Gly Ser Gly Glu Met Met Thr Arg Ser Pro Val Lys Val
   370 375 380
   Thr Leu Ser Glu Gly Pro His His Val Ala Leu Phe Lys Asp Ser Ser
   385 390 395 400
   Arg Glu Phe Asp Leu Thr Lys Glu Glu Asp Leu Ala Ala Leu Arg His
   405 410 415
   Glu Ile Glu Leu Arg Met Arg Lys Asn Val Lys Glu Gly Cys Thr Val
   420 425 430
   Ser Pro Glu Thr Ile Ser Leu Asn Val Lys Gly Pro Gly Leu Gln Arg
   435 440 445
   Met Val Leu Val Asp Leu Pro Gly Val Ile Asn Thr Val Thr Ser Gly
   450 455 460
   Met Ala Pro Asp Thr Lys Glu Thr Ile Phe Ser Ile Ser Lys Ala Tyr
   465 470 475 480
   Met Gln Asn Pro Asn Ala Ile Ile Leu Cys Ile Gln Asp Gly Ser Val
   485 490 495
   Asp Ala Glu Arg Ser Ile Val Thr Asp Leu Val Ser Gln Met Asp Pro
   500 505 510
   His Gly Arg Arg Thr Ile Phe Val Leu Thr Lys Val Asp Leu Ala Glu
   515 520 525
   Lys Asn Val Ala Ser Pro Ser Arg Ile Gln Gln Ile Ile Glu Gly Lys
   530 535 540
   Leu Phe Pro Met Lys Ala Leu Gly Tyr Phe Ala Val Val Thr Gly Lys
   545 550 555 560
   Gly Asn Ser Ser Glu Ser Ile Glu Ala Ile Arg Glu Tyr Glu Glu Glu
   565 570 575
   Phe Phe Gln Asn Ser Lys Leu Leu Lys Thr Ser Met Leu Lys Ala His
   580 585 590
   Gln Val Thr Thr Arg Asn Leu Ser Leu Ala Val Ser Asp Cys Phe Trp
   595 600 605
   Lys Met Val Arg Glu Ser Val Glu Gln Gln Ala Asp Ser Phe Lys Ala
   610 615 620
   Thr Arg Phe Asn Leu Glu Thr Glu Trp Lys Asn Asn Tyr Pro Arg Leu
   625 630 635 640
   Arg Glu Leu Asp Arg Asn Glu Leu Phe Glu Lys Ala Lys Asn Glu Ile
   645 650 655
   Leu Asp Glu Val Ile Ser Leu Ser Gln Val Thr Pro Lys His Trp Glu
   660 665 670
   Glu Ile Leu Gln Gln Ser Leu Trp Glu Arg Val Ser Thr His Val Ile
   675 680 685
   Glu Asn Ile Tyr Leu Pro Ala Ala Gln Thr Met Asn Ser Gly Thr Phe
   690 695 700
   Asn Thr Thr Val Asp Ile Lys Leu Lys Gln Trp Thr Asp Lys Gln Leu
   705 710 715 720
   Pro Asn Lys Ala Val Glu Val Ala Trp Glu Thr Leu Gln Glu Glu Phe
   725 730 735
   Ser Arg Phe Met Thr Glu Pro Lys Gly Lys Glu His Asp Asp Ile Phe
   740 745 750
   Asp Lys Leu Lys Glu Ala Val Lys Glu Glu Ser Ile Lys Arg His Lys
   755 760 765
   Trp Asn Asp Phe Ala Glu Asp Ser Leu Arg Val Ile Gln His Asn Ala
       770                 775                 780
   Leu Glu Asp Arg Ser Ile Ser Asp Lys Gln Gln Trp Asp Ala Ala Ile
   785                 790                 795                 800
   Tyr Phe Met Glu Glu Ala Leu Gln Ala Arg Leu Lys Asp Thr Glu Asn
                   805                 810                 815
   Ala Ile Glu Asn Met Val Gly Pro Asp Trp Lys Lys Arg Trp Leu Tyr
               820                 825                 830
   Trp Lys Asn Arg Thr Gln Glu Gln Cys Val His Asn Glu Thr Lys Asn
           835                 840                 845
   Glu Leu Glu Lys Met Leu Lys Cys Asn Glu Glu His Pro Ala Tyr Leu
       850                 855                 860
   Ala Ser Asp Glu Ile Thr Thr Val Arg Lys Asn Leu Glu Ser Arg Gly
   865                 870                 875                 880
   Val Glu Val Asp Pro Ser Leu Ile Lys Asp Thr Trp His Gln Val Tyr
                   885                 890                 895
   Arg Arg His Phe Leu Lys Thr Ala Leu Asn His Cys Asn Leu Cys Arg
               900                 905                 910
   Arg Gly Phe Tyr Tyr Tyr Gln Arg His Phe Val Asp Ser Glu Leu Glu
           915                 920                 925
   Cys Asn Asp Val Val Leu Phe Trp Arg Ile Gln Arg Met Leu Ala Ile
       930                 935                 940
   Thr Ala Asn Thr Leu Arg Gln Gln Leu Thr Asn Thr Glu Val Arg Arg
   945                 950                 955                 960
   Leu Glu Lys Asn Val Lys Glu Val Leu Glu Asp Phe Ala Glu Asp Gly
                   965                 970                 975
   Glu Lys Lys Ile Lys Leu Leu Thr Gly Lys Arg Val Gln Leu Ala Glu
               980                 985                 990
   Asp Leu Lys Lys Val Arg Glu Ile  Gln Glu Lys Leu Asp  Ala Phe Ile
           995                 1000                1005
   Glu Ala  Leu His Gln Glu Lys
   1010 1015
<210> 3
   <211> 2994
   <212> DNA
   <213> Homo sapiens
<220>
   <221> CDS
   <222> (1)..(2994)
<400> 3
   atg tgg cga cta cgt cgg gcc gct gtg gcc tgt gag gtc tgc cag tct      48
   Met Trp Arg Leu Arg Arg Ala Ala Val Ala Cys Glu Val Cys Gln Ser
   1               5                   10                  15
   tta gtg aaa cac agc tct gga ata aaa gga agt tta cca cta caa aaa      96
   Leu Val Lys His Ser Ser Gly Ile Lys Gly Ser Leu Pro Leu Gln Lys
               20                  25                  30
   cta cat ctg gtt tca cga agc att tat cat tca cat cat cct acc tta     144
   Leu His Leu Val Ser Arg Ser Ile Tyr His Ser His His Pro Thr Leu
           35                  40                  45
   aag ctt caa cga ccc caa tta agg aca tcc ttt cag cag ttc tct tct     192
   Lys Leu Gln Arg Pro Gln Leu Arg Thr Ser Phe Gln Gln Phe Ser Ser
       50                  55                  60
   ctg aca aac ctt cct tta cgt aaa ctg aaa ttc tct cca att aaa tat     240
   Leu Thr Asn Leu Pro Leu Arg Lys Leu Lys Phe Ser Pro Ile Lys Tyr
   65                  70                  75                  80
   ggc tac cag cct cgc agg aat ttt tgg cca gca aga tta gct acg aga     288
   Gly Tyr Gln Pro Arg Arg Asn Phe Trp Pro Ala Arg Leu Ala Thr Arg
                   85                  90                  95
   ctc tta aaa ctt cgc tat ctc ata cta gga tcg gct gtt ggg ggt ggc     336
   Leu Leu Lys Leu Arg Tyr Leu Ile Leu Gly Ser Ala Val Gly Gly Gly
               100                 105                 110
   tac aca gcc aaa aag act ttt gat cag tgg aaa gat atg ata ccg gac     384
   Tyr Thr Ala Lys Lys Thr Phe Asp Gln Trp Lys Asp Met Ile Pro Asp
           115                 120                 125
   ctt agt gaa tat aaa tgg att gtg cct gac att gtg tgg gaa att gat     432
   Leu Ser Glu Tyr Lys Trp Ile Val Pro Asp Ile Val Trp Glu Ile Asp
       130                 135                 140
   gag tat atc gat ttt gag aaa att aga aaa gcc ctt cct aat tca gaa     480
   Glu Tyr Ile Asp Phe Glu Lys Ile Arg Lys Ala Leu Pro Asn Ser Glu
   145                 150                 155                 160
   gac ctt gta aag tta gca cca gac ttt gac aag att gtt gaa agc ctt     528
   Asp Leu Val Lys Leu Ala Pro Asp Phe Asp Lys Ile Val Glu Ser Leu
                   165                 170                 175
   agc tta ttg aag gac ttt ttt acc tca ggt tct ccg gaa gaa acg gcg     576
   Ser Leu Leu Lys Asp Phe Phe Thr Ser Gly Ser Pro Glu Glu Thr Ala
               180                 185                 190
   ttt aga gca aca gat cgt gga tct gaa agt gac aag cat ttt aga aag        624
   Phe Arg Ala Thr Asp Arg Gly Ser Glu Ser Asp Lys His Phe Arg Lys
           195                 200                 205
   ggt ctg ctt ggt gag ctc att ctc tta caa caa caa att caa gag cat        672
   Gly Leu Leu Gly Glu Leu Ile Leu Leu Gln Gln Gln Ile Gln Glu His
       210                 215                 220
   gaa gag gaa gcg cgc aga gcc gct ggc caa tat agc acg agc tat gcc        720
   Glu Glu Glu Ala Arg Arg Ala Ala Gly Gln Tyr Ser Thr Ser Tyr Ala
   225                 230                 235                 240
   caa cag aag cgc aag gtg tca gac aaa gag aaa att gac caa ctt cag        768
   Gln Gln Lys Arg Lys Val Ser Asp Lys Glu Lys Ile Asp Gln Leu Gln
                   245                 250                 255
   gaa gaa ctt ctg cac act cag ttg aag tat cag aga atc ttg gaa cga        816
   Glu Glu Leu Leu His Thr Gln Leu Lys Tyr Gln Arg Ile Leu Glu Arg
               260                 265                 270
   tta gaa aag gag aac aaa gaa ttg aga aaa tta gta ttg cag aaa gat        864
   Leu Glu Lys Glu Asn Lys Glu Leu Arg Lys Leu Val Leu Gln Lys Asp
           275                 280                 285
   gac aaa ggc att cat cat aga aag ctt aag aaa tct ttg att gac atg        912
   Asp Lys Gly Ile His His Arg Lys Leu Lys Lys Ser Leu Ile Asp Met
       290                 295                 300
   tat tct gaa gtt ctt gat gtt ctc tct gat tat gat gcc agt tat aat        960
   Tyr Ser Glu Val Leu Asp Val Leu Ser Asp Tyr Asp Ala Ser Tyr Asn
   305                 310                 315                 320
   acg caa gat cat ctg cca cgg gtt gtt gtg gtt gga gat cag agt gct       1008
   Thr Gln Asp His Leu Pro Arg Val Val Val Val Gly Asp Gln Ser Ala
                   325                 330                 335
   gga aag act agt gtg ttg gaa atg att gcc caa gct cga ata ttc cca       1056
   Gly Lys Thr Ser Val Leu Glu Met Ile Ala Gln Ala Arg Ile Phe Pro
               340                 345                 350
   aga gga tct ggg gag atg atg aca cgt tct cca gtt aag gtg act ctg       1104
   Arg Gly Ser Gly Glu Met Met Thr Arg Ser Pro Val Lys Val Thr Leu
           355                 360                 365
   agt gaa ggt cct cac cat gtg gcc cta ttt aaa gat agt tct cgg gag       1152
   Ser Glu Gly Pro His His Val Ala Leu Phe Lys Asp Ser Ser Arg Glu
       370                 375                 380
   ttt gat ctt acc aaa gaa gaa gat ctt gca gca tta aga cat gaa ata       1200
   Phe Asp Leu Thr Lys Glu Glu Asp Leu Ala Ala Leu Arg His Glu Ile
   385                 390                 395                 400
   gaa ctt cga atg agg aaa aat gtg aaa gaa ggc tgt acc gtt agc cct       1248
   Glu Leu Arg Met Arg Lys Asn Val Lys Glu Gly Cys Thr Val Ser Pro
                   405                 410                 415
   gag acc ata tcc tta aat gta aaa ggc cct gga cta cag agg atg gtg       1296
   Glu Thr Ile Ser Leu Asn Val Lys Gly Pro Gly Leu Gln Arg Met Val
                   420                     425                     430
   ctt gtt gac tta cca ggt gtg att aat act gtg aca tca ggc atg gct      1344
   Leu Val Asp Leu Pro Gly Val Ile Asn Thr Val Thr Ser Gly Met Ala
           435                 440                 445
   cct gac aca aag gaa act att ttc agt atc agc aaa gct tac atg cag      1392
   Pro Asp Thr Lys Glu Thr Ile Phe Ser Ile Ser Lys Ala Tyr Met Gln
       450                     455                     460
   aat cct aat gcc atc ata ctg tgt att caa gat gga tct gtg gat gct      1440
   Asn Pro Asn Ala Ile Ile Leu Cys Ile Gln Asp Gly Ser Val Asp Ala
   465                     470                     475                     480
   gaa cgc agt att gtt aca gac ttg gtc agt caa atg gac cct cat gga      1488
   Glu Arg Ser Ile Val Thr Asp Leu Val Ser Gln Met Asp Pro His Gly
                   485                     490                     495
   agg aga acc ata ttc gtt ttg acc aaa gta gac ctg gca gag aaa aat      1536
   Arg Arg Thr Ile Phe Val Leu Thr Lys Val Asp Leu Ala Glu Lys Asn
               500                     505                     510
   gta gcc agt cca agc agg att cag cag ata att gaa gga aag ctc ttc      1584
   Val Ala Ser Pro Ser Arg Ile Gln Gln Ile Ile Glu Gly Lys Leu Phe
           515                     520                     525
   cca atg aaa gct tta ggt tat ttt gct gtt gta aca gga aaa ggg aac      1632
   Pro Met Lys Ala Leu Gly Tyr Phe Ala Val Val Thr Gly Lys Gly Asn
       530                     535                     540
   agc tct gaa agc att gaa gct ata aga gaa tat gaa gaa gag ttt ttt      1680
   Ser Ser Glu Ser Ile Glu Ala Ile Arg Glu Tyr Glu Glu Glu Phe Phe
   545                     550                     555                     560
   cag aat tca aag ctc cta aag aca agc atg cta aag gca cac caa gtg      1728
   Gln Asn Ser Lys Leu Leu Lys Thr Ser Met Leu Lys Ala His Gln Val
                   565                     570                     575
   act aca aga aat tta agc ctt gca gta tca gac tgc ttt tgg aaa atg      1776
   Thr Thr Arg Asn Leu Ser Leu Ala Val Ser Asp Cys Phe Trp Lys Met
               580                     585                     590
   gta cga gag tct gtt gaa caa cag gct gat agt ttc aaa gca aca cgt      1824
   Val Arg Glu Ser Val Glu Gln Gln Ala Asp Ser Phe Lys Ala Thr Arg
           595                     600                     605
   ttt aac ctt gaa act gaa tgg aag aat aac tat cct cgc ctg cgg gaa      1872
   Phe Asn Leu Glu Thr Glu Trp Lys Asn Asn Tyr Pro Arg Leu Arg Glu
       610                     615                     620
   ctt gac cgg aat gaa cta ttt gaa aaa gct aaa aat gaa atc ctt gat      1920
   Leu Asp Arg Asn Glu Leu Phe Glu Lys Ala Lys Asn Glu Ile Leu Asp
   625                     630                     635                     640
   gaa gtt atc agt ctg agc cag gtt aca cca aaa cat tgg gag gaa atc      1968
   Glu Val Ile Ser Leu Ser Gln Val Thr Pro Lys His Trp Glu Glu Ile
                   645                     650                     655
   ctt caa caa tct ttg tgg gaa aga gta tca act cat gtg att gaa aac      2016
   Leu Gln Gln Ser Leu Trp Glu Arg Val Ser Thr His Val Ile Glu Asn
               660                 665                 670
   atc tac ctt cca gct gcg cag acc atg aat tca gga act ttt aac acc      2064
   Ile Tyr Leu Pro Ala Ala Gln Thr Met Asn Ser Gly Thr Phe Asn Thr
           675                 680                 685
   aca gtg gat atc aag ctt aaa cag tgg act gat aaa caa ctt cct aat      2112
   Thr Val Asp Ile Lys Leu Lys Gln Trp Thr Asp Lys Gln Leu Pro Asn
       690                 695                 700
   aaa gca gta gag gtt gct tgg gag acc cta caa gaa gaa ttt tcc cgc      2160
   Lys Ala Val Glu Val Ala Trp Glu Thr Leu Gln Glu Glu Phe Ser Arg
   705                 710                 715                 720
   ttt atg aca gaa ccg aaa ggg aaa gag cat gat gac ata ttt gat aaa      2208
   Phe Met Thr Glu Pro Lys Gly Lys Glu His Asp Asp Ile Phe Asp Lys
                   725                 730                 735
   ctt aaa gag gcc gtt aag gaa gaa agt att aaa cga cac aag tgg aat      2256
   Leu Lys Glu Ala Val Lys Glu Glu Ser Ile Lys Arg His Lys Trp Asn
               740                 745                 750
   gac ttt gcg gag gac agc ttg agg gtt att caa cac aat gct ttg gaa      2304
   Asp Phe Ala Glu Asp Ser Leu Arg Val Ile Gln His Asn Ala Leu Glu
           755                 760                 765
   gac cga tcc ata tct gat aaa cag caa tgg gat gca gct att tat ttt      2352
   Asp Arg Ser Ile Ser Asp Lys Gln Gln Trp Asp Ala Ala Ile Tyr Phe
       770                 775                 780
   atg gaa gag gct ctg cag gct cgt ctc aag gat act gaa aat gca att      2400
   Met Glu Glu Ala Leu Gln Ala Arg Leu Lys Asp Thr Glu Asn Ala Ile
   785                 790                 795                 800
   gaa aac atg gtg ggt cca gac tgg aaa aag agg tgg tta tac tgg aag      2448
   Glu Asn Met Val Gly Pro Asp Trp Lys Lys Arg Trp Leu Tyr Trp Lys
                   805                 810                 815
   aat cgg acc caa gaa cag tgt gtt cac aat gaa acc aag aat gaa ttg      2496
   Asn Arg Thr Gln Glu Gln Cys Val His Asn Glu Thr Lys Asn Glu Leu
               820                 825                 830
   gag aag atg ttg aaa tgt aat gag gag cac cca gct tat ctt gca agt      2544
   Glu Lys Met Leu Lys Cys Asn Glu Glu His Pro Ala Tyr Leu Ala Ser
           835                 840                 845
   gat gaa ata acc aca gtc cgg aag aac ctt gaa tcc cga gga gta gaa      2592
   Asp Glu Ile Thr Thr Val Arg Lys Asn Leu Glu Ser Arg Gly Val Glu
       850                 855                 860
   gta gat cca agc ttg att aag gat act tgg cat caa gtt tat aga aga      2640
   Val Asp Pro Ser Leu Ile Lys Asp Thr Trp His Gln Val Tyr Arg Arg
   865                 870                 875                 880
   cat ttt tta aaa aca gct cta aac cat tgt aac ctt tgt cga aga ggt      2688
   His Phe Leu Lys Thr Ala Leu Asn His Cys Asn Leu Cys Arg Arg Gly
                   885                 890                 895
   ttt tat tac tac caa agg cat ttt gta gat tct gag ttg gaa tgc aat      2736
   Phe Tyr Tyr Tyr Gln Arg His Phe Val Asp Ser Glu Leu Glu Cys Asn
               900                       905                       910
   gat gtg gtc ttg ttt tgg cgt ata cag cgc atg ctt gct atc acc gca      2784
   Asp Val Val Leu Phe Trp Arg Ile Gln Arg Met Leu Ala Ile Thr Ala
           915                     920                     925
   aat act tta agg caa caa ctt aca aat act gaa gtt agg cga tta gag      2832
   Asn Thr Leu Arg Gln Gln Leu Thr Asn Thr Glu Val Arg Arg Leu Glu
       930                     935                     940
   aaa aat gtt aaa gag gta ttg gaa gat ttt gct gaa gat ggt gag aag      2880
   Lys Asn Val Lys Glu Val Leu Glu Asp Phe Ala Glu Asp Gly Glu Lys
   945                     950                     955                 960
   aag att aaa ttg ctt act ggt aaa cgc gtt caa ctg gcg gaa gac ctc      2928
   Lys Ile Lys Leu Leu Thr Gly Lys Arg Val Gln Leu Ala Glu Asp Leu
                   965                     970                     975
   aag aaa gtt aga gaa att caa gaa aaa ctt gat gct ttc att gaa gct      2976
   Lys Lys Val Arg Glu Ile Gln Glu Lys Leu Asp Ala Phe Ile Glu Ala
               980                     985                     990
   ctt cat cag gag aaa taa                                              2994
   Leu His Gln Glu Lys
           995
<210> 4
   <211> 997
   <212> PRT
   <213> Homo sapiens
<400> 4
   Met Trp Arg Leu Arg Arg Ala Ala Val Ala Cys Glu Val Cys Gln Ser
   1               5                   10                  15
   Leu Val Lys His Ser Ser Gly Ile Lys Gly Ser Leu Pro Leu Gln Lys
               20                  25                  30
   Leu His Leu Val Ser Arg Ser Ile Tyr His Ser His His Pro Thr Leu
           35                  40                  45
   Lys Leu Gln Arg Pro Gln Leu Arg Thr Ser Phe Gln Gln Phe Ser Ser
       50                  55                  60
   Leu Thr Asn Leu Pro Leu Arg Lys Leu Lys Phe Ser Pro Ile Lys Tyr
   65                  70                  75                  80
   Gly Tyr Gln Pro Arg Arg Asn Phe Trp Pro Ala Arg Leu Ala Thr Arg
                   85                  90                  95
   Leu Leu Lys Leu Arg Tyr Leu Ile Leu Gly Ser Ala Val Gly Gly Gly
               100                 105                 110
   Tyr Thr Ala Lys Lys Thr Phe Asp Gln Trp Lys Asp Met Ile Pro Asp
   115 120 125
   Leu Ser Glu Tyr Lys Trp Ile Val Pro Asp Ile Val Trp Glu Ile Asp
   130 135 140
   Glu Tyr Ile Asp Phe Glu Lys Ile Arg Lys Ala Leu Pro Asn Ser Glu
   145 150 155 160
   Asp Leu Val Lys Leu Ala Pro Asp Phe Asp Lys Ile Val Glu Ser Leu
   165 170 175
   Ser Leu Leu Lys Asp Phe Phe Thr Ser Gly Ser Pro Glu Glu Thr Ala
   180 185 190
   Phe Arg Ala Thr Asp Arg Gly Ser Glu Ser Asp Lys His Phe Arg Lys
   195 200 205
   Gly Leu Leu Gly Glu Leu Ile Leu Leu Gln Gln Gln Ile Gln Glu His
   210 215 220
   Glu Glu Glu Ala Arg Arg Ala Ala Gly Gln Tyr Ser Thr Ser Tyr Ala
   225 230 235 240
   Gln Gln Lys Arg Lys Val Ser Asp Lys Glu Lys Ile Asp Gln Leu Gln
   245 250 255
   Glu Glu Leu Leu His Thr Gln Leu Lys Tyr Gln Arg Ile Leu Glu Arg
   260 265 270
   Leu Glu Lys Glu Asn Lys Glu Leu Arg Lys Leu Val Leu Gln Lys Asp
   275 280 285
   Asp Lys Gly Ile His His Arg Lys Leu Lys Lys Ser Leu Ile Asp Met
   290 295 300
   Tyr Ser Glu Val Leu Asp Val Leu Ser Asp Tyr Asp Ala Ser Tyr Asn
   305 310 315 320
   Thr Gln Asp His Leu Pro Arg Val Val Val Val Gly Asp Gln Ser Ala
   325 330 335
   Gly Lys Thr Ser Val Leu Glu Met Ile Ala Gln Ala Arg Ile Phe Pro
   340 345 350
   Arg Gly Ser Gly Glu Met Met Thr Arg Ser Pro Val Lys Val Thr Leu
           355                 360                 365
   Ser Glu Gly Pro His His Val Ala Leu Phe Lys Asp Ser Ser Arg Glu
       370                 375                 380
   Phe Asp Leu Thr Lys Glu Glu Asp Leu Ala Ala Leu Arg His Glu Ile
   385                 390                 395                 400
   Glu Leu Arg Met Arg Lys Asn Val Lys Glu Gly Cys Thr Val Ser Pro
                   405                 410                 415
   Glu Thr Ile Ser Leu Asn Val Lys Gly Pro Gly Leu Gln Arg Met Val
               420                 425                 430
   Leu Val Asp Leu Pro Gly Val Ile Asn Thr Val Thr Ser Gly Met Ala
           435                 440                 445
   Pro Asp Thr Lys Glu Thr Ile Phe Ser Ile Ser Lys Ala Tyr Met Gln
       450                 455                 460
   Asn Pro Asn Ala Ile Ile Leu Cys Ile Gln Asp Gly Ser Val Asp Ala
   465                 470                 475                 480
   Glu Arg Ser Ile Val Thr Asp Leu Val Ser Gln Met Asp Pro His Gly
                   485                 490                 495
   Arg Arg Thr Ile Phe Val Leu Thr Lys Val Asp Leu Ala Glu Lys Asn
               500                 505                 510
   Val Ala Ser Pro Ser Arg Ile Gln Gln Ile Ile Glu Gly Lys Leu Phe
           515                 520                 525
   Pro Met Lys Ala Leu Gly Tyr Phe Ala Val Val Thr Gly Lys Gly Asn
       530                 535                 540
   Ser Ser Glu Ser Ile Glu Ala Ile Arg Glu Tyr Glu Glu Glu Phe Phe
   545                 550                 555                 560
   Gln Asn Ser Lys Leu Leu Lys Thr Ser Met Leu Lys Ala His Gln Val
                   565                 570                 575
   Thr Thr Arg Asn Leu Ser Leu Ala Val Ser Asp Cys Phe Trp Lys Met
               580                 585                 590
   Val Arg Glu Ser Val Glu Gln Gln Ala Asp Ser Phe Lys Ala Thr Arg
   595 600 605
   Phe Asn Leu Glu Thr Glu Trp Lys Asn Asn Tyr Pro Arg Leu Arg Glu
   610 615 620
   Leu Asp Arg Asn Glu Leu Phe Glu Lys Ala Lys Asn Glu Ile Leu Asp
   625 630 635 640
   Glu Val Ile Ser Leu Ser Gln Val Thr Pro Lys His Trp Glu Glu Ile
   645 650 655
   Leu Gln Gln Ser Leu Trp Glu Arg Val Ser Thr His Val Ile Glu Asn
   660 665 670
   Ile Tyr Leu Pro Ala Ala Gln Thr Met Asn Ser Gly Thr Phe Asn Thr
   675 680 685
   Thr Val Asp Ile Lys Leu Lys Gln Trp Thr Asp Lys Gln Leu Pro Asn
   690 695 700
   Lys Ala Val Glu Val Ala Trp Glu Thr Leu Gln Glu Glu Phe Ser Arg
   705 710 715 720
   Phe Met Thr Glu Pro Lys Gly Lys Glu His Asp Asp Ile Phe Asp Lys
   725 730 735
   Leu Lys Glu Ala Val Lys Glu Glu Ser Ile Lys Arg His Lys Trp Asn
   740 745 750
   Asp Phe Ala Glu Asp Ser Leu Arg Val Ile Gln His Asn Ala Leu Glu
   755 760 765
   Asp Arg Ser Ile Ser Asp Lys Gln Gln Trp Asp Ala Ala Ile Tyr Phe
   770 775 780
   Met Glu Glu Ala Leu Gln Ala Arg Leu Lys Asp Thr Glu Asn Ala Ile
   785 790 795 800
   Glu Asn Met Val Gly Pro Asp Trp Lys Lys Arg Trp Leu Tyr Trp Lys
   805 810 815
   Asn Arg Thr Gln Glu Gln Cys Val His Asn Glu Thr Lys Asn Glu Leu
   820 825 830
   Glu Lys Met Leu Lys Cys Asn Glu Glu His Pro Ala Tyr Leu Ala Ser
           835                 840                 845
   Asp Glu Ile Thr Thr Val Arg Lys Asn Leu Glu Ser Arg Gly Val Glu
       850                 855                 860
   Val Asp Pro Ser Leu Ile Lys Asp Thr Trp His Gln Val Tyr Arg Arg
   865                 870                 875                 880
   His Phe Leu Lys Thr Ala Leu Asn His Cys Asn Leu Cys Arg Arg Gly
                   885                 890                 895
   Phe Tyr Tyr Tyr Gln Arg His Phe Val Asp Ser Glu Leu Glu Cys Asn
               900                 905                 910
   Asp Val Val Leu Phe Trp Arg Ile Gln Arg Met Leu Ala Ile Thr Ala
           915                 920                 925
   Asn Thr Leu Arg Gln Gln Leu Thr Asn Thr Glu Val Arg Arg Leu Glu
       930                 935                 940
   Lys Asn Val Lys Glu Val Leu Glu Asp Phe Ala Glu Asp Gly Glu Lys
   945                 950                 955                 960
   Lys Ile Lys Leu Leu Thr Gly Lys Arg Val Gln Leu Ala Glu Asp Leu
                   965                 970                 975
   Lys Lys Val Arg Glu Ile Gln Glu Lys Leu Asp Ala Phe Ile Glu Ala
               980                 985                 990
   Leu His Gln Glu Lys
           995
<210> 5
   <211> 2940
   <212> DNA
   <213> Homo sapiens
<220>
   <221> CDS
   <222> (1)..(2940)
<400> 5
   atg tgg cga cta cgt cgg gcc gct gtg gcc tgt gag gtc tgc cag tct       48
   Met Trp Arg Leu Arg Arg Ala Ala Val Ala Cys Glu Val Cys Gln Ser
   1               5                   10                  15
   tta gtg aaa cac agc tct gga ata aaa gga agt tta cca cta caa aaa        96
   Leu Val Lys His Ser Ser Gly Ile Lys Gly Ser Leu Pro Leu Gln Lys
               20                  25                  30
   cta cat ctg gtt tca cga agc att tat cat tca cat cat cct acc tta       144
   Leu His Leu Val Ser Arg Ser Ile Tyr His Ser His His Pro Thr Leu
           35                  40                  45
   aag ctt caa cga ccc caa tta agg aca tcc ttt cag cag ttc tct tct       192
   Lys Leu Gln Arg Pro Gln Leu Arg Thr Ser Phe Gln Gln Phe Ser Ser
       50                  55                  60
   ctg aca aac ctt cct tta cgt aaa ctg aaa ttc tct cca att aaa tat       240
   Leu Thr Asn Leu Pro Leu Arg Lys Leu Lys Phe Ser Pro Ile Lys Tyr
   65                  70                  75                  80
   ggc tac cag cct cgc agg aat ttt tgg cca gca aga tta gct acg aga       288
   Gly Tyr Gln Pro Arg Arg Asn Phe Trp Pro Ala Arg Leu Ala Thr Arg
                   85                  90                  95
   ctc tta aaa ctt cgc tat ctc ata cta gga tcg gct gtt ggg ggt ggc       336
   Leu Leu Lys Leu Arg Tyr Leu Ile Leu Gly Ser Ala Val Gly Gly Gly
               100                 105                 110
   tac aca gcc aaa aag act ttt gat cag tgg aaa gat atg ata ccg gac       384
   Tyr Thr Ala Lys Lys Thr Phe Asp Gln Trp Lys Asp Met Ile Pro Asp
           115                 120                 125
   ctt agt gaa tat aaa tgg att gtg cct gac att gtg tgg gaa att gat       432
   Leu Ser Glu Tyr Lys Trp Ile Val Pro Asp Ile Val Trp Glu Ile Asp
       130                 135                 140
   gag tat atc gat ttt ggt cac aaa ttg gtt agt gaa gtc ata gga gct       480
   Glu Tyr Ile Asp Phe Gly His Lys Leu Val Ser Glu Val Ile Gly Ala
   145                 150                 155                 160
   tct gac cta ctt ctc ttg tta ggt tct ccg gaa gaa acg gcg ttt aga       528
   Ser Asp Leu Leu Leu Leu Leu Gly Ser Pro Glu Glu Thr Ala Phe Arg
                   165                 170                 175
   gca aca gat cgt gga tct gaa agt gac aag cat ttt aga aag ggt ctg       576
   Ala Thr Asp Arg Gly Ser Glu Ser Asp Lys His Phe Arg Lys Gly Leu
               180                 185                 190
   ctt ggt gag ctc att ctc tta caa caa caa att caa gag cat gaa gag       624
   Leu Gly Glu Leu Ile Leu Leu Gln Gln Gln Ile Gln Glu His Glu Glu
           195                 200                 205
   gaa gcg cgc aga gcc gct ggc caa tat agc acg agc tat gcc caa cag       672
   Glu Ala Arg Arg Ala Ala Gly Gln Tyr Ser Thr Ser Tyr Ala Gln Gln
       210                 215                 220
   aag cgc aag gtg tca gac aaa gag aaa att gac caa ctt cag gaa gaa       720
   Lys Arg Lys Val Ser Asp Lys Glu Lys Ile Asp Gln Leu Gln Glu Glu
   225                 230                 235                 240
   ctt ctg cac act cag ttg aag tat cag aga atc ttg gaa cga tta gaa       768
   Leu Leu His Thr Gln Leu Lys Tyr Gln Arg Ile Leu Glu Arg Leu Glu
                   245                 250                 255
   aag gag aac aaa gaa ttg aga aaa tta gta ttg cag aaa gat gac aaa        816
   Lys Glu Asn Lys Glu Leu Arg Lys Leu Val Leu Gln Lys Asp Asp Lys
               260                 265                 270
   ggc att cat cat aga aag ctt aag aaa tct ttg att gac atg tat tct        864
   Gly Ile His His Arg Lys Leu Lys Lys Ser Leu Ile Asp Met Tyr Ser
           275                 280                 285
   gaa gtt ctt gat gtt ctc tct gat tat gat gcc agt tat aat acg caa        912
   Glu Val Leu Asp Val Leu Ser Asp Tyr Asp Ala Ser Tyr Asn Thr Gln
       290                 295                 300
   gat cat ctg cca cgg gtt gtt gtg gtt gga gat cag agt gct gga aag        960
   Asp His Leu Pro Arg Val Val Val Val Gly Asp Gln Ser Ala Gly Lys
   305                 310                 315                 320
   act agt gtg ttg gaa atg att gcc caa gct cga ata ttc cca aga gga       1008
   Thr Ser Val Leu Glu Met Ile Ala Gln Ala Arg Ile Phe Pro Arg Gly
                   325                 330                 335
   tct ggg gag atg atg aca cgt tct cca gtt aag gtg act ctg agt gaa       1056
   Ser Gly Glu Met Met Thr Arg Ser Pro Val Lys Val Thr Leu Ser Glu
               340                 345                 350
   ggt cct cac cat gtg gcc cta ttt aaa gat agt tct cgg gag ttt gat       1104
   Gly Pro His His Val Ala Leu Phe Lys Asp Ser Ser Arg Glu Phe Asp
           355                 360                 365
   ctt acc aaa gaa gaa gat ctt gca gca tta aga cat gaa ata gaa ctt       1152
   Leu Thr Lys Glu Glu Asp Leu Ala Ala Leu Arg His Glu Ile Glu Leu
       370                 375                 380
   cga atg agg aaa aat gtg aaa gaa ggc tgt acc gtt agc cct gag acc       1200
   Arg Met Arg Lys Asn Val Lys Glu Gly Cys Thr Val Ser Pro Glu Thr
   385                 390                 395                 400
   ata tcc tta aat gta aaa ggc cct gga cta cag agg atg gtg ctt gtt       1248
   Ile Ser Leu Asn Val Lys Gly Pro Gly Leu Gln Arg Met Val Leu Val
                   405                 410                 415
   gac tta cca ggt gtg att aat act gtg aca tca ggc atg gct cct gac       1296
   Asp Leu Pro Gly Val Ile Asn Thr Val Thr Ser Gly Met Ala Pro Asp
               420                 425                 430
   aca aag gaa act att ttc agt atc agc aaa gct tac atg cag aat cct       1344
   Thr Lys Glu Thr Ile Phe Ser Ile Ser Lys Ala Tyr Met Gln Asn Pro
           435                 440                 445
   aat gcc atc ata ctg tgt att caa gat gga tct gtg gat gct gaa cgc       1392
   Asn Ala Ile Ile Leu Cys Ile Gln Asp Gly Ser Val Asp Ala Glu Arg
       450                 455                 460
   agt att gtt aca gac ttg gtc agt caa atg gac cct cat gga agg aga       1440
   Ser Ile Val Thr Asp Leu Val Ser Gln Met Asp Pro His Gly Arg Arg
   465                 470                 475                 480
   acc ata ttc gtt ttg acc aaa gta gac ctg gca gag aaa aat gta gcc       1488
   Thr Ile Phe Val Leu Thr Lys Val Asp Leu Ala Glu Lys Asn Val Ala
                   485                 490                 495
   agt cca agc agg att cag cag ata att gaa gga aag ctc ttc cca atg      1536
   Ser Pro Ser Arg Ile Gln Gln Ile Ile Glu Gly Lys Leu Phe Pro Met
               500                 505                 510
   aaa gct tta ggt tat ttt gct gtt gta aca gga aaa ggg aac agc tct      1584
   Lys Ala Leu Gly Tyr Phe Ala Val Val Thr Gly Lys Gly Asn Ser Ser
           515                 520                 525
   gaa agc att gaa gct ata aga gaa tat gaa gaa gag ttt ttt cag aat      1632
   Glu Ser Ile Glu Ala Ile Arg Glu Tyr Glu Glu Glu Phe Phe Gln Asn
       530                 535                 540
   tca aag ctc cta aag aca agc atg cta aag gca cac caa gtg act aca      1680
   Ser Lys Leu Leu Lys Thr Ser Met Leu Lys Ala His Gln Val Thr Thr
   545                 550                 555                 560
   aga aat tta agc ctt gca gta tca gac tgc ttt tgg aaa atg gta cga      1728
   Arg Asn Leu Ser Leu Ala Val Ser Asp Cys Phe Trp Lys Met Val Arg
                   565                 570                 575
   gag tct gtt gaa caa cag gct gat agt ttc aaa gca aca cgt ttt aac      1776
   Glu Ser Val Glu Gln Gln Ala Asp Ser Phe Lys Ala Thr Arg Phe Asn
               580                 585                 590
   ctt gaa act gaa tgg aag aat aac tat cct cgc ctg cgg gaa ctt gac      1824
   Leu Glu Thr Glu Trp Lys Asn Asn Tyr Pro Arg Leu Arg Glu Leu Asp
           595                 600                 605
   cgg aat gaa cta ttt gaa aaa gct aaa aat gaa atc ctt gat gaa gtt      1872
   Arg Asn Glu Leu Phe Glu Lys Ala Lys Asn Glu Ile Leu Asp Glu Val
       610                 615                 620
   atc agt ctg agc cag gtt aca cca aaa cat tgg gag gaa atc ctt caa      1920
   Ile Ser Leu Ser Gln Val Thr Pro Lys His Trp Glu Glu Ile Leu Gln
   625                 630                 635                 640
   caa tct ttg tgg gaa aga gta tca act cat gtg att gaa aac atc tac      1968
   Gln Ser Leu Trp Glu Arg Val Ser Thr His Val Ile Glu Asn Ile Tyr
                   645                 650                 655
   ctt cca gct gcg cag acc atg aat tca gga act ttt aac acc aca gtg      2016
   Leu Pro Ala Ala Gln Thr Met Asn Ser Gly Thr Phe Asn Thr Thr Val
               660                 665                 670
   gat atc aag ctt aaa cag tgg act gat aaa caa ctt cct aat aaa gca      2064
   Asp Ile Lys Leu Lys Gln Trp Thr Asp Lys Gln Leu Pro Asn Lys Ala
           675                 680                 685
   gta gag gtt gct tgg gag acc cta caa gaa gaa ttt tcc cgc ttt atg      2112
   Val Glu Val Ala Trp Glu Thr Leu Gln Glu Glu Phe Ser Arg Phe Met
       690                 695                 700
   aca gaa ccg aaa ggg aaa gag cat gat gac ata ttt gat aaa ctt aaa      2160
   Thr Glu Pro Lys Gly Lys Glu His Asp Asp Ile Phe Asp Lys Leu Lys
   705                 710                 715                 720
   gag gcc gtt aag gaa gaa agt att aaa cga cac aag tgg aat gac ttt      2208
   Glu Ala Val Lys Glu Glu Ser Ile Lys Arg His Lys Trp Asn Asp Phe
                   725                 730                 735
   gcg gag gac agc ttg agg gtt att caa cac aat gct ttg gaa gac cga      2256
   Ala Glu Asp Ser Leu Arg Val Ile Gln His Asn Ala Leu Glu Asp Arg
               740                 745                 750
   tcc ata tct gat aaa cag caa tgg gat gca gct att tat ttt atg gaa      2304
   Ser Ile Ser Asp Lys Gln Gln Trp Asp Ala Ala Ile Tyr Phe Met Glu
           755                 760                 765
   gag gct ctg cag gct cgt ctc aag gat act gaa aat gca att gaa aac      2352
   Glu Ala Leu Gln Ala Arg Leu Lys Asp Thr Glu Asn Ala Ile Glu Asn
       770                 775                 780
   atg gtg ggt cca gac tgg aaa aag agg tgg tta tac tgg aag aat cgg      2400
   Met Val Gly Pro Asp Trp Lys Lys Arg Trp Leu Tyr Trp Lys Asn Arg
   785                 790                 795                 800
   acc caa gaa cag tgt gtt cac aat gaa acc aag aat gaa ttg gag aag      2448
   Thr Gln Glu Gln Cys Val His Asn Glu Thr Lys Asn Glu Leu Glu Lys
                   805                 810                 815
   atg ttg aaa tgt aat gag gag cac cca gct tat ctt gca agt gat gaa      2496
   Met Leu Lys Cys Asn Glu Glu His Pro Ala Tyr Leu Ala Ser Asp Glu
               820                 825                 830
   ata acc aca gtc cgg aag aac ctt gaa tcc cga gga gta gaa gta gat      2544
   Ile Thr Thr Val Arg Lys Asn Leu Glu Ser Arg Gly Val Glu Val Asp
           835                 840                 845
   cca agc ttg att aag gat act tgg cat caa gtt tat aga aga cat ttt      2592
   Pro Ser Leu Ile Lys Asp Thr Trp His Gln Val Tyr Arg Arg His Phe
       850                 855                 860
   tta aaa aca gct cta aac cat tgt aac ctt tgt cga aga ggt ttt tat      2640
   Leu Lys Thr Ala Leu Asn His Cys Asn Leu Cys Arg Arg Gly Phe Tyr
   865                 870                 875                 880
   tac tac caa agg cat ttt gta gat tct gag ttg gaa tgc aat gat gtg      2688
   Tyr Tyr Gln Arg His Phe Val Asp Ser Glu Leu Glu Cys Asn Asp Val
                   885                 890                 895
   gtc ttg ttt tgg cgt ata cag cgc atg ctt gct atc acc gca aat act      2736
   Val Leu Phe Trp Arg Ile Gln Arg Met Leu Ala Ile Thr Ala Asn Thr
               900                 905                 910
   tta agg caa caa ctt aca aat act gaa gtt agg cga tta gag aaa aat      2784
   Leu Arg Gln Gln Leu Thr Asn Thr Glu Val Arg Arg Leu Glu Lys Asn
           915                 920                 925
   gtt aaa gag gta ttg gaa gat ttt gct gaa gat ggt gag aag aag att      2832
   Val Lys Glu Val Leu Glu Asp Phe Ala Glu Asp Gly Glu Lys Lys Ile
       930                 935                 940
   aaa ttg ctt act ggt aaa cgc gtt caa ctg gcg gaa gac ctc aag aaa      2880
   Lys Leu Leu Thr Gly Lys Arg Val Gln Leu Ala Glu Asp Leu Lys Lys
   945                 950                 955                 960
   gtt aga gaa att caa gaa aaa ctt gat gct ttc att gaa gct ctt cat      2928
   Val Arg Glu Ile Gln Glu Lys Leu Asp Ala Phe Ile Glu Ala Leu His
                   965                 970                 975
   cag gag aaa taa 2940
   Gln Glu Lys
<210> 6
   <211> 979
   <212> PRT
   <213> Homo sapiens
<400> 6
   Met Trp Arg Leu Arg Arg Ala Ala Val Ala Cys Glu Val Cys Gln Ser
   1               5                   10                  15
   Leu Val Lys His Ser Ser Gly Ile Lys Gly Ser Leu Pro Leu Gln Lys
               20                  25                  30
   Leu His Leu Val Ser Arg Ser Ile Tyr His Ser His His Pro Thr Leu
           35                  40                  45
   Lys Leu Gln Arg Pro Gln Leu Arg Thr Ser Phe Gln Gln Phe Ser Ser
       50                  55                  60
   Leu Thr Asn Leu Pro Leu Arg Lys Leu Lys Phe Ser Pro Ile Lys Tyr
   65                  70                  75                  80
   Gly Tyr Gln Pro Arg Arg Asn Phe Trp Pro Ala Arg Leu Ala Thr Arg
                   85                  90                  95
   Leu Leu Lys Leu Arg Tyr Leu Ile Leu Gly Ser Ala Val Gly Gly Gly
               100                 105                 110
   Tyr Thr Ala Lys Lys Thr Phe Asp Gln Trp Lys Asp Met Ile Pro Asp
           115                 120                 125
   Leu Ser Glu Tyr Lys Trp Ile Val Pro Asp Ile Val Trp Glu Ile Asp
       130                 135                 140
   Glu Tyr Ile Asp Phe Gly His Lys Leu Val Ser Glu Val Ile Gly Ala
   145                 150                 155                 160
   Ser Asp Leu Leu Leu Leu Leu Gly Ser Pro Glu Glu Thr Ala Phe Arg
                   165                 170                 175
   Ala Thr Asp Arg Gly Ser Glu Ser Asp Lys His Phe Arg Lys Gly Leu
               180                 185                 190
   Leu Gly Glu Leu Ile Leu Leu Gln Gln Gln Ile Gln Glu His Glu Glu
   195 200 205
   Glu Ala Arg Arg Ala Ala Gly Gln Tyr Ser Thr Ser Tyr Ala Gln Gln
   210 215 220
   Lys Arg Lys Val Ser Asp Lys Glu Lys Ile Asp Gln Leu Gln Glu Glu
   225 230 235 240
   Leu Leu His Thr Gln Leu Lys Tyr Gln Arg Ile Leu Glu Arg Leu Glu
   245 250 255
   Lys Glu Asn Lys Glu Leu Arg Lys Leu Val Leu Gln Lys Asp Asp Lys
   260 265 270
   Gly Ile His His Arg Lys Leu Lys Lys Ser Leu Ile Asp Met Tyr Ser
   275 280 285
   Glu Val Leu Asp Val Leu Ser Asp Tyr Asp Ala Ser Tyr Asn Thr Gln
   290 295 300
   Asp His Leu Pro Arg Val Val Val Val Gly Asp Gln Ser Ala Gly Lys
   305 310 315 320
   Thr Ser Val Leu Glu Met Ile Ala Gln Ala Arg Ile Phe Pro Arg Gly
   325 330 335
   Ser Gly Glu Met Met Thr Arg Ser Pro Val Lys Val Thr Leu Ser Glu
   340 345 350
   Gly Pro His His Val Ala Leu Phe Lys Asp Ser Ser Arg Glu Phe Asp
   355 360 365
   Leu Thr Lys Glu Glu Asp Leu Ala Ala Leu Arg His Glu Ile Glu Leu
   370 375 380
   Arg Met Arg Lys Asn Val Lys Glu Gly Cys Thr Val Ser Pro Glu Thr
   385 390 395 400
   Ile Ser Leu Asn Val Lys Gly Pro Gly Leu Gln Arg Met Val Leu Val
   405 410 415
   Asp Leu Pro Gly Val Ile Asn Thr Val Thr Ser Gly Met Ala Pro Asp
   420 425 430
   Thr Lys Glu Thr Ile Phe Ser Ile Ser Lys Ala Tyr Met Gln Asn Pro
   435 440 445
   Asn Ala Ile Ile Leu Cys Ile Gln Asp Gly Ser Val Asp Ala Glu Arg
   450 455 460
   Ser Ile Val Thr Asp Leu Val Ser Gln Met Asp Pro His Gly Arg Arg
   465 470 475 480
   Thr Ile Phe Val Leu Thr Lys Val Asp Leu Ala Glu Lys Asn Val Ala
   485 490 495
   Ser Pro Ser Arg Ile Gln Gln Ile Ile Glu Gly Lys Leu Phe Pro Met
   500 505 510
   Lys Ala Leu Gly Tyr Phe Ala Val Val Thr Gly Lys Gly Asn Ser Ser
   515 520 525
   Glu Ser Ile Glu Ala Ile Arg Glu Tyr Glu Glu Glu Phe Phe Gln Asn
   530 535 540
   Ser Lys Leu Leu Lys Thr Ser Met Leu Lys Ala His Gln Val Thr Thr
   545 550 555 560
   Arg Asn Leu Ser Leu Ala Val Ser Asp Cys Phe Trp Lys Met Val Arg
   565 570 575
   Glu Ser Val Glu Gln Gln Ala Asp Ser Phe Lys Ala Thr Arg Phe Asn
   580 585 590
   Leu Glu Thr Glu Trp Lys Asn Asn Tyr Pro Arg Leu Arg Glu Leu Asp
   595 600 605
   Arg Asn Glu Leu Phe Glu Lys Ala Lys Asn Glu Ile Leu Asp Glu Val
   610 615 620
   Ile Ser Leu Ser Gln Val Thr Pro Lys His Trp Glu Glu Ile Leu Gln
   625 630 635 640
   Gln Ser Leu Trp Glu Arg Val Ser Thr His Val Ile Glu Asn Ile Tyr
   645 650 655
   Leu Pro Ala Ala Gln Thr Met Asn Ser Gly Thr Phe Asn Thr Thr Val
   660 665 670
   Asp Ile Lys Leu Lys Gln Trp Thr Asp Lys Gln Leu Pro Asn Lys Ala
   675 680 685
   Val Glu Val Ala Trp Glu Thr Leu Gln Glu Glu Phe Ser Arg Phe Met
   690 695 700
   Thr Glu Pro Lys Gly Lys Glu His Asp Asp Ile Phe Asp Lys Leu Lys
   705 710 715 720
   Glu Ala Val Lys Glu Glu Ser Ile Lys Arg His Lys Trp Asn Asp Phe
   725 730 735
   Ala Glu Asp Ser Leu Arg Val Ile Gln His Asn Ala Leu Glu Asp Arg
   740 745 750
   Ser Ile Ser Asp Lys Gln Gln Trp Asp Ala Ala Ile Tyr Phe Met Glu
   755 760 765
   Glu Ala Leu Gln Ala Arg Leu Lys Asp Thr Glu Asn Ala Ile Glu Asn
   770 775 780
   Met Val Gly Pro Asp Trp Lys Lys Arg Trp Leu Tyr Trp Lys Asn Arg
   785 790 795 800
   Thr Gln Glu Gln Cys Val His Asn Glu Thr Lys Asn Glu Leu Glu Lys
   805 810 815
   Met Leu Lys Cys Asn Glu Glu His Pro Ala Tyr Leu Ala Ser Asp Glu
   820 825 830
   Ile Thr Thr Val Arg Lys Asn Leu Glu Ser Arg Gly Val Glu Val Asp
   835 840 845
   Pro Ser Leu Ile Lys Asp Thr Trp His Gln Val Tyr Arg Arg His Phe
   850 855 860
   Leu Lys Thr Ala Leu Asn His Cys Asn Leu Cys Arg Arg Gly Phe Tyr
   865 870 875 880
   Tyr Tyr Gln Arg His Phe Val Asp Ser Glu Leu Glu Cys Asn Asp Val
   885 890 895
   Val Leu Phe Trp Arg Ile Gln Arg Met Leu Ala Ile Thr Ala Asn Thr
   900 905 910
   Leu Arg Gln Gln Leu Thr Asn Thr Glu Val Arg Arg Leu Glu Lys Asn
           915                 920                 925
   Val Lys Glu Val Leu Glu Asp Phe Ala Glu Asp Gly Glu Lys Lys Ile
       930                 935                 940
   Lys Leu Leu Thr Gly Lys Arg Val Gln Leu Ala Glu Asp Leu Lys Lys
   945                 950                 955                 960
   Val Arg Glu Ile Gln Glu Lys Leu Asp Ala Phe Ile Glu Ala Leu His
                   965                 970                 975
   Gln Glu Lys
<210> 7
   <211> 2937
   <212> DNA
   <213> Homo sapiens
<220>
   <221> CDS
   <222> (1)..(2937)
<400> 7
   atg tgg cga cta cgt cgg gcc gct gtg gcc tgt gag gtc tgc cag tct       48
   Met Trp Arg Leu Arg Arg Ala Ala Val Ala Cys Glu Val Cys Gln Ser
   1               5                   10                  15
   tta gtg aaa cac agc tct gga ata aaa gga agt tta cca cta caa aaa       96
   Leu Val Lys His Ser Ser Gly Ile Lys Gly Ser Leu Pro Leu Gln Lys
               20                  25                  30
   cta cat ctg gtt tca cga agc att tat cat tca cat cat cct acc tta      144
   Leu His Leu Val Ser Arg Ser Ile Tyr His Ser His His Pro Thr Leu
           35                  40                  45
   aag ctt caa cga ccc caa tta agg aca tcc ttt cag cag ttc tct tct      192
   Lys Leu Gln Arg Pro Gln Leu Arg Thr Ser Phe Gln Gln Phe Ser Ser
       50                  55                  60
   ctg aca aac ctt cct tta cgt aaa ctg aaa ttc tct cca att aaa tat      240
   Leu Thr Asn Leu Pro Leu Arg Lys Leu Lys Phe Ser Pro Ile Lys Tyr
   65                  70                  75                  80
   ggc tac cag cct cgc agg aat ttt tgg cca gca aga tta gct acg aga      288
   Gly Tyr Gln Pro Arg Arg Asn Phe Trp Pro Ala Arg Leu Ala Thr Arg
                   85                  90                  95
   ctc tta aaa ctt cgc tat ctc ata cta gga tcg gct gtt ggg ggt ggc      336
   Leu Leu Lys Leu Arg Tyr Leu Ile Leu Gly Ser Ala Val Gly Gly Gly
               100                 105                 110
   tac aca gcc aaa aag act ttt gat cag tgg aaa gat atg ata ccg gac       384
   Tyr Thr Ala Lys Lys Thr Phe Asp Gln Trp Lys Asp Met Ile Pro Asp
           115                 120                 125
   ctt agt gaa tat aaa tgg att gtg cct gac att gtg tgg gaa att gat       432
   Leu Ser Glu Tyr Lys Trp Ile Val Pro Asp Ile Val Trp Glu Ile Asp
       130                 135                 140
   gag tat atc gat ttt gag aaa att aga aaa gcc ctt cct aat tca gaa       480
   Glu Tyr Ile Asp Phe Glu Lys Ile Arg Lys Ala Leu Pro Asn Ser Glu
   145                 150                 155                 160
   gac ctt gta aag tta gca cca gac ttt gac aag att gtt gaa agc ctt       528
   Asp Leu Val Lys Leu Ala Pro Asp Phe Asp Lys Ile Val Glu Ser Leu
                   165                 170                 175
   agc tta ttg aag gac ttt ttt acc tca ggt cac aaa ttg gtt agt gaa       576
   Ser Leu Leu Lys Asp Phe Phe Thr Ser Gly His Lys Leu Val Ser Glu
               180                 185                 190
   gtc ata gga gct tct gac cta ctt ctc ttg tta ggt tct ccg gaa gaa       624
   Val Ile Gly Ala Ser Asp Leu Leu Leu Leu Leu Gly Ser Pro Glu Glu
           195                 200                 205
   acg gcg ttt aga gca aca gat cgt gga tct gaa agt gac aag cat ttt       672
   Thr Ala Phe Arg Ala Thr Asp Arg Gly Ser Glu Ser Asp Lys His Phe
       210                 215                 220
   aga aag gtg tca gac aaa gag aaa att gac caa ctt cag gaa gaa ctt       720
   Arg Lys Val Ser Asp Lys Glu Lys Ile Asp Gln Leu Gln Glu Glu Leu
   225                 230                 235                 240
   ctg cac act cag ttg aag tat cag aga atc ttg gaa cga tta gaa aag       768
   Leu His Thr Gln Leu Lys Tyr Gln Arg Ile Leu Glu Arg Leu Glu Lys
                   245                 250                 255
   gag aac aaa gaa ttg aga aaa tta gta ttg cag aaa gat gac aaa ggc       816
   Glu Asn Lys Glu Leu Arg Lys Leu Val Leu Gln Lys Asp Asp Lys Gly
               260                 265                 270
   att cat cat aga aag ctt aag aaa tct ttg att gac atg tat tct gaa       864
   Ile His His Arg Lys Leu Lys Lys Ser Leu Ile Asp Met Tyr Ser Glu
           275                 280                 285
   gtt ctt gat gtt ctc tct gat tat gat gcc agt tat aat acg caa gat       912
   Val Leu Asp Val Leu Ser Asp Tyr Asp Ala Ser Tyr Asn Thr Gln Asp
       290                 295                 300
   cat ctg cca cgg gtt gtt gtg gtt gga gat cag agt gct gga aag act       960
   His Leu Pro Arg Val Val Val Val Gly Asp Gln Ser Ala Gly Lys Thr
   305                 310                 315                 320
   agt gtg ttg gaa atg att gcc caa gct cga ata ttc cca aga gga tct      1008
   Ser Val Leu Glu Met Ile Ala Gln Ala Arg Ile Phe Pro Arg Gly Ser
                   325                 330                 335
   ggg gag atg atg aca cgt tct cca gtt aag gtg act ctg agt gaa ggt      1056
   Gly Glu Met Met Thr Arg Ser Pro Val Lys Val Thr Leu Ser Glu Gly
               340                 345                 350
   cct cac cat gtg gcc cta ttt aaa gat agt tct cgg gag ttt gat ctt      1104
   Pro His His Val Ala Leu Phe Lys Asp Ser Ser Arg Glu Phe Asp Leu
           355                 360                 365
   acc aaa gaa gaa gat ctt gca gca tta aga cat gaa ata gaa ctt cga      1152
   Thr Lys Glu Glu Asp Leu Ala Ala Leu Arg His Glu Ile Glu Leu Arg
       370                 375                 380
   atg agg aaa aat gtg aaa gaa ggc tgt acc gtt agc cct gag acc ata      1200
   Met Arg Lys Asn Val Lys Glu Gly Cys Thr Val Ser Pro Glu Thr Ile
   385                 390                 395                 400
   tcc tta aat gta aaa ggc cct gga cta cag agg atg gtg ctt gtt gac      1248
   Ser Leu Asn Val Lys Gly Pro Gly Leu Gln Arg Met Val Leu Val Asp
                   405                 410                 415
   tta cca ggt gtg att aat act gtg aca tca ggc atg gct cct gac aca      1296
   Leu Pro Gly Val Ile Asn Thr Val Thr Ser Gly Met Ala Pro Asp Thr
               420                 425                 430
   aag gaa act att ttc agt atc agc aaa gct tac atg cag aat cct aat      1344
   Lys Glu Thr Ile Phe Ser Ile Ser Lys Ala Tyr Met Gln Asn Pro Asn
           435                 440                 445
   gcc atc ata ctg tgt att caa gat gga tct gtg gat gct gaa cgc agt      1392
   Ala Ile Ile Leu Cys Ile Gln Asp Gly Ser Val Asp Ala Glu Arg Ser
       450                 455                 460
   att gtt aca gac ttg gtc agt caa atg gac cct cat gga agg aga acc      1440
   Ile Val Thr Asp Leu Val Ser Gln Met Asp Pro His Gly Arg Arg Thr
   465                 470                 475                 480
   ata ttc gtt ttg acc aaa gta gac ctg gca gag aaa aat gta gcc agt      1488
   Ile Phe Val Leu Thr Lys Val Asp Leu Ala Glu Lys Asn Val Ala Ser
                   485                 490                 495
   cca agc agg att cag cag ata att gaa gga aag ctc ttc cca atg aaa      1536
   Pro Ser Arg Ile Gln Gln Ile Ile Glu Gly Lys Leu Phe Pro Met Lys
               500                 505                 510
   gct tta ggt tat ttt gct gtt gta aca gga aaa ggg aac agc tct gaa      1584
   Ala Leu Gly Tyr Phe Ala Val Val Thr Gly Lys Gly Asn Ser Ser Glu
           515                 520                 525
   agc att gaa gct ata aga gaa tat gaa gaa gag ttt ttt cag aat tca      1632
   Ser Ile Glu Ala Ile Arg Glu Tyr Glu Glu Glu Phe Phe Gln Asn Ser
       530                 535                 540
   aag ctc cta aag aca agc atg cta aag gca cac caa gtg act aca aga      1680
   Lys Leu Leu Lys Thr Ser Met Leu Lys Ala His Gln Val Thr Thr Arg
   545                 550                 555                 560
   aat tta agc ctt gca gta tca gac tgc ttt tgg aaa atg gta cga gag      1728
   Asn Leu Ser Leu Ala Val Ser Asp Cys Phe Trp Lys Met Val Arg Glu
                   565                 570                 575
   tct gtt gaa caa cag gct gat agt ttc aaa gca aca cgt ttt aac ctt      1776
   Ser Val Glu Gln Gln Ala Asp Ser Phe Lys Ala Thr Arg Phe Asn Leu
               580                 585                 590
   gaa act gaa tgg aag aat aac tat cct cgc ctg cgg gaa ctt gac cgg      1824
   Glu Thr Glu Trp Lys Asn Asn Tyr Pro Arg Leu Arg Glu Leu Asp Arg
           595                 600                 605
   aat gaa cta ttt gaa aaa gct aaa aat gaa atc ctt gat gaa gtt atc      1872
   Asn Glu Leu Phe Glu Lys Ala Lys Asn Glu Ile Leu Asp Glu Val Ile
       610                 615                 620
   agt ctg agc cag gtt aca cca aaa cat tgg gag gaa atc ctt caa caa      1920
   Ser Leu Ser Gln Val Thr Pro Lys His Trp Glu Glu Ile Leu Gln Gln
   625                 630                 635                 640
   tct ttg tgg gaa aga gta tca act cat gtg att gaa aac atc tac ctt      1968
   Ser Leu Trp Glu Arg Val Ser Thr His Val Ile Glu Asn Ile Tyr Leu
                   645                 650                 655
   cca gct gcg cag acc atg aat tca gga act ttt aac acc aca gtg gat      2016
   Pro Ala Ala Gln Thr Met Asn Ser Gly Thr Phe Asn Thr Thr Val Asp
               660                 665                 670
   atc aag ctt aaa cag tgg act gat aaa caa ctt cct aat aaa gca gta      2064
   Ile Lys Leu Lys Gln Trp Thr Asp Lys Gln Leu Pro Asn Lys Ala Val
           675                 680                 685
   gag gtt gct tgg gag acc cta caa gaa gaa ttt tcc cgc ttt atg aca      2112
   Glu Val Ala Trp Glu Thr Leu Gln Glu Glu Phe Ser Arg Phe Met Thr
       690                 695                 700
   gaa ccg aaa ggg aaa gag cat gat gac ata ttt gat aaa ctt aaa gag      2160
   Glu Pro Lys Gly Lys Glu His Asp Asp Ile Phe Asp Lys Leu Lys Glu
   705                 710                 715                 720
   gcc gtt aag gaa gaa agt att aaa cga cac aag tgg aat gac ttt gcg      2208
   Ala Val Lys Glu Glu Ser Ile Lys Arg His Lys Trp Asn Asp Phe Ala
                   725                 730                 735
   gag gac agc ttg agg gtt att caa cac aat gct ttg gaa gac cga tcc      2256
   Glu Asp Ser Leu Arg Val Ile Gln His Asn Ala Leu Glu Asp Arg Ser
               740                 745                 750
   ata tct gat aaa cag caa tgg gat gca gct att tat ttt atg gaa gag      2304
   Ile Ser Asp Lys Gln Gln Trp Asp Ala Ala Ile Tyr Phe Met Glu Glu
           755                 760                 765
   gct ctg cag gct cgt ctc aag gat act gaa aat gca att gaa aac atg      2352
   Ala Leu Gln Ala Arg Leu Lys Asp Thr Glu Asn Ala Ile Glu Asn Met
       770                 775                 780
   gtg ggt cca gac tgg aaa aag agg tgg tta tac tgg aag aat cgg acc      2400
   Val Gly Pro Asp Trp Lys Lys Arg Trp Leu Tyr Trp Lys Asn Arg Thr
   785                 790                 795                 800
   caa gaa cag tgt gtt cac aat gaa acc aag aat gaa ttg gag aag atg      2448
   Gln Glu Gln Cys Val His Asn Glu Thr Lys Asn Glu Leu Glu Lys Met
                   805                 810                 815
   ttg aaa tgt aat gag gag cac cca gct tat ctt gca agt gat gaa ata      2496
   Leu Lys Cys Asn Glu Glu His Pro Ala Tyr Leu Ala Ser Asp Glu Ile
               820                 825                 830
   acc aca gtc cgg aag aac ctt gaa tcc cga gga gta gaa gta gat cca      2544
   Thr Thr Val Arg Lys Asn Leu Glu Ser Arg Gly Val Glu Val Asp Pro
           835                 840                 845
   agc ttg att aag gat act tgg cat caa gtt tat aga aga cat ttt tta      2592
   Ser Leu Ile Lys Asp Thr Trp His Gln Val Tyr Arg Arg His Phe Leu
       850                 855                 860
   aaa aca gct cta aac cat tgt aac ctt tgt cga aga ggt ttt tat tac      2640
   Lys Thr Ala Leu Asn His Cys Asn Leu Cys Arg Arg Gly Phe Tyr Tyr
   865                 870                 875                 880
   tac caa agg cat ttt gta gat tct gag ttg gaa tgc aat gat gtg gtc      2688
   Tyr Gln Arg His Phe Val Asp Ser Glu Leu Glu Cys Asn Asp Val Val
                   885                 890                 895
   ttg ttt tgg cgt ata cag cgc atg ctt gct atc acc gca aat act tta      2736
   Leu Phe Trp Arg Ile Gln Arg Met Leu Ala Ile Thr Ala Asn Thr Leu
               900                 905                 910
   agg caa caa ctt aca aat act gaa gtt agg cga tta gag aaa aat gtt      2784
   Arg Gln Gln Leu Thr Asn Thr Glu Val Arg Arg Leu Glu Lys Asn Val
           915                 920                 925
   aaa gag gta ttg gaa gat ttt gct gaa gat ggt gag aag aag att aaa      2832
   Lys Glu Val Leu Glu Asp Phe Ala Glu Asp Gly Glu Lys Lys Ile Lys
       930                 935                 940
   ttg ctt act ggt aaa cgc gtt caa ctg gcg gaa gac ctc aag aaa gtt      2880
   Leu Leu Thr Gly Lys Arg Val Gln Leu Ala Glu Asp Leu Lys Lys Val
   945                 950                 955                 960
   aga gaa att caa gaa aaa ctt gat gct ttc att gaa gct ctt cat cag      2928
   Arg Glu Ile Gln Glu Lys Leu Asp Ala Phe Ile Glu Ala Leu His Gln
                   965                 970                 975
   gag aaa taa                                                          2937
   Glu Lys
<210> 8
   <211> 978
   <212> PRT
   <213> Homo sapiens
<400> 8
   Met Trp Arg Leu Arg Arg Ala Ala Val Ala Cys Glu Val Cys Gln Ser
   1               5                   10                  15
   Leu Val Lys His Ser Ser Gly Ile Lys Gly Ser Leu Pro Leu Gln Lys
               20                  25                  30
   Leu His Leu Val Ser Arg Ser Ile Tyr His Ser His His Pro Thr Leu
           35                  40                  45
   Lys Leu Gln Arg Pro Gln Leu Arg Thr Ser Phe Gln Gln Phe Ser Ser
       50                  55                  60
   Leu Thr Asn Leu Pro Leu Arg Lys Leu Lys Phe Ser Pro Ile Lys Tyr
   65                  70                  75                  80
   Gly Tyr Gln Pro Arg Arg Asn Phe Trp Pro Ala Arg Leu Ala Thr Arg
                   85                  90                  95
   Leu Leu Lys Leu Arg Tyr Leu Ile Leu Gly Ser Ala Val Gly Gly Gly
               100                 105                 110
   Tyr Thr Ala Lys Lys Thr Phe Asp Gln Trp Lys Asp Met Ile Pro Asp
           115                 120                 125
   Leu Ser Glu Tyr Lys Trp Ile Val Pro Asp Ile Val Trp Glu Ile Asp
       130                 135                 140
   Glu Tyr Ile Asp Phe Glu Lys Ile Arg Lys Ala Leu Pro Asn Ser Glu
   145                 150                 155                 160
   Asp Leu Val Lys Leu Ala Pro Asp Phe Asp Lys Ile Val Glu Ser Leu
                   165                 170                 175
   Ser Leu Leu Lys Asp Phe Phe Thr Ser Gly His Lys Leu Val Ser Glu
               180                 185                 190
   Val Ile Gly Ala Ser Asp Leu Leu Leu Leu Leu Gly Ser Pro Glu Glu
           195                 200                 205
   Thr Ala Phe Arg Ala Thr Asp Arg Gly Ser Glu Ser Asp Lys His Phe
       210                 215                 220
   Arg Lys Val Ser Asp Lys Glu Lys Ile Asp Gln Leu Gln Glu Glu Leu
   225                 230                 235                 240
   Leu His Thr Gln Leu Lys Tyr Gln Arg Ile Leu Glu Arg Leu Glu Lys
                   245                 250                 255
   Glu Asn Lys Glu Leu Arg Lys Leu Val Leu Gln Lys Asp Asp Lys Gly
               260                 265                 270
   Ile His His Arg Lys Leu Lys Lys Ser Leu Ile Asp Met Tyr Ser Glu
           275                 280                 285
   Val Leu Asp Val Leu Ser Asp Tyr Asp Ala Ser Tyr Asn Thr Gln Asp
       290                 295                 300
   His Leu Pro Arg Val Val Val Val Gly Asp Gln Ser Ala Gly Lys Thr
   305                 310                 315                 320
   Ser Val Leu Glu Met Ile Ala Gln Ala Arg Ile Phe Pro Arg Gly Ser
                   325                 330                 335
   Gly Glu Met Met Thr Arg Ser Pro Val Lys Val Thr Leu Ser Glu Gly
               340                 345                 350
   Pro His His Val Ala Leu Phe Lys Asp Ser Ser Arg Glu Phe Asp Leu
           355                 360                 365
   Thr Lys Glu Glu Asp Leu Ala Ala Leu Arg His Glu Ile Glu Leu Arg
       370                 375                 380
   Met Arg Lys Asn Val Lys Glu Gly Cys Thr Val Ser Pro Glu Thr Ile
   385                 390                 395                 400
   Ser Leu Asn Val Lys Gly Pro Gly Leu Gln Arg Met Val Leu Val Asp
                   405                 410                 415
   Leu Pro Gly Val Ile Asn Thr Val Thr Ser Gly Met Ala Pro Asp Thr
               420                 425                 430
   Lys Glu Thr Ile Phe Ser Ile Ser Lys Ala Tyr Met Gln Asn Pro Asn
           435                 440                 445
   Ala Ile Ile Leu Cys Ile Gln Asp Gly Ser Val Asp Ala Glu Arg Ser
       450                 455                 460
   Ile Val Thr Asp Leu Val Ser Gln Met Asp Pro His Gly Arg Arg Thr
   465                 470                 475                 480
   Ile Phe Val Leu Thr Lys Val Asp Leu Ala Glu Lys Asn Val Ala Ser
                   485                 490                 495
   Pro Ser Arg Ile Gln Gln Ile Ile Glu Gly Lys Leu Phe Pro Met Lys
               500                 505                 510
   Ala Leu Gly Tyr Phe Ala Val Val Thr Gly Lys Gly Asn Ser Ser Glu
           515                 520                 525
   Ser Ile Glu Ala Ile Arg Glu Tyr Glu Glu Glu Phe Phe Gln Asn Ser
   530 535 540
   Lys Leu Leu Lys Thr Ser Met Leu Lys Ala His Gln Val Thr Thr Arg
   545 550 555 560
   Asn Leu Ser Leu Ala Val Ser Asp Cys Phe Trp Lys Met Val Arg Glu
   565 570 575
   Ser Val Glu Gln Gln Ala Asp Ser Phe Lys Ala Thr Arg Phe Asn Leu
   580 585 590
   Glu Thr Glu Trp Lys Asn Asn Tyr Pro Arg Leu Arg Glu Leu Asp Arg
   595 600 605
   Asn Glu Leu Phe Glu Lys Ala Lys Asn Glu Ile Leu Asp Glu Val Ile
   610 615 620
   Ser Leu Ser Gln Val Thr Pro Lys His Trp Glu Glu Ile Leu Gln Gln
   625 630 635 640
   Ser Leu Trp Glu Arg Val Ser Thr His Val Ile Glu Asn Ile Tyr Leu
   645 650 655
   Pro Ala Ala Gln Thr Met Asn Ser Gly Thr Phe Asn Thr Thr Val Asp
   660 665 670
   Ile Lys Leu Lys Gln Trp Thr Asp Lys Gln Leu Pro Asn Lys Ala Val
   675 680 685
   Glu Val Ala Trp Glu Thr Leu Gln Glu Glu Phe Ser Arg Phe Met Thr
   690 695 700
   Glu Pro Lys Gly Lys Glu His Asp Asp Ile Phe Asp Lys Leu Lys Glu
   705 710 715 720
   Ala Val Lys Glu Glu Ser Ile Lys Arg His Lys Trp Asn Asp Phe Ala
   725 730 735
   Glu Asp Ser Leu Arg Val Ile Gln His Asn Ala Leu Glu Asp Arg Ser
   740 745 750
   Ile Ser Asp Lys Gln Gln Trp Asp Ala Ala Ile Tyr Phe Met Glu Glu
   755 760 765
   Ala Leu Gln Ala Arg Leu Lys Asp Thr Glu Asn Ala Ile Glu Asn Met
       770                 775                 780
   Val Gly Pro Asp Trp Lys Lys Arg Trp Leu Tyr Trp Lys Asn Arg Thr
   785                 790                 795                 800
   Gln Glu Gln Cys Val His Asn Glu Thr Lys Asn Glu Leu Glu Lys Met
                   805                 810                 815
   Leu Lys Cys Asn Glu Glu His Pro Ala Tyr Leu Ala Ser Asp Glu Ile
               820                 825                 830
   Thr Thr Val Arg Lys Asn Leu Glu Ser Arg Gly Val Glu Val Asp Pro
           835                 840                 845
   Ser Leu Ile Lys Asp Thr Trp His Gln Val Tyr Arg Arg His Phe Leu
       850                 855                 860
   Lys Thr Ala Leu Asn His Cys Asn Leu Cys Arg Arg Gly Phe Tyr Tyr
   865                 870                 875                 880
   Tyr Gln Arg His Phe Val Asp Ser Glu Leu Glu Cys Asn Asp Val Val
                   885                 890                 895
   Leu Phe Trp Arg Ile Gln Arg Met Leu Ala Ile Thr Ala Asn Thr Leu
               900                 905                 910
   Arg Gln Gln Leu Thr Asn Thr Glu Val Arg Arg Leu Glu Lys Asn Val
           915                 920                 925
   Lys Glu Val Leu Glu Asp Phe Ala Glu Asp Gly Glu Lys Lys Ile Lys
       930                 935                 940
   Leu Leu Thr Gly Lys Arg Val Gln Leu Ala Glu Asp Leu Lys Lys Val
   945                 950                 955                 960
   Arg Glu Ile Gln Glu Lys Leu Asp Ala Phe Ile Glu Ala Leu His Gln
                   965                 970                 975
   Glu Lys
<210> 9
   <211> 2886
   <212> DNA
   <213> Homo sapiens
<220>
   <221> CDS
   <222> (1)..(2886)
<400> 9
   atg tgg cga cta cgt cgg gcc gct gtg gcc tgt gag gtc tgc cag tct      48
   Met Trp Arg Leu Arg Arg Ala Ala Val Ala Cys Glu Val Cys Gln Ser
   1               5                   10                  15
   tta gtg aaa cac agc tct gga ata aaa gga agt tta cca cta caa aaa      96
   Leu Val Lys His Ser Ser Gly Ile Lys Gly Ser Leu Pro Leu Gln Lys
               20                  25                  30
   cta cat ctg gtt tca cga agc att tat cat tca cat cat cct acc tta      144
   Leu His Leu Val Ser Arg Ser Ile Tyr His Ser His His Pro Thr Leu
           35                  40                  45
   aag ctt caa cga ccc caa tta agg aca tcc ttt cag cag ttc tct tct      192
   Lys Leu Gln Arg Pro Gln Leu Arg Thr Ser Phe Gln Gln Phe Ser Ser
       50                  55                  60
   ctg aca aac ctt cct tta cgt aaa ctg aaa ttc tct cca att aaa tat      240
   Leu Thr Asn Leu Pro Leu Arg Lys Leu Lys Phe Ser Pro Ile Lys Tyr
   65                  70                  75                  80
   ggc tac cag cct cgc agg aat ttt tgg cca gca aga tta gct acg aga      288
   Gly Tyr Gln Pro Arg Arg Asn Phe Trp Pro Ala Arg Leu Ala Thr Arg
                   85                  90                  95
   ctc tta aaa ctt cgc tat ctc ata cta gga tcg gct gtt ggg ggt ggc      336
   Leu Leu Lys Leu Arg Tyr Leu Ile Leu Gly Ser Ala Val Gly Gly Gly
               100                 105                 110
   tac aca gcc aaa aag act ttt gat cag tgg aaa gat atg ata ccg gac      384
   Tyr Thr Ala Lys Lys Thr Phe Asp Gln Trp Lys Asp Met Ile Pro Asp
           115                 120                 125
   ctt agt gaa tat aaa tgg att gtg cct gac att gtg tgg gaa att gat      432
   Leu Ser Glu Tyr Lys Trp Ile Val Pro Asp Ile Val Trp Glu Ile Asp
       130                 135                 140
   gag tat atc gat ttt ggt tct ccg gaa gaa acg gcg ttt aga gca aca      480
   Glu Tyr Ile Asp Phe Gly Ser Pro Glu Glu Thr Ala Phe Arg Ala Thr
   145                 150                 155                 160
   gat cgt gga tct gaa agt gac aag cat ttt aga aag ggt ctg ctt ggt      528
   Asp Arg Gly Ser Glu Ser Asp Lys His Phe Arg Lys Gly Leu Leu Gly
                   165                 170                 175
   gag ctc att ctc tta caa caa caa att caa gag cat gaa gag gaa gcg      576
   Glu Leu Ile Leu Leu Gln Gln Gln Ile Gln Glu His Glu Glu Glu Ala
               180                 185                 190
   cgc aga gcc gct ggc caa tat agc acg agc tat gcc caa cag aag cgc      624
   Arg Arg Ala Ala Gly Gln Tyr Ser Thr Ser Tyr Ala Gln Gln Lys Arg
           195                 200                 205
   aag gtg tca gac aaa gag aaa att gac caa ctt cag gaa gaa ctt ctg      672
   Lys Val Ser Asp Lys Glu Lys Ile Asp Gln Leu Gln Glu Glu Leu Leu
       210                 215                 220
   cac act cag ttg aag tat cag aga atc ttg gaa cga tta gaa aag gag      720
   His Thr Gln Leu Lys Tyr Gln Arg Ile Leu Glu Arg Leu Glu Lys Glu
   225                 230                 235                 240
   aac aaa gaa ttg aga aaa tta gta ttg cag aaa gat gac aaa ggc att      768
   Asn Lys Glu Leu Arg Lys Leu Val Leu Gln Lys Asp Asp Lys Gly Ile
                   245                 250                 255
   cat cat aga aag ctt aag aaa tct ttg att gac atg tat tct gaa gtt      816
   His His Arg Lys Leu Lys Lys Ser Leu Ile Asp Met Tyr Ser Glu Val
               260                 265                 270
   ctt gat gtt ctc tct gat tat gat gcc agt tat aat acg caa gat cat      864
   Leu Asp Val Leu Ser Asp Tyr Asp Ala Ser Tyr Asn Thr Gln Asp His
           275                 280                 285
   ctg cca cgg gtt gtt gtg gtt gga gat cag agt gct gga aag act agt      912
   Leu Pro Arg Val Val Val Val Gly Asp Gln Ser Ala Gly Lys Thr Ser
       290                 295                 300
   gtg ttg gaa atg att gcc caa gct cga ata ttc cca aga gga tct ggg      960
   Val Leu Glu Met Ile Ala Gln Ala Arg Ile Phe Pro Arg Gly Ser Gly
   305                 310                 315                 320
   gag atg atg aca cgt tct cca gtt aag gtg act ctg agt gaa ggt cct     1008
   Glu Met Met Thr Arg Ser Pro Val Lys Val Thr Leu Ser Glu Gly Pro
                   325                 330                 335
   cac cat gtg gcc cta ttt aaa gat agt tct cgg gag ttt gat ctt acc     1056
   His His Val Ala Leu Phe Lys Asp Ser Ser Arg Glu Phe Asp Leu Thr
               340                 345                 350
   aaa gaa gaa gat ctt gca gca tta aga cat gaa ata gaa ctt cga atg     1104
   Lys Glu Glu Asp Leu Ala Ala Leu Arg His Glu Ile Glu Leu Arg Met
           355                 360                 365
   agg aaa aat gtg aaa gaa ggc tgt acc gtt agc cct gag acc ata tcc     1152
   Arg Lys Asn Val Lys Glu Gly Cys Thr Val Ser Pro Glu Thr Ile Ser
       370                 375                 380
   tta aat gta aaa ggc cct gga cta cag agg atg gtg ctt gtt gac tta     1200
   Leu Asn Val Lys Gly Pro Gly Leu Gln Arg Met Val Leu Val Asp Leu
   385                 390                 395                 400
   cca ggt gtg att aat act gtg aca tca ggc atg gct cct gac aca aag     1248
   Pro Gly Val Ile Asn Thr Val Thr Ser Gly Met Ala Pro Asp Thr Lys
                   405                 410                 415
   gaa act att ttc agt atc agc aaa gct tac atg cag aat cct aat gcc     1296
   Glu Thr Ile Phe Ser Ile Ser Lys Ala Tyr Met Gln Asn Pro Asn Ala
               420                 425                 430
   atc ata ctg tgt att caa gat gga tct gtg gat gct gaa cgc agt att     1344
   Ile Ile Leu Cys Ile Gln Asp Gly Ser Val Asp Ala Glu Arg Ser Ile
           435                 440                 445
   gtt aca gac ttg gtc agt caa atg gac cct cat gga agg aga acc ata     1392
   Val Thr Asp Leu Val Ser Gln Met Asp Pro His Gly Arg Arg Thr Ile
       450                 455                 460
   ttc gtt ttg acc aaa gta gac ctg gca gag aaa aat gta gcc agt cca      1440
   Phe Val Leu Thr Lys Val Asp Leu Ala Glu Lys Asn Val Ala Ser Pro
   465                 470                 475                 480
   agc agg att cag cag ata att gaa gga aag ctc ttc cca atg aaa gct      1488
   Ser Arg Ile Gln Gln Ile Ile Glu Gly Lys Leu Phe Pro Met Lys Ala
                   485                 490                 495
   tta ggt tat ttt gct gtt gta aca gga aaa ggg aac agc tct gaa agc      1536
   Leu Gly Tyr Phe Ala Val Val Thr Gly Lys Gly Asn Ser Ser Glu Ser
               500                 505                 510
   att gaa gct ata aga gaa tat gaa gaa gag ttt ttt cag aat tca aag      1584
   Ile Glu Ala Ile Arg Glu Tyr Glu Glu Glu Phe Phe Gln Asn Ser Lys
           515                 520                 525
   ctc cta aag aca agc atg cta aag gca cac caa gtg act aca aga aat      1632
   Leu Leu Lys Thr Ser Met Leu Lys Ala His Gln Val Thr Thr Arg Asn
       530                 535                 540
   tta agc ctt gca gta tca gac tgc ttt tgg aaa atg gta cga gag tct      1680
   Leu Ser Leu Ala Val Ser Asp Cys Phe Trp Lys Met Val Arg Glu Ser
   545                 550                 555                 560
   gtt gaa caa cag gct gat agt ttc aaa gca aca cgt ttt aac ctt gaa      1728
   Val Glu Gln Gln Ala Asp Ser Phe Lys Ala Thr Arg Phe Asn Leu Glu
                   565                 570                 575
   act gaa tgg aag aat aac tat cct cgc ctg cgg gaa ctt gac cgg aat      1776
   Thr Glu Trp Lys Asn Asn Tyr Pro Arg Leu Arg Glu Leu Asp Arg Asn
               580                 585                 590
   gaa cta ttt gaa aaa gct aaa aat gaa atc ctt gat gaa gtt atc agt      1824
   Glu Leu Phe Glu Lys Ala Lys Asn Glu Ile Leu Asp Glu Val Ile Ser
           595                 600                 605
   ctg agc cag gtt aca cca aaa cat tgg gag gaa atc ctt caa caa tct      1872
   Leu Ser Gln Val Thr Pro Lys His Trp Glu Glu Ile Leu Gln Gln Ser
       610                 615                 620
   ttg tgg gaa aga gta tca act cat gtg att gaa aac atc tac ctt cca      1920
   Leu Trp Glu Arg Val Ser Thr His Val Ile Glu Asn Ile Tyr Leu Pro
   625                 630                 635                 640
   gct gcg cag acc atg aat tca gga act ttt aac acc aca gtg gat atc      1968
   Ala Ala Gln Thr Met Asn Ser Gly Thr Phe Asn Thr Thr Val Asp Ile
                   645                 650                 655
   aag ctt aaa cag tgg act gat aaa caa ctt cct aat aaa gca gta gag      2016
   Lys Leu Lys Gln Trp Thr Asp Lys Gln Leu Pro Asn Lys Ala Val Glu
               660                 665                 670
   gtt gct tgg gag acc cta caa gaa gaa ttt tcc cgc ttt atg aca gaa      2064
   Val Ala Trp Glu Thr Leu Gln Glu Glu Phe Ser Arg Phe Met Thr Glu
           675                 680                 685
   ccg aaa ggg aaa gag cat gat gac ata ttt gat aaa ctt aaa gag gcc      2112
   Pro Lys Gly Lys Glu His Asp Asp Ile Phe Asp Lys Leu Lys Glu Ala
       690                 695                 700
   gtt aag gaa gaa agt att aaa cga cac aag tgg aat gac ttt gcg gag      2160
   Val Lys Glu Glu Ser Ile Lys Arg His Lys Trp Asn Asp Phe Ala Glu
   705                 710                 715                 720
   gac agc ttg agg gtt att caa cac aat gct ttg gaa gac cga tcc ata      2208
   Asp Ser Leu Arg Val Ile Gln His Asn Ala Leu Glu Asp Arg Ser Ile
                   725                 730                 735
   tct gat aaa cag caa tgg gat gca gct att tat ttt atg gaa gag gct      2256
   Ser Asp Lys Gln Gln Trp Asp Ala Ala Ile Tyr Phe Met Glu Glu Ala
               740                 745                 750
   ctg cag gct cgt ctc aag gat act gaa aat gca att gaa aac atg gtg      2304
   Leu Gln Ala Arg Leu Lys Asp Thr Glu Asn Ala Ile Glu Asn Met Val
           755                 760                 765
   ggt cca gac tgg aaa aag agg tgg tta tac tgg aag aat cgg acc caa      2352
   Gly Pro Asp Trp Lys Lys Arg Trp Leu Tyr Trp Lys Asn Arg Thr Gln
       770                 775                 780
   gaa cag tgt gtt cac aat gaa acc aag aat gaa ttg gag aag atg ttg      2400
   Glu Gln Cys Val His Asn Glu Thr Lys Asn Glu Leu Glu Lys Met Leu
   785                 790                 795                 800
   aaa tgt aat gag gag cac cca gct tat ctt gca agt gat gaa ata acc      2448
   Lys Cys Asn Glu Glu His Pro Ala Tyr Leu Ala Ser Asp Glu Ile Thr
                   805                 810                 815
   aca gtc cgg aag aac ctt gaa tcc cga gga gta gaa gta gat cca agc      2496
   Thr Val Arg Lys Asn Leu Glu Ser Arg Gly Val Glu Val Asp Pro Ser
               820                 825                 830
   ttg att aag gat act tgg cat caa gtt tat aga aga cat ttt tta aaa      2544
   Leu Ile Lys Asp Thr Trp His Gln Val Tyr Arg Arg His Phe Leu Lys
           835                 840                 845
   aca gct cta aac cat tgt aac ctt tgt cga aga ggt ttt tat tac tac      2592
   Thr Ala Leu Asn His Cys Asn Leu Cys Arg Arg Gly Phe Tyr Tyr Tyr
       850                 855                 860
   caa agg cat ttt gta gat tct gag ttg gaa tgc aat gat gtg gtc ttg      2640
   Gln Arg His Phe Val Asp Ser Glu Leu Glu Cys Asn Asp Val Val Leu
   865                 870                 875                 880
   ttt tgg cgt ata cag cgc atg ctt gct atc acc gca aat act tta agg      2688
   Phe Trp Arg Ile Gln Arg Met Leu Ala Ile Thr Ala Asn Thr Leu Arg
                   885                 890                 895
   caa caa ctt aca aat act gaa gtt agg cga tta gag aaa aat gtt aaa      2736
   Gln Gln Leu Thr Asn Thr Glu Val Arg Arg Leu Glu Lys Asn Val Lys
               900                 905                 910
   gag gta ttg gaa gat ttt gct gaa gat ggt gag aag aag att aaa ttg      2784
   Glu Val Leu Glu Asp Phe Ala Glu Asp Gly Glu Lys Lys Ile Lys Leu
           915                 920                 925
   ctt act ggt aaa cgc gtt caa ctg gcg gaa gac ctc aag aaa gtt aga      2832
   Leu Thr Gly Lys Arg Val Gln Leu Ala Glu Asp Leu Lys Lys Val Arg
       930                 935                 940
   gaa att caa gaa aaa ctt gat gct ttc att gaa gct ctt cat cag gag        2880
   Glu Ile Gln Glu Lys Leu Asp Ala Phe Ile Glu Ala Leu His Gln Glu
   945                 950                 955                 960
   aaa taa                                                                 2886
   Lys
<210> 10
   <211> 961
   <212> PRT
   <213> Homo sapiens
<400> 10
   Met Trp Arg Leu Arg Arg Ala Ala Val Ala Cys Glu Val Cys Gln Ser
   1               5                   10                  15
   Leu Val Lys His Ser Ser Gly Ile Lys Gly Ser Leu Pro Leu Gln Lys
               20                  25                  30
   Leu His Leu Val Ser Arg Ser Ile Tyr His Ser His His Pro Thr Leu
           35                  40                  45
   Lys Leu Gln Arg Pro Gln Leu Arg Thr Ser Phe Gln Gln Phe Ser Ser
       50                  55                  60
   Leu Thr Asn Leu Pro Leu Arg Lys Leu Lys Phe Ser Pro Ile Lys Tyr
   65                  70                  75                  80
   Gly Tyr Gln Pro Arg Arg Asn Phe Trp Pro Ala Arg Leu Ala Thr Arg
                   85                  90                  95
   Leu Leu Lys Leu Arg Tyr Leu Ile Leu Gly Ser Ala Val Gly Gly Gly
               100                 105                 110
   Tyr Thr Ala Lys Lys Thr Phe Asp Gln Trp Lys Asp Met Ile Pro Asp
           115                 120                 125
   Leu Ser Glu Tyr Lys Trp Ile Val Pro Asp Ile Val Trp Glu Ile Asp
       130                 135                 140
   Glu Tyr Ile Asp Phe Gly Ser Pro Glu Glu Thr Ala Phe Arg Ala Thr
   145                 150                 155                 160
   Asp Arg Gly Ser Glu Ser Asp Lys His Phe Arg Lys Gly Leu Leu Gly
   165 170 175
   Glu Leu Ile Leu Leu Gln Gln Gln Ile Gln Glu His Glu Glu Glu Ala
   180 185 190
   Arg Arg Ala Ala Gly Gln Tyr Ser Thr Ser Tyr Ala Gln Gln Lys Arg
   195 200 205
   Lys Val Ser Asp Lys Glu Lys Ile Asp Gln Leu Gln Glu Glu Leu Leu
   210 215 220
   His Thr Gln Leu Lys Tyr Gln Arg Ile Leu Glu Arg Leu Glu Lys Glu
   225 230 235 240
   Asn Lys Glu Leu Arg Lys Leu Val Leu Gln Lys Asp Asp Lys Gly Ile
   245 250 255
   His His Arg Lys Leu Lys Lys Ser Leu Ile Asp Met Tyr Ser Glu Val
   260 265 270
   Leu Asp Val Leu Ser Asp Tyr Asp Ala Ser Tyr Asn Thr Gln Asp His
   275 280 285
   Leu Pro Arg Val Val Val Val Gly Asp Gln Ser Ala Gly Lys Thr Ser
   290 295 300
   Val Leu Glu Met Ile Ala Gln Ala Arg Ile Phe Pro Arg Gly Ser Gly
   305 310 315 320
   Glu Met Met Thr Arg Ser Pro Val Lys Val Thr Leu Ser Glu Gly Pro
   325 330 335
   His His Val Ala Leu Phe Lys Asp Ser Ser Arg Glu Phe Asp Leu Thr
   340 345 350
   Lys Glu Glu Asp Leu Ala Ala Leu Arg His Glu Ile Glu Leu Arg Met
   355 360 365
   Arg Lys Asn Val Lys Glu Gly Cys Thr Val Ser Pro Glu Thr Ile Ser
   370 375 380
   Leu Asn Val Lys Gly Pro Gly Leu Gln Arg Met Val Leu Val Asp Leu
   385 390 395 400
   Pro Gly Val Ile Asn Thr Val Thr Ser Gly Met Ala Pro Asp Thr Lys
   405 410 415
   Glu Thr Ile Phe Ser Ile Ser Lys Ala Tyr Met Gln Asn Pro Asn Ala
   420 425 430
   Ile Ile Leu Cys Ile Gln Asp Gly Ser Val Asp Ala Glu Arg Ser Ile
   435 440 445
   Val Thr Asp Leu Val Ser Gln Met Asp Pro His Gly Arg Arg Thr Ile
   450 455 460
   Phe Val Leu Thr Lys Val Asp Leu Ala Glu Lys Asn Val Ala Ser Pro
   465 470 475 480
   Ser Arg Ile Gln Gln Ile Ile Glu Gly Lys Leu Phe Pro Met Lys Ala
   485 490 495
   Leu Gly Tyr Phe Ala Val Val Thr Gly Lys Gly Asn Ser Ser Glu Ser
   500 505 510
   Ile Glu Ala Ile Arg Glu Tyr Glu Glu Glu Phe Phe Gln Asn Ser Lys
   515 520 525
   Leu Leu Lys Thr Ser Met Leu Lys Ala His Gln Val Thr Thr Arg Asn
   530 535 540
   Leu Ser Leu Ala Val Ser Asp Cys Phe Trp Lys Met Val Arg Glu Ser
   545 550 555 560
   Val Glu Gln Gln Ala Asp Ser Phe Lys Ala Thr Arg Phe Asn Leu Glu
   565 570 575
   Thr Glu Trp Lys Asn Asn Tyr Pro Arg Leu Arg Glu Leu Asp Arg Asn
   580 585 590
   Glu Leu Phe Glu Lys Ala Lys Asn Glu Ile Leu Asp Glu Val Ile Ser
   595 600 605
   Leu Ser Gln Val Thr Pro Lys His Trp Glu Glu Ile Leu Gln Gln Ser
   610 615 620
   Leu Trp Glu Arg Val Ser Thr His Val Ile Glu Asn Ile Tyr Leu Pro
   625 630 635 640
   Ala Ala Gln Thr Met Asn Ser Gly Thr Phe Asn Thr Thr Val Asp Ile
   645 650 655
   Lys Leu Lys Gln Trp Thr Asp Lys Gln Leu Pro Asn Lys Ala Val Glu
   660 665 670
   Val Ala Trp Glu Thr Leu Gln Glu Glu Phe Ser Arg Phe Met Thr Glu
   675 680 685
   Pro Lys Gly Lys Glu His Asp Asp Ile Phe Asp Lys Leu Lys Glu Ala
   690 695 700
   Val Lys Glu Glu Ser Ile Lys Arg His Lys Trp Asn Asp Phe Ala Glu
   705 710 715 720
   Asp Ser Leu Arg Val Ile Gln His Asn Ala Leu Glu Asp Arg Ser Ile
   725 730 735
   Ser Asp Lys Gln Gln Trp Asp Ala Ala Ile Tyr Phe Met Glu Glu Ala
   740 745 750
   Leu Gln Ala Arg Leu Lys Asp Thr Glu Asn Ala Ile Glu Asn Met Val
   755 760 765
   Gly Pro Asp Trp Lys Lys Arg Trp Leu Tyr Trp Lys Asn Arg Thr Gln
   770 775 780
   Glu Gln Cys Val His Asn Glu Thr Lys Asn Glu Leu Glu Lys Met Leu
   785 790 795 800
   Lys Cys Asn Glu Glu His Pro Ala Tyr Leu Ala Ser Asp Glu Ile Thr
   805 810 815
   Thr Val Arg Lys Asn Leu Glu Ser Arg Gly Val Glu Val Asp Pro Ser
   820 825 830
   Leu Ile Lys Asp Thr Trp His Gln Val Tyr Arg Arg His Phe Leu Lys
   835 840 845
   Thr Ala Leu Asn His Cys Asn Leu Cys Arg Arg Gly Phe Tyr Tyr Tyr
   850 855 860
   Gln Arg His Phe Val Asp Ser Glu Leu Glu Cys Asn Asp Val Val Leu
   865 870 875 880
   Phe Trp Arg Ile Gln Arg Met Leu Ala Ile Thr Ala Asn Thr Leu Arg
   885 890 895
   Gln Gln Leu Thr Asn Thr Glu Val Arg Arg Leu Glu Lys Asn Val Lys
   900 905 910
   Glu Val Leu Glu Asp Phe Ala Glu Asp Gly Glu Lys Lys Ile Lys Leu
   915 920 925
   Leu Thr Gly Lys Arg Val Gln Leu Ala Glu Asp Leu Lys Lys Val Arg
   930 935 940
   Glu Ile Gln Glu Lys Leu Asp Ala Phe Ile Glu Ala Leu His Gln Glu
   945 950 955 960
   Lys
<210> 11
   <211> 2829
   <212> DNA
   <213> Homo sapiens
<220>
   <221> CDS
   <222> (1)..(2829)
<400> 11
   atg tgg cga cta cgt cgg gcc gct gtg gcc tgt gag gtc tgc cag tct 48
   Met Trp Arg Leu Arg Arg Ala Ala Val Ala Cys Glu Val Cys Gln Ser
   1 5 10 15
   tta gtg aaa cac agc tct gga ata aaa gga agt tta cca cta caa aaa 96
   Leu Val Lys His Ser Ser Gly Ile Lys Gly Ser Leu Pro Leu Gln Lys
   20 25 30
   cta cat ctg gtt tca cga agc att tat cat tca cat cat cct acc tta 144
   Leu His Leu Val Ser Arg Ser Ile Tyr His Ser His His Pro Thr Leu
   35 40 45
   aag ctt caa cga ccc caa tta agg aca tcc ttt cag cag ttc tct tct 192
   Lys Leu Gln Arg Pro Gln Leu Arg Thr Ser Phe Gln Gln Phe Ser Ser
   50 55 60
   ctg aca aac ctt cct tta cgt aaa ctg aaa ttc tct cca att aaa tat 240
   Leu Thr Asn Leu Pro Leu Arg Lys Leu Lys Phe Ser Pro Ile Lys Tyr
   65 70 75 80
   ggc tac cag cct cgc agg aat ttt tgg cca gca aga tta gct acg aga 288
   Gly Tyr Gln Pro Arg Arg Asn Phe Trp Pro Ala Arg Leu Ala Thr Arg
   85 90 95
   ctc tta aaa ctt cgc tat ctc ata cta gga tcg gct gtt ggg ggt ggc 336
   Leu Leu Lys Leu Arg Tyr Leu Ile Leu Gly Ser Ala Val Gly Gly Gly
                   100                 105                 110
   tac aca gcc aaa aag act ttt gat cag tgg aaa gat atg ata ccg gac        384
   Tyr Thr Ala Lys Lys Thr Phe Asp Gln Trp Lys Asp Met Ile Pro Asp
               115                 120                 125
   ctt agt gaa tat aaa tgg att gtg cct gac att gtg tgg gaa att gat        432
   Leu Ser Glu Tyr Lys Trp Ile Val Pro Asp Ile Val Trp Glu Ile Asp
       130                 135                 140
   gag tat atc gat ttt ggt cac aaa ttg gtt agt gaa gtc ata gga gct        480
   Glu Tyr Ile Asp Phe Gly His Lys Leu Val Ser Glu Val Ile Gly Ala
   145                 150                 155                 160
   tct gac cta ctt ctc ttg tta ggt tct ccg gaa gaa acg gcg ttt aga        528
   Ser Asp Leu Leu Leu Leu Leu Gly Ser Pro Glu Glu Thr Ala Phe Arg
                   165                 170                 175
   gca aca gat cgt gga tct gaa agt gac aag cat ttt aga aag gtg tca        576
   Ala Thr Asp Arg Gly Ser Glu Ser Asp Lys His Phe Arg Lys Val Ser
               180                 185                 190
   gac aaa gag aaa att gac caa ctt cag gaa gaa ctt ctg cac act cag        624
   Asp Lys Glu Lys Ile Asp Gln Leu Gln Glu Glu Leu Leu His Thr Gln
           195                 200                 205
   ttg aag tat cag aga atc ttg gaa cga tta gaa aag gag aac aaa gaa        672
   Leu Lys Tyr Gln Arg Ile Leu Glu Arg Leu Glu Lys Glu Asn Lys Glu
       210                 215                 220
   ttg aga aaa tta gta ttg cag aaa gat gac aaa ggc att cat cat aga        720
   Leu Arg Lys Leu Val Leu Gln Lys Asp Asp Lys Gly Ile His His Arg
   225                 230                 235                 240
   aag ctt aag aaa tct ttg att gac atg tat tct gaa gtt ctt gat gtt        768
   Lys Leu Lys Lys Ser Leu Ile Asp Met Tyr Ser Glu Val Leu Asp Val
                   245                 250                 255
   ctc tct gat tat gat gcc agt tat aat acg caa gat cat ctg cca cgg        816
   Leu Ser Asp Tyr Asp Ala Ser Tyr Asn Thr Gln Asp His Leu Pro Arg
               260                 265                 270
   gtt gtt gtg gtt gga gat cag agt gct gga aag act agt gtg ttg gaa        864
   Val Val Val Val Gly Asp Gln Ser Ala Gly Lys Thr Ser Val Leu Glu
           275                 280                 285
   atg att gcc caa gct cga ata ttc cca aga gga tct ggg gag atg atg        912
   Met Ile Ala Gln Ala Arg Ile Phe Pro Arg Gly Ser Gly Glu Met Met
       290                 295                 300
   aca cgt tct cca gtt aag gtg act ctg agt gaa ggt cct cac cat gtg        960
   Thr Arg Ser Pro Val Lys Val Thr Leu Ser Glu Gly Pro His His Val
   305                 310                 315                 320
   gcc cta ttt aaa gat agt tct cgg gag ttt gat ctt acc aaa gaa gaa       1008
   Ala Leu Phe Lys Asp Ser Ser Arg Glu Phe Asp Leu Thr Lys Glu Glu
                   325                 330                 335
   gat ctt gca gca tta aga cat gaa ata gaa ctt cga atg agg aaa aat       1056
   Asp Leu Ala Ala Leu Arg His Glu Ile Glu Leu Arg Met Arg Lys Asn
               340                     345                     350
   gtg aaa gaa ggc tgt acc gtt agc cct gag acc ata tcc tta aat gta      1104
   Val Lys Glu Gly Cys Thr Val Ser Pro Glu Thr Ile Ser Leu Asn Val
           355                 360                     365
   aaa ggc cct gga cta cag agg atg gtg ctt gtt gac tta cca ggt gtg      1152
   Lys Gly Pro Gly Leu Gln Arg Met Val Leu Val Asp Leu Pro Gly Val
       370                 375                     380
   att aat act gtg aca tca ggc atg gct cct gac aca aag gaa act att      1200
   Ile Asn Thr Val Thr Ser Gly Met Ala Pro Asp Thr Lys Glu Thr Ile
   385                 390                     395                 400
   ttc agt atc agc aaa gct tac atg cag aat cct aat gcc atc ata ctg      1248
   Phe Ser Ile Ser Lys Ala Tyr Met Gln Asn Pro Asn Ala Ile Ile Leu
                   405                     410                 415
   tgt att caa gat gga tct gtg gat gct gaa cgc agt att gtt aca gac      1296
   Cys Ile Gln Asp Gly Ser Val Asp Ala Glu Arg Ser Ile Val Thr Asp
               420                     425                 430
   ttg gtc agt caa atg gac cct cat gga agg aga acc ata ttc gtt ttg      1344
   Leu Val Ser Gln Met Asp Pro His Gly Arg Arg Thr Ile Phe Val Leu
           435                 440                     445
   acc aaa gta gac ctg gca gag aaa aat gta gcc agt cca agc agg att      1392
   Thr Lys Val Asp Leu Ala Glu Lys Asn Val Ala Ser Pro Ser Arg Ile
       450                 455                     460
   cag cag ata att gaa gga aag ctc ttc cca atg aaa gct tta ggt tat      1440
   Gln Gln Ile Ile Glu Gly Lys Leu Phe Pro Met Lys Ala Leu Gly Tyr
   465                 470                     475                 480
   ttt gct gtt gta aca gga aaa ggg aac agc tct gaa agc att gaa gct      1488
   Phe Ala Val Val Thr Gly Lys Gly Asn Ser Ser Glu Ser Ile Glu Ala
                   485                     490                 495
   ata aga gaa tat gaa gaa gag ttt ttt cag aat tca aag ctc cta aag      1536
   Ile Arg Glu Tyr Glu Glu Glu Phe Phe Gln Asn Ser Lys Leu Leu Lys
               500                     505                 510
   aca agc atg cta aag gca cac caa gtg act aca aga aat tta agc ctt      1584
   Thr Ser Met Leu Lys Ala His Gln Val Thr Thr Arg Asn Leu Ser Leu
           515                 520                     525
   gca gta tca gac tgc ttt tgg aaa atg gta cga gag tct gtt gaa caa      1632
   Ala Val Ser Asp Cys Phe Trp Lys Met Val Arg Glu Ser Val Glu Gln
       530                 535                     540
   cag gct gat agt ttc aaa gca aca cgt ttt aac ctt gaa act gaa tgg      1680
   Gln Ala Asp Ser Phe Lys Ala Thr Arg Phe Asn Leu Glu Thr Glu Trp
   545                 550                     555                 560
   aag aat aac tat cct cgc ctg cgg gaa ctt gac cgg aat gaa cta ttt      1728
   Lys Asn Asn Tyr Pro Arg Leu Arg Glu Leu Asp Arg Asn Glu Leu Phe
                   565                     570                 575
   gaa aaa gct aaa aat gaa atc ctt gat gaa gtt atc agt ctg agc cag      1776
   Glu Lys Ala Lys Asn Glu Ile Leu Asp Glu Val Ile Ser Leu Ser Gln
               580                 585                 590
   gtt aca cca aaa cat tgg gag gaa atc ctt caa caa tct ttg tgg gaa      1824
   Val Thr Pro Lys His Trp Glu Glu Ile Leu Gln Gln Ser Leu Trp Glu
           595                 600                 605
   aga gta tca act cat gtg att gaa aac atc tac ctt cca gct gcg cag      1872
   Arg Val Ser Thr His Val Ile Glu Asn Ile Tyr Leu Pro Ala Ala Gln
       610                 615                 620
   acc atg aat tca gga act ttt aac acc aca gtg gat atc aag ctt aaa      1920
   Thr Met Asn Ser Gly Thr Phe Asn Thr Thr Val Asp Ile Lys Leu Lys
   625                 630                 635                 640
   cag tgg act gat aaa caa ctt cct aat aaa gca gta gag gtt gct tgg      1968
   Gln Trp Thr Asp Lys Gln Leu Pro Asn Lys Ala Val Glu Val Ala Trp
                   645                 650                 655
   gag acc cta caa gaa gaa ttt tcc cgc ttt atg aca gaa ccg aaa ggg      2016
   Glu Thr Leu Gln Glu Glu Phe Ser Arg Phe Met Thr Glu Pro Lys Gly
               660                 665                 670
   aaa gag cat gat gac ata ttt gat aaa ctt aaa gag gcc gtt aag gaa      2064
   Lys Glu His Asp Asp Ile Phe Asp Lys Leu Lys Glu Ala Val Lys Glu
           675                 680                 685
   gaa agt att aaa cga cac aag tgg aat gac ttt gcg gag gac agc ttg      2112
   Glu Ser Ile Lys Arg His Lys Trp Asn Asp Phe Ala Glu Asp Ser Leu
       690                 695                 700
   agg gtt att caa cac aat gct ttg gaa gac cga tcc ata tct gat aaa      2160
   Arg Val Ile Gln His Asn Ala Leu Glu Asp Arg Ser Ile Ser Asp Lys
   705                 710                 715                 720
   cag caa tgg gat gca gct att tat ttt atg gaa gag gct ctg cag gct      2208
   Gln Gln Trp Asp Ala Ala Ile Tyr Phe Met Glu Glu Ala Leu Gln Ala
                   725                 730                 735
   cgt ctc aag gat act gaa aat gca att gaa aac atg gtg ggt cca gac      2256
   Arg Leu Lys Asp Thr Glu Asn Ala Ile Glu Asn Met Val Gly Pro Asp
               740                 745                 750
   tgg aaa aag agg tgg tta tac tgg aag aat cgg acc caa gaa cag tgt      2304
   Trp Lys Lys Arg Trp Leu Tyr Trp Lys Asn Arg Thr Gln Glu Gln Cys
           755                 760                 765
   gtt cac aat gaa acc aag aat gaa ttg gag aag atg ttg aaa tgt aat      2352
   Val His Asn Glu Thr Lys Asn Glu Leu Glu Lys Met Leu Lys Cys Asn
       770                 775                 780
   gag gag cac cca gct tat ctt gca agt gat gaa ata acc aca gtc cgg      2400
   Glu Glu His Pro Ala Tyr Leu Ala Ser Asp Glu Ile Thr Thr Val Arg
   785                 790                 795                 800
   aag aac ctt gaa tcc cga gga gta gaa gta gat cca agc ttg att aag      2448
   Lys Asn Leu Glu Ser Arg Gly Val Glu Val Asp Pro Ser Leu Ile Lys
                   805                 810                 815
   gat act tgg cat caa gtt tat aga aga cat ttt tta aaa aca gct cta      2496
   Asp Thr Trp His Gln Val Tyr Arg Arg His Phe Leu Lys Thr Ala Leu
               820                     825                     830
   aac cat tgt aac ctt tgt cga aga ggt ttt tat tac tac caa agg cat      2544
   Asn His Cys Asn Leu Cys Arg Arg Gly Phe Tyr Tyr Tyr Gln Arg His
           835                     840                     845
   ttt gta gat tct gag ttg gaa tgc aat gat gtg gtc ttg ttt tgg cgt      2592
   Phe Val Asp Ser Glu Leu Glu Cys Asn Asp Val Val Leu Phe Trp Arg
       850                     855                     860
   ata cag cgc atg ctt gct atc acc gca aat act tta agg caa caa ctt      2640
   Ile Gln Arg Met Leu Ala Ile Thr Ala Asn Thr Leu Arg Gln Gln Leu
   865                     870                     875                     880
   aca aat act gaa gtt agg cga tta gag aaa aat gtt aaa gag gta ttg      2688
   Thr Asn Thr Glu Val Arg Arg Leu Glu Lys Asn Val Lys Glu Val Leu
                   885                     890                     895
   gaa gat ttt gct gaa gat ggt gag aag aag att aaa ttg ctt act ggt      2736
   Glu Asp Phe Ala Glu Asp Gly Glu Lys Lys Ile Lys Leu Leu Thr Gly
               900                     905                     910
   aaa cgc gtt caa ctg gcg gaa gac ctc aag aaa gtt aga gaa att caa      2784
   Lys Arg Val Gln Leu Ala Glu Asp Leu Lys Lys Val Arg Glu Ile Gln
           915                     920                     925
   gaa aaa ctt gat gct ttc att gaa gct ctt cat cag gag aaa taa          2829
   Glu Lys Leu Asp Ala Phe Ile Glu Ala Leu His Gln Glu Lys
       930                     935                     940
<210> 12
   <211> 942
   <212> PRT
   <213> Homo sapiens
<400> 12
   Met Trp Arg Leu Arg Arg Ala Ala Val Ala Cys Glu Val Cys Gln Ser
   1               5                   10                  15
   Leu Val Lys His Ser Ser Gly Ile Lys Gly Ser Leu Pro Leu Gln Lys
               20                  25                  30
   Leu His Leu Val Ser Arg Ser Ile Tyr His Ser His His Pro Thr Leu
           35                  40                  45
   Lys Leu Gln Arg Pro Gln Leu Arg Thr Ser Phe Gln Gln Phe Ser Ser
       50                  55                  60
   Leu Thr Asn Leu Pro Leu Arg Lys Leu Lys Phe Ser Pro Ile Lys Tyr
   65                  70                  75                  80
   Gly Tyr Gln Pro Arg Arg Asn Phe Trp Pro Ala Arg Leu Ala Thr Arg
                   85                  90                  95
   Leu Leu Lys Leu Arg Tyr Leu Ile Leu Gly Ser Ala Val Gly Gly Gly
   100 105 110
   Tyr Thr Ala Lys Lys Thr Phe Asp Gln Trp Lys Asp Met Ile Pro Asp
   115 120 125
   Leu Ser Glu Tyr Lys Trp Ile Val Pro Asp Ile Val Trp Glu Ile Asp
   130 135 140
   Glu Tyr Ile Asp Phe Gly His Lys Leu Val Ser Glu Val Ile Gly Ala
   145 150 155 160
   Ser Asp Leu Leu Leu Leu Leu Gly Ser Pro Glu Glu Thr Ala Phe Arg
   165 170 175
   Ala Thr Asp Arg Gly Ser Glu Ser Asp Lys His Phe Arg Lys Val Ser
   180 185 190
   Asp Lys Glu Lys Ile Asp Gln Leu Gln Glu Glu Leu Leu His Thr Gln
   195 200 205
   Leu Lys Tyr Gln Arg Ile Leu Glu Arg Leu Glu Lys Glu Asn Lys Glu
   210 215 220
   Leu Arg Lys Leu Val Leu Gln Lys Asp Asp Lys Gly Ile His His Arg
   225 230 235 240
   Lys Leu Lys Lys Ser Leu Ile Asp Met Tyr Ser Glu Val Leu Asp Val
   245 250 255
   Leu Ser Asp Tyr Asp Ala Ser Tyr Asn Thr Gln Asp His Leu Pro Arg
   260 265 270
   Val Val Val Val Gly Asp Gln Ser Ala Gly Lys Thr Ser Val Leu Glu
   275 280 285
   Met Ile Ala Gln Ala Arg Ile Phe Pro Arg Gly Ser Gly Glu Met Met
   290 295 300
   Thr Arg Ser Pro Val Lys Val Thr Leu Ser Glu Gly Pro His His Val
   305 310 315 320
   Ala Leu Phe Lys Asp Ser Ser Arg Glu Phe Asp Leu Thr Lys Glu Glu
   325 330 335
   Asp Leu Ala Ala Leu Arg His Glu Ile Glu Leu Arg Met Arg Lys Asn
   340 345 350
   Val Lys Glu Gly Cys Thr Val Ser Pro Glu Thr Ile Ser Leu Asn Val
   355 360 365
   Lys Gly Pro Gly Leu Gln Arg Met Val Leu Val Asp Leu Pro Gly Val
   370 375 380
   Ile Asn Thr Val Thr Ser Gly Met Ala Pro Asp Thr Lys Glu Thr Ile
   385 390 395 400
   Phe Ser Ile Ser Lys Ala Tyr Met Gln Asn Pro Asn Ala Ile Ile Leu
   405 410 415
   Cys Ile Gln Asp Gly Ser Val Asp Ala Glu Arg Ser Ile Val Thr Asp
   420 425 430
   Leu Val Ser Gln Met Asp Pro His Gly Arg Arg Thr Ile Phe Val Leu
   435 440 445
   Thr Lys Val Asp Leu Ala Glu Lys Asn Val Ala Ser Pro Ser Arg Ile
   450 455 460
   Gln Gln Ile Ile Glu Gly Lys Leu Phe Pro Met Lys Ala Leu Gly Tyr
   465 470 475 480
   Phe Ala Val Val Thr Gly Lys Gly Asn Ser Ser Glu Ser Ile Glu Ala
   485 490 495
   Ile Arg Glu Tyr Glu Glu Glu Phe Phe Gln Asn Ser Lys Leu Leu Lys
   500 505 510
   Thr Ser Met Leu Lys Ala His Gln Val Thr Thr Arg Asn Leu Ser Leu
   515 520 525
   Ala Val Ser Asp Cys Phe Trp Lys Met Val Arg Glu Ser Val Glu Gln
   530 535 540
   Gln Ala Asp Ser Phe Lys Ala Thr Arg Phe Asn Leu Glu Thr Glu Trp
   545 550 555 560
   Lys Asn Asn Tyr Pro Arg Leu Arg Glu Leu Asp Arg Asn Glu Leu Phe
   565 570 575
   Glu Lys Ala Lys Asn Glu Ile Leu Asp Glu Val Ile Ser Leu Ser Gln
   580 585 590
   Val Thr Pro Lys His Trp Glu Glu Ile Leu Gln Gln Ser Leu Trp Glu
   595 600 605
   Arg Val Ser Thr His Val Ile Glu Asn Ile Tyr Leu Pro Ala Ala Gln
   610 615 620
   Thr Met Asn Ser Gly Thr Phe Asn Thr Thr Val Asp Ile Lys Leu Lys
   625 630 635 640
   Gln Trp Thr Asp Lys Gln Leu Pro Asn Lys Ala Val Glu Val Ala Trp
   645 650 655
   Glu Thr Leu Gln Glu Glu Phe Ser Arg Phe Met Thr Glu Pro Lys Gly
   660 665 670
   Lys Glu His Asp Asp Ile Phe Asp Lys Leu Lys Glu Ala Val Lys Glu
   675 680 685
   Glu Ser Ile Lys Arg His Lys Trp Asn Asp Phe Ala Glu Asp Ser Leu
   690 695 700
   Arg Val Ile Gln His Asn Ala Leu Glu Asp Arg Ser Ile Ser Asp Lys
   705 710 715 720
   Gln Gln Trp Asp Ala Ala Ile Tyr Phe Met Glu Glu Ala Leu Gln Ala
   725 730 735
   Arg Leu Lys Asp Thr Glu Asn Ala Ile Glu Asn Met Val Gly Pro Asp
   740 745 750
   Trp Lys Lys Arg Trp Leu Tyr Trp Lys Asn Arg Thr Gln Glu Gln Cys
   755 760 765
   Val His Asn Glu Thr Lys Asn Glu Leu Glu Lys Met Leu Lys Cys Asn
   770 775 780
   Glu Glu His Pro Ala Tyr Leu Ala Ser Asp Glu Ile Thr Thr Val Arg
   785 790 795 800
   Lys Asn Leu Glu Ser Arg Gly Val Glu Val Asp Pro Ser Leu Ile Lys
   805 810 815
   Asp Thr Trp His Gln Val Tyr Arg Arg His Phe Leu Lys Thr Ala Leu
               820                 825                 830
   Asn His Cys Asn Leu Cys Arg Arg Gly Phe Tyr Tyr Tyr Gln Arg His
           835                 840                 845
   Phe Val Asp Ser Glu Leu Glu Cys Asn Asp Val Val Leu Phe Trp Arg
       850                 855                 860
   Ile Gln Arg Met Leu Ala Ile Thr Ala Asn Thr Leu Arg Gln Gln Leu
   865                 870                 875                 880
   Thr Asn Thr Glu Val Arg Arg Leu Glu Lys Asn Val Lys Glu Val Leu
                   885                 890                 895
   Glu Asp Phe Ala Glu Asp Gly Glu Lys Lys Ile Lys Leu Leu Thr Gly
               900                 905                 910
   Lys Arg Val Gln Leu Ala Glu Asp Leu Lys Lys Val Arg Glu Ile Gln
           915                 920                 925
   Glu Lys Leu Asp Ala Phe Ile Glu Ala Leu His Gln Glu Lys
       930                 935                 940
<210> 13
   <211> 2775
   <212> DNA
   <213> Homo sapiens
<220>
   <221> CDS
   <222> (1)..(2775)
<400> 13
   atg tgg cga cta cgt cgg gcc gct gtg gcc tgt gag gtc tgc cag tct      48
   Met Trp Arg Leu Arg Arg Ala Ala Val Ala Cys Glu Val Cys Gln Ser
   1               5                   10                  15
   tta gtg aaa cac agc tct gga ata aaa gga agt tta cca cta caa aaa      96
   Leu Val Lys His Ser Ser Gly Ile Lys Gly Ser Leu Pro Leu Gln Lys
               20                  25                  30
   cta cat ctg gtt tca cga agc att tat cat tca cat cat cct acc tta     144
   Leu His Leu Val Ser Arg Ser Ile Tyr His Ser His His Pro Thr Leu
           35                  40                  45
   aag ctt caa cga ccc caa tta agg aca tcc ttt cag cag ttc tct tct     192
   Lys Leu Gln Arg Pro Gln Leu Arg Thr Ser Phe Gln Gln Phe Ser Ser
       50                  55                  60
   ctg aca aac ctt cct tta cgt aaa ctg aaa ttc tct cca att aaa tat       240
   Leu Thr Asn Leu Pro Leu Arg Lys Leu Lys Phe Ser Pro Ile Lys Tyr
   65                  70                  75                  80
   ggc tac cag cct cgc agg aat ttt tgg cca gca aga tta gct acg aga       288
   Gly Tyr Gln Pro Arg Arg Asn Phe Trp Pro Ala Arg Leu Ala Thr Arg
                   85                  90                  95
   ctc tta aaa ctt cgc tat ctc ata cta gga tcg gct gtt ggg ggt ggc       336
   Leu Leu Lys Leu Arg Tyr Leu Ile Leu Gly Ser Ala Val Gly Gly Gly
               100                 105                 110
   tac aca gcc aaa aag act ttt gat cag tgg aaa gat atg ata ccg gac       384
   Tyr Thr Ala Lys Lys Thr Phe Asp Gln Trp Lys Asp Met Ile Pro Asp
           115                 120                 125
   ctt agt gaa tat aaa tgg att gtg cct gac att gtg tgg gaa att gat       432
   Leu Ser Glu Tyr Lys Trp Ile Val Pro Asp Ile Val Trp Glu Ile Asp
       130                 135                 140
   gag tat atc gat ttt ggt tct ccg gaa gaa acg gcg ttt aga gca aca       480
   Glu Tyr Ile Asp Phe Gly Ser Pro Glu Glu Thr Ala Phe Arg Ala Thr
   145                 150                 155                 160
   gat cgt gga tct gaa agt gac aag cat ttt aga aag gtg tca gac aaa       528
   Asp Arg Gly Ser Glu Ser Asp Lys His Phe Arg Lys Val Ser Asp Lys
                   165                 170                 175
   gag aaa att gac caa ctt cag gaa gaa ctt ctg cac act cag ttg aag       576
   Glu Lys Ile Asp Gln Leu Gln Glu Glu Leu Leu His Thr Gln Leu Lys
               180                 185                 190
   tat cag aga atc ttg gaa cga tta gaa aag gag aac aaa gaa ttg aga       624
   Tyr Gln Arg Ile Leu Glu Arg Leu Glu Lys Glu Asn Lys Glu Leu Arg
           195                 200                 205
   aaa tta gta ttg cag aaa gat gac aaa ggc att cat cat aga aag ctt       672
   Lys Leu Val Leu Gln Lys Asp Asp Lys Gly Ile His His Arg Lys Leu
       210                 215                 220
   aag aaa tct ttg att gac atg tat tct gaa gtt ctt gat gtt ctc tct       720
   Lys Lys Ser Leu Ile Asp Met Tyr Ser Glu Val Leu Asp Val Leu Ser
   225                 230                 235                 240
   gat tat gat gcc agt tat aat acg caa gat cat ctg cca cgg gtt gtt       768
   Asp Tyr Asp Ala Ser Tyr Asn Thr Gln Asp His Leu Pro Arg Val Val
                   245                 250                 255
   gtg gtt gga gat cag agt gct gga aag act agt gtg ttg gaa atg att       816
   Val Val Gly Asp Gln Ser Ala Gly Lys Thr Ser Val Leu Glu Met Ile
               260                 265                 270
   gcc caa gct cga ata ttc cca aga gga tct ggg gag atg atg aca cgt       864
   Ala Gln Ala Arg Ile Phe Pro Arg Gly Ser Gly Glu Met Met Thr Arg
           275                 280                 285
   tct cca gtt aag gtg act ctg agt gaa ggt cct cac cat gtg gcc cta       912
   Ser Pro Val Lys Val Thr Leu Ser Glu Gly Pro His His Val Ala Leu
       290                 295                 300
   ttt aaa gat agt tct cgg gag ttt gat ctt acc aaa gaa gaa gat ctt        960
   Phe Lys Asp Ser Ser Arg Glu Phe Asp Leu Thr Lys Glu Glu Asp Leu
   305                 310                 315                 320
   gca gca tta aga cat gaa ata gaa ctt cga atg agg aaa aat gtg aaa       1008
   Ala Ala Leu Arg His Glu Ile Glu Leu Arg Met Arg Lys Asn Val Lys
                   325                 330                 335
   gaa ggc tgt acc gtt agc cct gag acc ata tcc tta aat gta aaa ggc       1056
   Glu Gly Cys Thr Val Ser Pro Glu Thr Ile Ser Leu Asn Val Lys Gly
               340                 345                 350
   cct gga cta cag agg atg gtg ctt gtt gac tta cca ggt gtg att aat       1104
   Pro Gly Leu Gln Arg Met Val Leu Val Asp Leu Pro Gly Val Ile Asn
           355                 360                 365
   act gtg aca tca ggc atg gct cct gac aca aag gaa act att ttc agt       1152
   Thr Val Thr Ser Gly Met Ala Pro Asp Thr Lys Glu Thr Ile Phe Ser
       370                 375                 380
   atc agc aaa gct tac atg cag aat cct aat gcc atc ata ctg tgt att       1200
   Ile Ser Lys Ala Tyr Met Gln Asn Pro Asn Ala Ile Ile Leu Cys Ile
   385                 390                 395                 400
   caa gat gga tct gtg gat gct gaa cgc agt att gtt aca gac ttg gtc       1248
   Gln Asp Gly Ser Val Asp Ala Glu Arg Ser Ile Val Thr Asp Leu Val
                   405                 410                 415
   agt caa atg gac cct cat gga agg aga acc ata ttc gtt ttg acc aaa       1296
   Ser Gln Met Asp Pro His Gly Arg Arg Thr Ile Phe Val Leu Thr Lys
               420                 425                 430
   gta gac ctg gca gag aaa aat gta gcc agt cca agc agg att cag cag       1344
   Val Asp Leu Ala Glu Lys Asn Val Ala Ser Pro Ser Arg Ile Gln Gln
           435                 440                 445
   ata att gaa gga aag ctc ttc cca atg aaa gct tta ggt tat ttt gct       1392
   Ile Ile Glu Gly Lys Leu Phe Pro Met Lys Ala Leu Gly Tyr Phe Ala
       450                 455                 460
   gtt gta aca gga aaa ggg aac agc tct gaa agc att gaa gct ata aga       1440
   Val Val Thr Gly Lys Gly Asn Ser Ser Glu Ser Ile Glu Ala Ile Arg
   465                 470                 475                 480
   gaa tat gaa gaa gag ttt ttt cag aat tca aag ctc cta aag aca agc       1488
   Glu Tyr Glu Glu Glu Phe Phe Gln Asn Ser Lys Leu Leu Lys Thr Ser
                   485                 490                 495
   atg cta aag gca cac caa gtg act aca aga aat tta agc ctt gca gta       1536
   Met Leu Lys Ala His Gln Val Thr Thr Arg Asn Leu Ser Leu Ala Val
               500                 505                 510
   tca gac tgc ttt tgg aaa atg gta cga gag tct gtt gaa caa cag gct       1584
   Ser Asp Cys Phe Trp Lys Met Val Arg Glu Ser Val Glu Gln Gln Ala
           515                 520                 525
   gat agt ttc aaa gca aca cgt ttt aac ctt gaa act gaa tgg aag aat       1632
   Asp Ser Phe Lys Ala Thr Arg Phe Asn Leu Glu Thr Glu Trp Lys Asn
       530                 535                 540
   aac tat cct cgc ctg cgg gaa ctt gac cgg aat gaa cta ttt gaa aaa      1680
   Asn Tyr Pro Arg Leu Arg Glu Leu Asp Arg Asn Glu Leu Phe Glu Lys
   545                 550                 555                 560
   gct aaa aat gaa atc ctt gat gaa gtt atc agt ctg agc cag gtt aca      1728
   Ala Lys Asn Glu Ile Leu Asp Glu Val Ile Ser Leu Ser Gln Val Thr
                   565                 570                 575
   cca aaa cat tgg gag gaa atc ctt caa caa tct ttg tgg gaa aga gta      1776
   Pro Lys His Trp Glu Glu Ile Leu Gln Gln Ser Leu Trp Glu Arg Val
               580                 585                 590
   tca act cat gtg att gaa aac atc tac ctt cca gct gcg cag acc atg      1824
   Ser Thr His Val Ile Glu Asn Ile Tyr Leu Pro Ala Ala Gln Thr Met
           595                 600                 605
   aat tca gga act ttt aac acc aca gtg gat atc aag ctt aaa cag tgg      1872
   Asn Ser Gly Thr Phe Asn Thr Thr Val Asp Ile Lys Leu Lys Gln Trp
       610                 615                 620
   act gat aaa caa ctt cct aat aaa gca gta gag gtt gct tgg gag acc      1920
   Thr Asp Lys Gln Leu Pro Asn Lys Ala Val Glu Val Ala Trp Glu Thr
   625                 630                 635                 640
   cta caa gaa gaa ttt tcc cgc ttt atg aca gaa ccg aaa ggg aaa gag      1968
   Leu Gln Glu Glu Phe Ser Arg Phe Met Thr Glu Pro Lys Gly Lys Glu
                   645                 650                 655
   cat gat gac ata ttt gat aaa ctt aaa gag gcc gtt aag gaa gaa agt      2016
   His Asp Asp Ile Phe Asp Lys Leu Lys Glu Ala Val Lys Glu Glu Ser
               660                 665                 670
   att aaa cga cac aag tgg aat gac ttt gcg gag gac agc ttg agg gtt      2064
   Ile Lys Arg His Lys Trp Asn Asp Phe Ala Glu Asp Ser Leu Arg Val
           675                 680                 685
   att caa cac aat gct ttg gaa gac cga tcc ata tct gat aaa cag caa      2112
   Ile Gln His Asn Ala Leu Glu Asp Arg Ser Ile Ser Asp Lys Gln Gln
       690                 695                 700
   tgg gat gca gct att tat ttt atg gaa gag gct ctg cag gct cgt ctc      2160
   Trp Asp Ala Ala Ile Tyr Phe Met Glu Glu Ala Leu Gln Ala Arg Leu
   705                 710                 715                 720
   aag gat act gaa aat gca att gaa aac atg gtg ggt cca gac tgg aaa      2208
   Lys Asp Thr Glu Asn Ala Ile Glu Asn Met Val Gly Pro Asp Trp Lys
                   725                 730                 735
   aag agg tgg tta tac tgg aag aat cgg acc caa gaa cag tgt gtt cac      2256
   Lys Arg Trp Leu Tyr Trp Lys Asn Arg Thr Gln Glu Gln Cys Val His
               740                 745                 750
   aat gaa acc aag aat gaa ttg gag aag atg ttg aaa tgt aat gag gag      2304
   Asn Glu Thr Lys Asn Glu Leu Glu Lys Met Leu Lys Cys Asn Glu Glu
           755                 760                 765
   cac cca gct tat ctt gca agt gat gaa ata acc aca gtc cgg aag aac      2352
   His Pro Ala Tyr Leu Ala Ser Asp Glu Ile Thr Thr Val Arg Lys Asn
       770                 775                 780
   ctt gaa tcc cga gga gta gaa gta gat cca agc ttg att aag gat act      2400
   Leu Glu Ser Arg Gly Val Glu Val Asp Pro Ser Leu Ile Lys Asp Thr
   785                 790                 795                 800
   tgg cat caa gtt tat aga aga cat ttt tta aaa aca gct cta aac cat      2448
   Trp His Gln Val Tyr Arg Arg His Phe Leu Lys Thr Ala Leu Asn His
                   805                 810                 815
   tgt aac ctt tgt cga aga ggt ttt tat tac tac caa agg cat ttt gta      2496
   Cys Asn Leu Cys Arg Arg Gly Phe Tyr Tyr Tyr Gln Arg His Phe Val
               820                 825                 830
   gat tct gag ttg gaa tgc aat gat gtg gtc ttg ttt tgg cgt ata cag      2544
   Asp Ser Glu Leu Glu Cys Asn Asp Val Val Leu Phe Trp Arg Ile Gln
           835                 840                 845
   cgc atg ctt gct atc acc gca aat act tta agg caa caa ctt aca aat      2592
   Arg Met Leu Ala Ile Thr Ala Asn Thr Leu Arg Gln Gln Leu Thr Asn
       850                 855                 860
   act gaa gtt agg cga tta gag aaa aat gtt aaa gag gta ttg gaa gat      2640
   Thr Glu Val Arg Arg Leu Glu Lys Asn Val Lys Glu Val Leu Glu Asp
   865                 870                 875                 880
   ttt gct gaa gat ggt gag aag aag att aaa ttg ctt act ggt aaa cgc      2688
   Phe Ala Glu Asp Gly Glu Lys Lys Ile Lys Leu Leu Thr Gly Lys Arg
                   885                 890                 895
   gtt caa ctg gcg gaa gac ctc aag aaa gtt aga gaa att caa gaa aaa      2736
   Val Gln Leu Ala Glu Asp Leu Lys Lys Val Arg Glu Ile Gln Glu Lys
               900                 905                 910
   ctt gat gct ttc att gaa gct ctt cat cag gag aaa taa                  2775
   Leu Asp Ala Phe Ile Glu Ala Leu His Gln Glu Lys
           915                 920
<210> 14
   <211> 924
   <212> PRT
   <213> Homo sapiens
<400> 14
   Met Trp Arg Leu Arg Arg Ala Ala Val Ala Cys Glu Val Cys Gln Ser
   1               5                   10                  15
   Leu Val Lys His Ser Ser Gly Ile Lys Gly Ser Leu Pro Leu Gln Lys
               20                  25                  30
   Leu His Leu Val Ser Arg Ser Ile Tyr His Ser His His Pro Thr Leu
           35                  40                  45
   Lys Leu Gln Arg Pro Gln Leu Arg Thr Ser Phe Gln Gln Phe Ser Ser
       50                  55                  60
   Leu Thr Asn Leu Pro Leu Arg Lys Leu Lys Phe Ser Pro Ile Lys Tyr
   65                  70                  75                  80
   Gly Tyr Gln Pro Arg Arg Asn Phe Trp Pro Ala Arg Leu Ala Thr Arg
                   85                  90                  95
   Leu Leu Lys Leu Arg Tyr Leu Ile Leu Gly Ser Ala Val Gly Gly Gly
               100                 105                 110
   Tyr Thr Ala Lys Lys Thr Phe Asp Gln Trp Lys Asp Met Ile Pro Asp
           115                 120                 125
   Leu Ser Glu Tyr Lys Trp Ile Val Pro Asp Ile Val Trp Glu Ile Asp
       130                 135                 140
   Glu Tyr Ile Asp Phe Gly Ser Pro Glu Glu Thr Ala Phe Arg Ala Thr
   145                 150                 155                 160
   Asp Arg Gly Ser Glu Ser Asp Lys His Phe Arg Lys Val Ser Asp Lys
                   165                 170                 175
   Glu Lys Ile Asp Gln Leu Gln Glu Glu Leu Leu His Thr Gln Leu Lys
               180                 185                 190
   Tyr Gln Arg Ile Leu Glu Arg Leu Glu Lys Glu Asn Lys Glu Leu Arg
           195                 200                 205
   Lys Leu Val Leu Gln Lys Asp Asp Lys Gly Ile His His Arg Lys Leu
       210                 215                 220
   Lys Lys Ser Leu Ile Asp Met Tyr Ser Glu Val Leu Asp Val Leu Ser
   225                 230                 235                 240
   Asp Tyr Asp Ala Ser Tyr Asn Thr Gln Asp His Leu Pro Arg Val Val
                   245                 250                 255
   Val Val Gly Asp Gln Ser Ala Gly Lys Thr Ser Val Leu Glu Met Ile
               260                 265                 270
   Ala Gln Ala Arg Ile Phe Pro Arg Gly Ser Gly Glu Met Met Thr Arg
           275                 280                 285
   Ser Pro Val Lys Val Thr Leu Ser Glu Gly Pro His His Val Ala Leu
       290                 295                 300
   Phe Lys Asp Ser Ser Arg Glu Phe Asp Leu Thr Lys Glu Glu Asp Leu
   305 310 315 320
   Ala Ala Leu Arg His Glu Ile Glu Leu Arg Met Arg Lys Asn Val Lys
   325 330 335
   Glu Gly Cys Thr Val Ser Pro Glu Thr Ile Ser Leu Asn Val Lys Gly
   340 345 350
   Pro Gly Leu Gln Arg Met Val Leu Val Asp Leu Pro Gly Val Ile Asn
   355 360 365
   Thr Val Thr Ser Gly Met Ala Pro Asp Thr Lys Glu Thr Ile Phe Ser
   370 375 380
   Ile Ser Lys Ala Tyr Met Gln Asn Pro Asn Ala Ile Ile Leu Cys Ile
   385 390 395 400
   Gln Asp Gly Ser Val Asp Ala Glu Arg Ser Ile Val Thr Asp Leu Val
   405 410 415
   Ser Gln Met Asp Pro His Gly Arg Arg Thr Ile Phe Val Leu Thr Lys
   420 425 430
   Val Asp Leu Ala Glu Lys Asn Val Ala Ser Pro Ser Arg Ile Gln Gln
   435 440 445
   Ile Ile Glu Gly Lys Leu Phe Pro Met Lys Ala Leu Gly Tyr Phe Ala
   450 455 460
   Val Val Thr Gly Lys Gly Asn Ser Ser Glu Ser Ile Glu Ala Ile Arg
   465 470 475 480
   Glu Tyr Glu Glu Glu Phe Phe Gln Asn Ser Lys Leu Leu Lys Thr Ser
   485 490 495
   Met Leu Lys Ala His Gln Val Thr Thr Arg Asn Leu Ser Leu Ala Val
   500 505 510
   Ser Asp Cys Phe Trp Lys Met Val Arg Glu Ser Val Glu Gln Gln Ala
   515 520 525
   Asp Ser Phe Lys Ala Thr Arg Phe Asn Leu Glu Thr Glu Trp Lys Asn
   530 535 540
   Asn Tyr Pro Arg Leu Arg Glu Leu Asp Arg Asn Glu Leu Phe Glu Lys
   545 550 555 560
   Ala Lys Asn Glu Ile Leu Asp Glu Val Ile Ser Leu Ser Gln Val Thr
   565 570 575
   Pro Lys His Trp Glu Glu Ile Leu Gln Gln Ser Leu Trp Glu Arg Val
   580 585 590
   Ser Thr His Val Ile Glu Asn Ile Tyr Leu Pro Ala Ala Gln Thr Met
   595 600 605
   Asn Ser Gly Thr Phe Asn Thr Thr Val Asp Ile Lys Leu Lys Gln Trp
   610 615 620
   Thr Asp Lys Gln Leu Pro Asn Lys Ala Val Glu Val Ala Trp Glu Thr
   625 630 635 640
   Leu Gln Glu Glu Phe Ser Arg Phe Met Thr Glu Pro Lys Gly Lys Glu
   645 650 655
   His Asp Asp Ile Phe Asp Lys Leu Lys Glu Ala Val Lys Glu Glu Ser
   660 665 670
   Ile Lys Arg His Lys Trp Asn Asp Phe Ala Glu Asp Ser Leu Arg Val
   675 680 685
   Ile Gln His Asn Ala Leu Glu Asp Arg Ser Ile Ser Asp Lys Gln Gln
   690 695 700
   Trp Asp Ala Ala Ile Tyr Phe Met Glu Glu Ala Leu Gln Ala Arg Leu
   705 710 715 720
   Lys Asp Thr Glu Asn Ala Ile Glu Asn Met Val Gly Pro Asp Trp Lys
   725 730 735
   Lys Arg Trp Leu Tyr Trp Lys Asn Arg Thr Gln Glu Gln Cys Val His
   740 745 750
   Asn Glu Thr Lys Asn Glu Leu Glu Lys Met Leu Lys Cys Asn Glu Glu
   755 760 765
   His Pro Ala Tyr Leu Ala Ser Asp Glu Ile Thr Thr Val Arg Lys Asn
   770 775 780
   Leu Glu Ser Arg Gly Val Glu Val Asp Pro Ser Leu Ile Lys Asp Thr
   785                 790                 795                 800
   Trp His Gln Val Tyr Arg Arg His Phe Leu Lys Thr Ala Leu Asn His
                   805                 810                 815
   Cys Asn Leu Cys Arg Arg Gly Phe Tyr Tyr Tyr Gln Arg His Phe Val
               820                 825                 830
   Asp Ser Glu Leu Glu Cys Asn Asp Val Val Leu Phe Trp Arg Ile Gln
           835                 840                 845
   Arg Met Leu Ala Ile Thr Ala Asn Thr Leu Arg Gln Gln Leu Thr Asn
       850                 855                 860
   Thr Glu Val Arg Arg Leu Glu Lys Asn Val Lys Glu Val Leu Glu Asp
   865                 870                 875                 880
   Phe Ala Glu Asp Gly Glu Lys Lys Ile Lys Leu Leu Thr Gly Lys Arg
                   885                 890                 895
   Val Gln Leu Ala Glu Asp Leu Lys Lys Val Arg Glu Ile Gln Glu Lys
               900                 905                 910
   Leu Asp Ala Phe Ile Glu Ala Leu His Gln Glu Lys
           915                 920
<210> 15
   <211> 2883
   <212> DNA
   <213> Homo sapiens
<220>
   <221> CDS
   <222> (1)..(2883)
<400> 15
   atg tgg cga cta cgt cgg gcc gct gtg gcc tgt gag gtc tgc cag tct      48
   Met Trp Arg Leu Arg Arg Ala Ala Val Ala Cys Glu Val Cys Gln Ser
   1               5                   10                  15
   tta gtg aaa cac agc tct gga ata aaa gga agt tta cca cta caa aaa      96
   Leu Val Lys His Ser Ser Gly Ile Lys Gly Ser Leu Pro Leu Gln Lys
               20                  25                  30
   cta cat ctg gtt tca cga agc att tat cat tca cat cat cct acc tta     144
   Leu His Leu Val Ser Arg Ser Ile Tyr His Ser His His Pro Thr Leu
           35                  40                  45
   aag ctt caa cga ccc caa tta agg aca tcc ttt cag cag ttc tct tct     192
   Lys Leu Gln Arg Pro Gln Leu Arg Thr Ser Phe Gln Gln Phe Ser Ser
        50                  55                  60
   ctg aca aac ctt cct tta cgt aaa ctg aaa ttc tct cca att aaa tat     240
   Leu Thr Asn Leu Pro Leu Arg Lys Leu Lys Phe Ser Pro Ile Lys Tyr
   65                  70                  75                  80
   ggc tac cag cct cgc agg aat ttt tgg cca gca aga tta gct acg aga     288
   Gly Tyr Gln Pro Arg Arg Asn Phe Trp Pro Ala Arg Leu Ala Thr Arg
                   85                  90                  95
   ctc tta aaa ctt cgc tat ctc ata cta gga tcg gct gtt ggg ggt ggc     336
   Leu Leu Lys Leu Arg Tyr Leu Ile Leu Gly Ser Ala Val Gly Gly Gly
               100                 105                 110
   tac aca gcc aaa aag act ttt gat cag tgg aaa gat atg ata ccg gac     384
   Tyr Thr Ala Lys Lys Thr Phe Asp Gln Trp Lys Asp Met Ile Pro Asp
           115                 120                 125
   ctt agt gaa tat aaa tgg att gtg cct gac att gtg tgg gaa att gat     432
   Leu Ser Glu Tyr Lys Trp Ile Val Pro Asp Ile Val Trp Glu Ile Asp
       130                 135                 140
   gag tat atc gat ttt gag aaa att aga aaa gcc ctt cct aat tca gaa     480
   Glu Tyr Ile Asp Phe Glu Lys Ile Arg Lys Ala Leu Pro Asn Ser Glu
   145                 150                 155                 160
   gac ctt gta aag tta gca cca gac ttt gac aag att gtt gaa agc ctt     528
   Asp Leu Val Lys Leu Ala Pro Asp Phe Asp Lys Ile Val Glu Ser Leu
                   165                 170                 175
   agc tta ttg aag gac ttt ttt acc tca ggt tct ccg gaa gaa acg gcg     576
   Ser Leu Leu Lys Asp Phe Phe Thr Ser Gly Ser Pro Glu Glu Thr Ala
               180                 185                 190
   ttt aga gca aca gat cgt gga tct gaa agt gac aag cat ttt aga aag     624
   Phe Arg Ala Thr Asp Arg Gly Ser Glu Ser Asp Lys His Phe Arg Lys
           195                 200                 205
   gtg tca gac aaa gag aaa att gac caa ctt cag gaa gaa ctt ctg cac     672
   Val Ser Asp Lys Glu Lys Ile Asp Gln Leu Gln Glu Glu Leu Leu His
       210                 215                 220
   act cag ttg aag tat cag aga atc ttg gaa cga tta gaa aag gag aac     720
   Thr Gln Leu Lys Tyr Gln Arg Ile Leu Glu Arg Leu Glu Lys Glu Asn
   225                 230                 235                 240
   aaa gaa ttg aga aaa tta gta ttg cag aaa gat gac aaa ggc att cat     768
   Lys Glu Leu Arg Lys Leu Val Leu Gln Lys Asp Asp Lys Gly Ile His
                   245                 250                 255
   cat aga aag ctt aag aaa tct ttg att gac atg tat tct gaa gtt ctt     816
   His Arg Lys Leu Lys Lys Ser Leu Ile Asp Met Tyr Ser Glu Val Leu
               260                 265                 270
   gat gtt ctc tct gat tat gat gcc agt tat aat acg caa gat cat ctg     864
   Asp Val Leu Ser Asp Tyr Asp Ala Ser Tyr Asn Thr Gln Asp His Leu
           275                 280                 285
   cca cgg gtt gtt gtg gtt gga gat cag agt gct gga aag act agt gtg      912
   Pro Arg Val Val Val Val Gly Asp Gln Ser Ala Gly Lys Thr Ser Val
       290                 295                 300
   ttg gaa atg att gcc caa gct cga ata ttc cca aga gga tct ggg gag      960
   Leu Glu Met Ile Ala Gln Ala Arg Ile Phe Pro Arg Gly Ser Gly Glu
   305                 310                 315                 320
   atg atg aca cgt tct cca gtt aag gtg act ctg agt gaa ggt cct cac     1008
   Met Met Thr Arg Ser Pro Val Lys Val Thr Leu Ser Glu Gly Pro His
                   325                 330                 335
   cat gtg gcc cta ttt aaa gat agt tct cgg gag ttt gat ctt acc aaa     1056
   His Val Ala Leu Phe Lys Asp Ser Ser Arg Glu Phe Asp Leu Thr Lys
               340                 345                 350
   gaa gaa gat ctt gca gca tta aga cat gaa ata gaa ctt cga atg agg     1104
   Glu Glu Asp Leu Ala Ala Leu Arg His Glu Ile Glu Leu Arg Met Arg
           355                 360                 365
   aaa aat gtg aaa gaa ggc tgt acc gtt agc cct gag acc ata tcc tta     1152
   Lys Asn Val Lys Glu Gly Cys Thr Val Ser Pro Glu Thr Ile Ser Leu
       370                 375                 380
   aat gta aaa ggc cct gga cta cag agg atg gtg ctt gtt gac tta cca     1200
   Asn Val Lys Gly Pro Gly Leu Gln Arg Met Val Leu Val Asp Leu Pro
   385                 390                 395                 400
   ggt gtg att aat act gtg aca tca ggc atg gct cct gac aca aag gaa     1248
   Gly Val Ile Asn Thr Val Thr Ser Gly Met Ala Pro Asp Thr Lys Glu
                   405                 410                 415
   act att ttc agt atc agc aaa gct tac atg cag aat cct aat gcc atc     1296
   Thr Ile Phe Ser Ile Ser Lys Ala Tyr Met Gln Asn Pro Asn Ala Ile
               420                 425                 430
   ata ctg tgt att caa gat gga tct gtg gat gct gaa cgc agt att gtt     1344
   Ile Leu Cys Ile Gln Asp Gly Ser Val Asp Ala Glu Arg Ser Ile Val
           435                 440                 445
   aca gac ttg gtc agt caa atg gac cct cat gga agg aga acc ata ttc     1392
   Thr Asp Leu Val Ser Gln Met Asp Pro His Gly Arg Arg Thr Ile Phe
       450                 455                 460
   gtt ttg acc aaa gta gac ctg gca gag aaa aat gta gcc agt cca agc     1440
   Val Leu Thr Lys Val Asp Leu Ala Glu Lys Asn Val Ala Ser Pro Ser
   465                 470                 475                 480
   agg att cag cag ata att gaa gga aag ctc ttc cca atg aaa gct tta     1488
   Arg Ile Gln Gln Ile Ile Glu Gly Lys Leu Phe Pro Met Lys Ala Leu
                   485                 490                 495
   ggt tat ttt gct gtt gta aca gga aaa ggg aac agc tct gaa agc att     1536
   Gly Tyr Phe Ala Val Val Thr Gly Lys Gly Asn Ser Ser Glu Ser Ile
               500                 505                 510
   gaa gct ata aga gaa tat gaa gaa gag ttt ttt cag aat tca aag ctc     1584
   Glu Ala Ile Arg Glu Tyr Glu Glu Glu Phe Phe Gln Asn Ser Lys Leu
           515                 520                 525
   cta aag aca agc atg cta aag gca cac caa gtg act aca aga aat tta      1632
   Leu Lys Thr Ser Met Leu Lys Ala His Gln Val Thr Thr Arg Asn Leu
       530                 535                 540
   agc ctt gca gta tca gac tgc ttt tgg aaa atg gta cga gag tct gtt      1680
   Ser Leu Ala Val Ser Asp Cys Phe Trp Lys Met Val Arg Glu Ser Val
   545                 550                 555                 560
   gaa caa cag gct gat agt ttc aaa gca aca cgt ttt aac ctt gaa act      1728
   Glu Gln Gln Ala Asp Ser Phe Lys Ala Thr Arg Phe Asn Leu Glu Thr
                   565                 570                 575
   gaa tgg aag aat aac tat cct cgc ctg cgg gaa ctt gac cgg aat gaa      1776
   Glu Trp Lys Asn Asn Tyr Pro Arg Leu Arg Glu Leu Asp Arg Asn Glu
               580                 585                 590
   cta ttt gaa aaa gct aaa aat gaa atc ctt gat gaa gtt atc agt ctg      1824
   Leu Phe Glu Lys Ala Lys Asn Glu Ile Leu Asp Glu Val Ile Ser Leu
           595                 600                 605
   agc cag gtt aca cca aaa cat tgg gag gaa atc ctt caa caa tct ttg      1872
   Ser Gln Val Thr Pro Lys His Trp Glu Glu Ile Leu Gln Gln Ser Leu
       610                 615                 620
   tgg gaa aga gta tca act cat gtg att gaa aac atc tac ctt cca gct      1920
   Trp Glu Arg Val Ser Thr His Val Ile Glu Asn Ile Tyr Leu Pro Ala
   625                 630                 635                 640
   gcg cag acc atg aat tca gga act ttt aac acc aca gtg gat atc aag      1968
   Ala Gln Thr Met Asn Ser Gly Thr Phe Asn Thr Thr Val Asp Ile Lys
                   645                 650                 655
   ctt aaa cag tgg act gat aaa caa ctt cct aat aaa gca gta gag gtt      2016
   Leu Lys Gln Trp Thr Asp Lys Gln Leu Pro Asn Lys Ala Val Glu Val
               660                 665                 670
   gct tgg gag acc cta caa gaa gaa ttt tcc cgc ttt atg aca gaa ccg      2064
   Ala Trp Glu Thr Leu Gln Glu Glu Phe Ser Arg Phe Met Thr Glu Pro
           675                 680                 685
   aaa ggg aaa gag cat gat gac ata ttt gat aaa ctt aaa gag gcc gtt      2112
   Lys Gly Lys Glu His Asp Asp Ile Phe Asp Lys Leu Lys Glu Ala Val
       690                 695                 700
   aag gaa gaa agt att aaa cga cac aag tgg aat gac ttt gcg gag gac      2160
   Lys Glu Glu Ser Ile Lys Arg His Lys Trp Asn Asp Phe Ala Glu Asp
   705                 710                 715                 720
   agc ttg agg gtt att caa cac aat gct ttg gaa gac cga tcc ata tct      2208
   Ser Leu Arg Val Ile Gln His Asn Ala Leu Glu Asp Arg Ser Ile Ser
                   725                 730                 735
   gat aaa cag caa tgg gat gca gct att tat ttt atg gaa gag gct ctg      2256
   Asp Lys Gln Gln Trp Asp Ala Ala Ile Tyr Phe Met Glu Glu Ala Leu
               740                 745                 750
   cag gct cgt ctc aag gat act gaa aat gca att gaa aac atg gtg ggt      2304
   Gln Ala Arg Leu Lys Asp Thr Glu Asn Ala Ile Glu Asn Met Val Gly
           755                 760                 765
   cca gac tgg aaa aag agg tgg tta tac tgg aag aat cgg acc caa gaa      2352
   Pro Asp Trp Lys Lys Arg Trp Leu Tyr Trp Lys Asn Arg Thr Gln Glu
       770                 775                     780
   cag tgt gtt cac aat gaa acc aag aat gaa ttg gag aag atg ttg aaa      2400
   Gln Cys Val His Asn Glu Thr Lys Asn Glu Leu Glu Lys Met Leu Lys
   785                 790                     795                 800
   tgt aat gag gag cac cca gct tat ctt gca agt gat gaa ata acc aca      2448
   Cys Asn Glu Glu His Pro Ala Tyr Leu Ala Ser Asp Glu Ile Thr Thr
                   805                     810                 815
   gtc cgg aag aac ctt gaa tcc cga gga gta gaa gta gat cca agc ttg      2496
   Val Arg Lys Asn Leu Glu Ser Arg Gly Val Glu Val Asp Pro Ser Leu
               820                 825                     830
   att aag gat act tgg cat caa gtt tat aga aga cat ttt tta aaa aca      2544
   Ile Lys Asp Thr Trp His Gln Val Tyr Arg Arg His Phe Leu Lys Thr
           835                     840                 845
   gct cta aac cat tgt aac ctt tgt cga aga ggt ttt tat tac tac caa      2592
   Ala Leu Asn His Cys Asn Leu Cys Arg Arg Gly Phe Tyr Tyr Tyr Gln
       850                 855                     860
   agg cat ttt gta gat tct gag ttg gaa tgc aat gat gtg gtc ttg ttt      2640
   Arg His Phe Val Asp Ser Glu Leu Glu Cys Asn Asp Val Val Leu Phe
   865                 870                     875                 880
   tgg cgt ata cag cgc atg ctt gct atc acc gca aat act tta agg caa      2688
   Trp Arg Ile Gln Arg Met Leu Ala Ile Thr Ala Asn Thr Leu Arg Gln
                   885                     890                 895
   caa ctt aca aat act gaa gtt agg cga tta gag aaa aat gtt aaa gag      2736
   Gln Leu Thr Asn Thr Glu Val Arg Arg Leu Glu Lys Asn Val Lys Glu
               900                 905                     910
   gta ttg gaa gat ttt gct gaa gat ggt gag aag aag att aaa ttg ctt      2784
   Val Leu Glu Asp Phe Ala Glu Asp Gly Glu Lys Lys Ile Lys Leu Leu
           915                     920                 925
   act ggt aaa cgc gtt caa ctg gcg gaa gac ctc aag aaa gtt aga gaa      2832
   Thr Gly Lys Arg Val Gln Leu Ala Glu Asp Leu Lys Lys Val Arg Glu
       930                 935                     940
   att caa gaa aaa ctt gat gct ttc att gaa gct ctt cat cag gag aaa      2880
   Ile Gln Glu Lys Leu Asp Ala Phe Ile Glu Ala Leu His Gln Glu Lys
   945                 950                     955                 960
   taa                                                                  2883
<210> 16
   <211> 960
   <212> PRT
   <213> Homo sapiens
<400> 16
   Met Trp Arg Leu Arg Arg Ala Ala Val Ala Cys Glu Val Cys Gln Ser
   1               5                   10                  15
   Leu Val Lys His Ser Ser Gly Ile Lys Gly Ser Leu Pro Leu Gln Lys
   20 25 30
   Leu His Leu Val Ser Arg Ser Ile Tyr His Ser His His Pro Thr Leu
   35 40 45
   Lys Leu Gln Arg Pro Gln Leu Arg Thr Ser Phe Gln Gln Phe Ser Ser
   50 55 60
   Leu Thr Asn Leu Pro Leu Arg Lys Leu Lys Phe Ser Pro Ile Lys Tyr
   65 70 75 80
   Gly Tyr Gln Pro Arg Arg Asn Phe Trp Pro Ala Arg Leu Ala Thr Arg
   85 90 95
   Leu Leu Lys Leu Arg Tyr Leu Ile Leu Gly Ser Ala Val Gly Gly Gly
   100 105 110
   Tyr Thr Ala Lys Lys Thr Phe Asp Gln Trp Lys Asp Met Ile Pro Asp
   115 120 125
   Leu Ser Glu Tyr Lys Trp Ile Val Pro Asp Ile Val Trp Glu Ile Asp
   130 135 140
   Glu Tyr Ile Asp Phe Glu Lys Ile Arg Lys Ala Leu Pro Asn Ser Glu
   145 150 155 160
   Asp Leu Val Lys Leu Ala Pro Asp Phe Asp Lys Ile Val Glu Ser Leu
   165 170 175
   Ser Leu Leu Lys Asp Phe Phe Thr Ser Gly Ser Pro Glu Glu Thr Ala
   180 185 190
   Phe Arg Ala Thr Asp Arg Gly Ser Glu Ser Asp Lys His Phe Arg Lys
   195 200 205
   Val Ser Asp Lys Glu Lys Ile Asp Gln Leu Gln Glu Glu Leu Leu His
   210 215 220
   Thr Gln Leu Lys Tyr Gln Arg Ile Leu Glu Arg Leu Glu Lys Glu Asn
   225 230 235 240
   Lys Glu Leu Arg Lys Leu Val Leu Gln Lys Asp Asp Lys Gly Ile His
   245 250 255
   His Arg Lys Leu Lys Lys Ser Leu Ile Asp Met Tyr Ser Glu Val Leu
   260 265 270
   Asp Val Leu Ser Asp Tyr Asp Ala Ser Tyr Asn Thr Gln Asp His Leu
   275 280 285
   Pro Arg Val Val Val Val Gly Asp Gln Ser Ala Gly Lys Thr Ser Val
   290 295 300
   Leu Glu Met Ile Ala Gln Ala Arg Ile Phe Pro Arg Gly Ser Gly Glu
   305 310 315 320
   Met Met Thr Arg Ser Pro Val Lys Val Thr Leu Ser Glu Gly Pro His
   325 330 335
   His Val Ala Leu Phe Lys Asp Ser Ser Arg Glu Phe Asp Leu Thr Lys
   340 345 350
   Glu Glu Asp Leu Ala Ala Leu Arg His Glu Ile Glu Leu Arg Met Arg
   355 360 365
   Lys Asn Val Lys Glu Gly Cys Thr Val Ser Pro Glu Thr Ile Ser Leu
   370 375 380
   Asn Val Lys Gly Pro Gly Leu Gln Arg Met Val Leu Val Asp Leu Pro
   385 390 395 400
   Gly Val Ile Asn Thr Val Thr Ser Gly Met Ala Pro Asp Thr Lys Glu
   405 410 415
   Thr Ile Phe Ser Ile Ser Lys Ala Tyr Met Gln Asn Pro Asn Ala Ile
   420 425 430
   Ile Leu Cys Ile Gln Asp Gly Ser Val Asp Ala Glu Arg Ser Ile Val
   435 440 445
   Thr Asp Leu Val Ser Gln Met Asp Pro His Gly Arg Arg Thr Ile Phe
   450 455 460
   Val Leu Thr Lys Val Asp Leu Ala Glu Lys Asn Val Ala Ser Pro Ser
   465 470 475 480
   Arg Ile Gln Gln Ile Ile Glu Gly Lys Leu Phe Pro Met Lys Ala Leu
   485 490 495
   Gly Tyr Phe Ala Val Val Thr Gly Lys Gly Asn Ser Ser Glu Ser Ile
               500                 505                 510
   Glu Ala Ile Arg Glu Tyr Glu Glu Glu Phe Phe Gln Asn Ser Lys Leu
           515                 520                 525
   Leu Lys Thr Ser Met Leu Lys Ala His Gln Val Thr Thr Arg Asn Leu
       530                 535                 540
   Ser Leu Ala Val Ser Asp Cys Phe Trp Lys Met Val Arg Glu Ser Val
   545                 550                 555                 560
   Glu Gln Gln Ala Asp Ser Phe Lys Ala Thr Arg Phe Asn Leu Glu Thr
                   565                 570                 575
   Glu Trp Lys Asn Asn Tyr Pro Arg Leu Arg Glu Leu Asp Arg Asn Glu
               580                 585                 590
   Leu Phe Glu Lys Ala Lys Asn Glu Ile Leu Asp Glu Val Ile Ser Leu
           595                 600                 605
   Ser Gln Val Thr Pro Lys His Trp Glu Glu Ile Leu Gln Gln Ser Leu
       610                 615                 620
   Trp Glu Arg Val Ser Thr His Val Ile Glu Asn Ile Tyr Leu Pro Ala
   625                 630                 635                 640
   Ala Gln Thr Met Asn Ser Gly Thr Phe Asn Thr Thr Val Asp Ile Lys
                   645                 650                 655
   Leu Lys Gln Trp Thr Asp Lys Gln Leu Pro Asn Lys Ala Val Glu Val
               660                 665                 670
   Ala Trp Glu Thr Leu Gln Glu Glu Phe Ser Arg Phe Met Thr Glu Pro
           675                 680                 685
   Lys Gly Lys Glu His Asp Asp Ile Phe Asp Lys Leu Lys Glu Ala Val
       690                 695                 700
   Lys Glu Glu Ser Ile Lys Arg His Lys Trp Asn Asp Phe Ala Glu Asp
   705                 710                 715                 720
   Ser Leu Arg Val Ile Gln His Asn Ala Leu Glu Asp Arg Ser Ile Ser
                   725                 730                 735
   Asp Lys Gln Gln Trp Asp Ala Ala Ile Tyr Phe Met Glu Glu Ala Leu
               740                 745                 750
   Gln Ala Arg Leu Lys Asp Thr Glu Asn Ala Ile Glu Asn Met Val Gly
           755                 760                 765
   Pro Asp Trp Lys Lys Arg Trp Leu Tyr Trp Lys Asn Arg Thr Gln Glu
       770                 775                 780
   Gln Cys Val His Asn Glu Thr Lys Asn Glu Leu Glu Lys Met Leu Lys
   785                 790                 795                 800
   Cys Asn Glu Glu His Pro Ala Tyr Leu Ala Ser Asp Glu Ile Thr Thr
                   805                 810                 815
   Val Arg Lys Asn Leu Glu Ser Arg Gly Val Glu Val Asp Pro Ser Leu
               820                 825                 830
   Ile Lys Asp Thr Trp His Gln Val Tyr Arg Arg His Phe Leu Lys Thr
           835                 840                 845
   Ala Leu Asn His Cys Asn Leu Cys Arg Arg Gly Phe Tyr Tyr Tyr Gln
       850                 855                 860
   Arg His Phe Val Asp Ser Glu Leu Glu Cys Asn Asp Val Val Leu Phe
   865                 870                 875                 880
   Trp Arg Ile Gln Arg Met Leu Ala Ile Thr Ala Asn Thr Leu Arg Gln
                   885                 890                 895
   Gln Leu Thr Asn Thr Glu Val Arg Arg Leu Glu Lys Asn Val Lys Glu
               900                 905                 910
   Val Leu Glu Asp Phe Ala Glu Asp Gly Glu Lys Lys Ile Lys Leu Leu
           915                 920                 925
   Thr Gly Lys Arg Val Gln Leu Ala Glu Asp Leu Lys Lys Val Arg Glu
       930                 935                 940
   Ile Gln Glu Lys Leu Asp Ala Phe Ile Glu Ala Leu His Gln Glu Lys
   945                 950                 955                 960
<210> 17
   <211> 15
   <212> PRT
   <213> artificial sequence
<220>
   <223> fragment of OPA1
<220>
   <221> soruce
   <222> (1)..(15)
   <223> OPA1
<400> 17
   Tyr Leu Ile Leu Gly Ser Ala Val Gly Gly Gly Tyr Thr Ala Lys
   1               5                   10                  15
<210> 18
   <211> 6
   <212> PRT
   <213> artificial sequence
<220>
   <223> fragment of OPA1
<220>
   <221> source
   <222> (1)..(6)
   <223> OPA1
<400> 18
   Thr Phe Asp Gln Trp Lys
   1               5
<210> 19
   <211> 10
   <212> PRT
   <213> artificial sequence
<220>
   <223> fragment of OPA1
<220>
   <221> source
   <222> (1)..(10)
   <223> OPA1
<400> 19
   Asp Met Ile Pro Asp Leu Ser Glu Tyr Lys
   1               5                   10
<210> 20
   <211> 18
   <212> PRT
   <213> artificial sequence
<220>
   <223> fragment of OPA1
<220>
   <221> source
   <222> (1)..(18)
   <223> OPA1
<400> 20
   Trp Ile Val Pro Asp Ile Val Trp Glu Ile Asp Glu Tyr Ile Asp Phe
   1               5                   10                  15
   Glu Lys
<210> 21
   <211> 7
   <212> PRT
   <213> artificial sequence
<220>
   <223> fragment of OPA1
<220>
   <221> source
   <222> (1)..(7)
   <223> OPA1
<400> 21
   Leu Ala Pro Asp Phe Asp Lys
   1               5
<210> 22
   <211> 9
   <212> PRT
   <213> artificial sequence
<220>
   <223> fragment of OPA1
<220>
   <221> source
   <222> (1)..(9)
   <223> OPA1
<400> 22
   Ile Val Glu Ser Leu Ser Leu Leu Lys
   1               5
<210> 23
   <211> 10
   <212> PRT
   <213> artificial sequence
<220>
   <223> fragment of OPA1
<220>
   <221> source
   <222> (1)..(10)
   <223> OPA1
<400> 23
   Ala Leu Pro Asn Ser Glu Asp Leu Val Lys
   1               5                   10
<210> 24
   <211> 14
   <212> PRT
   <213> artificial sequence
<220>
   <223> fragment of OPA1
<220>
   <221> source
   <222> (1)..(14)
   <223> OPA1
<400> 24
   Asp Phe Phe Thr Ser Gly Ser Pro Glu Glu Thr Ala Phe Arg
   1               5                   10
<210> 25
   <211> 13
   <212> PRT
   <213> artificial sequence
<220>
   <223> fragment of OPA1
<220>
   <221> source
   <222> (1)..(13)
   <223> OPA1
<400> 25
   Thr Arg Leu Leu Lys Leu Arg Tyr Leu Ile Leu Gly Ser
   1               5                   10
<210> 26
   <211> 21
   <212> PRT
   <213> artificial sequence
<220>
   <223> fragment of OPA1
<220>
   <221> source
   <222> (1)..(21)
   <223> OPA1
<400> 26
   Gly Leu Leu Gly Glu Leu Ile Leu Leu Gln Gln Gln Ile Gln Glu His
   1               5                   10                  15
   Glu Glu Glu Ala Arg
               20
<210> 27
   <211> 13
   <212> PRT
   <213> artificial sequence
<220>
   <223> fragment of OPA1
<220>
   <221> source
   <222> (1)..(13)
   <223> OPA1
<400> 27
   Ala Ala Gly Gln Tyr Ser Thr Ser Tyr Ala Gln Gln Lys
   1               5                   10
<210> 28
   <211> 14
   <212> PRT
   <213> artificial sequence
<220>
   <223> fragment of OPA1
<220>
   <221> source
   <222> (1)..(14)
   <223> OPA1
<400> 28
   Ile Asp Gln Leu Gln Glu Glu Leu Leu His Thr Gln Leu Lys
   1               5                   10
<210> 29
   <211> 24
   <212> PRT
   <213> artificial sequence
<220>
   <223> fragment of OPA1
<220>
   <221> source
   <222> (1)..(24)
   <223> OPA1
<400> 29
   Cys Asp Leu Lys Lys Val Arg Glu Ile Gln Glu Lys Leu Asp Ala Phe
   1               5                   10                  15
   Ile Glu Ala Leu His Gln Glu Lys
               20
<210> 30
   <211> 23
   <212> PRT
   <213> artificial sequence
<220>
   <223> fragment of OPA1
<220>
   <221> source
   <222> (1)..(23)
   <223> OPA1
<400> 30
   Asp Leu Lys Lys Val Arg Glu Ile Gln Glu Lys Leu Asp Ala Phe Ile
   1               5                   10                  15
   Glu Ala Leu His Gln Glu Lys
               20
<210> 31
   <211> 37
   <212> PRT
   <213> artificial sequence
<220>
   <223> fragment of OPA1
<220>
   <221> source
   <222> (1)..(37)
   <223> OPA1
<400> 31
   Glu Tyr Lys Trp Ile Val Pro Asp Ile Val Trp Glu Ile Asp Glu Tyr
   1               5                   10                  15
   Ile Asp Phe Gly His Lys Leu Val Ser Glu Val Ile Gly Ala Ser Asp
               20                  25                  30
   Leu Leu Leu Leu Leu
           35
<210> 32
   <211> 42
   <212> PRT
   <213> artificial sequence
<220>
   <223> fragment of OPA1
<220>
   <221> source
   <222> (1)..(42)
   <223> OPA1
<400> 32
   Glu Tyr Lys Trp Ile Val Pro Asp Ile Val Trp Glu Ile Asp Glu Tyr
   1               5                   10                  15
   Ile Asp Phe Gly Ser Pro Glu Glu Thr Ala Phe Arg Ala Thr Asp Arg
               20                  25                  30
   Gly Ser Glu Ser Asp Lys His Phe Arg Lys
           35                  40
<210> 33
   <211> 59
   <212> PRT
   <213> artificial sequence
<220>
   <223> fragment of OPA1
<220>
   <221> source
   <222> (1)..(59)
   <223> OPA1
<400> 33
   Glu Lys Ile Arg Lys Ala Leu Pro Asn Ser Glu Asp Leu Val Lys Leu
   1               5                   10                  15
   Ala Pro Asp Phe Asp Lys Ile Val Glu Ser Leu Ser Leu Leu Lys Asp
               20                  25                  30
   Phe Phe Thr Ser Gly Ser Pro Glu Glu Thr Ala Phe Arg Ala Thr Asp
           35                  40                  45
   Arg Gly Ser Glu Ser Asp Lys His Phe Arg Lys
       50                  55
<210> 34
   <211> 60
   <212> PRT
   <213> artificial sequence
<220>
   <223> fragment of OPA1
<220>
   <221> source
   <222> (1)..(60)
   <223> OPA1
<400> 34
   Gly Ser Pro Glu Glu Thr Ala Phe Arg Ala Thr Asp Arg Gly Ser Glu
   1               5                   10                  15
   Ser Asp Lys His Phe Arg Lys Val Ser Asp Lys Glu Lys Ile Asp Gln
               20                  25                  30
   Leu Gln Glu Glu Leu Leu His Thr Gln Leu Lys Tyr Gln Arg Ile Leu
           35                  40                  45
   Glu Arg Leu Glu Lys Glu Asn Lys Glu Leu Arg Lys
       50                  55                  60
<210> 35
   <211> 13
   <212> PRT
   <213> artificial sequence
<220>
   <223> fragment of OPA1
<220>
   <221> source
   <222> (1)..(13)
   <223> OPA1
<400> 35
   Ala Ala Val Ala Cys Glu Val Cys Gln Ser Leu Val Lys
   1 5 10
<210> 36
   <211> 6
   <212> PRT
   <213> artificial sequence
<220>
   <223> fragment of OPA1
<220>
   <221> source
   <222> (1)..(6)
   <223> OPA1
<400> 36
   His Ser Ser Gly Ile Lys
   1 5
<210> 37
   <211> 7
   <212> PRT
   <213> artificial sequence
<220>
   <223> fragment of OPA1
<220>
   <221> source
   <222> (1)..(7)
   <223> OPA1
<400> 37
   Gly Ser Leu Pro Leu Gln Lys
   1 5
<210> 38
   <211> 6
   <212> PRT
   <213> artificial sequence
<220>
   <223> fragment of OPA1
<220>
   <221> source
   <222> (1)..(6)
   <223> OPA1
<400> 38
   Leu His Leu Val Ser Arg
   1 5
<210> 39
   <211> 11
   <212> PRT
   <213> artificial sequence
<220>
   <223> fragment of OPA1
<220>
   <221> source
   <222> (1)..(11)
   <223> OPA1
<400> 39
   Ser Ile Tyr His Ser His His Pro Thr Leu Lys
   1 5 10
<210> 40
   <211> 15
   <212> PRT
   <213> artificial sequence
<220>
   <223> fragment of OPA1
<220>
   <221> source
   <222> (1)..(15)
   <223> OPA1
<400> 40
   Thr Ser Phe Gln Gln Phe Ser Ser Leu Thr Asn Leu Pro Leu Arg
   1 5 10 15
<210> 41
   <211> 5
   <212> PRT
   <213> artificial sequence
<220>
   <223> fragment of OPA1
<220>
   <221> source
   <222> (1)..(5)
   <223> OPA1
<400> 41
   Phe Ser Pro Ile Lys
   1 5
<210> 42
   <211> 6
   <212> PRT
   <213> artificial sequence
<220>
   <223> fragment of OPA1
<220>
   <221> source
   <222> (1)..(6)
   <223> OPA1
<400> 42
   Tyr Gly Tyr Gln Pro Arg
   1               5
<210> 43
   <211> 6
   <212> PRT
   <213> artificial sequence
<220>
   <223> fragment of OPA1
<220>
   <221> source
   <222> (1)..(6)
   <223> OPA1
<400> 43
   Asn Phe Trp Pro Ala Arg
   1               5
<210> 44
   <211> 24
   <212> PRT
   <213> artificial sequence
<220>
   <223> fragment of OPA1
<220>
   <221> source
   <222> (1)..(24)
   <223> OPA1
<400> 44
   Leu Val Ser Glu Val Ile Gly Ala Ser Asp Leu Leu Leu Leu Leu Gly
   1               5                   10                  15
   Ser Pro Glu Glu Thr Ala Phe Arg
               20
<210> 45
   <211> 6
   <212> PRT
   <213> artificial sequence
<220>
   <223> fragment of OPA1
<220>
   <221> source
   <222> (1)..(6)
   <223> OPA1
<400> 45
   Gly Ser Glu Ser Asp Lys
   1               5
<210> 46
   <211> 21
   <212> PRT
   <213> artificial sequence
<220>
   <223> fragment of OPA1
<220>
   <221> source
   <222> (1)..(21)
   <223> OPA1
<400> 46
   Gly Leu Leu Gly Glu Leu Ile Leu Leu Gln Gln Gln Ile Gln Glu His
   1               5                   10                  15
   Glu Glu Glu Ala Arg
               20
<210> 47
   <211> 5
   <212> PRT
   <213> artificial sequence
<220>
   <223> fragment of OPA1
<220>
   <221> source
   <222> (1)..(5)
   <223> OPA1
<400> 47
   Leu Val Leu Gln Lys
   1               5
<210> 48
   <211> 5
   <212> PRT
   <213> artificial sequence
<220>
   <223> fragment of OPA1
<220>
   <221> source
   <222> (1)..(5)
   <223> OPA1
<400> 48
   Gly Ile His His Arg
   1 5
<210> 49
   <211> 28
   <212> PRT
   <213> artificial sequence
<220>
   <223> fragment of OPA1
<220>
   <221> source
   <222> (1)..(28)
   <223> OPA1
<400> 49
   Ser Leu Ile Asp Met Tyr Ser Glu Val Leu Asp Val Leu Ser Asp Tyr
   1 5 10 15
   Asp Ala Ser Tyr Asn Thr Gln Asp His Leu Pro Arg
   20 25
<210> 50
   <211> 11
   <212> PRT
   <213> artificial sequence
<220>
   <223> fragment of OPA1
<220>
   <221> source
   <222> (1)..(11)
   <223> OPA1
<400> 50
   Val Val Val Val Gly Asp Gln Ser Ala Gly Lys
   1 5 10
<210> 51
   <211> 11
   <212> PRT
   <213> artificial sequence
<220>
   <223> fragment of OPA1
<220>
   <221> source
   <222> (1)..(11)
   <223> OPA1
<400> 51
   Thr Ser Val Leu Glu Met Ile Ala Gln Ala Arg
   1               5                   10
<210> 52
   <211> 8
   <212> PRT
   <213> artificial sequence
<220>
   <223> fragment of OPA1
<220>
   <221> source
   <222> (1)..(8)
   <223> OPA1
<400> 52
   Gly Ser Gly Glu Met Met Thr Arg
   1               5
<210> 53
   <211> 14
   <212> PRT
   <213> artificial sequence
<220>
   <223> fragment of OPA1
<220>
   <221> source
   <222> (1)..(14)
   <223> OPA1
<400> 53
   Val Thr Leu Ser Glu Gly Pro His His Val Ala Leu Phe Lys
   1               5                   10
<210> 54
   <211> 6
   <212> PRT
   <213> artificial sequence
<220>
   <223> fragment of OPA1
<220>
   <221> source
   <222> (1)..(6)
   <223> OPA1
<400> 54
   Glu Phe Asp Leu Thr Lys
   1               5
<210> 55
   <211> 8
   <212> PRT
   <213> artificial sequence
<220>
   <223> fragment of OPA1
<220>
   <221> source
   <222> (1)..(8)
   <223> OPA1
<400> 55
   Glu Glu Asp Leu Ala Ala Leu Arg
   1               5
<210> 56
   <211> 6
   <212> PRT
   <213> artificial sequence
<220>
   <223> fragment of OPA1
<220>
   <221> source
   <222> (1)..(6)
   <223> OPA1
<400> 56
   His Glu Ile Glu Leu Arg
   1               5
<210> 57
   <211> 15
   <212> PRT
   <213> artificial sequence
<220>
   <223> fragment of OPA1
<220>
   <221> source
   <222> (1)..(15)
   <223> OPA1
<400> 57
   Glu Gly Cys Thr Val Ser Pro Glu Thr Ile Ser Leu Asn Val Lys
   1 5 10 15
<210> 58
   <211> 6
   <212> PRT
   <213> artificial sequence
<220>
   <223> fragment of OPA1
<220>
   <221> source
   <222> (1)..(6)
   <223> OPA1
<400> 58
   Gly Pro Gly Leu Gln Arg
   1 5
<210> 59
   <211> 22
   <212> PRT
   <213> artificial sequence
<220>
   <223> fragment of OPA1
<220>
   <221> source
   <222> (1)..(22)
   <223> OPA1
<400> 59
   Met Val Leu Val Asp Leu Pro Gly Val Ile Asn Thr Val Thr Ser Gly
   1 5 10 15
   Met Ala Pro Asp Thr Lys
   20
<210> 60
   <211> 8
   <212> PRT
   <213> artificial sequence
<220>
   <223> fragment of OPA1
<220>
   <221> source
   <222> (1)..(8)
   <223> OPA1
<400> 60
   Glu Thr Ile Phe Ser Ile Ser Lys
   1 5
<210> 61
   <211> 22
   <212> PRT
   <213> artificial sequence
<220>
   <223> fragment of OPA1
<220>
   <221> source
   <222> (1)..(22)
   <223> OPA1
<400> 61
   Ala Tyr Met Gln Asn Pro Asn Ala Ile Ile Leu Cys Ile Gln Asp Gly
   1 5 10 15
   Ser Val Asp Ala Glu Arg
   20
<210> 62
   <211> 15
   <212> PRT
   <213> artificial sequence
<220>
   <223> fragment of OPA1
<220>
   <221> source
   <222> (1)..(15)
   <223> OPA1
<400> 62
   Ser Ile Val Thr Asp Leu Val Ser Gln Met Asp Pro His Gly Arg
   1 5 10 15
<210> 63
   <211> 7
   <212> PRT
   <213> artificial sequence
<220>
   <223> fragment of OPA1
<220>
   <221> source
   <222> (1)..(7)
   <223> OPA1
<400> 63
   Thr Ile Phe Val Leu Thr Lys
   1 5
<210> 64
   <211> 6
   <212> PRT
   <213> artificial sequence
<220>
   <223> fragment of OPA1
<220>
   <221> source
   <222> (1)..(6)
   <223> OPA1
<400> 64
   Val Asp Leu Ala Glu Lys
   1 5
<210> 65
   <211> 7
   <212> PRT
   <213> artificial sequence
<220>
   <223> fragment of OPA1
<400> 65
   Asn Val Ala Ser Pro Ser Arg
   1 5
<210> 66
   <211> 8
   <212> PRT
   <213> artificial sequence
<220>
   <223> fragment of OPA1
<220>
   <221> source
   <222> (1)..(8)
   <223> OPA1
<400> 66
   Ile Gln Gln Ile Ile Glu Gly Lys
   1 5
<210> 67
   <211> 5
   <212> PRT
   <213> artificial sequence
<220>
   <223> fragment of OPA1
<220>
   <221> source
   <222> (1)..(5)
   <223> OPA1
<400> 67
   Leu Phe Pro Met Lys
   1 5
<210> 68
   <211> 11
   <212> PRT
   <213> artificial sequence
<220>
   <223> fragment of OPA1
<220>
   <221> source
   <222> (1)..(11)
   <223> OPA1
<400> 68
   Ala Leu Gly Tyr Phe Ala Val Val Thr Gly Lys
   1 5 10
<210> 69
   <211> 11
   <212> PRT
   <213> artificial sequence
<220>
   <223> fragment of OPA1
<220>
   <221> source
   <222> (1)..(11)
   <223> OPA1
<400> 69
   Gly Asn Ser Ser Glu Ser Ile Glu Ala Ile Arg
   1 5 10
<210> 70
   <211> 11
   <212> PRT
   <213> artificial sequence
<220>
   <223> fragment of OPA1
<220>
   <221> source
   <222> (1)..(11)
   <223> OPA1
<400> 70
   Glu Tyr Glu Glu Glu Phe Phe Gln Asn Ser Lys
   1 5 10
<210> 71
   <211> 5
   <212> PRT
   <213> artificial sequence
<220>
   <223> fragment of OPA1
<220>
   <221> source
   <222> (1)..(5)
   <223> OPA1
<400> 71
   Thr Ser Met Leu Lys
   1 5
<210> 72
   <211> 7
   <212> PRT
   <213> artificial sequence
<220>
   <223> fragment of OPA1
<220>
   <221> source
   <222> (1)..(7)
   <223> OPA1
<400> 72
   Ala His Gln Val Thr Thr Arg
   1 5
<210> 73
   <211> 12
   <212> PRT
   <213> artificial sequence
<220>
   <223> fragment of OPA1
<220>
   <221> source
   <222> (1)..(12)
   <223> OPA1
<400> 73
   Asn Leu Ser Leu Ala Val Ser Asp Cys Phe Trp Lys
   1 5 10
<210> 74
   <211> 11
   <212> PRT
   <213> artificial sequence
<220>
   <223> fragment of OPA1
<220>
   <221> source
   <222> (1)..(11)
   <223> OPA1
<400> 74
   Glu Ser Val Glu Gln Gln Ala Asp Ser Phe Lys
   1 5 10
<210> 75
   <211> 8
   <212> PRT
   <213> artificial sequence
<220>
   <223> fragment of OPA1
<220>
   <221> source
   <222> (1)..(8)
   <223> OPA1
<400> 75
   Phe Asn Leu Glu Thr Glu Trp Lys
   1 5
<210> 76
   <211> 5
   <212> PRT
   <213> artificial sequence
<220>
   <223> fragment of OPA1
<220>
   <221> source
   <222> (1)..(5)
   <223> OPA1
<400> 76
   Asn Asn Tyr Pro Arg
   1               5
<210> 77
   <211> 6
   <212> PRT
   <213> artificial sequence
<220>
   <223> fragment of OPA1
<220>
   <221> source
   <222> (1)..(6)
   <223> OPA1
<400> 77
   Asn Glu Leu Phe Glu Lys
   1               5
<210> 78
   <211> 16
   <212> PRT
   <213> artificial sequence
<220>
   <223> fragment of OPA1
<220>
   <221> source
   <222> (1)..(16)
   <223> OPA1
<400> 78
   Asn Glu Ile Leu Asp Glu Val Ile Ser Leu Ser Gln Val Thr Pro Lys
   1               5                   10                  15
<210> 79
   <211> 13
   <212> PRT
   <213> artificial sequence
<220>
   <223> fragment of OPA1
<400> 79
   His Trp Glu Glu Ile Leu Gln Gln Ser Leu Trp Glu Arg
   1 5 10
<210> 80
   <211> 29
   <212> PRT
   <213> artificial sequence
<220>
   <223> fragment of OPA1
<220>
   <221> source
   <222> (1)..(29)
   <223> OPA1
<400> 80
   Val Ser Thr His Val Ile Glu Asn Ile Tyr Leu Pro Ala Ala Gln Thr
   1 5 10 15
   Met Asn Ser Gly Thr Phe Asn Thr Thr Val Asp Ile Lys
   20 25
<210> 81
   <211> 5
   <212> PRT
   <213> artificial sequence
<220>
   <223> fragment of OPA1
<220>
   <221> source
   <222> (1)..(5)
   <223> OPA1
<400> 81
   Gln Trp Thr Asp Lys
   1 5
<210> 82
   <211> 5
   <212> PRT
   <213> artificial sequence
<220>
   <223> fragment of OPA1
<220>
   <221> source
   <222> (1)..(5)
   <223> OPA1
<400> 82
   Gln Leu Pro Asn Lys
   1 5
<210> 83
   <211> 15
   <212> PRT
   <213> artificial sequence
<220>
   <223> fragment of OPA1
<220>
   <221> source
   <222> (1)..(15)
   <223> OPA1
<400> 83
   Ala Val Glu Val Ala Trp Glu Thr Leu Gln Glu Glu Phe Ser Arg
   1 5 10 15
<210> 84
   <211> 6
   <212> PRT
   <213> artificial sequence
<220>
   <223> fragment of OPA1
<220>
   <221> source
   <222> (1)..(6)
   <223> OPA1
<400> 84
   Phe Met Thr Glu Pro Lys
   1 5
<210> 85
   <211> 8
   <212> PRT
   <213> artificial sequence
<220>
   <223> fragment of OPA1
<220>
   <221> source
   <222> (1)..(8)
   <223> OPA1
<400> 85
   Glu His Asp Asp Ile Phe Asp Lys
   1 5
<210> 86
   <211> 5
   <212> PRT
   <213> artificial sequence
<220>
   <223> fragment of OPA1
<220>
   <221> source
   <222> (1)..(5)
   <223> OPA1
<400> 86
   Glu Glu Ser Ile Lys
   1               5
<210> 87
   <211> 10
   <212> PRT
   <213> artificial sequence
<220>
   <223> fragment of OPA1
<220>
   <221> source
   <222> (1)..(10)
   <223> OPA1
<400> 87
   Trp Asn Asp Phe Ala Glu Asp Ser Leu Arg
   1               5                   10
<210> 88
   <211> 10
   <212> PRT
   <213> artificial sequence
<220>
   <223> fragment of OPA1
<220>
   <221> source
   <222> (1)..(10)
   <223> OPA1
<400> 88
   Val Ile Gln His Asn Ala Leu Glu Asp Arg
   1               5                   10
<210> 89
   <211> 5
   <212> PRT
   <213> artificial sequence
<220>
   <223> fragment of OPA1
<220>
   <221> source
   <222> (1)..(5)
   <223> OPA1
<400> 89
   Ser Ile Ser Asp Lys
   1 5
<210> 90
   <211> 17
   <212> PRT
   <213> artificial sequence
<220>
   <223> fragment of OPA1
<220>
   <221> source
   <222> (1)..(17)
   <223> OPA1
<400> 90
   Gln Gln Trp Asp Ala Ala Ile Tyr Phe Met Glu Glu Ala Leu Gln Ala
   1 5 10 15
   Arg
<210> 91
   <211> 15
   <212> PRT
   <213> artificial sequence
<220>
   <223> fragment of OPA1
<220>
   <221> source
   <222> (1)..(15)
   <223> OPA1
<400> 91
   Asp Thr Glu Asn Ala Ile Glu Asn Met Val Gly Pro Asp Trp Lys
   1 5 10 15
<210> 92
   <211> 5
   <212> PRT
   <213> artificial sequence
<220>
   <223> fragment of OPA1
<220>
   <221> source
   <222> (1)..(5)
   <223> OPA1
<400> 92
   Trp Leu Tyr Trp Lys
   1               5
<210> 93
   <211> 11
   <212> PRT
   <213> artificial sequence
<220>
   <223> fragment of OPA1
<220>
   <221> source
   <222> (1)..(11)
   <223> OPA1
<400> 93
   Thr Gln Glu Gln Cys Val His Asn Glu Thr Lys
   1               5                   10
<210> 94
   <211> 5
   <212> PRT
   <213> artificial sequence
<220>
   <223> fragment of OPA1
<220>
   <221> source
   <222> (1)..(5)
   <223> OPA1
<400> 94
   Asn Glu Leu Glu Lys
   1               5
<210> 95
   <211> 18
   <212> PRT
   <213> artificial sequence
<220>
   <223> fragment of OPA1
<220>
   <221> source
   <222> (1)..(18)
   <223> OPA1
<400> 95
   Cys Asn Glu Glu His Pro Ala Tyr Leu Ala Ser Asp Glu Ile Thr Thr
   1               5                   10                  15
   Val Arg
<210> 96
   <211> 5
   <212> PRT
   <213> artificial sequence
<220>
   <223> fragment of OPA1
<220>
   <221> source
   <222> (1)..(5)
   <223> OPA1
<400> 96
   Asn Leu Glu Ser Arg
   1               5
<210> 97
   <211> 10
   <212> PRT
   <213> artificial sequence
<220>
   <223> fragment of OPA1
<220>
   <221> source
   <222> (1)..(10)
   <223> OPA1
<400> 97
   Gly Val Glu Val Asp Pro Ser Leu Ile Lys
   1               5                   10
<210> 98
   <211> 8
   <212> PRT
   <213> artificial sequence
<220>
   <223> fragment of OPA1
<220>
   <221> source
   <222> (1)..(8)
   <223> OPA1
<400> 98
   Asp Thr Trp His Gln Val Tyr Arg
   1 5
<210> 99
   <211> 10
   <212> PRT
   <213> artificial sequence
<220>
   <223> fragment of OPA1
<220>
   <221> source
   <222> (1)..(10)
   <223> OPA1
<400> 99
   Thr Ala Leu Asn His Cys Asn Leu Cys Arg
   1 5 10
<210> 100
   <211> 7
   <212> PRT
   <213> artificial sequence
<220>
   <223> fragment of OPA1
<220>
   <221> source
   <222> (1)..(7)
   <223> OPA1
<400> 100
   Gly Phe Tyr Tyr Tyr Gln Arg
   1 5
<210> 101
   <211> 17
   <212> PRT
   <213> artificial sequence
<220>
   <223> fragment of OPA1
<220>
   <221> source
   <222> (1)..(17)
   <223> OPA1
<400> 101
   His Phe Val Asp Ser Glu Leu Glu Cys Asn Asp Val Val Leu Phe Trp
   1               5                   10                  15
   Arg
<210> 102
   <211> 10
   <212> PRT
   <213> artificial sequence
<220>
   <223> fragment of OPA1
<220>
   <221> source
   <222> (1)..(10)
   <223> OPA1
<400> 102
   Met Leu Ala Ile Thr Ala Asn Thr Leu Arg
   1               5                   10
<210> 103
   <211> 9
   <212> PRT
   <213> artificial sequence
<220>
   <223> fragment of OPA1
<220>
   <221> source
   <222> (1)..(9)
   <223> OPA1
<400> 103
   Gln Gln Leu Thr Asn Thr Glu Val Arg
   1               5
<210> 104
   <211> 12
   <212> PRT
   <213> artificial sequence
<220>
   <223> fragment of OPA1
<220>
   <221> source
   <222> (1)..(12)
   <223> OPA1
<400> 104
   Glu Val Leu Glu Asp Phe Ala Glu Asp Gly Glu Lys
   1 5 10
<210> 105
   <211> 5
   <212> PRT
   <213> artificial sequence
<220>
   <223> fragment of OPA1
<220>
   <221> source
   <222> (1)..(5)
   <223> OPA1
<400> 105
   Leu Leu Thr Gly Lys
   1 5
<210> 106
   <211> 8
   <212> PRT
   <213> artificial sequence
<220>
   <223> fragment of OPA1
<220>
   <221> source
   <222> (1)..(8)
   <223> OPA1
<400> 106
   Val Gln Leu Ala Glu Asp Leu Lys
   1 5
<210> 107
   <211> 5
   <212> PRT
   <213> artificial sequence
<220>
   <223> fragment of OPA1
<220>
   <221> source
   <222> (1)..(5)
   <223> OPA1
<400> 107
   Glu Ile Gln Glu Lys
   1 5
<210> 108
   <211> 12
   <212> PRT
   <213> artificial sequence
<220>
   <223> fragment of OPA1
<220>
   <221> source
   <222> (1)..(12)
   <223> OPA1
<400> 108
   Leu Asp Ala Phe Ile Glu Ala Leu His Gln Glu Lys
   1 5 10
<210> 109
   <211> 25
   <212> PRT
   <213> artificial sequence
<220>
   <223> fragment of OPA1
<220>
   <221> source
   <222> (1)..(25)
   <223> OPA1
<400> 109
   Trp Ile Val Pro Asp Ile Val Trp Glu Ile Asp Glu Tyr Ile Asp Phe
   1 5 10 15
   Gly Ser Pro Glu Glu Thr Ala Phe Arg
   20 25
<210> 110
   <211> 11
   <212> PRT
   <213> artificial sequence
<220>
   <223> fragment of OPA1
<220>
   <221> source
   <222> (1)..(11)
   <223> OPA1
<400> 110
   Leu Asp Ala Phe Ile Glu Ala Leu His Gln Glu
   1 5 10
<210> 111
   <211> 20
   <212> PRT
   <213> artificial sequence
<220>
   <223> fragment of OPA1
<220>
   <221> source
   <222> (1)..(20)
<400> 111
   Phe Trp Pro Ala Arg Leu Ala Thr Arg Leu Leu Lys Leu Arg Tyr Leu
   1 5 10 15
   Ile Leu Gly Ser
   20